# EUROPEAN PATENT SPECIFICATION

(11) **EP 3 630 735 B1**
(45) Date of publication and mention of the grant of the patent: **05.05.2021**
(21) Application number: 18726487.4
(22) Date of filing: 29.05.2018
(51) Int. Cl.: C07D 257/06, A01N 43/713, A01N 43/653, A01N 43/82, C07D 249/14, C07D 271/08, C07D 271/113

(54) **BENZAMIDE COMPOUNDS AND THEIR USE AS HERBICIDES**
BENZAMIDVERBINDUNGEN UND DEREN VERWENDUNG ALS HERBIZIDE
COMPOSÉS DE BENZADINE ET LEUR UTILISATION COMME HERBICIDES

(30) Priority: 30.05.2017 EP 17173421
(43) Date of publication of application: 08.04.2020
(73) Proprietor: BASF SE, 67056 Ludwigshafen am Rhein (DE)
(72) Inventor: KORDES, Markus, 67056 Ludwigshafen (DE); ZIERKE, Thomas, 67056 Ludwigshafen (DE); SEITZ, Thomas, 67056 Ludwigshafen (DE); NIELSON, Ryan Louis, 67117 Limburgerhof (DE)
(74) Representative: BASF IP Association
(86) International application number: PCT/EP2018/064047
(87) International publication number: WO 2018/219936

(56) References cited:
- WO-A1-2011/035874
- WO-A1-2012/028579
- WO-A1-2013/017559
- WO-A1-2013/124245

### SEQUENCE LISTING

<110> BASF SE
<120> Benzamide compounds and their use as herbicides II
<130> 170395WO01-58254-PCT
<160> 69
<170> BiSSAP 1.3
<210> 1
   <211> 1305
   <212> DNA
   <213> Hordeum vulgare
<400> 1
   atgccgccca cccccaccac ccccgcggct accggcgccg ccgccgcggt gacgccggag      60
   cacgcgcgac cgcaccgaat ggtccgcttc aacccgcgca gcgaccgctt ccacacgctc     120
   tccttccacc acgtcgagtt ctggtgcgcg gacgccgcct ccgccgccgg ccgcttcgcg     180
   ttcgcgctcg gcgcgccgct cgccgccagg tccgacctct ccacggggaa ctccgcgcac     240
   gcctcccagc tgctccgctc gggctccctc gccttcctct tcaccgcgcc ctacgccaac     300
   ggctgcgacg ccgccaccgc ctccctgccc tccttctccg ccgacgccgc gcgccggttc     360
   tccgccgacc acgggatcgc ggtgcgctcc gtagcgctgc gcgtcgcaga cgccgccgag     420
   gccttccgcg ccagcgtcga cgggggcgcg cgcccggcct tcgcccccgt ggacctcggc     480
   cgcggcttcg gcttcgcgga ggtcgagctc tacggcgacg tcgtgctccg cttcgtcagc     540
   cacccggacg gcacggacgt gcccttcttg ccggggttcg agggcgtgac caacccggac     600
   gccgtggact acggcctgac gcggttcgac cacgtcgtcg gcaacgtccc ggagcttgcc     660
   cccgccgcag cctacatcgc cgggttcacg ggcttccacg agttcgccga gttcacggcg     720
   gaggacgtgg gcacgaccga gagcgggctc aactcggtgg tgctcgccaa caactcggag     780
   ggcgtgctgc tgccgctcaa cgagccggtg cacggcacca agcgccggag ccagatacag     840
   acgttcctgg aacaccacgg cggcccgggc gtgcagcaca tcgcggtggc cagcagtgac     900
   gtgctcagga cgctcaggaa gatgcgtgcg cgctccgcca tgggcggctt cgacttcctg     960
   ccacccccgc tgccgaagta ctacgaaggc gtgcgacgcc ttgccgggga tgtcctctcg    1020
   gaggcgcaga tcaaggaatg ccaggagctg ggtgtgctcg tcgataggga cgaccaaggg    1080
   gtgttgctcc aaatcttcac caagccagta ggggacaggc cgaccttgtt cctggagatg    1140
   atccagagga tcgggtgcat ggagaaggac gagagagggg aagagtacca gaagggtggc    1200
   tgcggcgggt tcggcaaagg caacttctcc gagctgttca agtccattga agattacgag    1260
   aagtcccttg aagccaagca atctgctgca gttcagggat catag                    1305
<210> 2
   <211> 434
   <212> PRT
   <213> Hordeum vulgare
<400> 2
   Met Pro Pro Thr Pro Thr Thr Pro Ala Ala Thr Gly Ala Ala Ala Ala
   1               5                   10                  15
   Val Thr Pro Glu His Ala Arg Pro His Arg Met Val Arg Phe Asn Pro
               20                  25                  30
   Arg Ser Asp Arg Phe His Thr Leu Ser Phe His His Val Glu Phe Trp
           35                  40                  45
   Cys Ala Asp Ala Ala Ser Ala Ala Gly Arg Phe Ala Phe Ala Leu Gly
       50                  55                  60
   Ala Pro Leu Ala Ala Arg Ser Asp Leu Ser Thr Gly Asn Ser Ala His
   65                  70                  75                  80
   Ala Ser Gln Leu Leu Arg Ser Gly Ser Leu Ala Phe Leu Phe Thr Ala
                   85                  90                  95
   Pro Tyr Ala Asn Gly Cys Asp Ala Ala Thr Ala Ser Leu Pro Ser Phe
               100                 105                 110
   Ser Ala Asp Ala Ala Arg Arg Phe Ser Ala Asp His Gly Ile Ala Val
           115                 120                 125
   Arg Ser Val Ala Leu Arg Val Ala Asp Ala Ala Glu Ala Phe Arg Ala
       130                 135                 140
   Ser Val Asp Gly Gly Ala Arg Pro Ala Phe Ala Pro Val Asp Leu Gly
   145                 150                 155                 160
   Arg Gly Phe Gly Phe Ala Glu Val Glu Leu Tyr Gly Asp Val Val Leu
                   165                 170                 175
   Arg Phe Val Ser His Pro Asp Gly Thr Asp Val Pro Phe Leu Pro Gly
               180                 185                 190
   Phe Glu Gly Val Thr Asn Pro Asp Ala Val Asp Tyr Gly Leu Thr Arg
           195                 200                 205
   Phe Asp His Val Val Gly Asn Val Pro Glu Leu Ala Pro Ala Ala Ala
       210                 215                 220
   Tyr Ile Ala Gly Phe Thr Gly Phe His Glu Phe Ala Glu Phe Thr Ala
   225                 230                 235                 240
   Glu Asp Val Gly Thr Thr Glu Ser Gly Leu Asn Ser Val Val Leu Ala
                   245                 250                 255
   Asn Asn Ser Glu Gly Val Leu Leu Pro Leu Asn Glu Pro Val His Gly
               260                 265                 270
   Thr Lys Arg Arg Ser Gln Ile Gln Thr Phe Leu Glu His His Gly Gly
           275                 280                 285
   Pro Gly Val Gln His Ile Ala Val Ala Ser Ser Asp Val Leu Arg Thr
       290                 295                 300
   Leu Arg Lys Met Arg Ala Arg Ser Ala Met Gly Gly Phe Asp Phe Leu
   305                 310                 315                 320
   Pro Pro Pro Leu Pro Lys Tyr Tyr Glu Gly Val Arg Arg Leu Ala Gly
                   325                 330                 335
   Asp Val Leu Ser Glu Ala Gln Ile Lys Glu Cys Gln Glu Leu Gly Val
               340                 345                 350
   Leu Val Asp Arg Asp Asp Gln Gly Val Leu Leu Gln Ile Phe Thr Lys
           355                 360                 365
   Pro Val Gly Asp Arg Pro Thr Leu Phe Leu Glu Met Ile Gln Arg Ile
       370                 375                 380
   Gly Cys Met Glu Lys Asp Glu Arg Gly Glu Glu Tyr Gln Lys Gly Gly
   385                 390                 395                 400
   Cys Gly Gly Phe Gly Lys Gly Asn Phe Ser Glu Leu Phe Lys Ser Ile
                   405                 410                 415
   Glu Asp Tyr Glu Lys Ser Leu Glu Ala Lys Gln Ser Ala Ala Val Gln
               420                 425                 430
   Gly Ser
<210> 3
   <211> 1470
   <212> DNA
   <213> Fragilariopsis cylindrus
<400> 3
   atgggtttct cttcagtatc gttatttccg ctatcgtggt cggcgtcggc agtaacatca      60
   acctatctcg agacgtccac tagtacacaa tcgaatgaag ctgctactac cacttcgaca     120
   acggcgtggg aaccaaaatt atcatcatta gaagaaagaa tagaaacgga aacgattgag     180
   tcgatcggtt tccatcatat agaattttat tgtggagatg ctcgtagtat ggcaaatcaa     240
   ttcgctgtct cattgggtat gtccgtcacg ggtatcaccg gccaatccac ggggaatgat     300
   caatgcattt cctatggatt acaaagtgga gagcagtttc gactattatt aactgctccc     360
   tattcacgag cgagagccac tactcgcgat gacgacgacg acgacgacga caacagtcct     420
   gatttggatg ccgacgctcc gatgccactc cctaattata atgtagaaga tgctcatact     480
   ttcttccaaa atcatggctt agcagctcga gcggttggca tagaagtcat ggatgccaaa     540
   aaagctttcg aggtatccgt ggccaatggc gcaattccag tactggaacc aacctttctt     600
   cccaacggat gctacatctc agaagttgaa ttgtacggtg acgttgtgtt gagatacgtg     660
   agtttcatca catcgaatga aaatcatact tataatgatg atgcatcaca accatttcta     720
   cctcatttag caccaataat tgatcaaagt aggaaagagg atgatgataa taatgatgat     780
   ggttttgggt tatataaaat cgaccatgcc gttgggaatg ttcccaattt acaagaggta     840
   tactcacata tccaaaaatt tacaggattt catgaatttg ctgaatttac atcagaagat     900
   gttggaactg tagactctgg attaaattct gttgttttag ccagtgacag tgaagcgatt     960
   ctgttaccta taaatgaacc aactaatgga cgacgaaaat cacaaattca aacgtatcta    1020
   gaacagaacg agggccctgg tctacaacat ttagcagtca aaacgaaaga tatattttca    1080
   accgtccgaa agatgcgaag aagtcaacaa ggtatgtcgg gatttgaatt gatgaaacga    1140
   ccgagtgagg aatattacaa agaacttcct gatcgacttg gtgatcaatt gacacccacg    1200
   cagtatcaag aattagagga acttggtatc cttgcggatt ccgatgagga aggaattttg    1260
   atgcaaattt ttaccaagcc cgtcggtgat cgacctacat tctttttgga actaattcaa     1320
   cgaatcggtt gcgtcattga gcatgacgat gacgacaggc aggagttatc agttgatctt    1380
   gaacgaccag gatgtggtgg ttttggtaag ggtaatttcc gagaactttt cagatcaatt    1440
   gaagagcacg agaaaacttt aaaggtatag                                     1470
<210> 4
   <211> 1467
   <212> DNA
   <213> Fragilariopsis cylindrus
<400> 4
   atgggtttta gcagcgttag cctgtttccg ctgagctggt cagcaagcgc agttaccagc      60
   acctatctgg aaaccagcac cagcacccag agcaatgaag cagcaaccac caccagtacc     120
   accgcatggg aaccgaaact gagcagcctg gaagaacgta ttgaaaccga aaccattgaa     180
   agcattggct ttcatcacat tgaattttat tgcggtgatg cacgtagcat ggcaaatcag     240
   tttgcagtta gcctgggtat gagcgttacc ggtattaccg gtcagagcac cggtaatgat     300
   cagtgtatta gctatggtct gcagagcggt gaacagtttc gtctgctgct gaccgcaccg     360
   tatagccgtg cacgtgcaac cacccgtgat gatgatgatg acgatgacga taatagtccg     420
   gatctggatg cagatgcacc gatgccgctg ccgaattata atgttgaaga tgcccatacc     480
   ttttttcaga atcatggtct ggcagcacgt gcagttggta ttgaagttat ggatgccaaa     540
   aaagcctttg aagttagcgt tgcaaatggt gcaattccgg ttctggaacc gacctttctg     600
   ccgaatggtt gttatatttc tgaagtggaa ctgtatggtg atgttgttct gcgttatgtg     660
   agctttatta ccagcaatga aaaccacacc tacaatgatg atgccagcca gccgtttctg     720
   ccgcatctgg caccgattat tgatcagagc cgtaaagaag atgatgataa taatgatgat     780
   ggctttggcc tgtataaaat tgatcatgcc gttggtaatg tgccgaatct gcaagaagtt     840
   tatagccata ttcagaaatt taccggcttt catgaatttg ccgaatttac cagcgaagat     900
   gttggcaccg ttgatagcgg tctgaatagc gttgttctgg caagcgatag cgaagcaatt     960
   ctgctgccga ttaatgaacc gaccaatggt cgtcgtaaaa gccagattca gacatatctg    1020
   gaacagaatg aaggtccggg tctgcagcat ctggccgtta aaaccaaaga tatttttagc    1080
   accgtgcgta aaatgcgtcg tagccagcag ggtatgagcg gttttgaact gatgaaacgt    1140
   ccgagcgaag aatattataa agaactgccg gatcgtctgg gtgatcagct gaccccgacc    1200
   cagtatcaag aattagaaga actgggtatt ctggcagata gtgatgaaga aggtattctg    1260
   atgcagattt ttaccaaacc ggttggtgat cgtccgacct ttttttttga actgattcag     1320
   cgtattggct gcgtgattga acatgatgat gatgatcgtc aagaactgag cgttgatctg    1380
   gaacgtccgg gttgtggtgg ttttggtaaa ggtaattttc gtgaactgtt tcgcagcatt    1440
   gaagaacatg aaaaaaccct gaaagtg                                         1467
<210> 5
   <211> 489
   <212> PRT
   <213> Fragilariopsis cylindrus
<400> 5
   Met Gly Phe Ser Ser Val Ser Leu Phe Pro Leu Ser Trp Ser Ala Ser
   1               5                   10                  15
   Ala Val Thr Ser Thr Tyr Leu Glu Thr Ser Thr Ser Thr Gln Ser Asn
               20                  25                  30
   Glu Ala Ala Thr Thr Thr Ser Thr Thr Ala Trp Glu Pro Lys Leu Ser
           35                  40                  45
   Ser Leu Glu Glu Arg Ile Glu Thr Glu Thr Ile Glu Ser Ile Gly Phe
       50                  55                  60
   His His Ile Glu Phe Tyr Cys Gly Asp Ala Arg Ser Met Ala Asn Gln
   65                  70                  75                  80
   Phe Ala Val Ser Leu Gly Met Ser Val Thr Gly Ile Thr Gly Gln Ser
                   85                  90                  95
   Thr Gly Asn Asp Gln Cys Ile Ser Tyr Gly Leu Gln Ser Gly Glu Gln
               100                 105                 110
   Phe Arg Leu Leu Leu Thr Ala Pro Tyr Ser Arg Ala Arg Ala Thr Thr
           115                 120                 125
   Arg Asp Asp Asp Asp Asp Asp Asp Asp Asn Ser Pro Asp Leu Asp Ala
       130                 135                 140
   Asp Ala Pro Met Pro Leu Pro Asn Tyr Asn Val Glu Asp Ala His Thr
   145                 150                 155                 160
   Phe Phe Gln Asn His Gly Leu Ala Ala Arg Ala Val Gly Ile Glu Val
                   165                 170                 175
   Met Asp Ala Lys Lys Ala Phe Glu Val Ser Val Ala Asn Gly Ala Ile
               180                 185                 190
   Pro Val Leu Glu Pro Thr Phe Leu Pro Asn Gly Cys Tyr Ile Ser Glu
           195                 200                 205
   Val Glu Leu Tyr Gly Asp Val Val Leu Arg Tyr Val Ser Phe Ile Thr
       210                 215                 220
   Ser Asn Glu Asn His Thr Tyr Asn Asp Asp Ala Ser Gln Pro Phe Leu
   225                 230                 235                 240
   Pro His Leu Ala Pro Ile Ile Asp Gln Ser Arg Lys Glu Asp Asp Asp
                   245                 250                 255
   Asn Asn Asp Asp Gly Phe Gly Leu Tyr Lys Ile Asp His Ala Val Gly
               260                 265                 270
   Asn Val Pro Asn Leu Gln Glu Val Tyr Ser His Ile Gln Lys Phe Thr
           275                 280                 285
   Gly Phe His Glu Phe Ala Glu Phe Thr Ser Glu Asp Val Gly Thr Val
       290                 295                 300
   Asp Ser Gly Leu Asn Ser Val Val Leu Ala Ser Asp Ser Glu Ala Ile
   305                 310                 315                 320
   Leu Leu Pro Ile Asn Glu Pro Thr Asn Gly Arg Arg Lys Ser Gln Ile
                   325                 330                 335
   Gln Thr Tyr Leu Glu Gln Asn Glu Gly Pro Gly Leu Gln His Leu Ala
               340                 345                 350
   Val Lys Thr Lys Asp Ile Phe Ser Thr Val Arg Lys Met Arg Arg Ser
           355                 360                 365
   Gln Gln Gly Met Ser Gly Phe Glu Leu Met Lys Arg Pro Ser Glu Glu
       370                 375                 380
   Tyr Tyr Lys Glu Leu Pro Asp Arg Leu Gly Asp Gln Leu Thr Pro Thr
   385                 390                 395                 400
   Gln Tyr Gln Glu Leu Glu Glu Leu Gly Ile Leu Ala Asp Ser Asp Glu
                   405                 410                 415
   Glu Gly Ile Leu Met Gln Ile Phe Thr Lys Pro Val Gly Asp Arg Pro
               420                 425                 430
   Thr Phe Phe Phe Glu Leu Ile Gln Arg Ile Gly Cys Val Ile Glu His
           435                 440                 445
   Asp Asp Asp Asp Arg Gln Glu Leu Ser Val Asp Leu Glu Arg Pro Gly
       450                 455                 460
   Cys Gly Gly Phe Gly Lys Gly Asn Phe Arg Glu Leu Phe Arg Ser Ile
   465                 470                 475                 480
   Glu Glu His Glu Lys Thr Leu Lys Val
                   485
<210> 6
   <211> 1341
   <212> DNA
   <213> Chlorella sp.
<400> 6
   atggtggtcg aggctgcggc cgcctccaac ggcaatggcg caggggagga ggtgttcagc      60
   aagaagctcg tgggatatga cggtttccag cgccacaacc cacgctccga ccgcttcccc     120
   atgcacaagt tccaccacgt cgagttctgg tgcggcgatg ccaccaccac cagctgcagg     180
   caagcgcgcc ccccagaagc acccatgttt gggtatgggc tgggcctgac tctggtggcc     240
   aagagcgacc agtccacggg caaccaccac tacgcgtcgt acgtcatgca gtcgggcgat     300
   cttgtgatgg cctttaccgc gccctacagc acccagacag acaagagcgg cagcagcccg     360
   cccgcagcgt acgaccagga cgccgcctac gccttcctca agaagcacgg catggcggtg     420
   cgcgcctttg gaatcctggt ggacgatgcc gcggaggcgt accgcatagc cactgcccac     480
   ggcggggtgg gtgtggcgcc acccaccacc cgcacggacg cagccagcgg caccagcctg     540
   acgtggagcg aggtgcagct gtacggcgac tgcgtgctgc gctttgtcag cggcgactac     600
   gagggcgcct tcatccccgg ctaccagccc gtggaagacg cgccccaagt ctcctacggc     660
   ctgcagcgcc tggaccatgc ggtgggcaac gtgccagagc tgatccctca agtggagtac     720
   atggctcgca gcctgggctg gcacgagttt gctgagttca ctgccgagga tgtgggcact     780
   gtggactcgg gcctcaactc catggtcatg gccaacaaca acgagatgat tctgctgccg     840
   gtcaacgagc ccacccacgg caccaagcgc aagagccaga tccagacctt cctggagcag     900
   aatgaggggc ccgggctgca gcacatggcc ctgaaaacag acgacatcgt agccaccatg     960
   cgacagctcc gggccaggtc tgcgtttggc ggcttcgact tcatgcccag gccttcgcct    1020
   gactactacc gcaagctgcc tgcccgcatc ggcagcctgc tgacggcgca gcagtacaag    1080
   gacgttgagg agctggggct gcttgtggac aaggatgacc agggcgtgct gctccagatc    1140
   ttcaccaagc cgctgggcga ccgacccacc gtgtttttcg aaatcatcca gcgcctgtgc    1200
   gccctggagc cgcaggcgcc caagagccag cgcggcgcgg tgccttccga ggtcggcggc    1260
   tgcggcggct ttggcaaggg caacttcagt gagctgttca agagcatcga ggtgtacgag    1320
   acggatctgg gcatcaacta a                                              1341
<210> 7
   <211> 1341
   <212> DNA
   <213> Chlorella sp
<400> 7
   atggttgttg aagcagcagc agcaagcaat ggtaatggtg ccggtgaaga agtgtttagc 60
   aaaaaactgg tgggctatga tggttttcag cgtcataatc cgcgtagcga tcgttttccg 120
   atgcataaat ttcatcatgt ggaattttgg tgtggtgatg caaccaccac cagttgtcgt 180
   caggcacgtc ctccggaagc accgatgttt ggttatggtc tgggtctgac cctggttgca 240
   aaaagcgatc agagcaccgg taatcatcat tatgcaagct atgttatgca gagcggtgat 300
   ctggttatgg catttaccgc accgtatagc acccagaccg ataaaagcgg tagcagtccg 360
   cctgcagcat acgatcagga tgcagcatac gcctttctga aaaaacatgg tatggcagtt 420
   cgtgcatttg gtattctggt tgatgatgca gcagaagcat atcgtattgc aaccgcacat 480
   ggtggtgttg gtgttgcacc tccgaccacc cgtaccgatg cagcaagcgg caccagcctg 540
   acctggtctg aagttcagct gtatggtgat tgtgttctgc gttttgttag cggtgattat 600
   gaaggtgcat ttattccggg ttatcagccg gttgaagatg caccgcaggt tagctatggt 660
   ctgcagcgtc tggatcatgc agttggtaat gttccggaac tgattccgca ggttgaatat 720
   atggcacgta gcctgggttg gcatgaattt gcagaattta ccgcagaaga tgttggcacc 780
   gttgatagcg gtctgaatag catggttatg gccaataaca atgaaatgat tctgctgccg 840
   gtgaatgaac cgacccacgg caccaaacgt aaaagccaga ttcagacctt tctggaacag 900
   aatgaaggtc cgggtctgca gcacatggca ctgaaaaccg atgatattgt tgcaaccatg 960
   cgtcagctgc gtgcacgtag cgcatttggt ggttttgatt ttatgcctcg tccgagtccg 1020
   gattattatc gtaaactgcc tgcacgtatt ggtagcctgc tgaccgcaca gcagtataaa 1080
   gatgttgaag aactgggtct gctggttgat aaagatgatc agggtgttct gctgcagatt 1140
   tttaccaaac cgctgggtga tcgtccgacc gttttttttg aaattattca gcgtctgtgt 1200
   gcactggaac cgcaggcacc gaaaagccag cgtggtgcag ttccgagcga agttggtggt 1260
   tgtggtggtt ttggtaaagg taattttagc gaactgttta aaagcattga agtgtatgaa 1320
   accgatctgg gcatcaatta a 1341
<210> 8
   <211> 446
   <212> PRT
   <213> Chlorella sp
<400> 8
   Met Val Val Glu Ala Ala Ala Ala Ser Asn Gly Asn Gly Ala Gly Glu
   1 5 10 15
   Glu Val Phe Ser Lys Lys Leu Val Gly Tyr Asp Gly Phe Gln Arg His
   20 25 30
   Asn Pro Arg Ser Asp Arg Phe Pro Met His Lys Phe His His Val Glu
   35 40 45
   Phe Trp Cys Gly Asp Ala Thr Thr Thr Ser Cys Arg Gln Ala Arg Pro
   50 55 60
   Pro Glu Ala Pro Met Phe Gly Tyr Gly Leu Gly Leu Thr Leu Val Ala
   65 70 75 80
   Lys Ser Asp Gln Ser Thr Gly Asn His His Tyr Ala Ser Tyr Val Met
   85 90 95
   Gln Ser Gly Asp Leu Val Met Ala Phe Thr Ala Pro Tyr Ser Thr Gln
   100 105 110
   Thr Asp Lys Ser Gly Ser Ser Pro Pro Ala Ala Tyr Asp Gln Asp Ala
   115 120 125
   Ala Tyr Ala Phe Leu Lys Lys His Gly Met Ala Val Arg Ala Phe Gly
   130 135 140
   Ile Leu Val Asp Asp Ala Ala Glu Ala Tyr Arg Ile Ala Thr Ala His
   145 150 155 160
   Gly Gly Val Gly Val Ala Pro Pro Thr Thr Arg Thr Asp Ala Ala Ser
   165 170 175
   Gly Thr Ser Leu Thr Trp Ser Glu Val Gln Leu Tyr Gly Asp Cys Val
   180 185 190
   Leu Arg Phe Val Ser Gly Asp Tyr Glu Gly Ala Phe Ile Pro Gly Tyr
   195 200 205
   Gln Pro Val Glu Asp Ala Pro Gln Val Ser Tyr Gly Leu Gln Arg Leu
   210 215 220
   Asp His Ala Val Gly Asn Val Pro Glu Leu Ile Pro Gln Val Glu Tyr
   225 230 235 240
   Met Ala Arg Ser Leu Gly Trp His Glu Phe Ala Glu Phe Thr Ala Glu
   245 250 255
   Asp Val Gly Thr Val Asp Ser Gly Leu Asn Ser Met Val Met Ala Asn
   260 265 270
   Asn Asn Glu Met Ile Leu Leu Pro Val Asn Glu Pro Thr His Gly Thr
   275 280 285
   Lys Arg Lys Ser Gln Ile Gln Thr Phe Leu Glu Gln Asn Glu Gly Pro
   290 295 300
   Gly Leu Gln His Met Ala Leu Lys Thr Asp Asp Ile Val Ala Thr Met
   305 310 315 320
   Arg Gln Leu Arg Ala Arg Ser Ala Phe Gly Gly Phe Asp Phe Met Pro
   325 330 335
   Arg Pro Ser Pro Asp Tyr Tyr Arg Lys Leu Pro Ala Arg Ile Gly Ser
   340 345 350
   Leu Leu Thr Ala Gln Gln Tyr Lys Asp Val Glu Glu Leu Gly Leu Leu
   355 360 365
   Val Asp Lys Asp Asp Gln Gly Val Leu Leu Gln Ile Phe Thr Lys Pro
   370 375 380
   Leu Gly Asp Arg Pro Thr Val Phe Phe Glu Ile Ile Gln Arg Leu Cys
   385 390 395 400
   Ala Leu Glu Pro Gln Ala Pro Lys Ser Gln Arg Gly Ala Val Pro Ser
   405 410 415
   Glu Val Gly Gly Cys Gly Gly Phe Gly Lys Gly Asn Phe Ser Glu Leu
   420 425 430
   Phe Lys Ser Ile Glu Val Tyr Glu Thr Asp Leu Gly Ile Asn
   435 440 445
<210> 9
   <211> 1257
   <212> DNA
   <213> Thalassiosira
<400> 9
   tttcatcata ttgaattctt cgccagtgat gcgcttacga cagccaagcg gtttgagcta      60
   gcgttgggat tgccaattac gtgttggagt tcattggcta cggggaacga tgtttgtgtt     120
   acctacggat tggaggggat gcaaactcga aagattgaaa ccgacaacgc aaacaagaac     180
   ggaggggcag gatccaatgt acaaatgact gcacctcttc ctcttccggg gtatgatatt     240
   gacaaagctc atgagtttta ctcgaagcac ggtttggcag tacgagctgt gggagtggaa     300
   gtgaaggatg caactgtagc ttatgcaaat gctgttgaga atggtgctac aggagtattg     360
   gagcctacga ttgttgaaaa ctttaacagc gatggagatt cgcagaagtg tcatatggct     420
   gaggtggaat tgtatggtga tgtggtgttg agattagtca gttttcatgg agattgtagt     480
   gccgaacaat ctacattcct tcctcacttg tcgccgtatc catccaacag caacaagaac     540
   aaaccaactt acggacttgc tcgtctagat cacacggtgg gcaatgttcc caacctcctc     600
   gcgacgcaac gatacattca aacattcacc aactaccatc ccttcgcaga gttcactccg     660
   gaagatgtgg gaacagtcga ctctggtctt aatagtgtag tacttgcatc agacaacgag     720
   aatgttttgc tgcctctcaa tgaacctacc gaaggtaaac gaaagagtca gattcaaaca     780
   tatctagagc agaacgaggg accgggactg cagcatattg ccatcaagac gaatgatatc     840
   tttgatacca ttgcaaagat gagacacgca gaagagaact ttggaggttt cgagttgatg     900
   aaacgtccat cggatgagta ttacaaagag ttgccttcga gattgggtga taagttgact     960
   gtcgagcaat acaaacagct ggaagagttg ggaatactgg cagatgcaga tgacgaaggt    1020
   atattgcttc aaatattcac aaagccattg ggagacaggc ccaccttgtt ccttgagatt    1080
   attcagcgaa ttgggtgtgt gttaccggat gacgatgaag caactgatga aggggaagct    1140
   aagaatgcac acaatagaat agtcagagaa cgtcccggat gtggtggatt tggtcagggc    1200
   aacttccgtg aactcttcaa agcaattgaa gaacatgaga agacactcaa ggtctaa       1257
<210> 10
   <211> 1257
   <212> DNA
   <213> Thalassiosira
<400> 10
   tttcatcata tcgaattttt tgcctcagat gcactgacca ccgcaaaacg ctttgaactg      60
   gcactgggtc tgccgattac ctgttggagc agcctggcaa ccggtaatga tgtttgtgtt     120
   acctatggtc tggaaggtat gcagacccgt aaaattgaaa ccgataacgc caataaaaac     180
   ggtggtgcag gtagcaatgt tcagatgacc gcaccgctgc cgctgcctgg ttatgatatt     240
   gataaagccc atgaattcta tagcaaacat ggtctggcag ttcgtgcagt tggtgttgaa     300
   gttaaagatg caaccgttgc ctatgcaaat gcagttgaaa atggtgcaac cggtgttctg     360
   gaaccgacca ttgttgaaaa ctttaatagt gatggcgata gccagaaatg tcacatggcc     420
   gaagttgaac tgtatggtga tgttgttctg cgtctggtta gctttcatgg tgattgtagc     480
   gcagaacaga gcacctttct gccgcatctg agcccgtatc cgagcaatag caataaaaac     540
   aaaccgacct atggcctggc acgtctggat cataccgttg gtaatgttcc gaatctgctg     600
   gcaacccagc gttatattca gacctttacc aactatcatc cgtttgcaga atttacaccg     660
   gaagatgttg gcaccgttga tagcggtctg aatagcgttg ttctggcaag cgataatgaa     720
   aatgttctgc tgccgctgaa tgaaccgacc gaaggtaaac gtaaaagcca gattcagacc     780
   tatctggaac agaatgaagg tccgggtctg cagcatattg caattaaaac caatgatatt     840
   tttgatacca ttgccaaaat gcgccatgcc gaagaaaatt ttggtggttt tgaactgatg     900
   aaacgtccgt ccgatgaata ctataaagaa ctgccgagcc gtctgggtga taaactgacc     960
   gttgaacagt ataaacagct ggaagaactg ggtattctgg cagatgcaga tgatgaaggt    1020
   attctgctgc agatttttac caaaccgctg ggtgatcgtc cgaccctgtt tctggaaatt    1080
   attcagcgta ttggttgtgt tctgccggat gatgatgaag caaccgatga aggtgaagca    1140
   aaaaatgccc ataatcgtat tgttcgtgaa cgtccgggtt gtggtggttt tggtcagggt    1200
   aattttcgcg aactgtttaa agccattgaa gaacatgaaa aaaccctgaa agtttaa        1257
<210> 11
   <211> 418
   <212> PRT
   <213> Thalassiosira pseudonana
<400> 11
   Phe His His Ile Glu Phe Phe Ala Ser Asp Ala Leu Thr Thr Ala Lys
   1               5                   10                  15
   Arg Phe Glu Leu Ala Leu Gly Leu Pro Ile Thr Cys Trp Ser Ser Leu
               20                  25                  30
   Ala Thr Gly Asn Asp Val Cys Val Thr Tyr Gly Leu Glu Gly Met Gln
           35                  40                  45
   Thr Arg Lys Ile Glu Thr Asp Asn Ala Asn Lys Asn Gly Gly Ala Gly
       50                  55                  60
   Ser Asn Val Gln Met Thr Ala Pro Leu Pro Leu Pro Gly Tyr Asp Ile
   65                  70                  75                  80
   Asp Lys Ala His Glu Phe Tyr Ser Lys His Gly Leu Ala Val Arg Ala
                   85                  90                  95
   Val Gly Val Glu Val Lys Asp Ala Thr Val Ala Tyr Ala Asn Ala Val
               100                 105                 110
   Glu Asn Gly Ala Thr Gly Val Leu Glu Pro Thr Ile Val Glu Asn Phe
           115                 120                 125
   Asn Ser Asp Gly Asp Ser Gln Lys Cys His Met Ala Glu Val Glu Leu
       130                 135                 140
   Tyr Gly Asp Val Val Leu Arg Leu Val Ser Phe His Gly Asp Cys Ser
   145                 150                 155                 160
   Ala Glu Gln Ser Thr Phe Leu Pro His Leu Ser Pro Tyr Pro Ser Asn
                   165                 170                 175
   Ser Asn Lys Asn Lys Pro Thr Tyr Gly Leu Ala Arg Leu Asp His Thr
               180                 185                 190
   Val Gly Asn Val Pro Asn Leu Leu Ala Thr Gln Arg Tyr Ile Gln Thr
           195                 200                 205
   Phe Thr Asn Tyr His Pro Phe Ala Glu Phe Thr Pro Glu Asp Val Gly
       210                 215                 220
   Thr Val Asp Ser Gly Leu Asn Ser Val Val Leu Ala Ser Asp Asn Glu
   225                 230                 235                 240
   Asn Val Leu Leu Pro Leu Asn Glu Pro Thr Glu Gly Lys Arg Lys Ser
                   245                 250                 255
   Gln Ile Gln Thr Tyr Leu Glu Gln Asn Glu Gly Pro Gly Leu Gln His
               260                 265                 270
   Ile Ala Ile Lys Thr Asn Asp Ile Phe Asp Thr Ile Ala Lys Met Arg
           275                 280                 285
   His Ala Glu Glu Asn Phe Gly Gly Phe Glu Leu Met Lys Arg Pro Ser
       290                 295                 300
   Asp Glu Tyr Tyr Lys Glu Leu Pro Ser Arg Leu Gly Asp Lys Leu Thr
   305                 310                 315                 320
   Val Glu Gln Tyr Lys Gln Leu Glu Glu Leu Gly Ile Leu Ala Asp Ala
                   325                 330                 335
   Asp Asp Glu Gly Ile Leu Leu Gln Ile Phe Thr Lys Pro Leu Gly Asp
               340                 345                 350
   Arg Pro Thr Leu Phe Leu Glu Ile Ile Gln Arg Ile Gly Cys Val Leu
           355                 360                 365
   Pro Asp Asp Asp Glu Ala Thr Asp Glu Gly Glu Ala Lys Asn Ala His
       370                 375                 380
   Asn Arg Ile Val Arg Glu Arg Pro Gly Cys Gly Gly Phe Gly Gln Gly
   385                 390                 395                 400
   Asn Phe Arg Glu Leu Phe Lys Ala Ile Glu Glu His Glu Lys Thr Leu
                   405                 410                 415
   Lys Val
<210> 12
   <211> 1092
   <212> DNA
   <213> Cyanothece
<400> 12
   atggaaatcg atcatattca tttctacgtt gaagatgcag cacatcaacg agattggttt 60
   attgataaaa tggggtttca atccatcagc aacagtatcc atgatgacac ttatagcgaa 120
   gtagtaggga atcagtctgt ttactttatc ttatcttctc ccctcaacga tgctagtcca 180
   gtttcttatt acttgaaatc tcatcctccg ggggttgctg atgttgcttt tcgtgttgac 240
   aatcttaatt ttttattaga caaagtatcc cgttttaagg tcgaaattat taatcaatct 300
   agtctaacag cttttcctct aaataaacca gtgaaattcg cgaaacttaa aggatggggt 360
   tctgtcaatc ataccttaat tgatcaggca agtcctagga cttttattag ctcaaaaatg 420
   attgctaaaa gcgatattat tgggattgat catgttgttt taaatgttcc tcaaggtgaa 480
   ctccccttag ccataaattg gtacaaaaat gtatttgatt ttataagtca tcaacagttc 540
   aacatccaaa cagaacattc ggggttatct agtgaagcct tagttgatag ttcaggaaaa 600
   gtacaattta atattaatca accaagttct actaattctc agattcagga atttttagac 660
   cataataacg gttcaggcat tcaacatatt ggtttaaaat caagtaatat tttacaaagt 720
   gttgcacaaa tgcgtcaaag gggattaccc tttttatccg ttcctaattc ctattaccaa 780
   aacctaaaag aattgattag aaaatcgaca atttcttgtt taagccaaca ggaactagaa 840
   caaattgaaa ctgaacaaat tctagtttgt tggccagaag ataacccgac ttcaatcctg 900
   atgcaaattt tcactcaacc catttttaag cagccgactt tcttttttga attaattcaa 960
   agacgcaacc aagcacaggg atttggccaa ggtaattttc aagcgttatt tgaagccata 1020
   gaatcagaac aaatcaagag aaatagggta tcctcacgag tcactttaca ggctgtaaca 1080
   ccccaatctt ga 1092
<210> 13
   <211> 1089
   <212> DNA
   <213> Cyanothece
<400> 13
   atggaaattg atcatattca tttttatgtg gaagatgcag cccatcagcg tgattggttt 60
   attgataaaa tgggctttca gagcattagc aatagcattc atgatgatac ctatagcgaa 120
   gttgtgggta atcagagcgt gtatttcatt ctgagcagtc cgctgaatga tgcaagtccg 180
   gttagctatt atctgaaaag ccatcctccg ggtgttgcag atgttgcatt tcgtgttgat      240
   aatctgaatt ttctgctgga taaagtgagc cgctttaaag tggaaatcat taatcagagc      300
   agcctgaccg catttccgct gaataaaccg gttaaatttg ccaaactgaa aggttggggt      360
   agcgttaatc ataccctgat tgatcaggca agtccgcgta cctttattag cagcaaaatg      420
   attgccaaaa gcgatattat tggcattgat catgtggttc tgaatgttcc gcagggtgaa      480
   ctgccgctgg caattaattg gtacaaaaat gtgtttgatt ttattagcca tcagcagttt      540
   aatattcaga ccgaacatag cggtctgagc agcgaagcac tggttgatag cagcggtaaa      600
   gttcagttta atattaatca gccgagcagc accaatagcc agattcaaga atttctggat      660
   cataataatg gcagcggcat tcagcatatt ggtctgaaaa gcagcaatat tctgcagagc      720
   gttgcacaga tgcgtcagcg tggtctgccg tttctgagcg ttccgaatag ctattatcag      780
   aatctgaaag aactgattcg caaaagcacc attagctgtc tgagccagca agaactggaa      840
   caaattgaaa ccgaacaaat tctggtttgt tggcctgaag ataatccgac cagcattctg      900
   atgcagattt ttacccagcc gatttttaaa cagccgacct ttttttttga actgattcag      960
   cgtcgtaatc aggcacaggg ttttggtcag ggtaattttc aggcactgtt tgaagcaatt     1020
   gaaagtgaac aaattaaacg taatcgtgtt agcagccgtg ttaccctgca ggcagttaca     1080
   ccgcagagc                                                             1089
<210> 14
   <211> 363
   <212> PRT
   <213> Cyanothece
<400> 14
   Met Glu Ile Asp His Ile His Phe Tyr Val Glu Asp Ala Ala His Gln
   1               5                   10                  15
   Arg Asp Trp Phe Ile Asp Lys Met Gly Phe Gln Ser Ile Ser Asn Ser
               20                  25                  30
   Ile His Asp Asp Thr Tyr Ser Glu Val Val Gly Asn Gln Ser Val Tyr
           35                  40                  45
   Phe Ile Leu Ser Ser Pro Leu Asn Asp Ala Ser Pro Val Ser Tyr Tyr
       50                  55                  60
   Leu Lys Ser His Pro Pro Gly Val Ala Asp Val Ala Phe Arg Val Asp
   65                  70                  75                  80
   Asn Leu Asn Phe Leu Leu Asp Lys Val Ser Arg Phe Lys Val Glu Ile
                   85                  90                  95
   Ile Asn Gln Ser Ser Leu Thr Ala Phe Pro Leu Asn Lys Pro Val Lys
               100                 105                 110
   Phe Ala Lys Leu Lys Gly Trp Gly Ser Val Asn His Thr Leu Ile Asp
           115                 120                 125
   Gln Ala Ser Pro Arg Thr Phe Ile Ser Ser Lys Met Ile Ala Lys Ser
       130                 135                 140
   Asp Ile Ile Gly Ile Asp His Val Val Leu Asn Val Pro Gln Gly Glu
   145                 150                 155                 160
   Leu Pro Leu Ala Ile Asn Trp Tyr Lys Asn Val Phe Asp Phe Ile Ser
                   165                 170                 175
   His Gln Gln Phe Asn Ile Gln Thr Glu His Ser Gly Leu Ser Ser Glu
               180                 185                 190
   Ala Leu Val Asp Ser Ser Gly Lys Val Gln Phe Asn Ile Asn Gln Pro
           195                 200                 205
   Ser Ser Thr Asn Ser Gln Ile Gln Glu Phe Leu Asp His Asn Asn Gly
       210                 215                 220
   Ser Gly Ile Gln His Ile Gly Leu Lys Ser Ser Asn Ile Leu Gln Ser
   225                 230                 235                 240
   Val Ala Gln Met Arg Gln Arg Gly Leu Pro Phe Leu Ser Val Pro Asn
                   245                 250                 255
   Ser Tyr Tyr Gln Asn Leu Lys Glu Leu Ile Arg Lys Ser Thr Ile Ser
               260                 265                 270
   Cys Leu Ser Gln Gln Glu Leu Glu Gln Ile Glu Thr Glu Gln Ile Leu
           275                 280                 285
   Val Cys Trp Pro Glu Asp Asn Pro Thr Ser Ile Leu Met Gln Ile Phe
       290                 295                 300
   Thr Gln Pro Ile Phe Lys Gln Pro Thr Phe Phe Phe Glu Leu Ile Gln
   305                 310                 315                 320
   Arg Arg Asn Gln Ala Gln Gly Phe Gly Gln Gly Asn Phe Gln Ala Leu
                   325                 330                 335
   Phe Glu Ala Ile Glu Ser Glu Gln Ile Lys Arg Asn Arg Val Ser Ser
               340                 345                 350
   Arg Val Thr Leu Gln Ala Val Thr Pro Gln Ser
           355                 360
<210> 15
   <211> 1068
   <212> DNA
   <213> Acaryochloris marina
<400> 15
   atggattttg atcatattca tttttatgtt catgattcca agcaatgcca gcgttggttt 60
   actaacgttt taggatttca atatcttggg agcaatacta cggctgatcg gcagattgaa 120
   gttgtctctt ctggggcgat tgtctgtata ttttccagcc ctctaaaccg aaccagccca 180
   gttgcccagt atctacaaca acaccctcct ggtgtcgttg atttggcttt cttggtcccg 240
   gacgttcagg ctacgcttac ctgcgctgtc cagtcaggag caaccctttt acaacctttg 300
   accgaagaaa aaaacgacca aggaacgtta acttggggaa aagtacgagg gtggggagcg 360
   ttagaacata ccttggtaga gcgaagaggg caaacctcca ctctgccatc cagcattttt 420
   cccatctcaa ttcatgggca tgatgcccat cagagtctat ttacccagat tgatcatggg 480
   gttttgaatg tgggtaagca tcagctgcaa gctgctgtga gttggtatca gcgcatattt 540
   ggatttaaaa ctcaccgata ttttgatatt caaacgcgtc gttcaggtct gcgcagtgaa 600
   gtgttgaccc atccccaagg ccaaatcaag tttccgatca atgaacccac ctcagcgaat 660
   tcccaaatcc aagaatttct agaggtcaat cggggggcag gtattcaaca tatcgcattg 720
   ggaacttcta atattgttga aacggttact cagcttaagc atcgagggct atccatccta 780
   gatattccac ccagctacta tcaacgctta cgacaccagt ttgagcaagt ctattcccac 840
   ctcgattggc atgccctgga aacacaacat attctggctg attttgagga agatgctgga 900
   gccggaattc tattgcaaac cttcacaaag cctatctttc cacaacccac ttttttcttt 960
   gaaattattg agcgccaacg gcaggcccaa gggttcggac aacgaaactt tttggccctt 1020
   tttcaagcca tggagcggga acaacagaaa cggggagtat tgctatag 1068
<210> 16
   <211> 1065
   <212> DNA
   <213> Acaryochloris marina
<400> 16
   atggattttg atcacatcca cttttatgtg catgacagca aacagtgtca gcgttggttt      60
   accaatgttc tgggttttca gtatctgggt agcaatacca ccgcagatcg tcagattgaa     120
   gttgttagca gcggtgcaat tgtttgcatt tttagcagtc cgctgaatcg taccagtccg     180
   gttgcacagt atctgcagca gcatccgcct ggtgttgttg atctggcatt tctggttccg     240
   gatgttcagg caaccctgac ctgtgcagtt cagagcggtg ccaccctgct gcagccgctg     300
   accgaagaaa aaaatgatca gggtacactg acctggggta aagttcgtgg ttggggtgca     360
   ctggaacaca ccctggttga acgtcgtggt cagaccagca ccctgccgag cagcattttt     420
   ccgattagca ttcatggtca tgatgcacat cagagcctgt ttacccagat tgatcatggt     480
   gttctgaatg ttggtaaaca tcagctgcag gcagcagtta gctggtatca gcgtattttt     540
   ggctttaaaa cccaccgcta ttttgatatt cagacccgtc gtagcggtct gcgtagcgaa     600
   gttctgaccc atccgcaggg tcagatcaaa tttccgatca atgaaccgac cagcgcaaat     660
   agccagattc aagaatttct ggaagttaat cgtggtgccg gtattcagca tattgcactg     720
   ggcaccagca atattgttga aaccgttacc cagctgaaac atcgtggtct gagcattctg     780
   gatattccgc ctagctatta tcagcgtctg cgtcatcagt ttgaacaggt ttatagccat     840
   ctggattggc atgccctgga aacccagcat attctggcag attttgaaga agatgcaggc     900
   gcaggtattc tgctgcaaac ctttaccaaa ccgatttttc cgcagccgac cttttttttc      960
   gaaattattg aacgtcagcg tcaggcacag ggttttggtc agcgcaattt tctggcactg    1020
   tttcaggcaa tggaacgtga acagcagaaa cgtggtgtgc tgctg                    1065
<210> 17
   <211> 355
   <212> PRT
   <213> Acaryochloris marina
<400> 17
   Met Asp Phe Asp His Ile His Phe Tyr Val His Asp Ser Lys Gln Cys
   1               5                   10                  15
   Gln Arg Trp Phe Thr Asn Val Leu Gly Phe Gln Tyr Leu Gly Ser Asn
               20                  25                  30
   Thr Thr Ala Asp Arg Gln Ile Glu Val Val Ser Ser Gly Ala Ile Val
           35                  40                  45
   Cys Ile Phe Ser Ser Pro Leu Asn Arg Thr Ser Pro Val Ala Gln Tyr
       50                  55                  60
   Leu Gln Gln His Pro Pro Gly Val Val Asp Leu Ala Phe Leu Val Pro
   65                  70                  75                  80
   Asp Val Gln Ala Thr Leu Thr Cys Ala Val Gln Ser Gly Ala Thr Leu
                   85                  90                  95
   Leu Gln Pro Leu Thr Glu Glu Lys Asn Asp Gln Gly Thr Leu Thr Trp
               100                 105                 110
   Gly Lys Val Arg Gly Trp Gly Ala Leu Glu His Thr Leu Val Glu Arg
           115                 120                 125
   Arg Gly Gln Thr Ser Thr Leu Pro Ser Ser Ile Phe Pro Ile Ser Ile
       130                 135                 140
   His Gly His Asp Ala His Gln Ser Leu Phe Thr Gln Ile Asp His Gly
   145                 150                 155                 160
   Val Leu Asn Val Gly Lys His Gln Leu Gln Ala Ala Val Ser Trp Tyr
                   165                 170                 175
   Gln Arg Ile Phe Gly Phe Lys Thr His Arg Tyr Phe Asp Ile Gln Thr
               180                 185                 190
   Arg Arg Ser Gly Leu Arg Ser Glu Val Leu Thr His Pro Gln Gly Gln
           195                 200                 205
   Ile Lys Phe Pro Ile Asn Glu Pro Thr Ser Ala Asn Ser Gln Ile Gln
       210                 215                 220
   Glu Phe Leu Glu Val Asn Arg Gly Ala Gly Ile Gln His Ile Ala Leu
   225                 230                 235                 240
   Gly Thr Ser Asn Ile Val Glu Thr Val Thr Gln Leu Lys His Arg Gly
                   245                 250                 255
   Leu Ser Ile Leu Asp Ile Pro Pro Ser Tyr Tyr Gln Arg Leu Arg His
               260                 265                 270
   Gln Phe Glu Gln Val Tyr Ser His Leu Asp Trp His Ala Leu Glu Thr
           275                 280                 285
   Gln His Ile Leu Ala Asp Phe Glu Glu Asp Ala Gly Ala Gly Ile Leu
       290                 295                 300
   Leu Gln Thr Phe Thr Lys Pro Ile Phe Pro Gln Pro Thr Phe Phe Phe
   305                 310                 315                 320
   Glu Ile Ile Glu Arg Gln Arg Gln Ala Gln Gly Phe Gly Gln Arg Asn
                   325                 330                 335
   Phe Leu Ala Leu Phe Gln Ala Met Glu Arg Glu Gln Gln Lys Arg Gly
               340                 345                 350
   Val Leu Leu
           355
<210> 18
   <211> 1020
   <212> DNA
   <213> Synechocystis sp. PCC 6803
<400> 18
   atggaattcg actatcttca tttatacgtt gacgattatc agtcagctca tcgttgttat      60
   caacgtcaat ggggtttcac ttgcgtaaat aaaattatta ctgaccaagg aattactggc     120
   atctaccaac aggggcaaat acttctgcta atttcggcat cggaatctag tttgagtaga     180
   tatgccgact atctccagaa acatcccccc ggcgtaggtg aagtcgcttg gcaggtggcc     240
   aattggcaaa aaattcagca tcaattatca gaattacaga tagaaaccac accagttatt     300
   catcctctga ctaaagcaga aggattaact tttttgctct ggggagatgt gcaccatagc     360
   atttatcctg ttcgttctga gctaaatcag aataaaacat tgcatggtgt tggtttaacg     420
   accatcgacc atgtggtgct aaacattgcc gccgatcaat ttacccaggc ttcccaatgg     480
   tatcaacagg tgtttggctg gtcggtgcag cagagtttta ctgtcaatac gccccattct     540
   ggtctgtata gcgaagccct ggccagtgcc aatgggaaag tccaatttaa cctcaattgt     600
   cccaccaata acagttccca aattcaaact tttttagcca ataaccatgg ggctggtatt     660
   caacatgtcg ctttttccac tacgagtatt acgcgaactg tggctcatct gcgggaaagg     720
   ggcgtaaatt ttttaaaaat ccccactggc tattatcaac agcaaagaaa cagtagctat     780
   tttaattatg caagtttgga ttgggatacc ttacagtgcc tagaaatttt gctggatgat     840
   caagataata cgggggagcg attactgcta caaattttta gtcagccttg ctatggagta     900
   ggcactctat tttgggaaat tattgaacgc cgccaccggg caaaaggatt tggtcaagga     960
   aactttcaag ctctctatga agcggtggag actttagaaa aacagttaga agtgccataa    1020
<210> 19
   <211> 1020
   <212> DNA
   <213> Synechocystis sp. PCC 6803
<400> 19
   atggaattcg attacctgca tctgtatgtt gatgactatc agagcgctca ccgttgttac      60
   cagcgtcagt ggggctttac ttgtgtgaac aaaatcatca ccgaccaggg tatcactggt     120
   atttaccagc agggtcagat cctgctgctg atcagcgctt ctgaatcttc cctctctcgc     180
   tatgccgatt acctccagaa acatccgcca ggtgtaggtg aagtcgcctg gcaggtcgca     240
   aactggcaga aaattcagca ccagctgtcc gaactgcaga ttgaaactac cccggtgatt     300
   cacccactga ccaaagcaga aggcctgact ttcctgctgt ggggtgacgt tcaccactcc     360
   atctacccag tacgtagcga gctgaaccag aacaaaaccc tgcatggcgt tggtctgacc     420
   actatcgatc acgtggttct gaacatcgca gcggaccagt tcacccaggc gagccagtgg     480
   tatcagcagg tattcggttg gtccgttcag cagtctttca cggttaacac cccgcattcc     540
   ggtctgtact ctgaagctct ggcgtctgcg aacggcaaag ttcagttcaa cctgaactgc     600
   ccgaccaaca acagctccca gattcagacc ttcctggcga acaaccacgg tgctggtatc     660
   cagcacgttg cattctctac tacctctatc acccgtacgg tcgctcacct gcgtgaacgt     720
   ggcgtgaact tcctgaaaat cccgaccggt tactatcagc agcagcgcaa cagctcctac     780
   ttcaactacg cgtctctgga ttgggatacc ctgcagtgcc tggagattct gctggacgac     840
   caggacaaca ctggcgaacg cctgctgctg cagatctttt ctcagccgtg ctatggcgtg     900
   ggtacgctgt tttgggaaat tatcgagcgc cgtcaccgtg ctaaaggctt tggccagggc     960
   aactttcagg cactgtacga ggcagtagaa accctggaaa aacagctcga agtgccataa    1020
<210> 20
   <211> 339
   <212> PRT
   <213> Synechocystis sp. PCC 6803
<400> 20
   Met Glu Phe Asp Tyr Leu His Leu Tyr Val Asp Asp Tyr Gln Ser Ala
   1               5                   10                  15
   His Arg Cys Tyr Gln Arg Gln Trp Gly Phe Thr Cys Val Asn Lys Ile
               20                  25                  30
   Ile Thr Asp Gln Gly Ile Thr Gly Ile Tyr Gln Gln Gly Gln Ile Leu
           35                  40                  45
   Leu Leu Ile Ser Ala Ser Glu Ser Ser Leu Ser Arg Tyr Ala Asp Tyr
       50                  55                  60
   Leu Gln Lys His Pro Pro Gly Val Gly Glu Val Ala Trp Gln Val Ala
   65                  70                  75                  80
   Asn Trp Gln Lys Ile Gln His Gln Leu Ser Glu Leu Gln Ile Glu Thr
                   85                  90                  95
   Thr Pro Val Ile His Pro Leu Thr Lys Ala Glu Gly Leu Thr Phe Leu
               100                 105                 110
   Leu Trp Gly Asp Val His His Ser Ile Tyr Pro Val Arg Ser Glu Leu
           115                 120                 125
   Asn Gln Asn Lys Thr Leu His Gly Val Gly Leu Thr Thr Ile Asp His
       130                 135                 140
   Val Val Leu Asn Ile Ala Ala Asp Gln Phe Thr Gln Ala Ser Gln Trp
   145                 150                 155                 160
   Tyr Gln Gln Val Phe Gly Trp Ser Val Gln Gln Ser Phe Thr Val Asn
                   165                 170                 175
   Thr Pro His Ser Gly Leu Tyr Ser Glu Ala Leu Ala Ser Ala Asn Gly
               180                 185                 190
   Lys Val Gln Phe Asn Leu Asn Cys Pro Thr Asn Asn Ser Ser Gln Ile
           195                 200                 205
   Gln Thr Phe Leu Ala Asn Asn His Gly Ala Gly Ile Gln His Val Ala
       210                 215                 220
   Phe Ser Thr Thr Ser Ile Thr Arg Thr Val Ala His Leu Arg Glu Arg
   225                 230                 235                 240
   Gly Val Asn Phe Leu Lys Ile Pro Thr Gly Tyr Tyr Gln Gln Gln Arg
                   245                 250                 255
   Asn Ser Ser Tyr Phe Asn Tyr Ala Ser Leu Asp Trp Asp Thr Leu Gln
               260                 265                 270
   Cys Leu Glu Ile Leu Leu Asp Asp Gln Asp Asn Thr Gly Glu Arg Leu
           275                 280                 285
   Leu Leu Gln Ile Phe Ser Gln Pro Cys Tyr Gly Val Gly Thr Leu Phe
       290                 295                 300
   Trp Glu Ile Ile Glu Arg Arg His Arg Ala Lys Gly Phe Gly Gln Gly
   305                 310                 315                 320
   Asn Phe Gln Ala Leu Tyr Glu Ala Val Glu Thr Leu Glu Lys Gln Leu
                   325                 330                 335
   Glu Val Pro
<210> 21
   <211> 1326
   <212> DNA
   <213> Alopecurus myosuroides
<400> 21
   atgcctccga ccaccgcaac cgcaaccggt gctgcagcag cagccgttac accggaacat      60
   gcagcacgtc gttttccgcg tgttgttcgt gttaatccgc gtagcgatcg ttttccggtt     120
   ctggcatttc atcatgttga attttggtgt gccgatgcag caagcgcagc aggtcgtttt     180
   agctttgcac tgggtgcacc gctggcagca cgtagcgatc tgagcaccgg taatagcagc     240
   catgcaagcc atctgctgcg tagtggtgca ctggcatttc tgtttaccgc accgtatgca     300
   ccgcctccgc aggatgcagc agatgcagcc gctaccgcca gcattccgag ctttagcacc     360
   gaagcagcac gtacctttag cagcgcacat ggtctggcag ttcgtagcgt tgcaattcgt     420
   gttgcagatg ccgcagaagc atttcatacc agcgttgcgg gtggtgcacg tccggcattt     480
   gcaccggcag atctgggtag cggttttggt ctggccgaag ttgaactgta tggtgatgtt     540
   gttctgcgtt ttgttagtca tccggatggt gatgatgttc cgtttctgcc gggttttgaa     600
   ggtgttagcc gtccgggtgc aatggattat ggtctgaccc gttttgatca tgttgttggt     660
   aatgttccgg aaatggcacc ggttgcagca tatatgaaag gttttaccgg ctttcatgaa     720
   tttgccgaat ttaccgcaga agatgttggc accgcagaaa gcggtctgaa tagcgttgtt     780
   ctggcaaata atagcgaagc agttctgctg ccgctgaatg aaccggtgca tggcaccaaa     840
   cgtcgtagcc agattcagac ctatctggat tatcatggtg gtccgggtgt tcagcatatt     900
   gcactggcaa gcagtgatgt tctgcgtacc ctgcgtgaaa tgcgtgcacg tagcgcaatg     960
   ggtggttttg aatttatggc accgccgcag gcaaaatatt atgaaggtgt tcgtcgtctg    1020
   gctggtgatg ttctgagcga agcacagatt aaagaatgtc aggaactggg cgttctggtt    1080
   gatcgtgatg atcagggtgt tctgctgcag atttttacca aaccggttgg tgatcgtcgt    1140
   ccgacctttt ttctggaaat gattcagcgt attggctgca tggaaaaaga tgaaattggc    1200
   caggaatatc agaaaggcgg ctgtggtggt tttggtaaag gtaattttag cgaactgttt    1260
   aaaagcattg aagattatga aaaaagcctg gaagccaaac agagcgcagt tgcacagcag    1320
   agctaa                                                               1326
<210> 22
   <211> 441
   <212> PRT
   <213> Alopecurus myosuroides
<400> 22
   Met Pro Pro Thr Thr Ala Thr Ala Thr Gly Ala Ala Ala Ala Ala Val
   1               5                   10                  15
   Thr Pro Glu His Ala Ala Arg Arg Phe Pro Arg Val Val Arg Val Asn
               20                  25                  30
   Pro Arg Ser Asp Arg Phe Pro Val Leu Ala Phe His His Val Glu Phe
           35                  40                  45
   Trp Cys Ala Asp Ala Ala Ser Ala Ala Gly Arg Phe Ser Phe Ala Leu
       50                  55                  60
   Gly Ala Pro Leu Ala Ala Arg Ser Asp Leu Ser Thr Gly Asn Ser Ser
   65                  70                  75                  80
   His Ala Ser His Leu Leu Arg Ser Gly Ala Leu Ala Phe Leu Phe Thr
                   85                  90                  95
   Ala Pro Tyr Ala Pro Pro Pro Gln Asp Ala Ala Asp Ala Ala Ala Thr
               100                 105                 110
   Ala Ser Ile Pro Ser Phe Ser Thr Glu Ala Ala Arg Thr Phe Ser Ser
           115                 120                 125
   Ala His Gly Leu Ala Val Arg Ser Val Ala Ile Arg Val Ala Asp Ala
       130                 135                 140
   Ala Glu Ala Phe His Thr Ser Val Ala Gly Gly Ala Arg Pro Ala Phe
   145                 150                 155                 160
   Ala Pro Ala Asp Leu Gly Ser Gly Phe Gly Leu Ala Glu Val Glu Leu
                   165                 170                 175
   Tyr Gly Asp Val Val Leu Arg Phe Val Ser His Pro Asp Gly Asp Asp
               180                 185                 190
   Val Pro Phe Leu Pro Gly Phe Glu Gly Val Ser Arg Pro Gly Ala Met
           195                 200                 205
   Asp Tyr Gly Leu Thr Arg Phe Asp His Val Val Gly Asn Val Pro Glu
       210                 215                 220
   Met Ala Pro Val Ala Ala Tyr Met Lys Gly Phe Thr Gly Phe His Glu
   225                 230                 235                 240
   Phe Ala Glu Phe Thr Ala Glu Asp Val Gly Thr Ala Glu Ser Gly Leu
                   245                 250                 255
   Asn Ser Val Val Leu Ala Asn Asn Ser Glu Ala Val Leu Leu Pro Leu
               260                 265                 270
   Asn Glu Pro Val His Gly Thr Lys Arg Arg Ser Gln Ile Gln Thr Tyr
           275                 280                 285
   Leu Asp Tyr His Gly Gly Pro Gly Val Gln His Ile Ala Leu Ala Ser
       290                 295                 300
   Ser Asp Val Leu Arg Thr Leu Arg Glu Met Arg Ala Arg Ser Ala Met
   305                 310                 315                 320
   Gly Gly Phe Glu Phe Met Ala Pro Pro Gln Ala Lys Tyr Tyr Glu Gly
                   325                 330                 335
   Val Arg Arg Leu Ala Gly Asp Val Leu Ser Glu Ala Gln Ile Lys Glu
               340                 345                 350
   Cys Gln Glu Leu Gly Val Leu Val Asp Arg Asp Asp Gln Gly Val Leu
           355                 360                 365
   Leu Gln Ile Phe Thr Lys Pro Val Gly Asp Arg Arg Pro Thr Phe Phe
       370                 375                 380
   Leu Glu Met Ile Gln Arg Ile Gly Cys Met Glu Lys Asp Glu Ile Gly
   385                 390                 395                 400
   Gln Glu Tyr Gln Lys Gly Gly Cys Gly Gly Phe Gly Lys Gly Asn Phe
                   405                 410                 415
   Ser Glu Leu Phe Lys Ser Ile Glu Asp Tyr Glu Lys Ser Leu Glu Ala
               420                 425                 430
   Lys Gln Ser Ala Val Ala Gln Gln Ser
           435                 440
<210> 23
   <211> 1323
   <212> DNA
   <213> Alopecurus myosuroides
<400> 23
   atgccaccaa ctactgctac tgctacaggt gctgctgctg cagctgttac tccagaacat      60
   gctgctagaa ggttcccaag agttgttaga gttaacccaa ggtctgatag gttcccagtt     120
   cttgctttcc atcatgttga gttttggtgt gctgatgctg cttctgctgc tggaagattt     180
   tcttttgctc ttggtgctcc acttgctgct agatctgatt tgtctactgg aaactcttct     240
   cacgcttctc accttttgag atctggtgct cttgctttcc ttttcactgc tccttatgct      300
   ccaccaccac aagatgctgc agatgcagca gctactgctt ctattccatc tttttcaact     360
   gaggctgcta ggactttctc ttctgctcat ggattggctg ttagatctgt ggctattaga     420
   gttgcagatg ctgcagaggc tttccatact tctgttgctg gtggtgctag accagctttt     480
   gctccagctg atcttggatc tggatttgga cttgctgagg ttgagcttta cggtgatgtt     540
   gttcttagat tcgtgtctca cccagatggt gatgatgttc catttcttcc aggattcgag     600
   ggtgttagta gaccaggtgc tatggattat ggactcacta ggttcgatca cgttgtggga     660
   aatgttccag aaatggctcc agttgctgct tacatgaagg gattcactgg atttcatgag     720
   ttcgctgagt tcactgctga ggatgttgga actgctgagt ctggacttaa ctctgttgtg     780
   cttgctaaca actctgaggc tgttcttttg ccacttaatg agccagttca cggcactaag     840
   agaagatctc agattcagac ttacctcgat taccatggtg gaccaggtgt tcaacatatt     900
   gctcttgctt catctgatgt gcttaggact cttagagaga tgagagctag atctgctatg     960
   ggaggatttg agtttatggc tccaccacaa gctaagtatt acgaaggtgt tagaaggctt    1020
   gctggtgatg ttctttctga ggctcaaatc aaagagtgcc aagagcttgg agttcttgtg    1080
   gatagagatg atcagggtgt gcttctccag attttcacta agccagttgg agataggcca    1140
   acattcttct tggagatgat tcagaggatc ggctgcatgg aaaaggatga gattggacaa    1200
   gagtaccaaa agggcggatg tggtggattt ggaaagggaa atttctccga gcttttcaag    1260
   tccatcgagg attacgagaa gtctcttgag gctaagcaat ctgctgttgc tcaacagtct    1320
   tga                                                                  1323
<210> 24
   <211> 440
   <212> PRT
   <213> Alopecurus myosuroides
<400> 24
   Met Pro Pro Thr Thr Ala Thr Ala Thr Gly Ala Ala Ala Ala Ala Val
   1               5                   10                  15
   Thr Pro Glu His Ala Ala Arg Arg Phe Pro Arg Val Val Arg Val Asn
               20                  25                  30
   Pro Arg Ser Asp Arg Phe Pro Val Leu Ala Phe His His Val Glu Phe
           35                  40                  45
   Trp Cys Ala Asp Ala Ala Ser Ala Ala Gly Arg Phe Ser Phe Ala Leu
       50                  55                  60
   Gly Ala Pro Leu Ala Ala Arg Ser Asp Leu Ser Thr Gly Asn Ser Ser
   65                  70                  75                  80
   His Ala Ser His Leu Leu Arg Ser Gly Ala Leu Ala Phe Leu Phe Thr
                   85                  90                  95
   Ala Pro Tyr Ala Pro Pro Pro Gln Asp Ala Ala Asp Ala Ala Ala Thr
               100                 105                 110
   Ala Ser Ile Pro Ser Phe Ser Thr Glu Ala Ala Arg Thr Phe Ser Ser
           115                 120                 125
   Ala His Gly Leu Ala Val Arg Ser Val Ala Ile Arg Val Ala Asp Ala
       130                 135                 140
   Ala Glu Ala Phe His Thr Ser Val Ala Gly Gly Ala Arg Pro Ala Phe
   145                 150                 155                 160
   Ala Pro Ala Asp Leu Gly Ser Gly Phe Gly Leu Ala Glu Val Glu Leu
                   165                 170                 175
   Tyr Gly Asp Val Val Leu Arg Phe Val Ser His Pro Asp Gly Asp Asp
               180                 185                 190
   Val Pro Phe Leu Pro Gly Phe Glu Gly Val Ser Arg Pro Gly Ala Met
           195                 200                 205
   Asp Tyr Gly Leu Thr Arg Phe Asp His Val Val Gly Asn Val Pro Glu
       210                 215                 220
   Met Ala Pro Val Ala Ala Tyr Met Lys Gly Phe Thr Gly Phe His Glu
   225                 230                 235                 240
   Phe Ala Glu Phe Thr Ala Glu Asp Val Gly Thr Ala Glu Ser Gly Leu
                   245                 250                 255
   Asn Ser Val Val Leu Ala Asn Asn Ser Glu Ala Val Leu Leu Pro Leu
               260                 265                 270
   Asn Glu Pro Val His Gly Thr Lys Arg Arg Ser Gln Ile Gln Thr Tyr
           275                 280                 285
   Leu Asp Tyr His Gly Gly Pro Gly Val Gln His Ile Ala Leu Ala Ser
       290                 295                 300
   Ser Asp Val Leu Arg Thr Leu Arg Glu Met Arg Ala Arg Ser Ala Met
   305                 310                 315                 320
   Gly Gly Phe Glu Phe Met Ala Pro Pro Gln Ala Lys Tyr Tyr Glu Gly
                   325                 330                 335
   Val Arg Arg Leu Ala Gly Asp Val Leu Ser Glu Ala Gln Ile Lys Glu
               340                 345                 350
   Cys Gln Glu Leu Gly Val Leu Val Asp Arg Asp Asp Gln Gly Val Leu
           355                 360                 365
   Leu Gln Ile Phe Thr Lys Pro Val Gly Asp Arg Pro Thr Phe Phe Leu
       370                 375                 380
   Glu Met Ile Gln Arg Ile Gly Cys Met Glu Lys Asp Glu Ile Gly Gln
   385                 390                 395                 400
   Glu Tyr Gln Lys Gly Gly Cys Gly Gly Phe Gly Lys Gly Asn Phe Ser
                   405                 410                 415
   Glu Leu Phe Lys Ser Ile Glu Asp Tyr Glu Lys Ser Leu Glu Ala Lys
               420                 425                 430
   Gln Ser Ala Val Ala Gln Gln Ser
           435                 440
<210> 25
   <211> 1323
   <212> DNA
   <213> Sorghum halapense
<400> 25
   atgcctccga ccccgaccac cgcagcagca acaggtgccg cagttgcagc agcaagcgca      60
   gaacaggcag catttcgtct ggttggtcat cgtaattttg ttcgtgttaa tccgcgtagc     120
   gatcgttttc ataccctggc atttcatcat gttgaactgt ggtgtgccga tgcagccagc     180
   gcagcaggtc gttttagctt tggtctgggt gcaccgctgg cagcacgtag cgatctgagc     240
   accggtaata ccgcacatgc aagcctgctg ctgcgttcag gtgcactggc atttctgttt     300
   accgcaccgt atgcccatgg tgctgatgca gcaaccgcaa gcctgccgag ctttagcgca     360
   gcagaagcac gtcgttttgc agcagatcat ggtctggcag ttcgtgccgt tgcactgcgt     420
   gttgcagatg ccgaagatgc atttcgtgca agcgttgcag ccggtgcacg tccggcattt     480
   gaaccggttg aactgggtct gggttttcgt ctggccgaag ttgaactgta tggtgatgtt     540
   gttctgcgtt atgttagcta tccggatgat gcagatgcaa gctttctgcc gggttttgtt     600
   ggtgttagca gtccgggtgc ggcagattat ggcctgcgtc gttttgatca tattgtgggt     660
   aatgttccgg aactggcacc ggcagcggca tattttgcag gttttaccgg ctttcatgaa     720
   tttgcagaat ttaccgcaga agatgttggc accaccgaaa gcggtctgaa tagcatggtt     780
   ctggcaaata atgccgaaaa tgttctgctg ccgctgaatg aaccggtgca tggcaccaaa     840
   cgtcgtagcc agattcagac ctttctggat catcatggtg gtccgggtgt tcagcacatg     900
   gcactggcaa gtgatgatgt gctgcgtacc ctgcgtgaaa tgcaggcatg tagtgcaatg     960
   ggtggttttg aatttatggc accgccggca ccggaatatt atgatggtgt tcgtcgtcgt    1020
   gccggtgatg ttctgaccga agcacagatt aaagaatgtc aggaactggg cgttctggtt    1080
   gatcgtgatg atcagggtgt tctgctgcag atttttacca aaccggttgg tgatcgcccg    1140
   accttttttc tggaaattat tcagcgtatt ggttgcatgg aaaaagatga aaaaggccag    1200
   gaatatcaga aaggcggttg tggtggtttt ggtaaaggta attttagcca gctgtttaaa    1260
   agcattgaag attatgaaaa aagcctggaa gcaaaacagg cagctgcagc acagggtccg    1320
   taa                                                                   1323
<210> 26
   <211> 440
   <212> PRT
   <213> Sorghum halapense
<400> 26
   Met Pro Pro Thr Pro Thr Thr Ala Ala Ala Thr Gly Ala Ala Val Ala
   1               5                   10                  15
   Ala Ala Ser Ala Glu Gln Ala Ala Phe Arg Leu Val Gly His Arg Asn
               20                  25                  30
   Phe Val Arg Val Asn Pro Arg Ser Asp Arg Phe His Thr Leu Ala Phe
           35                  40                  45
   His His Val Glu Leu Trp Cys Ala Asp Ala Ala Ser Ala Ala Gly Arg
       50                  55                  60
   Phe Ser Phe Gly Leu Gly Ala Pro Leu Ala Ala Arg Ser Asp Leu Ser
   65                  70                  75                  80
   Thr Gly Asn Thr Ala His Ala Ser Leu Leu Leu Arg Ser Gly Ala Leu
                   85                  90                  95
   Ala Phe Leu Phe Thr Ala Pro Tyr Ala His Gly Ala Asp Ala Ala Thr
               100                 105                 110
   Ala Ser Leu Pro Ser Phe Ser Ala Ala Glu Ala Arg Arg Phe Ala Ala
           115                 120                 125
   Asp His Gly Leu Ala Val Arg Ala Val Ala Leu Arg Val Ala Asp Ala
       130                 135                 140
   Glu Asp Ala Phe Arg Ala Ser Val Ala Ala Gly Ala Arg Pro Ala Phe
   145                 150                 155                 160
   Glu Pro Val Glu Leu Gly Leu Gly Phe Arg Leu Ala Glu Val Glu Leu
                   165                 170                 175
   Tyr Gly Asp Val Val Leu Arg Tyr Val Ser Tyr Pro Asp Asp Ala Asp
               180                 185                 190
   Ala Ser Phe Leu Pro Gly Phe Val Gly Val Ser Ser Pro Gly Ala Ala
           195                 200                 205
   Asp Tyr Gly Leu Arg Arg Phe Asp His Ile Val Gly Asn Val Pro Glu
       210                 215                 220
   Leu Ala Pro Ala Ala Ala Tyr Phe Ala Gly Phe Thr Gly Phe His Glu
   225                 230                 235                 240
   Phe Ala Glu Phe Thr Ala Glu Asp Val Gly Thr Thr Glu Ser Gly Leu
                   245                 250                 255
   Asn Ser Met Val Leu Ala Asn Asn Ala Glu Asn Val Leu Leu Pro Leu
               260                 265                 270
   Asn Glu Pro Val His Gly Thr Lys Arg Arg Ser Gln Ile Gln Thr Phe
           275                 280                 285
   Leu Asp His His Gly Gly Pro Gly Val Gln His Met Ala Leu Ala Ser
       290                 295                 300
   Asp Asp Val Leu Arg Thr Leu Arg Glu Met Gln Ala Cys Ser Ala Met
   305                 310                 315                 320
   Gly Gly Phe Glu Phe Met Ala Pro Pro Ala Pro Glu Tyr Tyr Asp Gly
                   325                 330                 335
   Val Arg Arg Arg Ala Gly Asp Val Leu Thr Glu Ala Gln Ile Lys Glu
               340                 345                 350
   Cys Gln Glu Leu Gly Val Leu Val Asp Arg Asp Asp Gln Gly Val Leu
           355                 360                 365
   Leu Gln Ile Phe Thr Lys Pro Val Gly Asp Arg Pro Thr Phe Phe Leu
       370                 375                 380
   Glu Ile Ile Gln Arg Ile Gly Cys Met Glu Lys Asp Glu Lys Gly Gln
   385                 390                 395                 400
   Glu Tyr Gln Lys Gly Gly Cys Gly Gly Phe Gly Lys Gly Asn Phe Ser
                   405                 410                 415
   Gln Leu Phe Lys Ser Ile Glu Asp Tyr Glu Lys Ser Leu Glu Ala Lys
               420                 425                 430
   Gln Ala Ala Ala Ala Gln Gly Pro
           435                 440
<210> 27
   <211> 1323
   <212> DNA
   <213> Sorghum halapense
<400> 27
   atgcctccga ccccgaccac cgcagcagca acaggtgccg cagttgcagc agcaagcgca      60
   gaacaggcag catttcgtct ggttggtcat cgtaattttg ttcgtgttaa tccgcgtagc     120
   gatcgttttc ataccctggc atttcatcat gttgaactgt ggtgtgccga tgcagccagc     180
   gcagcaggtc gttttagctt tggtctgggt gcaccgctgg cagcacgtag cgatctgagc     240
   accggtaata ccgcacatgc aagcctgctg ctgcgttcag gtgcactggc atttctgttt     300
   accgcaccgt atgcccatgg tgctgatgca gcaaccgcaa gcctgccgag ctttagcgca     360
   gcagaagcac gtcgttttgc agcagatcat ggtctggcag ttcgtgccgt tgcactgcgt     420
   gttgcagatg ccgaagatgc atttcgtgca agcgttgcag ccggtgcacg tccggcattt     480
   gaaccggttg aactgggtct gggttttcgt ctggccgaag ttgaactgta tggtgatgtt     540
   gttctgcgtt atgttagcta tccggatgat gcagatgcaa gctttctgcc gggttttgtt     600
   ggtgttacca gtccgggtgc ggcagattat ggcctgaaac gttttgatca tattgtgggt     660
   aatgttccgg aactggcacc ggcagcggca tattttgcag gttttaccgg ctttcatgaa     720
   tttgcagaat ttaccgcaga agatgttggc accaccgaaa gcggtctgaa tagcatggtt     780
   ctggcaaata atgccgaaaa tgttctgctg ccgctgaatg aaccggtgca tggcaccaaa     840
   cgtcgtagcc agattcagac ctttctggat catcatggtg gtccgggtgt tcagcacatg     900
   gcactggcaa gtgatgatgt gctgcgtacc ctgcgtgaaa tgcaggcacg tagtgcaatg     960
   ggtggttttg aatttatggc accgccggca ccggaatatt atgatggtgt tcgtcgtcgt    1020
   gccggtgatg ttctgaccga agcacagatt aaagaatgtc aggaactggg cgttctggtt    1080
   gatcgtgatg atcagggtgt tctgctgcag atttttacca aaccggttgg tgatcgcccg    1140
   accttttttc tggaaattat tcagcgtatt ggttgcatgg aaaaagatga aaaaggccag    1200
   gaatatcaga aaggcggttg tggtggtttt ggtaaaggta attttagcca gctgtttaaa    1260
   agcattgaag attatgaaaa aagcctggaa gcaaaacagg cagctgcagc acagggtccg    1320
   taa                                                                  1323
<210> 28
   <211> 453
   <212> PRT
   <213> Sorghum halapense
<400> 28
   Met Pro Pro Thr Pro Thr Thr Ala Ala Ala Thr Gly Ala Ala Val Ala
   1               5                   10                  15
   Ala Ala Ser Ala Glu Gln Ala Ala Phe Arg Leu Val Gly His Arg Asn
               20                  25                  30
   Phe Val Arg Val Asn Pro Arg Ser Asp Arg Phe His Thr Leu Ala Phe
           35                  40                  45
   His His Val Glu Leu Trp Cys Ala Asp Ala Ala Ser Ala Ala Gly Arg
       50                  55                  60
   Phe Ser Phe Gly Leu Gly Ala Pro Leu Ala Ala Arg Ser Asp Leu Ser
   65                  70                  75                  80
   Thr Gly Asn Thr Ala His Ala Ser Leu Leu Leu Arg Ser Gly Ala Leu
                   85                  90                  95
   Ala Phe Leu Phe Thr Ala Pro Tyr Ala His Gly Ala Asp Ala Ala Thr
               100                 105                 110
   Ala Ser Leu Pro Ser Phe Ser Ala Ala Glu Ala Arg Arg Phe Ala Ala
           115                 120                 125
   Asp His Gly Leu Ala Val Arg Ala Val Ala Leu Arg Val Ala Asp Ala
       130                 135                 140
   Glu Asp Ala Phe Arg Ala Ser Val Ala Ala Gly Ala Arg Pro Ala Phe
   145                 150                 155                 160
   Glu Pro Val Glu Leu Gly Leu Gly Phe Arg Leu Ala Glu Val Glu Leu
                   165                 170                 175
   Tyr Gly Asp Val Val Leu Arg Tyr Val Ser Tyr Pro Thr Thr Arg Thr
               180                 185                 190
   Arg Pro Ser Cys Arg Gly Ser Trp Ala Asp Asp Ala Asp Ala Ser Phe
           195                 200                 205
   Leu Pro Gly Phe Val Gly Val Thr Ser Pro Gly Ala Ala Asp Tyr Gly
       210                 215                 220
   Leu Lys Arg Phe Asp His Ile Val Gly Asn Val Pro Glu Leu Ala Pro
   225                 230                 235                 240
   Ala Ala Ala Tyr Phe Ala Gly Phe Thr Gly Phe His Glu Phe Ala Glu
                   245                 250                 255
   Phe Thr Ala Glu Asp Val Gly Thr Thr Glu Ser Gly Leu Asn Ser Met
               260                 265                 270
   Val Leu Ala Asn Asn Ala Glu Asn Val Leu Leu Pro Leu Asn Glu Pro
           275                 280                 285
   Val His Gly Thr Lys Arg Arg Ser Gln Ile Gln Thr Phe Leu Asp His
       290                 295                 300
   His Gly Gly Pro Gly Val Gln His Met Ala Leu Ala Ser Asp Asp Val
   305                 310                 315                 320
   Leu Arg Thr Leu Arg Glu Met Gln Ala Arg Ser Ala Met Gly Gly Phe
                   325                 330                 335
   Glu Phe Met Ala Pro Pro Ala Pro Glu Tyr Tyr Asp Gly Val Arg Arg
               340                 345                 350
   Arg Ala Gly Asp Val Leu Thr Glu Ala Gln Ile Lys Glu Cys Gln Glu
           355                 360                 365
   Leu Gly Val Leu Val Asp Arg Asp Asp Gln Gly Val Leu Leu Gln Ile
       370                 375                 380
   Phe Thr Lys Pro Val Gly Asp Arg Pro Thr Phe Phe Leu Glu Ile Ile
   385                 390                 395                 400
   Gln Arg Ile Gly Cys Met Glu Lys Asp Glu Lys Gly Gln Glu Tyr Gln
                   405                 410                 415
   Lys Gly Gly Cys Gly Gly Phe Gly Lys Gly Asn Phe Ser Gln Leu Phe
               420                 425                 430
   Lys Ser Ile Glu Asp Tyr Glu Lys Ser Leu Glu Ala Lys Gln Ala Ala
           435                 440                 445
   Ala Ala Gln Gly Pro
       450
<210> 29
   <211> 1311
   <212> DNA
   <213> Poa annua
<400> 29
   atgcctccga ccaccgcaac cgccaccgca gcagcaaccg ttacaccgga acatgcagca      60
   cgtcgttttc cgcgtgttgt tcgtgttaat ccgcgtagcg atcgttttcc ggttctgagc     120
   tttcatcatg ttgaattttg gtgtgccgat gcagcaagcg cagcaggtcg ttttagcttt     180
   gcactgggtg caccgctggc agcacgtagc gatctgagca ccggtaatag cgcacatgca     240
   agcctgctgc tgcgttcagg tgcactggca tttctgttta ccgcaccgta tgcaccgcag     300
   ccgcaggatg cagataccgc aagcattccg agctttagcg cagatgcagc acgtgcattt     360
   agcgcagcac atggtctggc agttcgtagc gttgcagttc gtgttgcaga tgccgcagat     420
   gcatttcgtg caagcattgc agccggtgca cgtccggcat ttgcaccggc agatctgggt     480
   cgtggttttg gtctggccga agttgaactg tatggtgatg ttgttctgcg ttttgttagc     540
   catccggatg cagatgatgc accgccgttt ctgccgggtt ttgaagcagt tagccgtcgt     600
   ccgggtgccg ttgattatgg tctgacccgt tttgatcatg ttgttggtaa tgttccggaa     660
   atgggtccgg tgattgatta tattaaaggc tttatgggct ttcatgaatt tgccgaattt     720
   accgcagaag atgttggcac caccgaaagc ggtctgaata gcgttgttct ggcaaataat     780
   agcgaagcag ttctgctgcc gctgaatgaa ccggtgcatg gcaccaaacg tcgtagccag     840
   attcagacct atctggaata tcatggtggt ccgggtgttc agcatattgc actggcaagc     900
   agtgatgttc tgcgtaccct gcgtgaaatg caggcacgtt cagcaatggg tggttttgaa     960
   tttatggcac cgccgcagcc gaaatattat gaaggtgttc gtcgtattgc cggtgatgtt    1020
   ctgagcgaag cacagattaa agaatgtcag gaactgggcg ttctggttga tcgtgatgat    1080
   cagggtgttc tgctgcagat ttttaccaaa ccggttggtg atcgtccgac cttttttctg    1140
   gaaatgattc agcgtattgg ctgcatggaa aaagatgaac gtggtcagga atatcagaaa    1200
   ggcggttgtg gcggttttgg taaaggtaat tttagcgaac tgtttaaaag cattgaagat    1260
   tatgaaaaaa gcctggaagc caaacagagc gcagttgcac agcagagcta a             1311
<210> 30
   <211> 436
   <212> PRT
   <213> Poa annua
<400> 30
   Met Pro Pro Thr Thr Ala Thr Ala Thr Ala Ala Ala Thr Val Thr Pro
   1               5                   10                  15
   Glu His Ala Ala Arg Arg Phe Pro Arg Val Val Arg Val Asn Pro Arg
               20                  25                  30
   Ser Asp Arg Phe Pro Val Leu Ser Phe His His Val Glu Phe Trp Cys
           35                  40                  45
   Ala Asp Ala Ala Ser Ala Ala Gly Arg Phe Ser Phe Ala Leu Gly Ala
       50                  55                  60
   Pro Leu Ala Ala Arg Ser Asp Leu Ser Thr Gly Asn Ser Ala His Ala
   65                  70                  75                  80
   Ser Leu Leu Leu Arg Ser Gly Ala Leu Ala Phe Leu Phe Thr Ala Pro
                   85                  90                  95
   Tyr Ala Pro Gln Pro Gln Asp Ala Asp Thr Ala Ser Ile Pro Ser Phe
               100                 105                 110
   Ser Ala Asp Ala Ala Arg Ala Phe Ser Ala Ala His Gly Leu Ala Val
           115                 120                 125
   Arg Ser Val Ala Val Arg Val Ala Asp Ala Ala Asp Ala Phe Arg Ala
       130                 135                 140
   Ser Ile Ala Ala Gly Ala Arg Pro Ala Phe Ala Pro Ala Asp Leu Gly
   145                 150                 155                 160
   Arg Gly Phe Gly Leu Ala Glu Val Glu Leu Tyr Gly Asp Val Val Leu
                   165                 170                 175
   Arg Phe Val Ser His Pro Asp Ala Asp Asp Ala Pro Pro Phe Leu Pro
               180                 185                 190
   Gly Phe Glu Ala Val Ser Arg Arg Pro Gly Ala Val Asp Tyr Gly Leu
           195                 200                 205
   Thr Arg Phe Asp His Val Val Gly Asn Val Pro Glu Met Gly Pro Val
       210                 215                 220
   Ile Asp Tyr Ile Lys Gly Phe Met Gly Phe His Glu Phe Ala Glu Phe
   225                 230                 235                 240
   Thr Ala Glu Asp Val Gly Thr Thr Glu Ser Gly Leu Asn Ser Val Val
                   245                 250                 255
   Leu Ala Asn Asn Ser Glu Ala Val Leu Leu Pro Leu Asn Glu Pro Val
               260                 265                 270
   His Gly Thr Lys Arg Arg Ser Gln Ile Gln Thr Tyr Leu Glu Tyr His
           275                 280                 285
   Gly Gly Pro Gly Val Gln His Ile Ala Leu Ala Ser Ser Asp Val Leu
       290                 295                 300
   Arg Thr Leu Arg Glu Met Gln Ala Arg Ser Ala Met Gly Gly Phe Glu
   305                 310                 315                 320
   Phe Met Ala Pro Pro Gln Pro Lys Tyr Tyr Glu Gly Val Arg Arg Ile
                   325                 330                 335
   Ala Gly Asp Val Leu Ser Glu Ala Gln Ile Lys Glu Cys Gln Glu Leu
               340                 345                 350
   Gly Val Leu Val Asp Arg Asp Asp Gln Gly Val Leu Leu Gln Ile Phe
           355                 360                 365
   Thr Lys Pro Val Gly Asp Arg Pro Thr Phe Phe Leu Glu Met Ile Gln
       370                 375                 380
   Arg Ile Gly Cys Met Glu Lys Asp Glu Arg Gly Gln Glu Tyr Gln Lys
   385                 390                 395                 400
   Gly Gly Cys Gly Gly Phe Gly Lys Gly Asn Phe Ser Glu Leu Phe Lys
                   405                 410                 415
   Ser Ile Glu Asp Tyr Glu Lys Ser Leu Glu Ala Lys Gln Ser Ala Val
               420                 425                 430
   Ala Gln Gln Ser
           435
<210> 31
   <211> 1305
   <212> DNA
   <213> Poa annua
<400> 31
   atgccaccaa ctactgctac tgctacagct gctgctactg ttactccaga acatgctgct      60
   agaaggttcc caagagttgt tagagttaac ccaaggtctg ataggttccc agttctttct     120
   ttccaccacg ttgaattttg gtgtgctgat gctgcttctg ctgctggaag attttctttt     180
   gctcttggtg ctccacttgc tgctagatct gatttgtcta ctggaaattc tgctcacgct     240
   tctttgcttt tgaggtctgg tgctcttgct ttccttttta ctgctcctta tgctccacaa     300
   ccacaggatg ctgatactgc atcaattcca tctttctcag ctgatgctgc aagggctttt     360
   tctgctgctc atggattggc tgttagatct gttgctgtta gagttgctga tgcagctgat     420
   gctttcagag cttctattgc tgcaggtgct agaccagctt ttgctccagc tgatcttgga     480
   agaggatttg gacttgctga ggttgagctt tacggtgatg ttgttcttag attcgtgtct     540
   cacccagatg ctgatgatgc tccatttctt ccaggatttg aggctgtttc tagaccaggt     600
   gctgttgatt atggactcac taggttcgat cacgttgtgg gaaatgttcc agaaatggga     660
   ccagtgatcg attacatcaa gggattcatg ggattccatg agttcgctga gtttactgct     720
   gaggatgttg gaactactga gtctggactt aactctgttg tgcttgctaa caactctgag     780
   gctgttcttt tgccacttaa tgagccagtt cacggcacta agagaagatc tcagattcag     840
   acttaccttg agtaccatgg tggaccaggt gttcaacata ttgctcttgc ttcatctgat     900
   gtgcttagga ctcttagaga gatgcaagct agatctgcta tgggaggatt tgagtttatg     960
   gctccaccac aacctaagta ttacgagggt gttagaagga ttgctggtga tgttctttcc    1020
   gaggctcaaa tcaaagagtg tcaagagctt ggagtgcttg tggatagaga tgatcagggt    1080
   gtgcttctcc agattttcac taagccagtt ggagataggc caacattctt cttggagatg    1140
   attcagagga tcggctgcat ggaaaaggat gagagaggtc aagagtatca aaagggcgga    1200
   tgtggtggat ttggaaaggg aaatttctcc gagcttttca agtccatcga ggattacgag    1260
   aagtctcttg aggctaagca atctgctgtt gctcaacagt cttga                   1305
<210> 32
   <211> 434
   <212> PRT
   <213> Poa annua
<400> 32
   Met Pro Pro Thr Thr Ala Thr Ala Thr Ala Ala Ala Thr Val Thr Pro
   1               5                   10                  15
   Glu His Ala Ala Arg Arg Phe Pro Arg Val Val Arg Val Asn Pro Arg
               20                  25                  30
   Ser Asp Arg Phe Pro Val Leu Ser Phe His His Val Glu Phe Trp Cys
           35                  40                  45
   Ala Asp Ala Ala Ser Ala Ala Gly Arg Phe Ser Phe Ala Leu Gly Ala
       50                  55                  60
   Pro Leu Ala Ala Arg Ser Asp Leu Ser Thr Gly Asn Ser Ala His Ala
   65                  70                  75                  80
   Ser Leu Leu Leu Arg Ser Gly Ala Leu Ala Phe Leu Phe Thr Ala Pro
                   85                  90                  95
   Tyr Ala Pro Gln Pro Gln Asp Ala Asp Thr Ala Ser Ile Pro Ser Phe
               100                 105                 110
   Ser Ala Asp Ala Ala Arg Ala Phe Ser Ala Ala His Gly Leu Ala Val
           115                 120                 125
   Arg Ser Val Ala Val Arg Val Ala Asp Ala Ala Asp Ala Phe Arg Ala
       130                 135                 140
   Ser Ile Ala Ala Gly Ala Arg Pro Ala Phe Ala Pro Ala Asp Leu Gly
   145                 150                 155                 160
   Arg Gly Phe Gly Leu Ala Glu Val Glu Leu Tyr Gly Asp Val Val Leu
                   165                 170                 175
   Arg Phe Val Ser His Pro Asp Ala Asp Asp Ala Pro Phe Leu Pro Gly
               180                 185                 190
   Phe Glu Ala Val Ser Arg Pro Gly Ala Val Asp Tyr Gly Leu Thr Arg
           195                 200                 205
   Phe Asp His Val Val Gly Asn Val Pro Glu Met Gly Pro Val Ile Asp
       210                 215                 220
   Tyr Ile Lys Gly Phe Met Gly Phe His Glu Phe Ala Glu Phe Thr Ala
   225                 230                 235                 240
   Glu Asp Val Gly Thr Thr Glu Ser Gly Leu Asn Ser Val Val Leu Ala
                   245                 250                 255
   Asn Asn Ser Glu Ala Val Leu Leu Pro Leu Asn Glu Pro Val His Gly
               260                 265                 270
   Thr Lys Arg Arg Ser Gln Ile Gln Thr Tyr Leu Glu Tyr His Gly Gly
           275                 280                 285
   Pro Gly Val Gln His Ile Ala Leu Ala Ser Ser Asp Val Leu Arg Thr
       290                 295                 300
   Leu Arg Glu Met Gln Ala Arg Ser Ala Met Gly Gly Phe Glu Phe Met
   305                 310                 315                 320
   Ala Pro Pro Gln Pro Lys Tyr Tyr Glu Gly Val Arg Arg Ile Ala Gly
                   325                 330                 335
   Asp Val Leu Ser Glu Ala Gln Ile Lys Glu Cys Gln Glu Leu Gly Val
               340                 345                 350
   Leu Val Asp Arg Asp Asp Gln Gly Val Leu Leu Gln Ile Phe Thr Lys
           355                 360                 365
   Pro Val Gly Asp Arg Pro Thr Phe Phe Leu Glu Met Ile Gln Arg Ile
       370                 375                 380
   Gly Cys Met Glu Lys Asp Glu Arg Gly Gln Glu Tyr Gln Lys Gly Gly
   385                 390                 395                 400
   Cys Gly Gly Phe Gly Lys Gly Asn Phe Ser Glu Leu Phe Lys Ser Ile
                   405                 410                 415
   Glu Asp Tyr Glu Lys Ser Leu Glu Ala Lys Gln Ser Ala Val Ala Gln
               420                 425                 430
   Gln Ser
<210> 33
   <211> 1323
   <212> DNA
   <213> Lolium multiflorum
<400> 33
   atgcctccga caccggcaac cgcaaccggt gctgcagcag cagcagttac accggaacat      60
   gcagcacgta gctttccgcg tgttgttcgt gttaatccgc gtagcgatcg ttttccggtt     120
   ctgagctttc atcatgttga actgtggtgt gccgatgcag caagcgcagc aggtcgtttt     180
   agctttgcac tgggtgctcc gctggcagcc cgtagcgatc tgagcaccgg taatagcgca     240
   catgcaagcc tgctgctgcg tagcggtgca ctggcatttc tgtttaccgc accgtatgca     300
   ccgcctccgc aggaagcagc aaccgcagct gcaaccgcaa gcattccgag ctttagcgca     360
   gatgcagccc gtacctttgc agcagcacat ggtctggcag ttcgtagcgt tggtgttcgt     420
   gttgccgatg cagcggaagc atttcgtgtt agcgttgccg gtggtgcacg tccggcattt     480
   gcaccggcag atctgggtca tggttttggt ctggccgaag ttgaactgta tggtgatgtt     540
   gttctgcgtt ttgttagcta tccggatgaa accgatctgc cgtttctgcc gggttttgaa     600
   cgtgttagca gtccgggtgc cgttgattat ggtctgaccc gttttgatca tgttgttggt     660
   aatgttccgg aaatggcacc ggttattgat tatatgaaag gctttctggg ctttcatgaa     720
   tttgcagaat ttaccgcaga agatgttggc accaccgaaa gcggtctgaa tagcgttgtt     780
   ctggcaaata atagcgaaaa tgttctgctg ccgctgaatg aaccggtgca tggcaccaaa     840
   cgtcgtagcc agattcagac ctatctggat tatcatggtg gtccgggtgt tcagcatatt     900
   gcactggcaa gcaccgatgt tctgcgtacc ctgcgtgaaa tgcgtgcacg taccccgatg     960
   ggtggttttg aatttatggc accgccgcag gcaaaatatt atgaaggtgt tcgtcgtatt    1020
   gccggtgatg ttctgagcga agaacaaatt aaagaatgtc aggaactggg cgttctggtt    1080
   gatcgtgatg atcagggtgt tctgctgcag atttttacca aaccggttgg tgatcgtccg    1140
   accttttttc tggaaatgat tcagcgtatt ggctgcatgg aaaaagatga agttggtcag    1200
   gaatatcaga aaggcggttg tggtggtttt ggtaaaggta attttagcga actgtttaaa    1260
   agcattgaag attatgaaaa aaccctggaa gccaaacaga gcgttgttgc acagaaaagc    1320
   taa                                                                  1323
<210> 34
   <211> 440
   <212> PRT
   <213> Lolium multiflorum
<400> 34
   Met Pro Pro Thr Pro Ala Thr Ala Thr Gly Ala Ala Ala Ala Ala Val
   1               5                   10                  15
   Thr Pro Glu His Ala Ala Arg Ser Phe Pro Arg Val Val Arg Val Asn
               20                  25                  30
   Pro Arg Ser Asp Arg Phe Pro Val Leu Ser Phe His His Val Glu Leu
           35                  40                  45
   Trp Cys Ala Asp Ala Ala Ser Ala Ala Gly Arg Phe Ser Phe Ala Leu
       50                  55                  60
   Gly Ala Pro Leu Ala Ala Arg Ser Asp Leu Ser Thr Gly Asn Ser Ala
   65                  70                  75                  80
   His Ala Ser Leu Leu Leu Arg Ser Gly Ala Leu Ala Phe Leu Phe Thr
                   85                  90                  95
   Ala Pro Tyr Ala Pro Pro Pro Gln Glu Ala Ala Thr Ala Ala Ala Thr
               100                 105                 110
   Ala Ser Ile Pro Ser Phe Ser Ala Asp Ala Ala Arg Thr Phe Ala Ala
           115                 120                 125
   Ala His Gly Leu Ala Val Arg Ser Val Gly Val Arg Val Ala Asp Ala
       130                 135                 140
   Ala Glu Ala Phe Arg Val Ser Val Ala Gly Gly Ala Arg Pro Ala Phe
   145                 150                 155                 160
   Ala Pro Ala Asp Leu Gly His Gly Phe Gly Leu Ala Glu Val Glu Leu
                   165                 170                 175
   Tyr Gly Asp Val Val Leu Arg Phe Val Ser Tyr Pro Asp Glu Thr Asp
               180                 185                 190
   Leu Pro Phe Leu Pro Gly Phe Glu Arg Val Ser Ser Pro Gly Ala Val
           195                 200                 205
   Asp Tyr Gly Leu Thr Arg Phe Asp His Val Val Gly Asn Val Pro Glu
       210                 215                 220
   Met Ala Pro Val Ile Asp Tyr Met Lys Gly Phe Leu Gly Phe His Glu
   225                 230                 235                 240
   Phe Ala Glu Phe Thr Ala Glu Asp Val Gly Thr Thr Glu Ser Gly Leu
                   245                 250                 255
   Asn Ser Val Val Leu Ala Asn Asn Ser Glu Asn Val Leu Leu Pro Leu
               260                 265                 270
   Asn Glu Pro Val His Gly Thr Lys Arg Arg Ser Gln Ile Gln Thr Tyr
           275                 280                 285
   Leu Asp Tyr His Gly Gly Pro Gly Val Gln His Ile Ala Leu Ala Ser
       290                 295                 300
   Thr Asp Val Leu Arg Thr Leu Arg Glu Met Arg Ala Arg Thr Pro Met
   305                 310                 315                 320
   Gly Gly Phe Glu Phe Met Ala Pro Pro Gln Ala Lys Tyr Tyr Glu Gly
                   325                 330                 335
   Val Arg Arg Ile Ala Gly Asp Val Leu Ser Glu Glu Gln Ile Lys Glu
               340                 345                 350
   Cys Gln Glu Leu Gly Val Leu Val Asp Arg Asp Asp Gln Gly Val Leu
           355                 360                 365
   Leu Gln Ile Phe Thr Lys Pro Val Gly Asp Arg Pro Thr Phe Phe Leu
       370                 375                 380
   Glu Met Ile Gln Arg Ile Gly Cys Met Glu Lys Asp Glu Val Gly Gln
   385                 390                 395                 400
   Glu Tyr Gln Lys Gly Gly Cys Gly Gly Phe Gly Lys Gly Asn Phe Ser
                   405                 410                 415
   Glu Leu Phe Lys Ser Ile Glu Asp Tyr Glu Lys Thr Leu Glu Ala Lys
               420                 425                 430
   Gln Ser Val Val Ala Gln Lys Ser
           435                 440
<210> 35
   <211> 1053
   <212> DNA
   <213> Synechococcus sp
<400> 35
   atgaacccgt ccattcgaat tgtccaaggg atccaccacc tgcacttcta cctttgggat      60
   ctgccccgtt ggcgggaaca cttttgtcgg gtttggggct tccgggtggc aagcgacgcc     120
   ggcaacaccc tggagctgga gcagggatcc ctgcgcttgc gcctgtctca gccggcacgg     180
   gcgggggacg aggtggaccg ccatttgcag cggcatgggc cgggggtggt ggatgtggcc     240
   ttggcggtgg gagagcagga gctaccggcc ttggcggagc tgttgcgggg ccgaggcgcc     300
   caactggcgt ggatcccggc agcagcggcg ctctgcctcc acacccccta cgggatccgg      360
   cattctctga tccctggccc cttggatgcc gcccctgccg aagcgggcct gttttcccac     420
   tgggatcacg tggtgttgaa cgtggagcag ggatccctgc aggcggcagc cgactggtat     480
   gggcgggtgc tgggctggcg gcggctgtac cgctacagca tcggcaccgc cacctccggc     540
   ctggaaagcg tggtggtggg ggatccggaa gcggggatcc aatgggccat caacgagccc     600
   acctgtgccg cttcccagat tcaggagttt ttgcatgccc atggcggccc gggcattcag     660
   cacgcggcgc tgcacagctc agacattgtt gccagcctgc gccggttgcg gcagggggga      720
   gtggactttt tgcaagtggc gccgcagtac tacaccagcc tggaaaggga gctggggttg     780
   gcgctccgtt ctgcccttgg gcaggccatc tcctggcaag acctggtgga gcagcagatc     840
   cttctggatg ctaccctgcc cgcttctgat ggccaggatc gcccccttct gctgcagacc     900
   tttacccagc ccctctttgg tcggcccacc tttttctttg aagtcattca acggctaggc     960
   ggggccacgg gctttggcga ggccaatttt caggctttgt tcgaggccct ggaacggcaa    1020
   cagcgacagc gacaccaggc gctgacccct tag                                 1053
<210> 36
   <211> 350
   <212> PRT
   <213> Synechococcus sp
<400> 36
   Met Asn Pro Ser Ile Arg Ile Val Gln Gly Ile His His Leu His Phe
   1               5                   10                  15
   Tyr Leu Trp Asp Leu Pro Arg Trp Arg Glu His Phe Cys Arg Val Trp
               20                  25                  30
   Gly Phe Arg Val Ala Ser Asp Ala Gly Asn Thr Leu Glu Leu Glu Gln
           35                  40                  45
   Gly Ser Leu Arg Leu Arg Leu Ser Gln Pro Ala Arg Ala Gly Asp Glu
       50                  55                  60
   Val Asp Arg His Leu Gln Arg His Gly Pro Gly Val Val Asp Val Ala
   65                  70                  75                  80
   Leu Ala Val Gly Glu Gln Glu Leu Pro Ala Leu Ala Glu Leu Leu Arg
                   85                  90                  95
   Gly Arg Gly Ala Gln Leu Ala Trp Ile Pro Ala Ala Ala Ala Leu Cys
               100                 105                 110
   Leu His Thr Pro Tyr Gly Ile Arg His Ser Leu Ile Pro Gly Pro Leu
           115                 120                 125
   Asp Ala Ala Pro Ala Glu Ala Gly Leu Phe Ser His Trp Asp His Val
       130                 135                 140
   Val Leu Asn Val Glu Gln Gly Ser Leu Gln Ala Ala Ala Asp Trp Tyr
   145                 150                 155                 160
   Gly Arg Val Leu Gly Trp Arg Arg Leu Tyr Arg Tyr Ser Ile Gly Thr
                   165                 170                 175
   Ala Thr Ser Gly Leu Glu Ser Val Val Val Gly Asp Pro Glu Ala Gly
               180                 185                 190
   Ile Gln Trp Ala Ile Asn Glu Pro Thr Cys Ala Ala Ser Gln Ile Gln
           195                 200                 205
   Glu Phe Leu His Ala His Gly Gly Pro Gly Ile Gln His Ala Ala Leu
       210                 215                 220
   His Ser Ser Asp Ile Val Ala Ser Leu Arg Arg Leu Arg Gln Gly Gly
   225                 230                 235                 240
   Val Asp Phe Leu Gln Val Ala Pro Gln Tyr Tyr Thr Ser Leu Glu Arg
                   245                 250                 255
   Glu Leu Gly Leu Ala Leu Arg Ser Ala Leu Gly Gln Ala Ile Ser Trp
               260                 265                 270
   Gln Asp Leu Val Glu Gln Gln Ile Leu Leu Asp Ala Thr Leu Pro Ala
           275                 280                 285
   Ser Asp Gly Gln Asp Arg Pro Leu Leu Leu Gln Thr Phe Thr Gln Pro
       290                 295                 300
   Leu Phe Gly Arg Pro Thr Phe Phe Phe Glu Val Ile Gln Arg Leu Gly
   305                 310                 315                 320
   Gly Ala Thr Gly Phe Gly Glu Ala Asn Phe Gln Ala Leu Phe Glu Ala
                   325                 330                 335
   Leu Glu Arg Gln Gln Arg Gln Arg His Gln Ala Leu Thr Pro
               340                 345                 350
<210> 37
   <211> 1149
   <212> DNA
   <213> Blepharisma japonicum
<400> 37
   atgacttatt acgacaagca agaaacgcgt ccagatcttg gcgaattcta tggtttccat      60
   cacgttcgtt tttacgtctc caactcagag caagccgctt cgttctacac atctcgcttt     120
   gggttttctc cggttgccta tgaaggattg gaaacaggaa accaaaaatt ctgtaccaat     180
   gtcgtccgaa gcaaccatgt agtcatcgct tttacctcag ctctcactcc tgaagacaat     240
   gaagtgaacc gtcacgttgg caagcatagt gatggagttc aagacattgc ctttagtgta     300
   agtgacgcaa gagggatgta tgagaaagcg atagctaaag gctgtaaaag cttccgtgag     360
   ccacaggttt tacaagatca atttggatct gttataatag cgtctctcca gacttatgga     420
   gacactgttc acacattagt ccaaaatgtc gactatacag gacccttttt gcctggcttc      480
   agagcaatca caaaagatga tccattaaac tctgcctttc ctcaggtaaa ttatgacatt     540
   attgatcatg ttgtaggaaa tcagcctggt ggcgatatga ctcctacagt agaatggtat     600
   gagaaatatc tagaatttca tcgatattgg tctgctgatg agtctgtaat ccataccgat     660
   tattcagcat taaggtctgt tgtggttgct gattgggatg aagtgatcaa aatgcctatt     720
   aatgagcctg ctgatggact tagaaaaagt caaatccaag aatatgtcga atattatggt     780
   ggagcaggcg tacaacatat tgccttaaaa gtcaatgata ttatttcagt aataagcacc     840
   ttaagggcta gaggtgtgga attcttagaa gttcctccta aatattatga tagcttaaga     900
   aaaagacttg cgcattctgc ggtacaaatt gaagaagact taaaaagaat tgaagacctt     960
   catattttgg ttgactttga cgaccgtggg tatttacttc agattttcac aaaaccagta    1020
   gaagacagac ctactctgtt ttatgaaatt attcaaagac ataataacaa tggattcgga    1080
   attggaaatt ttaaagccct atttgaatca ttggaacaag agcaagaaag aagaggtaat    1140
   ttgatctaa                                                             1149
<210> 38
   <211> 350
   <212> PRT
   <213> Blephrisman japonicum
<400> 38
   Met Asn Pro Ser Ile Arg Ile Val Gln Gly Ile His His Leu His Phe
   1               5                   10                  15
   Tyr Leu Trp Asp Leu Pro Arg Trp Arg Glu His Phe Cys Arg Val Trp
               20                  25                  30
   Gly Phe Arg Val Ala Ser Asp Ala Gly Asn Thr Leu Glu Leu Glu Gln
           35                  40                  45
   Gly Ser Leu Arg Leu Arg Leu Ser Gln Pro Ala Arg Ala Gly Asp Glu
           50                  55                  60
   Val Asp Arg His Leu Gln Arg His Gly Pro Gly Val Val Asp Val Ala
   65                  70                  75                  80
   Leu Ala Val Gly Glu Gln Glu Leu Pro Ala Leu Ala Glu Leu Leu Arg
                       85                  90                  95
   Gly Arg Gly Ala Gln Leu Ala Trp Ile Pro Ala Ala Ala Ala Leu Cys
               100                 105                 110
   Leu His Thr Pro Tyr Gly Ile Arg His Ser Leu Ile Pro Gly Pro Leu
           115                 120                 125
   Asp Ala Ala Pro Ala Glu Ala Gly Leu Phe Ser His Trp Asp His Val
       130                 135                 140
   Val Leu Asn Val Glu Gln Gly Ser Leu Gln Ala Ala Ala Asp Trp Tyr
   145                 150                 155                 160
   Gly Arg Val Leu Gly Trp Arg Arg Leu Tyr Arg Tyr Ser Ile Gly Thr
                       165                 170                 175
   Ala Thr Ser Gly Leu Glu Ser Val Val Val Gly Asp Pro Glu Ala Gly
               180                 185                 190
   Ile Gln Trp Ala Ile Asn Glu Pro Thr Cys Ala Ala Ser Gln Ile Gln
           195                 200                 205
   Glu Phe Leu His Ala His Gly Gly Pro Gly Ile Gln His Ala Ala Leu
       210                 215                 220
   His Ser Ser Asp Ile Val Ala Ser Leu Arg Arg Leu Arg Gln Gly Gly
   225                 230                 235                 240
   Val Asp Phe Leu Gln Val Ala Pro Gln Tyr Tyr Thr Ser Leu Glu Arg
                       245                 250                 255
   Glu Leu Gly Leu Ala Leu Arg Ser Ala Leu Gly Gln Ala Ile Ser Trp
               260                 265                 270
   Gln Asp Leu Val Glu Gln Gln Ile Leu Leu Asp Ala Thr Leu Pro Ala
           275                 280                 285
   Ser Asp Gly Gln Asp Arg Pro Leu Leu Leu Gln Thr Phe Thr Gln Pro
       290                 295                 300
   Leu Phe Gly Arg Pro Thr Phe Phe Phe Glu Val Ile Gln Arg Leu Gly
   305                 310                 315                 320
   Gly Ala Thr Gly Phe Gly Glu Ala Asn Phe Gln Ala Leu Phe Glu Ala
                       325                 330                 335
   Leu Glu Arg Gln Gln Arg Gln Arg His Gln Ala Leu Thr Pro
               340                 345                 350
<210> 39
   <211> 1107
   <212> DNA
   <213> Picrophilus torridus
<400> 39
   atgtatggca aaaatttaat ctcagaacta agggaaaagg agatctttaa acgattacat      60
   cacgtggaat tttacgttag cagtgccaaa acatggtcat atttcatgaa caggggtctt     120
   ggatttaaaa cagtggcata tgccggtcca gaaaccggga taagggacaa gatatcctat     180
   gttatgtccc agggcactgc aaggatatct tttacatcat caatgaatga tgatagctat     240
   atatcgaatc atgttaaaaa acacggggat ggcgtaaagg atatagcact tgaggtcgat     300
   gatctggacg aggcaaaaag cctgatagaa aagtatggaa caaaggtttc aaaaataaat     360
   gaaataaagg atggaaatgg aaagataaga actgcagaga taaaaacgta cggtgaaacc     420
   gttcatacat taatagaaac cggggattac aatggcgtat tcatgcccgg ttatgaggaa     480
   tctgaaataa attcaaaaaa cactgggata aaaaagatcg atcatatagt tggaaatgtc     540
   tatgagggcg agatggatag ctgggttaat ttttacatag aaaaacttgg ctttgagcat     600
   ttaataacct ttgatgataa agatataaga actgattaca gcgcattaag atcaaaggtt     660
   gtaaaataca atgacgatat cgtatttcca ataaatgagc ctgcaaaggg cttaagaaaa     720
   tcacagatag aggaatatct tgactattac aggtctgagg gcgttcagca catagcactg     780
   ttaactgatg atataataaa aactgtatcc atgatggagg aaaacggcat agaattttta     840
   aaaacaccag gatcatacta tgaatcccta tcatcaagga taggctcaat agacgaggat     900
   ttaaatgaaa tagagaaaca taacatactt gtggatcgtg atgagaacgg atacctatta     960
   cagatcttca caaagcctgt tactgacagg ccaacgttct tctttgaggt catacagaga    1020
   aagggtgcaa ggtcattcgg caacggtaac tttaaggcac tttttgaggc gatagaaagg    1080
   gagcaggcaa agagaggaaa cctatga                                        1107
<210> 40
   <211> 368
   <212> PRT
   <213> Picrophilus torridus
<400> 40
   Met Tyr Gly Lys Asn Leu Ile Ser Glu Leu Arg Glu Lys Glu Ile Phe
   1               5                   10                  15
   Lys Arg Leu His His Val Glu Phe Tyr Val Ser Ser Ala Lys Thr Trp
               20                  25                  30
   Ser Tyr Phe Met Asn Arg Gly Leu Gly Phe Lys Thr Val Ala Tyr Ala
           35                  40                  45
   Gly Pro Glu Thr Gly Ile Arg Asp Lys Ile Ser Tyr Val Met Ser Gln
       50                  55                  60
   Gly Thr Ala Arg Ile Ser Phe Thr Ser Ser Met Asn Asp Asp Ser Tyr
   65                  70                  75                  80
   Ile Ser Asn His Val Lys Lys His Gly Asp Gly Val Lys Asp Ile Ala
                   85                  90                  95
   Leu Glu Val Asp Asp Leu Asp Glu Ala Lys Ser Leu Ile Glu Lys Tyr
               100                 105                 110
   Gly Thr Lys Val Ser Lys Ile Asn Glu Ile Lys Asp Gly Asn Gly Lys
           115                 120                 125
   Ile Arg Thr Ala Glu Ile Lys Thr Tyr Gly Glu Thr Val His Thr Leu
       130                 135                 140
   Ile Glu Thr Gly Asp Tyr Asn Gly Val Phe Met Pro Gly Tyr Glu Glu
   145                 150                 155                 160
   Ser Glu Ile Asn Ser Lys Asn Thr Gly Ile Lys Lys Ile Asp His Ile
                   165                 170                 175
   Val Gly Asn Val Tyr Glu Gly Glu Met Asp Ser Trp Val Asn Phe Tyr
               180                 185                 190
   Ile Glu Lys Leu Gly Phe Glu His Leu Ile Thr Phe Asp Asp Lys Asp
           195                 200                 205
   Ile Arg Thr Asp Tyr Ser Ala Leu Arg Ser Lys Val Val Lys Tyr Asn
       210                 215                 220
   Asp Asp Ile Val Phe Pro Ile Asn Glu Pro Ala Lys Gly Leu Arg Lys
   225                 230                 235                 240
   Ser Gln Ile Glu Glu Tyr Leu Asp Tyr Tyr Arg Ser Glu Gly Val Gln
                   245                 250                 255
   His Ile Ala Leu Leu Thr Asp Asp Ile Ile Lys Thr Val Ser Met Met
               260                 265                 270
   Glu Glu Asn Gly Ile Glu Phe Leu Lys Thr Pro Gly Ser Tyr Tyr Glu
           275                 280                 285
   Ser Leu Ser Ser Arg Ile Gly Ser Ile Asp Glu Asp Leu Asn Glu Ile
       290                 295                 300
   Glu Lys His Asn Ile Leu Val Asp Arg Asp Glu Asn Gly Tyr Leu Leu
   305                 310                 315                 320
   Gln Ile Phe Thr Lys Pro Val Thr Asp Arg Pro Thr Phe Phe Phe Glu
                   325                 330                 335
   Val Ile Gln Arg Lys Gly Ala Arg Ser Phe Gly Asn Gly Asn Phe Lys
               340                 345                 350
   Ala Leu Phe Glu Ala Ile Glu Arg Glu Gln Ala Lys Arg Gly Asn Leu
           355                 360                 365
<210> 41
   <211> 1164
   <212> DNA
   <213> Kordia algicida
<400> 41
   atggcagcag aaataaaaaa cttaaaagat ttacaaaata cagaatacgg actcaaaaaa      60
   ttatttgacg aagcagaaga ctttcttcca cttttaggaa cagactacgt agaattatac     120
   gtcgggaacg ccaaacaatc ggcacatttc tacaaaacgg cttttggttt tcaatcagaa     180
   gcttacgcag gattggaaac aggattaacc gacagagttt catacgtatt aaaacaagat     240
   aaaattcgct tggtcttaac aacaccatta ggaaaaggtg gcgaaatcaa tgagcatatc     300
   gatttacacg gcgatggcgt aaaagtagta gcactttggg tagaagatgc tacaaaagcc     360
   tttgaagaaa cgaccaaaag aggcgcaaaa ccgtacatgg aaccaacaaa agaagaagat     420
   gaaaacggat atgtaattcg ctcaggaatc tatacgtacg gagaaacggt tcatgttttt     480
   gtagaacgta aaaactataa cggagtcttt ttaccaggat atcaaagatg ggaatctcac     540
   tacaatccgg agccagttgg cttaaaattc atcgatcaca tggtaggaaa tgtaggttgg     600
   ggagaaatga aagaatggtg tgaattctac gcgaaagtaa tgggatttgc gcaaattatc     660
   tcctttacag atgatgatat ttctaccgat tttactgcgt tgatgagtaa agtaatgagt     720
   aatggaaatg gtagaatcaa atttccaatc aatgaacccg cagaaggaaa aaagaaatcg     780
   caaattgaag aatatctaga cttttacaat ggttcaggag tacaacatat tgcggttgct     840
   acagacaata ttattgatac ggtttcgcaa atgcgcgaac gtggagtaga attcttatac     900
   gttccagata catattatga tgacttgtta gaacgtgttg gcgacatcga tgaagatgta     960
   gaagaactca aaaaacacgg aatcttaatt gatcgtgatg aagaaggata cttattgcag    1020
   ttatttacca aaaccattgt agacagacca acaatgttct ttgaagtcat tcagcgtaaa    1080
   ggcgcacaat catttggagt aggaaacttt aaagctttat ttgaagcgat agaaagagaa    1140
   caagctgctc gcggaacatt gtaa                                           1164
<210> 42
   <211> 368
   <212> PRT
   <213> Kordia algicida
<400> 42
   Met Tyr Gly Lys Asn Leu Ile Ser Glu Leu Arg Glu Lys Glu Ile Phe
   1               5                   10                  15
   Lys Arg Leu His His Val Glu Phe Tyr Val Ser Ser Ala Lys Thr Trp
               20                  25                  30
   Ser Tyr Phe Met Asn Arg Gly Leu Gly Phe Lys Thr Val Ala Tyr Ala
           35                  40                  45
   Gly Pro Glu Thr Gly Ile Arg Asp Lys Ile Ser Tyr Val Met Ser Gln
       50                  55                  60
   Gly Thr Ala Arg Ile Ser Phe Thr Ser Ser Met Asn Asp Asp Ser Tyr
   65                  70                  75                  80
   Ile Ser Asn His Val Lys Lys His Gly Asp Gly Val Lys Asp Ile Ala
                   85                  90                  95
   Leu Glu Val Asp Asp Leu Asp Glu Ala Lys Ser Leu Ile Glu Lys Tyr
               100                 105                 110
   Gly Thr Lys Val Ser Lys Ile Asn Glu Ile Lys Asp Gly Asn Gly Lys
           115                 120                 125
   Ile Arg Thr Ala Glu Ile Lys Thr Tyr Gly Glu Thr Val His Thr Leu
       130                 135                 140
   Ile Glu Thr Gly Asp Tyr Asn Gly Val Phe Met Pro Gly Tyr Glu Glu
   145                 150                 155                 160
   Ser Glu Ile Asn Ser Lys Asn Thr Gly Ile Lys Lys Ile Asp His Ile
                   165                 170                 175
   Val Gly Asn Val Tyr Glu Gly Glu Met Asp Ser Trp Val Asn Phe Tyr
               180                 185                 190
   Ile Glu Lys Leu Gly Phe Glu His Leu Ile Thr Phe Asp Asp Lys Asp
           195                 200                 205
   Ile Arg Thr Asp Tyr Ser Ala Leu Arg Ser Lys Val Val Lys Tyr Asn
       210                 215                 220
   Asp Asp Ile Val Phe Pro Ile Asn Glu Pro Ala Lys Gly Leu Arg Lys
   225                 230                 235                 240
   Ser Gln Ile Glu Glu Tyr Leu Asp Tyr Tyr Arg Ser Glu Gly Val Gln
                   245                 250                 255
   His Ile Ala Leu Leu Thr Asp Asp Ile Ile Lys Thr Val Ser Met Met
               260                 265                 270
   Glu Glu Asn Gly Ile Glu Phe Leu Lys Thr Pro Gly Ser Tyr Tyr Glu
           275                 280                 285
   Ser Leu Ser Ser Arg Ile Gly Ser Ile Asp Glu Asp Leu Asn Glu Ile
       290                 295                 300
   Glu Lys His Asn Ile Leu Val Asp Arg Asp Glu Asn Gly Tyr Leu Leu
   305                 310                 315                 320
   Gln Ile Phe Thr Lys Pro Val Thr Asp Arg Pro Thr Phe Phe Phe Glu
                   325                 330                 335
   Val Ile Gln Arg Lys Gly Ala Arg Ser Phe Gly Asn Gly Asn Phe Lys
               340                 345                 350
   Ala Leu Phe Glu Ala Ile Glu Arg Glu Gln Ala Lys Arg Gly Asn Leu
           355                 360                 365
<210> 43
   <211> 1206
   <212> DNA
   <213> Rhodococcus sp.
<400> 43
   atgacgatcg agcagactct caccgacaag gaacgcctgg caggtctcga cctcggccag      60
   ctcgagcagt tggtcgggct cgtcgagtac gacggcaccc gcgacccgtt cccggtcagc     120
   ggctgggatg ccgtcgtctg ggtggtcggc aacgccaccc agaccgccca ctacttccag     180
   tccgcgttcg ggatgaccct cgtcgcctac tccggaccca ccaccggcaa ccgcgaccac      240
   cacagcttcg tcctcgaatc cggggccgtc cgcttcgtca tcaaaggcgc cgtgaacccg      300
   gacagccccc tgatcgacca ccaccgcacc cacggcgacg gcgtcgtcga catcgccctc      360
   gccgtccccg acgtcgacaa gtgcatcgcc cacgcccgcg cccagggcgc caccgtcctc      420
   gacgaacccc acgacgtgac cgacgaccac ggcaccgtcc gcctcgccgc gatcgccacc      480
   tacggcgaca cccgccacac cctcgtcgac cgcagccact acaccggccc ctacctgccc      540
   ggctacaccg cccgcacctc cggccacacc aaacgggacg gggcacccaa gcgcctgttc      600
   caggccctcg accacgtcgt cggcaacgtc gaactcggca agatggacca ctgggtcgac      660
   ttctacaacc gggtcatggg ctttacgaac atggccgagt tcgtcggcga ggacatcgcc      720
   accgactact ccgcgctgat gagcaaggtc gtctccaacg gcaaccaccg ggtcaagttc      780
   cccctcaacg aacccgccct cgccaagaaa cgctcgcaga tcgacgaata cctcgacttc      840
   taccgcggcc ccggcgccca gcacctggcc ctggccacca atgacatcct caccgccgtc      900
   gaccagctga ccgccgaggg cgtcgagttc ctggccaccc ccgactccta ctacgaggac      960
   cccgaactgc gggcccggat cggcaacgtc cgcgccccca tcgccgaact gcagaaacgc     1020
   ggcatcctcg tcgaccgcga cgaagacggc tacctgctgc agatcttcac caaacccctc     1080
   gtcgaccggc ccaccgtgtt cttcgaactc atcgaacgcc acggctccct cggcttcggc     1140
   atcggcaact tcaaagccct cttcgaggcc atcgaacgcg aacaagccgc ccgcggaaac     1200
   ttctga                                                                1206
<210> 44
   <211> 401
   <212> PRT
   <213> Rhodococcus sp.
<400> 44
   Met Thr Ile Glu Gln Thr Leu Thr Asp Lys Glu Arg Leu Ala Gly Leu
   1               5                   10                  15
   Asp Leu Gly Gln Leu Glu Gln Leu Val Gly Leu Val Glu Tyr Asp Gly
               20                  25                  30
   Thr Arg Asp Pro Phe Pro Val Ser Gly Trp Asp Ala Val Val Trp Val
           35                  40                  45
   Val Gly Asn Ala Thr Gln Thr Ala His Tyr Phe Gln Ser Ala Phe Gly
       50                  55                  60
   Met Thr Leu Val Ala Tyr Ser Gly Pro Thr Thr Gly Asn Arg Asp His
   65                  70                  75                  80
   His Ser Phe Val Leu Glu Ser Gly Ala Val Arg Phe Val Ile Lys Gly
                   85                  90                  95
   Ala Val Asn Pro Asp Ser Pro Leu Ile Asp His His Arg Thr His Gly
               100                 105                 110
   Asp Gly Val Val Asp Ile Ala Leu Ala Val Pro Asp Val Asp Lys Cys
           115                 120                 125
   Ile Ala His Ala Arg Ala Gln Gly Ala Thr Val Leu Asp Glu Pro His
       130                 135                 140
   Asp Val Thr Asp Asp His Gly Thr Val Arg Leu Ala Ala Ile Ala Thr
   145                 150                 155                 160
   Tyr Gly Asp Thr Arg His Thr Leu Val Asp Arg Ser His Tyr Thr Gly
                   165                 170                 175
   Pro Tyr Leu Pro Gly Tyr Thr Ala Arg Thr Ser Gly His Thr Lys Arg
               180                 185                 190
   Asp Gly Ala Pro Lys Arg Leu Phe Gln Ala Leu Asp His Val Val Gly
           195                 200                 205
   Asn Val Glu Leu Gly Lys Met Asp His Trp Val Asp Phe Tyr Asn Arg
       210                 215                 220
   Val Met Gly Phe Thr Asn Met Ala Glu Phe Val Gly Glu Asp Ile Ala
   225                 230                 235                 240
   Thr Asp Tyr Ser Ala Leu Met Ser Lys Val Val Ser Asn Gly Asn His
                   245                 250                 255
   Arg Val Lys Phe Pro Leu Asn Glu Pro Ala Leu Ala Lys Lys Arg Ser
               260                 265                 270
   Gln Ile Asp Glu Tyr Leu Asp Phe Tyr Arg Gly Pro Gly Ala Gln His
           275                 280                 285
   Leu Ala Leu Ala Thr Asn Asp Ile Leu Thr Ala Val Asp Gln Leu Thr
       290                 295                 300
   Ala Glu Gly Val Glu Phe Leu Ala Thr Pro Asp Ser Tyr Tyr Glu Asp
   305                 310                 315                 320
   Pro Glu Leu Arg Ala Arg Ile Gly Asn Val Arg Ala Pro Ile Ala Glu
                   325                 330                 335
   Leu Gln Lys Arg Gly Ile Leu Val Asp Arg Asp Glu Asp Gly Tyr Leu
               340                 345                 350
   Leu Gln Ile Phe Thr Lys Pro Leu Val Asp Arg Pro Thr Val Phe Phe
           355                 360                 365
   Glu Leu Ile Glu Arg His Gly Ser Leu Gly Phe Gly Ile Gly Asn Phe
       370                 375                 380
   Lys Ala Leu Phe Glu Ala Ile Glu Arg Glu Gln Ala Ala Arg Gly Asn
   385                 390                 395                 400
   Phe
<210> 45
   <211> 1209
   <212> DNA
   <213> Rhodococcus sp
<400> 45
   atgactaccg ccgacattcg cctgacgccc cgcgaggtgg ccgcacatct ggagaccgac      60
   gagctccggc agttggtcgg gctcgtcgaa cacgacgacg cgtcggatcc gtttcccgtg     120
   gtcgcgatgg atgccgtggt gttcgtgtgc ggcaacgcga cgcagagcac gcagtacttc     180
   gtctccacgt ggggcatgac cctcgtcgcc tacgccgggc cggagaccgg tcagcgctcg     240
   cacaagtcct tcgtcctcga gtcggggtcg gcacggttcg tgctgcacgg cgccgtcgat     300
   ccgaagagcc cgctcgcgga ccatcaccgg gcgcacggcg acggcgtggt ggacctggcg     360
   atggaagttc tcgacgtcga ccgctgcatc gcgcatgcac gctcgcaggg ggccaccatt     420
   ctcgaggagc cgcgcgacgt cacggatcag ttcggcaccg tgcggctcgc ggcgatcgcc     480
   acgtacggca gcacccggca caccatcgtc gaccgaagcc gatacgacgg cccctacctc     540
   cccggattcg tcgcgcgctc cagcggtttc gcggcgcgac cgggtaaacc cccgcgattg     600
   ttccaggcgc tcgaccacgc cgtcggcaac gtcgagatgg gccggatgga tcactgggtc     660
   cggttctaca accgcgtcat gggcttcacg aacatggccg aattcgtcgg cgacgacatc     720
   gccacggagt actcggcgct gatgtcgaag gtcgtggcga acggcaatca ccgggtgaag     780
   ttcccgctca acgaacccgc ggtgggaaag aagaagtcgc agatcgacga atatctcgag     840
   ttctacggtg agccgggctg ccagcatctg gccctcgcga ccggagacat cctcgcgacg     900
   gtggacgcgt tgcgggccga gggtgtcgaa ttcctgaaca cacccgacgc gtactacgag     960
   gacccacagc tgcgcgcccg gatcggcagg gtgcgggtgc cggtggagga actgcagaag    1020
   cgcggaatcc tcgtcgaccg cgacgaggac ggatacctcc tgcagatctt caccaaaccg    1080
   ctcggcgacc ggccgaccgt gttcttcgag gtgatcgaac ggcacggttc gctcgggttc    1140
   ggggcgggta acttccaggc cctgttcgaa tccatcgagc gtgagcaggc ggcgcgcggc    1200
   aatctgtga                                                           1209
<210> 46
   <211> 402
   <212> PRT
   <213> Rhodococcus sp
<400> 46
   Met Thr Thr Ala Asp Ile Arg Leu Thr Pro Arg Glu Val Ala Ala His
   1               5                   10                  15
   Leu Glu Thr Asp Glu Leu Arg Gln Leu Val Gly Leu Val Glu His Asp
               20                  25                  30
   Asp Ala Ser Asp Pro Phe Pro Val Val Ala Met Asp Ala Val Val Phe
           35                  40                  45
   Val Cys Gly Asn Ala Thr Gln Ser Thr Gln Tyr Phe Val Ser Thr Trp
       50                  55                  60
   Gly Met Thr Leu Val Ala Tyr Ala Gly Pro Glu Thr Gly Gln Arg Ser
   65                  70                  75                  80
   His Lys Ser Phe Val Leu Glu Ser Gly Ser Ala Arg Phe Val Leu His
                   85                  90                  95
   Gly Ala Val Asp Pro Lys Ser Pro Leu Ala Asp His His Arg Ala His
               100                 105                 110
   Gly Asp Gly Val Val Asp Leu Ala Met Glu Val Leu Asp Val Asp Arg
           115                 120                 125
   Cys Ile Ala His Ala Arg Ser Gln Gly Ala Thr Ile Leu Glu Glu Pro
       130                 135                 140
   Arg Asp Val Thr Asp Gln Phe Gly Thr Val Arg Leu Ala Ala Ile Ala
   145                 150                 155                 160
   Thr Tyr Gly Ser Thr Arg His Thr Ile Val Asp Arg Ser Arg Tyr Asp
                   165                 170                 175
   Gly Pro Tyr Leu Pro Gly Phe Val Ala Arg Ser Ser Gly Phe Ala Ala
               180                 185                 190
   Arg Pro Gly Lys Pro Pro Arg Leu Phe Gln Ala Leu Asp His Ala Val
           195                 200                 205
   Gly Asn Val Glu Met Gly Arg Met Asp His Trp Val Arg Phe Tyr Asn
       210                 215                 220
   Arg Val Met Gly Phe Thr Asn Met Ala Glu Phe Val Gly Asp Asp Ile
   225                 230                 235                 240
   Ala Thr Glu Tyr Ser Ala Leu Met Ser Lys Val Val Ala Asn Gly Asn
                   245                 250                 255
   His Arg Val Lys Phe Pro Leu Asn Glu Pro Ala Val Gly Lys Lys Lys
               260                 265                 270
   Ser Gln Ile Asp Glu Tyr Leu Glu Phe Tyr Gly Glu Pro Gly Cys Gln
           275                 280                 285
   His Leu Ala Leu Ala Thr Gly Asp Ile Leu Ala Thr Val Asp Ala Leu
       290                 295                 300
   Arg Ala Glu Gly Val Glu Phe Leu Asn Thr Pro Asp Ala Tyr Tyr Glu
   305                 310                 315                 320
   Asp Pro Gln Leu Arg Ala Arg Ile Gly Arg Val Arg Val Pro Val Glu
                   325                 330                 335
   Glu Leu Gln Lys Arg Gly Ile Leu Val Asp Arg Asp Glu Asp Gly Tyr
               340                 345                 350
   Leu Leu Gln Ile Phe Thr Lys Pro Leu Gly Asp Arg Pro Thr Val Phe
           355                 360                 365
   Phe Glu Val Ile Glu Arg His Gly Ser Leu Gly Phe Gly Ala Gly Asn
       370                 375                 380
   Phe Gln Ala Leu Phe Glu Ser Ile Glu Arg Glu Gln Ala Ala Arg Gly
   385                 390                 395                 400
   Asn Leu
<210> 47
   <211> 1395
   <212> DNA
   <213> Scenedesmus obliquus
<400> 47
   atgggcgcag gtggagctca gggcccgaag gttgagctgg ttggctacgc caatttcgtt      60
   cgcaacaacc ctcgcagcga taagttccct gtgcacaagt ttcaccacat cgagttttgg     120
   tgcgcggacg ccaccaacac cttcaagcgc ttccagcatg gcctgggcat gaccctagtg     180
   gccaagtccg accacagcac aggcaacagc aagtactgca gctacgtctt gcaaagcaac     240
   gacctcgtgt tcaccttcac agcaccctac tcgcgcaagt gcgcagcagc agcgccttct     300
   agcagcgagc cgctgccaga ttatgaccag cagcaggcct ttgaattcat ctgcacccac     360
   ggcctggcgg caagggctgt aggcctgcag gtgggcgatg cagcgcaggc gtacgaggtt     420
   tctgtggcaa acggcgcgaa gggtgtgcgt ccacccacca agctggagga tggcggcggc     480
   tgtgctgtgg tcagcgaggt gctgctgtat ggtgacgtgg tgctgcgcta catcagcggc     540
   aagtgggagg gcccctacct gccgggctac acagccacgc ctgatgagcc gcagatctgc     600
   tacggcctgc accgcctgga ccacgccgtg ggcaatgtgc ccaagctcat cgagcacttg     660
   gagcacgtca ttggcttcac aggcttccac gagtttgccg agtttgtggc ggaggacgtg     720
   ggcactgtgg acagcgggct gaacagcatg gtgctggcca gcaacaacga gatggtgctg     780
   ctgcccatga acgagcccac ctttggcacc aagcgcaagt cgcagatcca gacgtacctg     840
   gagcagaacg agggccccgg gctgcagcac ctggcgctga agacgcacga catcctgtcc     900
   accatgcgcg agatgcacgc acgctcgcgc tgcggcggct tcgagttcca ggcggcaccc     960
   gggcacgact actacaagcg cgtggcggag aaagtgggtg acgtgctgtc cccggaggag    1020
   tgggcagccg ttgagcagct gggcatcctg gtggaccagg acgaccaggg cgtgctgctg    1080
   cagatcttca ccaagccgct gggggacagg cccaccatct tcattgagat cattgagcgg    1140
   cgcggctgcc tcaaggagag cgcggcacag gcaggcagtg cggcagcagc agcagcagag    1200
   cctacagcag ctggcggtga tgcagatgca gatggagcag cagcagcagt ggctgacaag    1260
   ttcaaagaca tcgtgcaggt gcgtgaggat ggcgtggttg tggagcaagc tgctggctgt    1320
   ggaggcttcg gcaaaggcaa cttcagcgag ctgttcaaaa gcattgagga gtacgagcgc    1380
   acgctgcagg tgtaa                                                     1395
<210> 48
   <211> 464
   <212> PRT
   <213> Scenedesmus obliquus
<400> 48
   Met Gly Ala Gly Gly Ala Gln Gly Pro Lys Val Glu Leu Val Gly Tyr
   1               5                   10                  15
   Ala Asn Phe Val Arg Asn Asn Pro Arg Ser Asp Lys Phe Pro Val His
               20                  25                  30
   Lys Phe His His Ile Glu Phe Trp Cys Ala Asp Ala Thr Asn Thr Phe
           35                  40                  45
   Lys Arg Phe Gln His Gly Leu Gly Met Thr Leu Val Ala Lys Ser Asp
       50                  55                  60
   His Ser Thr Gly Asn Ser Lys Tyr Cys Ser Tyr Val Leu Gln Ser Asn
   65                  70                  75                  80
   Asp Leu Val Phe Thr Phe Thr Ala Pro Tyr Ser Arg Lys Cys Ala Ala
                   85                  90                  95
   Ala Ala Pro Ser Ser Ser Glu Pro Leu Pro Asp Tyr Asp Gln Gln Gln
               100                 105                 110
   Ala Phe Glu Phe Ile Cys Thr His Gly Leu Ala Ala Arg Ala Val Gly
           115                 120                 125
   Leu Gln Val Gly Asp Ala Ala Gln Ala Tyr Glu Val Ser Val Ala Asn
       130                 135                 140
   Gly Ala Lys Gly Val Arg Pro Pro Thr Lys Leu Glu Asp Gly Gly Gly
   145                 150                 155                 160
   Cys Ala Val Val Ser Glu Val Leu Leu Tyr Gly Asp Val Val Leu Arg
                   165                 170                 175
   Tyr Ile Ser Gly Lys Trp Glu Gly Pro Tyr Leu Pro Gly Tyr Thr Ala
               180                 185                 190
   Thr Pro Asp Glu Pro Gln Ile Cys Tyr Gly Leu His Arg Leu Asp His
           195                 200                 205
   Ala Val Gly Asn Val Pro Lys Leu Ile Glu His Leu Glu His Val Ile
       210                 215                 220
   Gly Phe Thr Gly Phe His Glu Phe Ala Glu Phe Val Ala Glu Asp Val
   225                 230                 235                 240
   Gly Thr Val Asp Ser Gly Leu Asn Ser Met Val Leu Ala Ser Asn Asn
                   245                 250                 255
   Glu Met Val Leu Leu Pro Met Asn Glu Pro Thr Phe Gly Thr Lys Arg
               260                 265                 270
   Lys Ser Gln Ile Gln Thr Tyr Leu Glu Gln Asn Glu Gly Pro Gly Leu
           275                 280                 285
   Gln His Leu Ala Leu Lys Thr His Asp Ile Leu Ser Thr Met Arg Glu
       290                 295                 300
   Met His Ala Arg Ser Arg Cys Gly Gly Phe Glu Phe Gln Ala Ala Pro
   305                 310                 315                 320
   Gly His Asp Tyr Tyr Lys Arg Val Ala Glu Lys Val Gly Asp Val Leu
                   325                 330                 335
   Ser Pro Glu Glu Trp Ala Ala Val Glu Gln Leu Gly Ile Leu Val Asp
               340                 345                 350
   Gln Asp Asp Gln Gly Val Leu Leu Gln Ile Phe Thr Lys Pro Leu Gly
           355                 360                 365
   Asp Arg Pro Thr Ile Phe Ile Glu Ile Ile Glu Arg Arg Gly Cys Leu
       370                 375                 380
   Lys Glu Ser Ala Ala Gln Ala Gly Ser Ala Ala Ala Ala Ala Ala Glu
   385                 390                 395                 400
   Pro Thr Ala Ala Gly Gly Asp Ala Asp Ala Asp Gly Ala Ala Ala Ala
                   405                 410                 415
   Val Ala Asp Lys Phe Lys Asp Ile Val Gln Val Arg Glu Asp Gly Val
               420                 425                 430
   Val Val Glu Gln Ala Ala Gly Cys Gly Gly Phe Gly Lys Gly Asn Phe
           435                 440                 445
   Ser Glu Leu Phe Lys Ser Ile Glu Glu Tyr Glu Arg Thr Leu Gln Val
       450                 455                 460
<210> 49
   <211> 1395
   <212> DNA
   <213> Scenedesmus obliquus
<400> 49
   atgggcgcag gtggagctca gggcccgaag gttgagctgg ttggctacgc caatttcgtt      60
   cgcaacaacc ctcgcagcga taagttccct gtgcacaagt ttcaccacat cgagttttgg     120
   tgcgcggacg ccaccaacac cttcaagcgc ttccagcatg gcctgggcat gaccctagtg     180
   gccaagtccg accacagcac aggcaacagc aagtactgca gctacgtctt gcaaagcaac     240
   gacctcgtgt tcaccttcac agcaccctac tcgcgcaagt gcgcagcagc agcgccttct     300
   agcagcgagc cgctgccaga ttatgaccag cagcaggcct ttgaattcat ctgcacccac     360
   ggcctggcgg caagggctgt aggcctgcag gtgggcgatg cagcgcaggc gtacgaggtt     420
   tctgtggcaa acggcgcgaa gggtgtgcgt ccacccacca agctggagga tggcggcggc     480
   tgtgctgtgg tcagcgaggt gctgctgtat ggtgacgtgg tgctgcgcta catcagcggc     540
   aagtgggagg gcccctacct gccgggctac acagccacgc ctgatgagcc gcagatctgc     600
   tacggcctgc accgcctgga ccacgccgtg ggcaatgtgc ccaagctcat cgagcacttg     660
   gagcacgtca ttggcttcac aggcttccac gagtttgccg agtttgtggc ggaggacgtg     720
   ggcactgtgg acagcgggct gaacagcatg gtgctggcca gcaacaacga gatggtgctg     780
   ctgcccatga acgagcccac ctttggcacc aagcgcaagt cgcagatcca gacgtacctg     840
   gagcagaacg agggccccgg gctgcagcac ctggcgctga agacgcacga catcctgtcc     900
   accatgcgcg agatgcacgc acgctcgcgc tgcggcggct tcgagttcca ggcggcaccc     960
   gggcacgact actacaagcg cgtggcggag aaagtgggtg acgtgctgtc cccggaggag    1020
   tgggcagccg ttgagcagct gggcatcctg gtggaccagg acgaccaggg cgtgctgctg    1080
   cagatcttca ccaagccgct gggggacagg cccaccatct tcattgagat cattgagcgg    1140
   cgcggctgcc tcaaggagag cgcggcacag gcaggcagtg cggcagcagc agcagcagag    1200
   cctacagcag ctggcggtga tgcagatgca gatggagcag cagcagcagt ggctgacaag    1260
   ttcaaagaca tcgtgcaggt gcgtgaggat ggcgtggttg tggagcaagc tgctggctgt    1320
   ggaggcttcg gcaaaggcaa cttcagcgag ctgttcaaaa gcattgagga gtacgagcgc    1380
   acgctgcagg tgtaa                                                     1395
<210> 50
   <211> 430
   <212> PRT
   <213> Scenedesmus obliquus
<400> 50
   Met Gly Ala Gly Gly Ala Gln Gly Pro Lys Val Glu Leu Val Gly Tyr
   1               5                   10                  15
   Ala Asn Phe Val Arg Asn Asn Pro Arg Ser Asp Lys Phe Pro Val His
               20                  25                  30
   Lys Phe His His Ile Glu Phe Trp Cys Ala Asp Ala Thr Asn Thr Phe
           35                  40                  45
   Lys Arg Phe Gln His Gly Leu Gly Met Thr Leu Val Ala Lys Ser Asp
       50                  55                  60
   His Ser Thr Gly Asn Ser Lys Tyr Cys Ser Tyr Val Leu Gln Ser Asn
   65                  70                  75                  80
   Asp Leu Val Phe Thr Phe Thr Ala Pro Tyr Ser Arg Lys Cys Ala Ala
                   85                  90                  95
   Ala Ala Pro Ser Ser Ser Glu Pro Leu Pro Asp Tyr Asp Gln Gln Gln
               100                 105                 110
   Ala Phe Glu Phe Ile Cys Thr His Gly Leu Ala Ala Arg Ala Val Gly
           115                 120                 125
   Leu Gln Val Gly Asp Ala Ala Gln Ala Tyr Glu Val Ser Val Ala Asn
       130                 135                 140
   Gly Ala Lys Gly Val Arg Pro Pro Thr Lys Leu Glu Asp Gly Gly Gly
   145                 150                 155                 160
   Cys Ala Val Val Ser Glu Val Leu Leu Tyr Gly Asp Val Val Leu Arg
                   165                 170                 175
   Tyr Ile Ser Gly Lys Trp Glu Gly Pro Tyr Leu Pro Gly Tyr Thr Ala
               180                 185                 190
   Thr Pro Asp Glu Pro Gln Ile Cys Tyr Gly Leu His Arg Leu Asp His
           195                 200                 205
   Ala Val Gly Asn Val Pro Lys Leu Ile Glu His Leu Glu His Val Ile
       210                 215                 220
   Gly Phe Thr Gly Phe His Glu Phe Ala Glu Phe Val Ala Glu Asp Val
   225                 230                 235                 240
   Gly Thr Val Asp Ser Gly Leu Asn Ser Met Val Leu Ala Ser Asn Asn
                   245                 250                 255
   Glu Met Val Leu Leu Pro Met Asn Glu Pro Thr Phe Gly Thr Lys Arg
               260                 265                 270
   Lys Ser Gln Ile Gln Thr Tyr Leu Glu Gln Asn Glu Gly Pro Gly Leu
           275                 280                 285
   Gln His Leu Ala Leu Lys Thr His Asp Ile Leu Ser Thr Met Arg Glu
       290                 295                 300
   Met His Ala Arg Ser Arg Cys Gly Gly Phe Glu Phe Gln Ala Ala Pro
   305                 310                 315                 320
   Gly His Asp Tyr Tyr Lys Arg Val Ala Glu Lys Val Gly Asp Val Leu
                   325                 330                 335
   Ser Pro Glu Glu Trp Ala Ala Val Glu Gln Leu Gly Ile Leu Val Asp
               340                 345                 350
   Gln Asp Asp Gln Gly Val Leu Leu Gln Ile Phe Thr Lys Pro Leu Gly
           355                 360                 365
   Asp Arg Pro Thr Ile Phe Ile Glu Ile Ile Glu Arg Arg Gly Cys Leu
       370                 375                 380
   Lys Glu Ser Ala Ala Gln Ala Gly Ser Ala Ala Ala Ala Val Glu Gln
   385                 390                 395                 400
   Ala Ala Gly Cys Gly Gly Phe Gly Lys Gly Asn Phe Ser Glu Leu Phe
                   405                 410                 415
   Lys Ser Ile Glu Glu Tyr Glu Arg Thr Leu Gln Val Leu Glu
               420                 425                 430
<210> 51
   <211> 1305
   <212> DNA
   <213> Hordeum vulgare
<400> 51
   atggcacctc caactccaac aacacctgcc gctactggtg cagccgcagc tgtaactcct      60
   gaacatgcga ggccacatcg gatggttcga ttcaatccga gatctgatag attccatact     120
   ctgagcttcc atcatgtgga attctggtgt gctgatgcag cttctgcagc tggacgtttc     180
   gcttttgccc ttggagctcc tttagcagcg agatcagact tgagcacagg aaacagtgca     240
   cacgcatctc aacttctgcg ttcaggaagc cttgcgttcc tgtttactgc accgtatgct     300
   aacggatgcg acgcagcaac tgcctcactt ccttctttca gtgcagatgc agctagacga     360
   ttctcagccg atcatggaat tgcagtcaga tctgtggctt tgcgagttgc tgatgctgcc     420
   gaagctttca gggcatcagt tgatggaggt gctaggcctg cttttgctcc tgtggacttg     480
   ggtagaggat ttggctttgc cgaggtcgaa ctctatggtg atgtggttct ccggtttgtc     540
   tctcacccag atggaacaga tgttcctttc ttgccagggt ttgagggagt gacaaaccca     600
   gatgcggtag attacggtct cacgagattc gaccatgtag tgggcaatgt accggaattg     660
   gctcctgcgg ctgcttacat agctggcttt acgggatttc acgaattcgc ggaattcacc     720
   gctgaggatg tcggaaccac agaatcaggg ctgaattccg tcgtccttgc caacaattcc     780
   gaaggggtat tgctgcctct taacgagcct gtgcatggca cgaaaagacg tagccagata     840
   cagaccttcc tagaacatca cggtggacca ggtgttcaac acattgctgt tgccagcagt     900
   gatgtactca ggacgcttcg taagatgaga gctaggagtg cgatgggagg gtttgacttt     960
   ctaccacctc cgctgccaaa atactatgag ggtgtgagga gactggctgg tgatgttttg    1020
   tctgaagcgc agatcaagga gtgtcaggaa ttaggggtgc tcgttgacag agatgatcaa    1080
   ggggtgcttc tccagatctt tactaagccg gttggtgata ggcctaccct ctttctagag    1140
   atgattcaac gtatcgggtg tatggaaaag gacgagagag gtgaggagta tcaaaagggt    1200
   ggatgcggcg gttttgggaa aggtaatttc tccgagctgt tcaagtcgat cgaagattac    1260
   gagaaatccc ttgaggcgaa acaatctgca gctgttcaag gatcg                    1305
<210> 52
   <211> 1338
   <212> DNA
   <213> Arabidopsis
<400> 52
   atgggccacc aaaacgccgc cgtttcagag aatcaaaacc atgatgacgg cgctgcgtcg      60
   tcgccgggat tcaagctcgt cggattttcc aagttcgtaa gaaagaatcc aaagtctgat     120
   aaattcaagg ttaagcgctt ccatcacatc gagttctggt gcggcgacgc aaccaacgtc     180
   gctcgtcgct tctcctgggg tctggggatg agattctccg ccaaatccga tctttccacc 240
   ggaaacatgg ttcacgcctc ttacctactc acctccggtg acctccgatt ccttttcact 300
   gctccttact ctccgtctct ctccgccgga gagattaaac cgacaaccac agcttctatc 360
   ccaagtttcg atcacggctc ttgtcgttcc ttcttctctt cacatggtct cggtgttaga 420
   gccgttgcga ttgaagtaga agacgcagag tcagctttct ccatcagtgt agctaatggc 480
   gctattcctt cgtcgcctcc tatcgtcctc aatgaagcag ttacgatcgc tgaggttaaa 540
   ctatacggcg atgttgttct ccgatatgtt agttacaaag cagaagatac cgaaaaatcc 600
   gaattcttgc cagggttcga gcgtgtagag gatgcgtcgt cgttcccatt ggattatggt 660
   atccggcggc ttgaccacgc cgtgggaaac gttcctgagc ttggtccggc tttaacttat 720
   gtagcggggt tcactggttt tcaccaattc gcagagttca cagcagacga cgttggaacc 780
   gccgagagcg gtttaaattc agcggtcctg gctagcaatg atgaaatggt tcttctaccg 840
   attaacgagc cagtgcacgg aacaaagagg aagagtcaga ttcagacgta tttggaacat 900
   aacgaaggcg cagggctaca acatctggct ctgatgagtg aagacatatt caggaccctg 960
   agagagatga ggaagaggag cagtattgga ggattcgact tcatgccttc tcctccgcct 1020
   acttactacc agaatctcaa gaaacgggtc ggcgacgtgc tcagcgatga tcagatcaag 1080
   gagtgtgagg aattagggat tcttgtagac agagatgatc aagggacgtt gcttcaaatc 1140
   ttcacaaaac cactaggtga caggccgacg atatttatag agataatcca gagagtagga 1200
   tgcatgatga aagatgagga agggaaggct taccagagtg gaggatgtgg tggttttggc 1260
   aaaggcaatt tctctgagct cttcaagtcc attgaagaat acgaaaagac tcttgaagcc 1320
   aaacagttag tgggatga 1338
<210> 53
   <211> 445
   <212> PRT
   <213> Arabidopsis
<400> 53
   Met Gly His Gln Asn Ala Ala Val Ser Glu Asn Gln Asn His Asp Asp
   1               5                   10                  15
   Gly Ala Ala Ser Ser Pro Gly Phe Lys Leu Val Gly Phe Ser Lys Phe
                   20                  25                  30
   Val Arg Lys Asn Pro Lys Ser Asp Lys Phe Lys Val Lys Arg Phe His
           35                  40                  45
   His Ile Glu Phe Trp Cys Gly Asp Ala Thr Asn Val Ala Arg Arg Phe
       50                  55                  60
   Ser Trp Gly Leu Gly Met Arg Phe Ser Ala Lys Ser Asp Leu Ser Thr
   65                  70                  75                  80
   Gly Asn Met Val His Ala Ser Tyr Leu Leu Thr Ser Gly Asp Leu Arg
                   85                  90                  95
   Phe Leu Phe Thr Ala Pro Tyr Ser Pro Ser Leu Ser Ala Gly Glu Ile
               100                 105                 110
   Lys Pro Thr Thr Thr Ala Ser Ile Pro Ser Phe Asp His Gly Ser Cys
           115                 120                 125
   Arg Ser Phe Phe Ser Ser His Gly Leu Gly Val Arg Ala Val Ala Ile
       130                 135                 140
   Glu Val Glu Asp Ala Glu Ser Ala Phe Ser Ile Ser Val Ala Asn Gly
   145                 150                 155                 160
   Ala Ile Pro Ser Ser Pro Pro Ile Val Leu Asn Glu Ala Val Thr Ile
                   165                 170                 175
   Ala Glu Val Lys Leu Tyr Gly Asp Val Val Leu Arg Tyr Val Ser Tyr
               180                 185                 190
   Lys Ala Glu Asp Thr Glu Lys Ser Glu Phe Leu Pro Gly Phe Glu Arg
           195                 200                 205
   Val Glu Asp Ala Ser Ser Phe Pro Leu Asp Tyr Gly Ile Arg Arg Leu
       210                 215                 220
   Asp His Ala Val Gly Asn Val Pro Glu Leu Gly Pro Ala Leu Thr Tyr
   225                 230                 235                 240
   Val Ala Gly Phe Thr Gly Phe His Gln Phe Ala Glu Phe Thr Ala Asp
                   245                 250                 255
   Asp Val Gly Thr Ala Glu Ser Gly Leu Asn Ser Ala Val Leu Ala Ser
               260                 265                 270
   Asn Asp Glu Met Val Leu Leu Pro Ile Asn Glu Pro Val His Gly Thr
           275                 280                 285
   Lys Arg Lys Ser Gln Ile Gln Thr Tyr Leu Glu His Asn Glu Gly Ala
       290                 295                 300
   Gly Leu Gln His Leu Ala Leu Met Ser Glu Asp Ile Phe Arg Thr Leu
   305                 310                 315                 320
   Arg Glu Met Arg Lys Arg Ser Ser Ile Gly Gly Phe Asp Phe Met Pro
                   325                 330                 335
   Ser Pro Pro Pro Thr Tyr Tyr Gln Asn Leu Lys Lys Arg Val Gly Asp
               340                 345                 350
   Val Leu Ser Asp Asp Gln Ile Lys Glu Cys Glu Glu Leu Gly Ile Leu
           355                 360                 365
   Val Asp Arg Asp Asp Gln Gly Thr Leu Leu Gln Ile Phe Thr Lys Pro
       370                 375                 380
   Leu Gly Asp Arg Pro Thr Ile Phe Ile Glu Ile Ile Gln Arg Val Gly
   385                 390                 395                 400
   Cys Met Met Lys Asp Glu Glu Gly Lys Ala Tyr Gln Ser Gly Gly Cys
                   405                 410                 415
   Gly Gly Phe Gly Lys Gly Asn Phe Ser Glu Leu Phe Lys Ser Ile Glu
               420                 425                 430
   Glu Tyr Glu Lys Thr Leu Glu Ala Lys Gln Leu Val Gly
           435                 440                 445
<210> 54
   <211> 1299
   <212> DNA
   <213> Chlamydomonas
<400> 54
   atgggcgctg gtggcgcttc taccacggta gcgaatggcg ggatcaagtt ggtagggcac      60
   aagaattttg tgcgctataa tccacaatcc gaccggtttg ctattaagag gttccatagc     120
   ttcgagttct ggtgcgcgga tgcgaccaac acatacaagc ggttctctta tggcctgggc     180
   atgccgctgg tcgccaagtc cgaccagtcc accaacaacc agctctttgc ctcctacgtg     240
   ctgcgctcca acgacctggt cttcaccttc accgcgccct acagccgcaa gtgcgcctcg     300
   gtcagcgagg gcgttccgct gcgtcactac aacatcgacc atgcgtatga gttcatcaac     360
   tcgcacgggc tggcggtgcg ggcagtaggc ctgctggtgg atgacgccaa gacggcgtac     420
   gaggtgtctg tggcgcacgg ggccaagggc gtgctgccgc cggtggagtt gcgggatgag     480
   gcgagcggca ccagccaggt catctcggag gtcattgttt acggggacgt cgttttccgc     540
   tacgtgtcgg gctccttcga gggccctttc atggccggct acacgccagt cacagactcg     600
   ccggtcgcgt cgattgggtt acagcgcgtg gaccacgcgg tgggcaacac acacgacctg     660
   atcaaggccg tggagtacat caccgggttc tgtggcttcc acgagttctc agagtttgtt     720
   gcggaggacg tgggcactgt ggacagcggc ctgaacagca tggtgcttgc caacaacgag     780
   gagaccatat tgatgcctgt gaacgagccc accttcggca cgccgcgcaa gagccaaatc     840
   cagacctacc tggagcagaa cgaggggccg gggctgcagc acctggcgct gctcagcaac     900
   gacatcttca ccaccctgcg ggagatgcgc gcgcgcagcg agctgggtgg cttcgagttc     960
   atgccgcggg caaatgcgaa gtactacaaa gacatgtacg cccgcatcgg cgactcgctc    1020
   acgccgcagc agtacaggga ggtggaggag ctgggcatcc tggtggacaa ggacgaccag    1080
   ggcgtgctgc tgcagatctt caccaagccg ctgggcgacc ggcccacggt gtttattgag    1140
   atcatccagc gtgtgggctg catgcgggag gtgaaggagc ctgctacggg cgctgtggtg    1200
   gggacggagc aggcggctgg ctgcggcggc ttcgggaaag gcaacttcgg cgccctcttc    1260
   aagtccattg aggactatga gcgcacccta aatgtgtag                            1299
<210> 55
   <211> 432
   <212> PRT
   <213> Chamydomonas
<400> 55
   Met Gly Ala Gly Gly Ala Ser Thr Thr Val Ala Asn Gly Gly Ile Lys
   1               5                   10                  15
   Leu Val Gly His Lys Asn Phe Val Arg Tyr Asn Pro Gln Ser Asp Arg
               20                  25                  30
   Phe Ala Ile Lys Arg Phe His Ser Phe Glu Phe Trp Cys Ala Asp Ala
           35                  40                  45
   Thr Asn Thr Tyr Lys Arg Phe Ser Tyr Gly Leu Gly Met Pro Leu Val
       50                  55                  60
   Ala Lys Ser Asp Gln Ser Thr Asn Asn Gln Leu Phe Ala Ser Tyr Val
   65                  70                  75                  80
   Leu Arg Ser Asn Asp Leu Val Phe Thr Phe Thr Ala Pro Tyr Ser Arg
                   85                  90                  95
   Lys Cys Ala Ser Val Ser Glu Gly Val Pro Leu Arg His Tyr Asn Ile
               100                 105                 110
   Asp His Ala Tyr Glu Phe Ile Asn Ser His Gly Leu Ala Val Arg Ala
           115                 120                 125
   Val Gly Leu Leu Val Asp Asp Ala Lys Thr Ala Tyr Glu Val Ser Val
       130                 135                 140
   Ala His Gly Ala Lys Gly Val Leu Pro Pro Val Glu Leu Arg Asp Glu
   145                 150                 155                 160
   Ala Ser Gly Thr Ser Gln Val Ile Ser Glu Val Ile Val Tyr Gly Asp
                   165                 170                 175
   Val Val Phe Arg Tyr Val Ser Gly Ser Phe Glu Gly Pro Phe Met Ala
               180                 185                 190
   Gly Tyr Thr Pro Val Thr Asp Ser Pro Val Ala Ser Ile Gly Leu Gln
           195                 200                 205
   Arg Val Asp His Ala Val Gly Asn Thr His Asp Leu Ile Lys Ala Val
       210                 215                 220
   Glu Tyr Ile Thr Gly Phe Cys Gly Phe His Glu Phe Ser Glu Phe Val
   225                 230                 235                 240
   Ala Glu Asp Val Gly Thr Val Asp Ser Gly Leu Asn Ser Met Val Leu
                   245                 250                 255
   Ala Asn Asn Glu Glu Thr Ile Leu Met Pro Val Asn Glu Pro Thr Phe
               260                 265                 270
   Gly Thr Pro Arg Lys Ser Gln Ile Gln Thr Tyr Leu Glu Gln Asn Glu
           275                 280                 285
   Gly Pro Gly Leu Gln His Leu Ala Leu Leu Ser Asn Asp Ile Phe Thr
       290                 295                 300
   Thr Leu Arg Glu Met Arg Ala Arg Ser Glu Leu Gly Gly Phe Glu Phe
   305                 310                 315                 320
   Met Pro Arg Ala Asn Ala Lys Tyr Tyr Lys Asp Met Tyr Ala Arg Ile
                   325                 330                 335
   Gly Asp Ser Leu Thr Pro Gln Gln Tyr Arg Glu Val Glu Glu Leu Gly
               340                 345                 350
   Ile Leu Val Asp Lys Asp Asp Gln Gly Val Leu Leu Gln Ile Phe Thr
           355                 360                 365
   Lys Pro Leu Gly Asp Arg Pro Thr Val Phe Ile Glu Ile Ile Gln Arg
       370                 375                 380
   Val Gly Cys Met Arg Glu Val Lys Glu Pro Ala Thr Gly Ala Val Val
   385                 390                 395                 400
   Gly Thr Glu Gln Ala Ala Gly Cys Gly Gly Phe Gly Lys Gly Asn Phe
                   405                 410                 415
   Gly Ala Leu Phe Lys Ser Ile Glu Asp Tyr Glu Arg Thr Leu Asn Val
               420                 425                 430
<210> 56
   <211> 1299
   <212> DNA
   <213> Chlamydomonas
<400> 56
   atgggagcgg gtggtgcagg caccggagat cgggaggggg gcattaagct cgtgggctac      60
   aagaatttcg tgcgccagaa cccgctttca gacaaattca ccgtccacaa gtttcatcac     120
   atcgatttct ggtgcggaga tgcaacaaac acatcgaagc ggttctccta cggcctgggc     180
   atgccgctgg tcgccaagtc cgaccagtcc accaacaacc agctctttgc ctcctacgtg     240
   ctgcgctcca acgacctggt cttcaccttc accgcgccct acagccgcaa gtgcgcctcg     300
   gtcagcgagg gcgttccgct gcgtcactac aacatcgacc atgcgtatga gttcatcaac     360
   tcgcacgggc tggcggtgcg ggcagtaggc ctgctggtgg atgacgccaa gacggcgtac     420
   gaggtgtctg tggcgcacgg ggccaagggc gtgctgccgc cggtggagct gcgggatgag     480
   gcgagcggca ccagccaggt catctcggag gtgctgctgt acggcgaggt cgtgctgcgc     540
   tacgtgtcgg gctccttcca gggccccttc ctggccggct acacgcccgt cacagactcg     600
   gccgtgacct ccttcggcct gcaacgtctg gaccacgcgg tgggcaacac ccatgacctg     660
   atcaaggccg tggagtacat caccggcttc acaggtttcc acgagttctc agagtttgtt     720
   gcggaggacg tgggcactgt ggacagcggc ctgaacagca tggtgctggc ctccaacaac     780
   gaggcagtgc tgctgcctgt gaacgagccc acctttggca cgccgcgcaa gagccaaatc     840
   cagacctacc tggagcagaa cgaggggccg gggctgcagc acctggcgct gctcagcaac      900
   gacatcttca ccaccctgcg ggagatgcgc gcgcgcagcg agctgggtgg cttcgagttc     960
   atgccacggg caaatgccaa gtactacaaa gacatgtacg cccgcatcgg cgactcgctc    1020
   acgccgcagc agtacaggga ggtggaggag ctgggcatcc tggtggacaa ggacgaccag    1080
   ggcgtgctgc tgcagatctt caccaagccg ctgggcgacc ggcccacggt gtttattgag    1140
   atcatccagc gtgtgggctg catgcgggag gtgaaagagc ctgctacggg cgctgtggtg    1200
   gggacggagc aggcggctgg ctgcggcggc ttcgggaaag gcaacttcgg tgccctcttc    1260
   aagtccattg aggactatga gcgcacctta aatgtttaa                           1299
<210> 57
   <211> 432
   <212> PRT
   <213> Chlamydomonas
<400> 57
   Met Gly Ala Gly Gly Ala Gly Thr Gly Asp Arg Glu Gly Gly Ile Lys
   1               5                   10                  15
   Leu Val Gly Tyr Lys Asn Phe Val Arg Gln Asn Pro Leu Ser Asp Lys
               20                  25                  30
   Phe Thr Val His Lys Phe His His Ile Asp Phe Trp Cys Gly Asp Ala
           35                  40                  45
   Thr Asn Thr Ser Lys Arg Phe Ser Tyr Gly Leu Gly Met Pro Leu Val
       50                  55                  60
   Ala Lys Ser Asp Gln Ser Thr Asn Asn Gln Leu Phe Ala Ser Tyr Val
   65                  70                  75                  80
   Leu Arg Ser Asn Asp Leu Val Phe Thr Phe Thr Ala Pro Tyr Ser Arg
                   85                  90                  95
   Lys Cys Ala Ser Val Ser Glu Gly Val Pro Leu Arg His Tyr Asn Ile
               100                 105                 110
   Asp His Ala Tyr Glu Phe Ile Asn Ser His Gly Leu Ala Val Arg Ala
           115                 120                 125
   Val Gly Leu Leu Val Asp Asp Ala Lys Thr Ala Tyr Glu Val Ser Val
       130                 135                 140
   Ala His Gly Ala Lys Gly Val Leu Pro Pro Val Glu Leu Arg Asp Glu
   145                 150                 155                 160
   Ala Ser Gly Thr Ser Gln Val Ile Ser Glu Val Leu Leu Tyr Gly Glu
                   165                 170                 175
   Val Val Leu Arg Tyr Val Ser Gly Ser Phe Gln Gly Pro Phe Leu Ala
               180                 185                 190
   Gly Tyr Thr Pro Val Thr Asp Ser Ala Val Thr Ser Phe Gly Leu Gln
           195                 200                 205
   Arg Leu Asp His Ala Val Gly Asn Thr His Asp Leu Ile Lys Ala Val
       210                 215                 220
   Glu Tyr Ile Thr Gly Phe Thr Gly Phe His Glu Phe Ser Glu Phe Val
   225                 230                 235                 240
   Ala Glu Asp Val Gly Thr Val Asp Ser Gly Leu Asn Ser Met Val Leu
                   245                 250                 255
   Ala Ser Asn Asn Glu Ala Val Leu Leu Pro Val Asn Glu Pro Thr Phe
               260                 265                 270
   Gly Thr Pro Arg Lys Ser Gln Ile Gln Thr Tyr Leu Glu Gln Asn Glu
           275                 280                 285
   Gly Pro Gly Leu Gln His Leu Ala Leu Leu Ser Asn Asp Ile Phe Thr
       290                 295                 300
   Thr Leu Arg Glu Met Arg Ala Arg Ser Glu Leu Gly Gly Phe Glu Phe
   305                 310                 315                 320
   Met Pro Arg Ala Asn Ala Lys Tyr Tyr Lys Asp Met Tyr Ala Arg Ile
                   325                 330                 335
   Gly Asp Ser Leu Thr Pro Gln Gln Tyr Arg Glu Val Glu Glu Leu Gly
               340                 345                 350
   Ile Leu Val Asp Lys Asp Asp Gln Gly Val Leu Leu Gln Ile Phe Thr
           355                 360                 365
   Lys Pro Leu Gly Asp Arg Pro Thr Val Phe Ile Glu Ile Ile Gln Arg
       370                 375                 380
   Val Gly Cys Met Arg Glu Val Lys Glu Pro Ala Thr Gly Ala Val Val
   385                 390                 395                 400
   Gly Thr Glu Gln Ala Ala Gly Cys Gly Gly Phe Gly Lys Gly Asn Phe
                   405                 410                 415
   Gly Ala Leu Phe Lys Ser Ile Glu Asp Tyr Glu Arg Thr Leu Asn Val
               420                 425                 430
<210> 58
   <211> 433
   <212> PRT
   <213> Physcomitrella
<400> 58
   Met Gly Leu Asp Lys Ser Glu Ser Glu Gly Ser Val Val Gly Pro Leu
   1               5                   10                  15
   His Leu Val Gly Cys Glu Arg Phe Val Arg Asn Asn Pro Lys Thr Asp
               20                  25                  30
   Arg Phe Gly Val Glu Arg Phe His His Val Glu Phe Trp Cys Gly Asp
           35                  40                  45
   Ala Ser Asn Thr Trp Arg Arg Phe Ser Trp Gly Leu Gly Met His Leu
       50                  55                  60
   Val Ala Lys Ser Asp Gln Thr Thr Gly Asn Gln Thr Tyr Cys Ser Tyr
   65                  70                  75                  80
   Ala Ile Gln Ser Asn Glu Leu Val Phe Ala Phe Thr Ala Pro Tyr Ser
                   85                  90                  95
   Ser Thr Ile Asp Gln Thr Asn Thr Lys Met Pro His Pro Gly Tyr Lys
               100                 105                 110
   Ser Asp Glu Ala Arg Ser Phe Thr Asp Ser His Gly Leu Ala Val Arg
           115                 120                 125
   Ala Val Gly Ile Leu Val Asp Asp Ala Asp Glu Ala Phe Arg Ile Ser
       130                 135                 140
   Val Glu His Gly Ala Val Ser Val Leu Glu Pro His Val Leu Ser Asp
   145                 150                 155                 160
   Asp Ala Lys Gly Gly Lys Met Val Met Ala Glu Val Lys Leu Tyr Gly
                   165                 170                 175
   Asp Val Val Leu Arg Tyr Val Ser Glu Gln Gly Tyr Lys Gly Ser Met
               180                 185                 190
   Leu Pro Asn Tyr Glu Glu Val Glu Ser Leu Pro Leu Ser Tyr Gly Leu
           195                 200                 205
   Val Arg Leu Asp His Ala Val Gly Asn Val His Asn Leu Ala Glu Ala
       210                 215                 220
   Val Asn Tyr Ile Ala Lys Phe Thr Gly Phe His Glu Phe Ala Glu Phe
   225                 230                 235                 240
   Thr Ala Gly Asp Val Gly Thr Thr Glu Ser Gly Leu Asn Ser Met Val
                   245                 250                 255
   Val Ala Ser Asn Asn Glu Met Val Leu Leu Pro Ile Asn Glu Pro Thr
               260                 265                 270
   Phe Gly Thr Lys Arg Lys Ser Gln Ile Gln Thr Tyr Leu Glu His Asn
           275                 280                 285
   Glu Gly Pro Gly Leu Gln His Leu Ala Leu Ile Cys Asp Asn Ile Phe
       290                 295                 300
   Ser Thr Leu Arg Glu Met Arg Thr Arg Thr His Ile Gly Gly Phe Asp
   305                 310                 315                 320
   Phe Met Pro Lys Pro Pro Pro Thr Tyr Tyr Lys Asn Leu Ala Asn Arg
                   325                 330                 335
   Val Gly Asp Ile Leu Thr Ala Glu Gln Ile Lys Glu Cys Asp Glu Leu
               340                 345                 350
   Gly Ile Leu Val Asp Lys Asp Asp Gln Gly Val Leu Leu Gln Ile Phe
           355                 360                 365
   Thr Lys Pro Val Gly Asp Arg Pro Ser Ile Phe Val Glu Ile Ile Gln
       370                 375                 380
   Arg Ile Gly Cys Met Asp Lys Asp Glu Ser Thr Gly Ala Thr Val Gln
   385                 390                 395                 400
   Lys Gly Gly Cys Gly Gly Phe Gly Lys Gly Asn Phe Ser Glu Leu Phe
                   405                 410                 415
   Lys Ser Ile Glu Glu Tyr Glu Lys Thr Leu Asp Gly Thr Leu Lys Val
               420                 425                 430
   His
<210> 59
   <211> 446
   <212> PRT
   <213> Oryza
<400> 59
   Met Pro Pro Thr Pro Thr Pro Thr Ala Thr Thr Gly Ala Val Ser Ala
   1               5                   10                  15
   Ala Ala Ala Ala Gly Glu Asn Ala Gly Phe Arg Leu Val Gly His Arg
               20                  25                  30
   Arg Phe Val Arg Ala Asn Pro Arg Ser Asp Arg Phe Gln Ala Leu Ala
           35                  40                  45
   Phe His His Val Glu Leu Trp Cys Ala Asp Ala Ala Ser Ala Ala Gly
       50                  55                  60
   Arg Phe Ala Phe Ala Leu Gly Ala Pro Leu Ala Ala Arg Ser Asp Leu
   65                  70                  75                  80
   Ser Thr Gly Asn Ser Ala His Ala Ser Leu Leu Leu Arg Ser Ala Ser
                   85                  90                  95
   Val Ala Phe Leu Phe Thr Ala Pro Tyr Gly Gly Asp His Gly Val Gly
               100                 105                 110
   Ala Asp Ala Ala Thr Thr Ala Ser Ile Pro Ser Phe Ser Pro Gly Ala
           115                 120                 125
   Ala Arg Arg Phe Ala Ala Asp His Gly Leu Ala Val His Ala Val Ala
       130                 135                 140
   Leu Arg Val Ala Asp Ala Ala Asp Ala Phe Arg Ala Ser Val Ala Ala
   145                 150                 155                 160
   Gly Ala Arg Pro Ala Phe Gln Pro Ala Asp Leu Gly Gly Gly Phe Gly
                   165                 170                 175
   Leu Ala Glu Val Glu Leu Tyr Gly Asp Val Val Leu Arg Phe Val Ser
               180                 185                 190
   His Pro Asp Gly Ala Asp Ala Pro Phe Leu Pro Gly Phe Glu Gly Val
           195                 200                 205
   Ser Asn Pro Gly Ala Val Asp Tyr Gly Leu Arg Arg Phe Asp His Val
       210                 215                 220
   Val Gly Asn Val Pro Glu Leu Ala Pro Val Ala Ala Tyr Ile Ser Gly
   225                 230                 235                 240
   Phe Thr Gly Phe His Glu Phe Ala Glu Phe Thr Ala Glu Asp Val Gly
                   245                 250                 255
   Thr Ala Glu Ser Gly Leu Asn Ser Val Val Leu Ala Asn Asn Ala Glu
               260                 265                 270
   Thr Val Leu Leu Pro Leu Asn Glu Pro Val His Gly Thr Lys Arg Arg
           275                 280                 285
   Ser Gln Ile Gln Thr Tyr Leu Asp His His Gly Gly Pro Gly Val Gln
       290                 295                 300
   His Ile Ala Leu Ala Ser Asp Asp Val Leu Gly Thr Leu Arg Glu Met
   305                 310                 315                 320
   Arg Ala Arg Ser Ala Met Gly Gly Phe Glu Phe Leu Ala Pro Pro Pro
                   325                 330                 335
   Pro Asn Tyr Tyr Asp Gly Val Arg Arg Arg Ala Gly Asp Val Leu Ser
               340                 345                 350
   Glu Glu Gln Ile Asn Glu Cys Gln Glu Leu Gly Val Leu Val Asp Arg
           355                 360                 365
   Asp Asp Gln Gly Val Leu Leu Gln Ile Phe Thr Lys Pro Val Gly Asp
       370                 375                 380
   Arg Pro Thr Phe Phe Leu Glu Met Ile Gln Arg Ile Gly Cys Met Glu
   385                 390                 395                 400
   Lys Asp Glu Ser Gly Gln Glu Tyr Gln Lys Gly Gly Cys Gly Gly Phe
                   405                 410                 415
   Gly Lys Gly Asn Phe Ser Glu Leu Phe Lys Ser Ile Glu Glu Tyr Glu
               420                 425                 430
   Lys Ser Leu Glu Ala Lys Gln Ala Pro Thr Val Gln Gly Ser
           435                 440                 445
<210> 60
   <211> 436
   <212> PRT
   <213> Triticum
<400> 60
   Met Pro Pro Thr Pro Thr Thr Pro Ala Ala Thr Gly Ala Gly Ala Ala
   1               5                   10                  15
   Ala Ala Val Thr Pro Glu His Ala Arg Pro Arg Arg Met Val Arg Phe
               20                  25                  30
   Asn Pro Arg Ser Asp Arg Phe His Thr Leu Ser Phe His His Val Glu
           35                  40                  45
   Phe Trp Cys Ala Asp Ala Ala Ser Ala Ala Gly Arg Phe Ala Phe Ala
       50                  55                  60
   Leu Gly Ala Pro Leu Ala Ala Arg Ser Asp Leu Ser Thr Gly Asn Ser
   65                  70                  75                  80
   Val His Ala Ser Gln Leu Leu Arg Ser Gly Asn Leu Ala Phe Leu Phe
                   85                  90                  95
   Thr Ala Pro Tyr Ala Asn Gly Cys Asp Ala Ala Thr Ala Ser Leu Pro
               100                 105                 110
   Ser Phe Ser Ala Asp Ala Ala Arg Arg Phe Ser Ala Asp His Gly Leu
           115                 120                 125
   Ala Val Arg Ser Ile Ala Leu Arg Val Ala Asp Ala Ala Glu Ala Phe
       130                 135                 140
   Arg Ala Ser Val Asp Gly Gly Ala Arg Pro Ala Phe Ser Pro Val Asp
   145                 150                 155                 160
   Leu Gly Arg Gly Phe Gly Phe Ala Glu Val Glu Leu Tyr Gly Asp Val
                   165                 170                 175
   Val Leu Arg Phe Val Ser His Pro Asp Asp Thr Asp Val Pro Phe Leu
               180                 185                 190
   Pro Gly Phe Glu Gly Val Ser Asn Pro Asp Ala Val Asp Tyr Gly Leu
           195                 200                 205
   Thr Arg Phe Asp His Val Val Gly Asn Val Pro Glu Leu Ala Pro Ala
       210                 215                 220
   Ala Ala Tyr Val Ala Gly Phe Ala Gly Phe His Glu Phe Ala Glu Phe
   225                 230                 235                 240
   Thr Thr Glu Asp Val Gly Thr Ala Glu Ser Gly Leu Asn Ser Met Val
                   245                 250                 255
   Leu Ala Asn Asn Ser Glu Gly Val Leu Leu Pro Leu Asn Glu Pro Val
               260                 265                 270
   His Gly Thr Lys Arg Arg Ser Gln Ile Gln Thr Phe Leu Glu His His
           275                 280                 285
   Gly Gly Ser Gly Val Gln His Ile Ala Val Ala Ser Ser Asp Val Leu
       290                 295                 300
   Arg Thr Leu Arg Glu Met Arg Ala Arg Ser Ala Met Gly Gly Phe Asp
   305                 310                 315                 320
   Phe Leu Pro Pro Arg Cys Arg Lys Tyr Tyr Glu Gly Val Arg Arg Ile
                   325                 330                 335
   Ala Gly Asp Val Leu Ser Glu Ala Gln Ile Lys Glu Cys Gln Glu Leu
               340                 345                 350
   Gly Val Leu Val Asp Arg Asp Asp Gln Gly Val Leu Leu Gln Ile Phe
           355                 360                 365
   Thr Lys Pro Val Gly Asp Arg Pro Thr Leu Phe Leu Glu Met Ile Gln
       370                 375                 380
   Arg Ile Gly Cys Met Glu Lys Asp Glu Arg Gly Glu Glu Tyr Gln Lys
   385                 390                 395                 400
   Gly Gly Cys Gly Gly Phe Gly Lys Gly Asn Phe Ser Glu Leu Phe Lys
                   405                 410                 415
   Ser Ile Glu Asp Tyr Glu Lys Ser Leu Glu Ala Lys Gln Ser Ala Ala
               420                 425                 430
   Val Gln Gly Ser
           435
<210> 61
   <211> 444
   <212> PRT
   <213> Zea
<400> 61
   Met Pro Pro Thr Pro Thr Ala Ala Ala Ala Gly Ala Ala Val Ala Ala
   1               5                   10                  15
   Ala Ser Ala Ala Glu Gln Ala Ala Phe Arg Leu Val Gly His Arg Asn
               20                  25                  30
   Phe Val Arg Phe Asn Pro Arg Ser Asp Arg Phe His Thr Leu Ala Phe
           35                  40                  45
   His His Val Glu Leu Trp Cys Ala Asp Ala Ala Ser Ala Ala Gly Arg
       50                  55                  60
   Phe Ser Phe Gly Leu Gly Ala Pro Leu Ala Ala Arg Ser Asp Leu Ser
   65                  70                  75                  80
   Thr Gly Asn Ser Ala His Ala Ser Leu Leu Leu Arg Ser Gly Ser Leu
                   85                  90                  95
   Ser Phe Leu Phe Thr Ala Pro Tyr Ala His Gly Ala Asp Ala Ala Thr
               100                 105                 110
   Ala Ala Leu Pro Ser Phe Ser Ala Ala Ala Ala Arg Arg Phe Ala Ala
           115                 120                 125
   Asp His Gly Leu Ala Val Arg Ala Val Ala Leu Arg Val Ala Asp Ala
       130                 135                 140
   Glu Glu Ala Phe Arg Thr Ser Val Ala Ala Gly Ala Arg Pro Ala Phe
   145                 150                 155                 160
   Gly Pro Val Asp Leu Gly Arg Gly Phe Arg Leu Ala Glu Val Glu Leu
                   165                 170                 175
   Tyr Gly Asp Val Val Leu Arg Tyr Val Ser Tyr Pro Asp Gly Ala Ala
               180                 185                 190
   Gly Glu Pro Phe Leu Pro Gly Phe Glu Gly Val Ala Ser Pro Gly Ala
           195                 200                 205
   Ala Asp Tyr Gly Leu Ser Arg Phe Asp His Ile Val Gly Asn Val Pro
       210                 215                 220
   Glu Leu Ala Pro Ala Ala Ala Tyr Phe Ala Gly Phe Thr Gly Phe His
   225                 230                 235                 240
   Glu Phe Ala Glu Phe Thr Thr Glu Asp Val Gly Thr Ala Glu Ser Gly
                   245                 250                 255
   Leu Asn Ser Met Val Leu Ala Asn Asn Ser Glu Asn Val Leu Leu Pro
               260                 265                 270
   Leu Asn Glu Pro Val His Gly Thr Lys Arg Arg Ser Gln Ile Gln Thr
           275                 280                 285
   Phe Leu Asp His His Gly Gly Pro Gly Val Gln His Met Ala Leu Ala
       290                 295                 300
   Ser Asp Asp Val Leu Arg Thr Leu Arg Glu Met Gln Ala Arg Ser Ala
   305                 310                 315                 320
   Met Gly Gly Phe Glu Phe Met Ala Pro Pro Thr Ser Asp Tyr Tyr Asp
                   325                 330                 335
   Gly Val Arg Arg Arg Ala Gly Asp Val Leu Thr Glu Ala Gln Ile Lys
               340                 345                 350
   Glu Cys Gln Glu Leu Gly Val Leu Val Asp Arg Asp Asp Gln Gly Val
           355                 360                 365
   Leu Leu Gln Ile Phe Pro Lys Pro Val Gly Asp Arg Pro Thr Leu Phe
       370                 375                 380
   Leu Glu Ile Ile Gln Arg Ile Gly Cys Met Glu Arg Asp Glu Lys Gly
   385                 390                 395                 400
   Gln Glu Tyr Gln Lys Gly Gly Cys Gly Gly Phe Gly Lys Gly Asn Phe
                   405                 410                 415
   Ser Gln Leu Phe Lys Ser Ile Glu Asp Tyr Glu Lys Ser Leu Glu Ala
               420                 425                 430
   Met Gln Ala Ala Ala Ala Ala Thr Ala Gln Gly Ser
           435                 440
<210> 62
   <211> 443
   <212> PRT
   <213> Glycine
<220>
   <221> VARIANT
   <222> 393..393
<400> 62
   Met Cys Asn Glu Ile Gln Ala Gln Ala Gln Ala Gln Ala Gln Pro Gly
   1               5                   10                  15
   Phe Lys Leu Val Gly Phe Lys Asn Phe Val Arg Thr Asn Pro Lys Ser
               20                  25                  30
   Asp Arg Phe Gln Val Asn Arg Phe His His Ile Glu Phe Trp Cys Thr
           35                  40                  45
   Asp Ala Thr Asn Ala Ser Arg Arg Phe Ser Trp Gly Leu Gly Met Pro
       50                  55                  60
   Ile Val Ala Lys Ser Asp Leu Ser Thr Gly Asn Gln Ile His Ala Ser
   65                  70                  75                  80
   Tyr Leu Leu Arg Ser Gly Asp Leu Ser Phe Leu Phe Ser Ala Pro Tyr
                   85                  90                  95
   Ser Pro Ser Leu Ser Ala Gly Ser Ser Ala Ala Ser Ser Ala Ser Ile
               100                 105                 110
   Pro Ser Phe Asp Ala Ala Thr Cys Leu Ala Phe Ala Ala Lys His Gly
           115                 120                 125
   Phe Gly Val Arg Ala Ile Ala Leu Glu Val Ala Asp Ala Glu Ala Ala
       130                 135                 140
   Phe Ser Ala Ser Val Ala Lys Gly Ala Glu Pro Ala Ser Pro Pro Val
   145                 150                 155                 160
   Leu Val Asp Asp Arg Thr Gly Phe Ala Glu Val Arg Leu Tyr Gly Asp
                   165                 170                 175
   Val Val Leu Arg Tyr Val Ser Tyr Lys Asp Ala Ala Pro Gln Ala Pro
               180                 185                 190
   His Ala Asp Pro Ser Arg Trp Phe Leu Pro Gly Phe Glu Ala Ala Ala
           195                 200                 205
   Ser Ser Ser Ser Phe Pro Glu Leu Asp Tyr Gly Ile Arg Arg Leu Asp
       210                 215                 220
   His Ala Val Gly Asn Val Pro Glu Leu Ala Pro Ala Val Arg Tyr Leu
   225                 230                 235                 240
   Lys Gly Phe Ser Gly Phe His Glu Phe Ala Glu Phe Thr Ala Glu Asp
                   245                 250                 255
   Val Gly Thr Ser Glu Ser Gly Leu Asn Ser Val Val Leu Ala Asn Asn
               260                 265                 270
   Ser Glu Thr Val Leu Leu Pro Leu Asn Glu Pro Val Tyr Gly Thr Lys
           275                 280                 285
   Arg Lys Ser Gln Ile Glu Thr Tyr Leu Glu His Asn Glu Gly Ala Gly
       290                 295                 300
   Val Gln His Leu Ala Leu Val Thr His Asp Ile Phe Thr Thr Leu Arg
   305                 310                 315                 320
   Glu Met Arg Lys Arg Ser Phe Leu Gly Gly Phe Glu Phe Met Pro Ser
                   325                 330                 335
   Pro Pro Pro Thr Tyr Tyr Ala Asn Leu His Asn Arg Ala Ala Asp Val
               340                 345                 350
   Leu Thr Val Asp Gln Ile Lys Gln Cys Glu Glu Leu Gly Ile Leu Val
           355                 360                 365
   Asp Arg Asp Asp Gln Gly Thr Leu Leu Gln Ile Phe Thr Lys Pro Val
       370                 375                 380
   Gly Asp Arg Pro Thr Ile Phe Ile Xaa Ile Ile Gln Arg Ile Gly Cys
   385                 390                 395                 400
   Met Val Glu Asp Glu Glu Gly Lys Val Tyr Gln Lys Gly Ala Cys Gly
                   405                 410                 415
   Gly Phe Gly Lys Gly Asn Phe Ser Glu Leu Phe Lys Ser Ile Glu Glu
               420                 425                 430
   Tyr Glu Lys Thr Leu Glu Ala Lys Arg Thr Ala
           435                 440
<210> 63
   <211> 445
   <212> PRT
   <213> Vitis
<400> 63
   Met Gly Lys Gln Asn Thr Thr Thr Asn Asn Pro Ala Pro Gly Phe Lys
   1               5                   10                  15
   Leu Val Gly Phe Ser Asn Phe Leu Arg Thr Asn Pro Met Ser Asp Arg
               20                  25                  30
   Phe Gly Val Lys Arg Phe His His Ile Glu Phe Trp Ser Thr Asp Ala
           35                  40                  45
   Thr Asn Leu Ala Arg Arg Phe Ser Trp Gly Leu Gly Met Pro Ile Val
       50                  55                  60
   Ala Lys Ser Asp Leu Ser Thr Gly Asn Val Ile His Ala Ser Tyr Leu
   65                  70                  75                  80
   Thr Arg Ser Gly Asp Leu Asn Phe Leu Phe Thr Ala Pro Tyr Ser Pro
                   85                  90                  95
   Ser Ile Ala Gly Asp Leu Glu Asn Ala Ala Ala Thr Ala Ser Ile Pro
               100                 105                 110
   Ser Phe Asp His Ser Ala Cys His Ala Phe Ala Ala Ser His Gly Leu
           115                 120                 125
   Gly Val Arg Ala Ile Ala Ile Glu Val Asp Asp Ala Glu Gly Ala Phe
       130                 135                 140
   His Thr Ser Val Ala His Gly Ala Arg Pro Met Ser Pro Pro Val Thr
   145                 150                 155                 160
   Met Gly Gly Ser Val Val Ile Ser Glu Val His Leu Tyr Gly Asp Ala
                   165                 170                 175
   Val Leu Arg Tyr Val Ser Tyr Lys Asn Pro Asn Pro Asn Ala Thr Ser
               180                 185                 190
   Asp Pro Ser Ser Trp Phe Leu Pro Gly Phe Glu Ala Val Asp Glu Gly
           195                 200                 205
   Ser Ser Phe Pro Val Asp Phe Gly Leu Arg Arg Val Asp His Thr Val
       210                 215                 220
   Gly Asn Val Pro Lys Leu Ala Pro Val Val Thr Tyr Leu Lys Gln Phe
   225                 230                 235                 240
   Thr Gly Phe His Glu Phe Ala Glu Phe Thr Ala Glu Asp Val Gly Thr
                   245                 250                 255
   Ser Glu Ser Gly Leu Asn Ser Val Val Leu Ala Ser Asn Asn Glu Met
               260                 265                 270
   Val Leu Leu Pro Leu Asn Glu Pro Val Phe Gly Thr Lys Arg Lys Ser
           275                 280                 285
   Gln Ile Gln Thr Tyr Leu Glu His Asn Glu Gly Pro Gly Val Gln His
       290                 295                 300
   Leu Ala Leu Met Ser Asp Asp Ile Phe Arg Thr Leu Arg Glu Met Arg
   305                 310                 315                 320
   Arg Arg Ser Gly Val Gly Gly Phe Asp Phe Met Pro Ser Pro Pro Pro
                   325                 330                 335
   Thr Tyr Tyr Arg Asn Val Lys Lys Arg Ala Gly Asp Val Leu Thr Asp
               340                 345                 350
   Asp Gln Ile Lys Glu Cys Glu Glu Leu Gly Ile Leu Val Asp Lys Asp
           355                 360                 365
   Asp Gln Gly Thr Leu Leu Gln Ile Phe Thr Lys Pro Leu Gly Asp Arg
       370                 375                 380
   Pro Thr Ile Phe Ile Glu Ile Ile Gln Arg Leu Gly Cys Met Val Lys
   385                 390                 395                 400
   Asp Asp Glu Gly Lys Val Ser Gln Lys Gly Gly Cys Gly Gly Phe Gly
                   405                 410                 415
   Lys Gly Asn Phe Ser Glu Leu Phe Lys Ser Ile Glu Glu Tyr Glu Lys
               420                 425                 430
   Thr Leu Gly Ala Lys Arg Ile Val Asp Pro Ala Pro Val
           435                 440                 445
<210> 64
   <211> 358
   <212> PRT
   <213> Pseudomonas fluorescens
<400> 64
   Met Ala Asp Gln Tyr Glu Asn Pro Met Gly Leu Met Gly Phe Glu Phe
   1               5                   10                  15
   Ile Glu Phe Ala Ser Pro Thr Pro Gly Thr Leu Glu Pro Ile Phe Glu
               20                  25                  30
   Ile Met Gly Phe Thr Lys Val Ala Thr His Arg Ser Lys Asn Val His
           35                  40                  45
   Leu Tyr Arg Gln Gly Glu Ile Asn Leu Ile Leu Asn Asn Gln Pro Asp
       50                  55                  60
   Ser Leu Ala Ser Tyr Phe Ala Ala Glu His Gly Pro Ser Val Cys Gly
   65                  70                  75                  80
   Met Ala Phe Arg Val Lys Asp Ser Gln Gln Ala Tyr Asn Arg Ala Leu
                   85                  90                  95
   Glu Leu Gly Ala Gln Pro Ile His Ile Glu Thr Gly Pro Met Glu Leu
               100                 105                 110
   Asn Leu Pro Ala Ile Lys Gly Ile Gly Gly Ala Pro Leu Tyr Leu Ile
           115                 120                 125
   Asp Arg Phe Gly Glu Gly Ser Ser Ile Tyr Asp Ile Asp Phe Val Tyr
       130                 135                 140
   Leu Glu Gly Val Asp Arg Asn Pro Val Gly Ala Gly Leu Lys Val Ile
   145                 150                 155                 160
   Asp His Leu Thr His Asn Val Tyr Arg Gly Arg Met Ala Tyr Trp Ala
                   165                 170                 175
   Asn Phe Tyr Glu Lys Leu Phe Asn Phe Arg Glu Ala Arg Tyr Phe Asp
               180                 185                 190
   Ile Lys Gly Glu Tyr Thr Gly Leu Thr Ser Lys Ala Met Ser Ala Pro
           195                 200                 205
   Asp Gly Met Ile Arg Ile Pro Leu Asn Glu Glu Ser Ser Lys Gly Ala
       210                 215                 220
   Gly Gln Ile Glu Glu Phe Leu Met Gln Phe Asn Gly Glu Gly Ile Gln
   225                 230                 235                 240
   His Val Ala Phe Leu Thr Glu Asp Leu Val Lys Thr Trp Asp Ala Leu
                   245                 250                 255
   Lys Lys Ile Gly Met Arg Phe Met Thr Ala Pro Pro Asp Thr Tyr Tyr
               260                 265                 270
   Glu Met Leu Glu Gly Arg Leu Pro Asn His Gly Glu Pro Val Asp Gln
           275                 280                 285
   Leu Gln Ala Arg Gly Ile Leu Leu Asp Gly Ser Ser Ile Glu Gly Asp
       290                 295                 300
   Lys Arg Leu Leu Leu Gln Ile Phe Ser Glu Thr Leu Met Gly Pro Val
   305                 310                 315                 320
   Phe Phe Glu Phe Ile Gln Arg Lys Gly Asp Asp Gly Phe Gly Glu Gly
                   325                 330                 335
   Asn Phe Lys Ala Leu Phe Glu Ser Ile Glu Arg Asp Gln Val Arg Arg
               340                 345                 350
   Gly Val Leu Thr Thr Asp
           355
<210> 65
   <211> 358
   <212> PRT
   <213> Pseudomonas fluorescens
<400> 65
   Met Ala Asp Leu Tyr Glu Asn Pro Met Gly Leu Met Gly Phe Glu Phe
   1               5                   10                  15
   Ile Glu Leu Ala Ser Pro Thr Pro Asn Thr Leu Glu Pro Ile Phe Glu
               20                  25                  30
   Ile Met Gly Phe Thr Lys Val Ala Thr His Arg Ser Lys Asp Val His
           35                  40                  45
   Leu Tyr Arg Gln Gly Ala Ile Asn Leu Ile Leu Asn Asn Glu Pro His
       50                  55                  60
   Ser Val Ala Ser Tyr Phe Ala Ala Glu His Gly Pro Ser Val Cys Gly
   65                  70                  75                  80
   Met Ala Phe Arg Val Lys Asp Ser Gln Lys Ala Tyr Lys Arg Ala Leu
                   85                  90                  95
   Glu Leu Gly Ala Gln Pro Ile His Ile Glu Thr Gly Pro Met Glu Leu
               100                 105                 110
   Asn Leu Pro Ala Ile Lys Gly Ile Gly Gly Ala Pro Leu Tyr Leu Ile
           115                 120                 125
   Asp Arg Phe Gly Glu Gly Ser Ser Ile Tyr Asp Ile Asp Phe Val Phe
       130                 135                 140
   Leu Glu Gly Val Asp Arg His Pro Val Gly Ala Gly Leu Lys Ile Ile
   145                 150                 155                 160
   Asp His Leu Thr His Asn Val Tyr Arg Gly Arg Met Ala Tyr Trp Ala
                   165                 170                 175
   Asn Phe Tyr Glu Lys Leu Phe Asn Phe Arg Glu Ile Arg Tyr Phe Asp
               180                 185                 190
   Ile Lys Gly Glu Tyr Thr Gly Leu Thr Ser Lys Ala Met Thr Ala Pro
           195                 200                 205
   Asp Gly Met Ile Arg Ile Pro Leu Asn Glu Glu Ser Ser Lys Gly Ala
       210                 215                 220
   Gly Gln Ile Glu Glu Phe Leu Met Gln Phe Asn Gly Glu Gly Ile Gln
   225                 230                 235                 240
   His Val Ala Phe Leu Ser Asp Asp Leu Ile Lys Thr Trp Asp His Leu
                   245                 250                 255
   Lys Ser Ile Gly Met Arg Phe Met Thr Ala Pro Pro Asp Thr Tyr Tyr
               260                 265                 270
   Glu Met Leu Glu Gly Arg Leu Pro Asn His Gly Glu Pro Val Gly Glu
           275                 280                 285
   Leu Gln Ala Arg Gly Ile Leu Leu Asp Gly Ser Ser Glu Ser Gly Asp
       290                 295                 300
   Lys Arg Leu Leu Leu Gln Ile Phe Ser Glu Thr Leu Met Gly Pro Val
   305                 310                 315                 320
   Phe Phe Glu Phe Ile Gln Arg Lys Gly Asp Asp Gly Phe Gly Glu Gly
                   325                 330                 335
   Asn Phe Lys Ala Leu Phe Glu Ser Ile Glu Arg Asp Gln Val Arg Arg
               340                 345                 350
   Gly Val Leu Ser Thr Asp
                               355
<210> 66
   <211> 440
   <212> PRT
   <213> Avena sativa
<400> 66
   Met Pro Pro Thr Pro Ala Thr Ala Thr Gly Ala Ala Ala Ala Ala Val
   1               5                   10                  15
   Thr Pro Glu His Ala Ala Arg Ser Phe Pro Arg Val Val Arg Val Asn
               20                  25                  30
   Pro Arg Ser Asp Arg Phe Pro Val Leu Ser Phe His His Val Glu Leu
           35                  40                  45
   Trp Cys Ala Asp Ala Ala Ser Ala Ala Gly Arg Phe Ser Phe Ala Leu
       50                  55                  60
   Gly Ala Pro Leu Ala Ala Arg Ser Asp Leu Ser Thr Gly Asn Ser Ala
   65                  70                  75                  80
   His Ala Ser Leu Leu Leu Arg Ser Gly Ala Leu Ala Phe Leu Phe Thr
                   85                  90                  95
   Ala Pro Tyr Ala Pro Pro Pro Gln Glu Ala Ala Thr Ala Ala Ala Thr
               100                 105                 110
   Ala Ser Ile Pro Ser Phe Ser Ala Asp Ala Ala Arg Thr Phe Ala Ala
           115                 120                 125
   Ala His Gly Leu Ala Val Arg Ser Val Gly Val Arg Val Ala Asp Ala
       130                 135                 140
   Ala Glu Ala Phe Arg Val Ser Val Ala Gly Gly Ala Arg Pro Ala Phe
   145                 150                 155                 160
   Ala Pro Ala Asp Leu Gly His Gly Phe Gly Leu Ala Glu Val Glu Leu
                   165                 170                 175
   Tyr Gly Asp Val Val Leu Arg Phe Val Ser Tyr Pro Asp Glu Thr Asp
               180                 185                 190
   Leu Pro Phe Leu Pro Gly Phe Glu Arg Val Ser Ser Pro Gly Ala Val
           195                 200                 205
   Asp Tyr Gly Leu Thr Arg Phe Asp His Val Val Gly Asn Val Pro Glu
       210                 215                 220
   Met Ala Pro Val Ile Asp Tyr Met Lys Gly Phe Leu Gly Phe His Glu
   225                 230                 235                 240
   Phe Ala Glu Phe Thr Ala Glu Asp Val Gly Thr Thr Glu Ser Gly Leu
                   245                 250                 255
   Asn Ser Val Val Leu Ala Asn Asn Ser Glu Ala Val Leu Leu Pro Leu
               260                 265                 270
   Asn Glu Pro Val His Gly Thr Lys Arg Arg Ser Gln Ile Gln Thr Tyr
           275                 280                 285
   Leu Glu Tyr His Gly Gly Pro Gly Val Gln His Ile Ala Leu Ala Ser
       290                 295                 300
   Asn Asp Val Leu Arg Thr Leu Arg Glu Met Arg Ala Arg Thr Pro Met
   305                 310                 315                 320
   Gly Gly Phe Glu Phe Met Ala Pro Pro Gln Ala Lys Tyr Tyr Glu Gly
                   325                 330                 335
   Val Arg Arg Ile Ala Gly Asp Val Leu Ser Glu Glu Gln Ile Lys Glu
               340                 345                 350
   Cys Gln Glu Leu Gly Val Leu Val Asp Arg Asp Asp Gln Gly Val Leu
           355                 360                 365
   Leu Gln Ile Phe Thr Lys Pro Val Gly Asp Arg Pro Thr Phe Phe Leu
       370                 375                 380
   Glu Met Ile Gln Arg Ile Gly Cys Met Glu Lys Asp Glu Val Gly Gln
   385                 390                 395                 400
   Glu Tyr Gln Lys Gly Gly Cys Gly Gly Phe Gly Lys Gly Asn Phe Ser
                   405                 410                 415
   Glu Leu Phe Lys Ser Ile Glu Asp Tyr Glu Lys Ser Leu Glu Val Lys
               420                 425                 430
   Gln Ser Val Val Ala Gln Lys Ser
                       435                 440
<210> 67
   <211> 444
   <212> PRT
   <213> Zea mays
<400> 67
   Met Gly Pro Thr Pro Thr Ala Ala Ala Ala Gly Ala Ala Val Ala Ala
   1               5                   10                  15
   Ala Ser Ala Ala Glu Gln Ala Ala Phe Arg Leu Val Gly His Arg Asn
               20                  25                  30
   Phe Val Arg Phe Asn Pro Arg Ser Asp Arg Phe His Thr Leu Ala Phe
           35                  40                  45
   His His Val Glu Leu Trp Cys Ala Asp Ala Ala Ser Ala Ala Gly Arg
       50                  55                  60
   Phe Ser Phe Gly Leu Gly Ala Pro Leu Ala Ala Arg Ser Asp Leu Ser
   65                  70                  75                  80
   Thr Gly Asn Ser Ala His Ala Ser Leu Leu Leu Arg Ser Gly Ser Leu
                   85                  90                  95
   Ser Phe Leu Phe Thr Ala Pro Tyr Ala His Gly Ala Asp Ala Ala Thr
               100                 105                 110
   Ala Ala Leu Pro Ser Phe Ser Ala Ala Ala Ala Arg Arg Phe Ala Ala
           115                 120                 125
   Asp His Gly Leu Ala Val Arg Ala Val Ala Leu Arg Val Ala Asp Ala
       130                 135                 140
   Glu Asp Ala Phe Arg Ala Ser Val Ala Ala Gly Ala Arg Pro Ala Phe
   145                 150                 155                 160
   Gly Pro Val Asp Leu Gly Arg Gly Phe Arg Leu Ala Glu Val Glu Leu
                   165                 170                 175
   Tyr Gly Asp Val Val Leu Arg Tyr Val Ser Tyr Pro Asp Gly Ala Ala
               180                 185                 190
   Gly Glu Pro Phe Leu Pro Gly Phe Glu Gly Val Ala Ser Pro Gly Ala
           195                 200                 205
   Ala Asp Tyr Gly Leu Ser Arg Phe Asp His Ile Val Gly Asn Val Pro
       210                 215                 220
   Glu Leu Ala Pro Ala Ala Ala Tyr Phe Ala Gly Phe Thr Gly Phe His
   225                 230                 235                 240
   Glu Phe Ala Glu Phe Thr Thr Glu Asp Val Gly Thr Ala Glu Ser Gly
                   245                 250                 255
   Leu Asn Ser Met Val Leu Ala Asn Asn Ser Glu Asn Val Leu Leu Pro
               260                 265                 270
   Leu Asn Glu Pro Val His Gly Thr Lys Arg Arg Ser Gln Ile Gln Thr
           275                 280                 285
   Phe Leu Asp His His Gly Gly Pro Gly Val Gln His Met Ala Leu Ala
       290                 295                 300
   Ser Asp Asp Val Leu Arg Thr Leu Arg Glu Met Gln Ala Arg Ser Ala
   305                 310                 315                 320
   Met Gly Gly Phe Glu Phe Met Ala Pro Pro Thr Ser Asp Tyr Tyr Asp
                   325                 330                 335
   Gly Val Arg Arg Arg Ala Gly Asp Val Leu Thr Glu Ala Gln Ile Lys
               340                 345                 350
   Glu Cys Gln Glu Leu Gly Val Leu Val Asp Arg Asp Asp Gln Gly Val
           355                 360                 365
   Leu Leu Gln Ile Phe Thr Lys Pro Val Gly Asp Arg Pro Thr Leu Phe
       370                 375                 380
   Leu Glu Ile Ile Gln Arg Ile Gly Cys Met Glu Lys Asp Glu Lys Gly
   385                 390                 395                 400
   Gln Glu Tyr Gln Lys Gly Gly Cys Gly Gly Phe Gly Lys Gly Asn Phe
                   405                 410                 415
   Ser Gln Leu Phe Lys Ser Ile Glu Asp Tyr Glu Lys Ser Leu Glu Ala
               420                 425                 430
   Lys Gln Ala Ala Ala Ala Ala Ala Ala Gln Gly Ser
   435 440
<210> 68
   <211> 1332
   <212> DNA
   <213> Zea mays
<400> 68
   atgggtccga ccccgaccgc aacagcagcc ggtgcagcag ttgcagcagc aagcgcagca      60
   gaacaggcag catttcgtct ggttggtcat cgtaattttg ttcgttttaa tccgcgttcc     120
   gatcgttttc ataccctggc atttcatcat gttgaactgt ggtgtgcaga tgcagcatca     180
   gcagcaggtc gttttagctt tgcactgggt gcaccgctgg cagcacgtag cgatctgagc     240
   accggtaata gcgcacatgc aagtctgctg ctgcgtagcg gtagcctgag ctttctgttt     300
   accgcaccgt atgcacatgg tgccgatgca gccaccgcag cactgccgag ctttagcgca     360
   gccgcagccc gtcgttttgc agcagatcat ggtctggcag ttcgtgcagt tgcactgcgt     420
   gttgcagatg ccgaagatgc atttcgtgca agcgttgcag cgggtgcacg tccggcattt     480
   ggtccggttg atctgggtcg tggttttcgc ctggccgaag tggaactgta tggtgatgtt     540
   gttctgcgtt atgttagcta tccggatggt gccgcaggcg aaccgtttct gcctggtttt     600
   gaaggtgttg caagtccggg tgcagccgat tatggcctga gccgttttga tcatattgtt     660
   ggtaatgtgc cggaactggc accggcagca gcatattttg caggttttac cggttttcat     720
   gaatttgccg aatttaccac cgaagatgtt ggcaccgcag aaagcggtct gaatagcatg     780
   gcactggcaa ataatagcga aaatgtactg ctgccgctga atgaaccggt gcatggcacc     840
   aaacgtcgta gccagattca gacctttctg gatcatcatg gtggtccggg tgttcagcat     900
   attgcactgg catcagatga tgtgctgcgt accctgcgtg aaatgcaggc acgtagtgca     960
   atgggtggct ttgaatttct gcctccgcct ccgagcgatt attatgatgg tgttcgtcgt    1020
   gaagccggtg atgttctgac cgaagcacag attaatgaat gtcaagaact gggtgttatg    1080
   gtggatcgtg atgatcaggg tgtcctgctg cagattttta ccaaaccggt tggtgatcgt    1140
   ccgacctttt ttctggaaat tattcagcgt attggctgca tggaaaaaga tgaaaaaggc    1200
   caagaatatc agaaaggtgg ttgtggtggt tttggcaaag ataactttag ccagctgttt    1260
   aaaagcatcg aggattatga aaaaagcctg gaagcaaaac aagccgcagc agccgcaacc    1320
   gcacagggta gc                                                        1332
<210> 69
   <211> 1332
   <212> DNA
   <213> Zea mays
<400> 69
   atgggtccga ccccgaccgc aacagcagcc ggtgcagcag ttgcagcagc aagcgcagca      60
   gaacaggcag catttcgtct ggttggtcat cgtaattttg ttcgttttaa tccgcgtagc     120
   gatcgttttc agaccctggc atttcatcat gttgaactgt ggtgtgcaga tgcagcatca     180
   gcagcaggtc gttttagctt tgcactgggt gttccgctgg cagcacgtag tgatctgagc     240
   accggtaata gcgcacatgc aagtctgctg ctgcgtagcg gtagcctgag cctgctgttt     300
   accgcaccgt atgcacatgg tgccgatgca gccaccgcag cactgccgag ctttagcgca     360
   gccgcagccc gtcgttttgc agcagatcat ggtctggcag ttcgtgcagt tgccctgcgt     420
   gttgcagatg ccgaagatgc atttcgtgca agcgttgcag cgggtgcacg tccggcattt     480
   ggtccggttg atctgggtcg tggttttcgc ctggccgaag tggaactgta tggtgatgtt     540
   gttctgcgtt atgttagcta tccggatggt gccgcaggcg aaccgtttct gcctggtttt     600
   gaaggtgttg caagtccggg tgcagccgat tatggcctga gccgttttga tcatattgtt     660
   ggtaatgtgc cggaactggc accggcagca gcatatatgg caggttttac cggttttcat     720
   gaatttgcag aatttaccac cgaagatgtt ggcaccaccg aaagcggtct gaatagcatg     780
   gcactggcaa ataatagcga aaatgtactg ctgccgctga atgaaccggt gcatggcacc     840
   aaacgtcgta gccagattca gacctttctg gatcatcatg gtggtccggg tgttcagcat     900
   attgcactgg catcagatga tgtgctgcgt accctgcgtg aaatgcgtgc acgtagcgca     960
   atgggtggct ttgaatttct gcctccgcct ctgagcgatt attatgatgg tgttcgtcgt    1020
   tgtgccggtg atgttctgac cgaagcacag attaatgaat gtcaagaact gggtgtgatg    1080
   gtggatcgtg atgatcaggg tgtcctgctg cagattttta ccaaaccggt tggtgatcgt    1140
   ccgacctttt ttctggaaat tattcagcgt attggctgca tggaaaaaga tgaaaaaggc    1200
   caagaatatc agaaaggtgg ttgtggtggt tttggcaaag gtaattttgg tcagctgttt    1260
   aaaagcatcg aggactatga aaaaagcctg gaagcaaaac aagccgcagc agccgcaacc    1320
   gcacagggta gc                                                        1332

## Description

The present invention relates to benzamide compounds and salts thereof and to compositions comprising the same. The invention also relates to the use of the benzamide compounds or of the compositions comprising such compounds for controlling unwanted vegetation. Furthermore, the invention relates to methods of applying such compounds.

For the purposes of controlling unwanted vegetation, especially in crops, there is an ongoing need for new herbicides which have high activities and selectivities together with a substantial lack of toxicity for humans and animals.

WO 2011/035874 describes N-(1,2,5-oxadiazol-3-yl)benzamides carrying 3 substituents in the 2-, 3- and 4-positions of the phenyl ring and their use as herbicides.

WO 2012/028579 describes N-(tetrazol-4-yl)- and N-(triazol-3-yl)arylcarboxylic acid amides carrying 3 substituents in the 2-, 3- and 4-positions of the aryl ring and their use as herbicides.

WO2013/017559 describes N-(tetrazol-5-yl)- and N-(triazol-5-yl)arylcarboxylic acid amides carrying 3 substituents in the 2-, 3- and 4-positions of the aryl ring and their use as herbicides.

WO2013/124245 describes N-(tetrazol-5-yl)-, N-(triazol-5-yl)- and N-(1,3,4-oxadiazol-2-yl)arylcarboxylic acid amides carrying 1, 2, or 3 substituents in the 2-, 3- and 5-positions of the aryl ring and a nitro group in the 4 position of the aryl ring and their use as herbicides.

The compounds of the prior art often suffer from insufficient herbicidal activity in particular at low application rates and/or unsatisfactory selectivity resulting in a low compatibility with crop plants.

Accordingly, it is an object of the present invention to provide further benzamide compounds having a strong herbicidal activity, in particular even at low application rates, a sufficiently low toxicity for humans and animals and/or a high compatibility with crop plants. The benzamide compounds should also show a broad activity spectrum against a large number of different unwanted plants.

These and further objectives are achieved by the compounds of formula I defined below and their agriculturally suitable salts.

Therefore, in a first aspect the present invention relates to compounds of formula I, 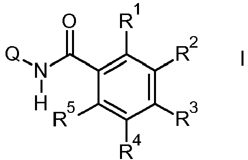 wherein
- Q: is Q¹ or Q² or Q³ or Q⁴, 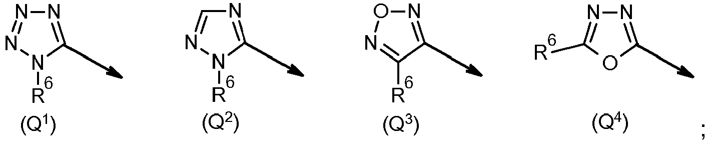
- R¹: is selected from the group consisting of cyano, halogen, nitro, C₁-C₈-alkyl, C₁-C₈-haloalkyl, C₁-C₄-alkoxy-C₁-C₄-alkyl, C₁-C₄-haloalkoxy-C₁-C₄-alkyl, C₁-C₈-alkoxy, C₁-C₄-alkoxy-C₁-C₄-alkoxy-Z¹, C₁-C₆-haloalkoxy, R^{1b}-S(O)ₖ-Z¹;
- R²: is R^{2c}R^{2d}NC(O)NR²ⁿ-Z²;
- R³: is selected from the group consisting of hydrogen, cyano, thiocyanato, halogen, hydroxy-Z², C₁-C₆-alkyl, C₂-C₈-alkenyl, C₂-C₈-alkynyl, C₃-C₁₀-cycloalkyl-Z², C₃-C₆-cycloalkenyl-Z², C₃-C₁₀-cycloalko_{X}y-Z², C₃-C₁₀-cycloalkyl-Ci-C₂-alkoxy, where the cyclic groups of the four aforementioned radicals are unsubstituted or partially or completely halogenated, C₁-C₄-cyanoalkyl, C₁-C₈-haloalkyl, C₂-C₈-haloalkenyl, C₃-C₈-haloalkynyl, C₁-Ca-alkoxy-Z², C₁-C₈-haloalkoxy-Z², C₁-C₄-alkoxy-C₁-C₄-alkoxy-Z², C₁-C₄-haloal koxy-C₁-C₄-alkoxy-Z², C₂-C₈-alkenyloxy-Z², C₂-C₃-alkynyloxy-Z², C₂-C₈-haloalkenyloxy-Z², C₃-C₈-haloalkynyloXy-Z², R^{2b}-S(O)ₖ-Z², R^{2c}-C(O)-Z², R^{2d}O-C(O)-Z², R^{2d}O-N=CH-Z², R^{2e}R^{2f}N-C(O)-Z², R^{2g}R^{2h}N-Z², R²²C(O)O-Z², R²⁵OC(O)O-Z², (R²²)₂NC(O)O-Z², R²⁵S(O)₂O-Z², R₂₂OS(O)₂-Z², (R²²)₂NS(O)₂-Z², R²⁵OC(O)N(R²²)-Z², (R²²)₂NC(O)N(R²²)-Z², (R²²)₂NS(O)₂N(R²²)-Z², (OH)₂P(O)-Z², (C₁-C₄-alkoxy)₂P(O)-Z², phenyl-Z^{2a}, heterocyclyl-Z^{2a}, where heterocyclyl is a 3-, 4-, 5- or 6-membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where cyclic groups in phenyl-Z^{2a} and heterocyclyl-Z^{2a} are unsubstituted or substituted by 1, 2, 3 or 4 groups R²¹, which are identical or different;
- R⁴: is selected from the group consisting of halogen, C₁-C₈-alkyl, cyano-Z¹, nitro, C₃-C₇-cycloalkyl, C₃-C₇-CyCloalkyl-C₁-C₄-alkyl, where the C₃-C₇-cycloalkyl groups in the two aforementioned radicals are unsubstituted or partially or completely halogenated, C₂-C₈-alkenyl, C₂-C₈-alkynyl, C₁-C₈-haloalkyl, C₁-C₃-alkylamino, C₁-C₃-dialkylamino, C₁-C₃-alkylamino-S(O)ₖ, C₁-C₃-alkylcarbonyl, C₁-C₈-alkoxy, C₁-C₄-alkoxy-C₁-C₄-alkyl, C₁-C₄-alkoxy-C₁-C₄-alkoxy-Z¹, C₁-C₄-alkylthio-C₁-C₄-alkyl, C₁-C₄-alkylthio-C₁-C₄-alkylthio-Z¹, C₂-C₆-alkenyloxy, C₂-C₆-alkynyloxy, C₁-C₆-haloalkoxy, C₁-C₄-haloalkoxy-C₁-C₄-alkyl, C₁-C₄-haloalkoxy-C₁-C₄-alkoxy-Z¹, R^{1b}-S(O)ₖ-Z¹, phenoxy-Z¹ and heterocy-clyloxy-Z¹, where heterocyclyloxy is an oxygen bound 5- or 6- membered monocyclic or 8-, 9- or 10-membered bicyclic saturated, partially unsaturated or aromatic hetero-cycle, which contains 1, 2, 3 or 4 heteroatoms as ring members, which are selected from the group consisting of O, N and S, where the cyclic groups in phenoxy and heterocyclyloxy are unsubstituted or substituted by 1, 2, 3 or 4 groups R¹¹, which are identical or different;
- R⁵: is hydrogen;
- R⁶: is selected from the group consisting of cyano, C₁-C₆-alkyl, C₃-C₇-CyCloalkyl, C₃-C₇-cycloalkyl-C₁-C₄-alkyl, where the C₃-C₇-CyCloalkyl groups of the two aforementioned radicals are unsubstituted or partially or completely halogenated, C₁-C₆-haloalkyl, C₂-C₆-alkenyl, C₂-C₆-alkynyl, C₂-C₆-haloalkenyl, C₃-C₆-haloalkynyl, C₁-C₄-alkoxy-C₁-C₄-alkyl, C₁-C₄-haloalkoxy-C₁-C₄-alkyl, R^{b}-S(O)ₙ-C₁-C₃-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
- R²¹: is selected from the group consisting of cyano, halogen, nitro, C₁-C₆-alkyl, C₃-C₇-cycloalkyl, C₃-C₇-halocycloalkyl, C₁-C₆-haloalkyl, C₂-C₆-alkenyl, C₂-C₆-haloalkenyl, C₂-C₆-alkynyl, C₃-C₆-haloalkynyl, C₁-C₆-alkoxy, C₁-C₄-alkoxy-C₁-C₄-alkyl, C₁-C₄-alkylthio-C₁-C₄-alkyl, C₁-C₄-haloalkoxy-C₁-C₄-alkyl, C₁-C₄-alkoxy-C₁-C₄-alkoxy, C₃-C₇-cycloalkoxy and C₁-C₆-haloalkoxy, or two radicals R²¹ bound to the same carbon atom together may form a group =O;
- Z¹, Z²: independently of each other are selected from the group consisting of a covalent bond and C₁-C₄-alkanediyl;
- Z^{2a}: is selected from the group consisting of a covalent bond, C₁-C₄-alkanediyl, O-C₁-C₄-alkanediyl, C₁-C₄-alkanediyl-O and C₁-C₄-alkanediyl-O-C₁-C₄-alkanediyl;
- R^{b}, R^{1b}, R^{2b}: independently of each other are selected from the group consisting of C₁-C₆-alkyl, C₃-C₇-CyCloalkyl, C₁-C₆-haloalkyl, C₂-C₆-alkenyl, C₂-C₆-haloalkenyl, C₂-C₆-alkynyl, C₃-C₆-haloalkynyl, phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
- R^{2c}: is selected from the group consisting of hydrogen, C₁-C₆-alkyl, C₃-C₇-CyCloalkyl, C₃-C₇-CyCloalkyl-C₁-C₄-alkyl, where the C₃-C₇-CyCloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl, benzyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
- R^{2d}: is selected from the group consisting of hydrogen, C₁-C₆-alkyl, C₃-C₇-CyCloalkyl, C₃-C₇-CyCloalkyl-C₁-C₄-alkyl, where the C₃-C₇-CyCloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)II-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
- R^{2c}, R^{2d}: together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
- R²ⁿ: is selected from the group consisting of hydrogen, C₁-C₆-alkyl, C₃-C₇-CyCloalkyl, C₃-C₇-CyCloalkyl-C₁-C₄-alkyl, where the C₃-C₇-CyCloalkyl groups in the two aforementioned radicals are unsubstituted or partially or completely halogenated, C₁-C₆-cyanoalkyl, C₁-C₆-haloalkyl, C₂-C₆-alkenyl, C₂-C₆-haloalkenyl, C₂-C₆-alkynyl, C₁-C₄-alkoxy-C₁-C₄-alkyl;
- R^{2e}, R^{2f}: independently of each other are selected from the group consisting of hydrogen, C₁-C₆-alkyl, C₃-C₇-CyCloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
- R^{2e}, R^{2f}: together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
- R^{2g}: is selected from the group consisting of hydrogen, C₁-C₆-alkyl, C₃-C₇-CyCloalkyl, C₃-C₇-CyCloalkyl-C₁-C₄-alkyl, where the C₃-C₇-CyCloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl. C₁-C₄-alkylsulfonyl, C₁-C₄-alkylcarbonyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
- R^{2h}: is selected from the group consisting of hydrogen, C₁-C₆-alkyl, C₃-C₇-CyCloalkyl, C₃-C₇-CyCloalkyl-C₁-C₄-alkyl, where the C₃-C₇-CyCloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylsulfonyl, C₁-C₄-alkylcarbonyl, a radical C(O)R^{k}, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
- R^{2g}, R^{2h}: together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
- R²²: is selected from the group consisting of hydrogen, C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-CyCloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl. C₁-C₆-haloalkyl, C₃-C₆-cycloalkyl, C(O)OR²³, C(O)N(R²³)₂, OR²³, N(R²³)₂, S(O)ₙR²⁴, S(O)₂OR²³, S(O)₂N(R²³)₂, and R²³O-C₁-C₆-alkyl, and where heterocyclyl bears 0,1 or 2 oxo groups;
- R²³: is selected from the group consisting of hydrogen, C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
- R²⁴: is selected from the group consisting of C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
- R²⁵: is selected from the group consisting of C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-cycloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl, C₁-C₆-haloalkyl, C₃-C₆-cycloalkyl, C(O)OR²³, C(O)N(R²³)₂, OR²³, N(R²³)₂, S(O)ₙR²⁴, S(O)₂OR²³, S(O)₂N(R²³)₂, and R²³O-C₁-C₆-alkyl, and where heterocyclyl bears 0, 1 or 2 oxo groups;
- k: is 0, 1 or 2;
- n: is 0, 1 or 2;
- R^{k}: has the meanings of R^{2c}.

The compounds of the present invention, i.e. the compounds of formula I and their agriculturally suitable salts, are particularly useful for controlling unwanted vegetation. Therefore, the invention also relates to the use of a compound of formula I or an agriculturally suitable salt thereof or of a composition comprising at least one compound of formula I or an agriculturally suitable salt thereof for combating or controlling unwanted vegetation.

The invention also relates to a composition comprising at least one compound of formula I, or a salt thereof, and at least one auxiliary. In particular, the invention relates to an agricultural composition comprising at least one compound of formula I or an agriculturally suitable salt thereof, and at least one auxiliary customary for crop protection formulations.

The present invention also relates to a method for combating or controlling unwanted vegetation, which method comprises allowing a herbicidally effective amount of at least one compound of formula I or a salt thereof, to act on unwanted plants, their seed and/or their habitat.

Depending on the substitution pattern, the compounds of formula I may have one or more centers of chirality, in which case they are present as mixtures of enantiomers or diastereomers. The invention provides both the pure enantiomers or pure diastereomers of the compounds of formula I, and their mixtures and the use according to the invention of the pure enantiomers or pure diastereomers of the compound of formula I or its mixtures. Suitable compounds of formula I also include all possible geometrical stereoisomers (cis/trans isomers) and mixtures thereof. Cis/trans isomers may be present with respect to an alkene, carbon-nitrogen double-bond, nitrogen-sulfur double bond or amide group. The term "stereoisomer(s)" encompasses both optical isomers, such as enantiomers or diastereomers, the latter existing due to more than one center of chirality in the molecule, as well as geometrical isomers (cis/trans isomers).

Depending on the substitution pattern, the compounds of formula I may be present in the form of their tautomers. Hence the invention also relates to the tautomers of compounds of formula I and the stereoisomers and salts of said tautomers.

The present invention moreover relates to compounds as defined herein, wherein one or more of the atoms depicted in formula I have been replaced by its stable, preferably nonradioactive isotope (e.g., hydrogen by deuterium, ¹²C by ¹³C, ¹⁴N by ¹⁵N, ¹⁶O by ¹⁸O) and in particular wherein at least one hydrogen atom has been replaced by a deuterium atom. Of course, the compounds according to the invention contain more of the respective isotope than this naturally occurs and thus is anyway present in the compounds of formula I.

The compounds of the present invention may be amorphous or may exist in one ore more different crystalline states (polymorphs) which may have different macroscopic properties such as stability or show different biological properties such as activities. The present invention includes both amorphous and crystalline compounds of formula I, their enantiomers or diastereomers, mixtures of different crystalline states of the respective compound of formula I, its enantiomers or diastereomers, as well as amorphous or crystalline salts thereof.

Salts of the compounds of the present invention are preferably agriculturally suitable salts. They can be formed in a customary method, e.g. by reacting the compound with an acid if the compound of the present invention has a basic functionality or by reacting the compound with a suitable base if the compound of the present invention has an acidic functionality.

Useful agriculturally suitable salts are especially the salts of those cations or the acid addition salts of those acids whose cations and anions, respectively, do not have any adverse effect on the herbicidal action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethylammonium, bis(2-hydroxyethyl)ammonium, benzyltrimethylammonium and benzl-triethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting compounds of the present invention with an acid of the corresponding anion, preferably with hydrochloric acid, hydrobromic acid, sulfuric acid, phosphoric acid or nitric acid.

The term "undesired vegetation" ("weeds") is understood to include any vegetation growing in non-crop-areas or at a crop plant site or locus of seeded and otherwise desired crop, where the vegetation is any plant species, including their germinant seeds, emerging seedlings and established vegetation, other than the seeded or desired crop (if any). Weeds, in the broadest sense, are plants considered undesirable in a particular location.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine or bromine.

The term "partially or completely halogenated" will be taken to mean that 1 or more, e.g. 1, 2, 3, 4 or 5 or all of the hydrogen atoms of a given radical have been replaced by a halogen atom, in particular by fluorine or chlorine. A partially or completely halogenated radical is termed below also "halo-radical". For example, partially or completely halogenated alkyl is also termed haloalkyl.

The term "alkyl" as used herein (and in the alkyl moieties of other groups comprising an alkyl group, e.g. alkoxy, alkylamino, alkylcarbonyl, alkoxycarbonyl, alkylthio, alkylsulfonyl and alkoxyalkyl) denotes in each case a straight-chain or branched alkyl group having usually from 1 to 10 carbon atoms, frequently from 1 to 6 carbon atoms, preferably 1 to 4 carbon atoms and in particular from 1 to 3 carbon atoms. Examples of C₁-C₄-alkyl are methyl, ethyl, n-propyl, isopropyl, n-butyl, 2-butyl (sec-butyl), isobutyl and tert-butyl. Examples for C₁-C₆-alkyl are, apart those mentioned for C₁-C₄-alkyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Examples for C₁-C₁₀-alkyl are, apart those mentioned for C₁-C₆-alkyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 1-methyloctyl, 2-methylheptyl, 1-ethylhexyl, 2-ethylhexyl, 1,2-dimethylhexyl, 1-propylpentyl, 2-propylpentyl, nonyl, decyl, 2-propylheptyl and 3-propylheptyl.

The term "alkylene" (or alkanediyl) as used herein in each case denotes an alkyl radical as defined above, wherein one hydrogen atom at any position of the carbon backbone is replaced by one further binding site, thus forming a bivalent moiety.

The term "haloalkyl" as used herein (and in the haloalkyl moieties of other groups comprising a haloalkyl group, e.g. haloalkoxy, haloalkylthio, haloalkylcarbonyl, haloalkylsulfonyl and haloalkylsulfinyl) denotes in each case a straight-chain or branched alkyl group having usually from 1 to 8 carbon atoms ("C₁-C₈-haloalkyl"), frequently from 1 to 6 carbon atoms ("C₁-C₆-haloalkyl"), more frequently 1 to 4 carbon atoms ("C₁-C₄-haloalkyl"), wherein the hydrogen atoms of this group are partially or totally replaced with halogen atoms. Preferred haloalkyl moieties are selected from C₁-C₄-haloalkyl, more preferably from C₁-C₂-haloalkyl, more preferably from halomethyl, in particular from C₁-C₂-fluoroalkyl. Halomethyl is methyl in which 1, 2 or 3 of the hydrogen atoms are replaced by halogen atoms. Examples are bromomethyl, chloromethyl, dichloromethyl, trichloromethyl, fluoromethyl, difluoromethyl, trifluoromethyl, chlorofluoromethyl, dichlorofluoromethyl, chlorodifluoromethyl and the like. Examples for C₁-C₂-fluoroalkyl are fluoromethyl, difluoromethyl, trifluoromethyl, 1-fluoroethyl, 2-fluoroethyl, 2,2-difluoroethyl, 2,2,2-trifluoroethyl, pentafluoroethyl, and the like. Examples for C₁-C₂-haloalkyl are, apart those mentioned for C₁-C₂-fluoroalkyl, chloromethyl, dichloromethyl, trichloromethyl, bromomethyl, chlorofluoromethyl, dichlorofluoromethyl, chlorodifluoromethyl, 1-chloroethyl, 2-chloroethyl, 2,2,-dichloroethyl, 2,2,2-trichloroethyl, 2-chloro-2-fluoroethyl, 2-chloro-2,2-difluoroethyl, 2,2-dichloro-2-fluoroethyl, 1-bromoethyl, and the like. Examples for C₁-C₄-haloalkyl are, apart those mentioned for C₁-C₂-haloalkyl, 1-fluoropropyl, 2-fluoropropyl, 3-fluoropropyl, 3,3-difluoropropyl, 3,3,3-trifluoropropyl, heptafluoropropyl, 1,1,1-trifluoroprop-2-yl, 3-chloropropyl, 4-chlorobutyl and the like.

The term "cycloalkyl" as used herein (and in the cycloalkyl moieties of other groups comprising a cycloalkyl group, e.g. cycloalkoxy and cycloalkylalkyl) denotes in each case a mono- or bicyclic cycloaliphatic radical having usually from 3 to 10 carbon atoms ("C₃-C₁₀-cycloalkyl"), preferably 3 to 7 carbon atoms ("C₃-C₇-CyCloalkyl") or in particular 3 to 6 carbon atoms ("C₃-C₆-cycloalkyl"). Examples of monocyclic radicals having 3 to 6 carbon atoms comprise cyclopropyl, cyclobutyl, cyclopentyl and cyclohexyl. Examples of monocyclic radicals having 3 to 7 carbon atoms comprise cyclopropyl, cyclobutyl, cyclopentyl, cyclohexyl and cycloheptyl. Examples of bicyclic radicals having 7 or 8 carbon atoms comprise bicyclo[2.1.1]hexyl, bicyclo[2.2.1]heptyl, bicyclo[3.1.1]heptyl, bicyclo[2.2.1]heptyl, bicyclo[2.2.2]octyl and bicyclo[3.2.1]octyl.

The term "halocycloalkyl" as used herein (and in the halocycloalkyl moieties of other groups comprising an halocycloalkyl group, e.g. halocycloalkylmethyl) denotes in each case a mono- or bicyclic cycloaliphatic radical having usually from 3 to 10 carbon atoms, preferably 3 to 7 carbon atoms or in particular 3 to 6 carbon atoms, wherein at least one, e.g. 1, 2, 3, 4 or 5 of the hydrogen atoms are replaced by halogen, in particular by fluorine or chlorine. Examples are 1- and 2- fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "cycloalkyl-alkyl" used herein denotes a cycloalkyl group, as defined above, which is bound to the remainder of the molecule via an alkylene group. The term "C₃-C₇-cycloalkyl-C₁-C₄-alkyl" refers to a C₃-C₇-Cycloalkyl group as defined above which is bound to the remainder of the molecule via a C₁-C₄-alkyl group, as defined above. Examples are cyclopropylmethyl, cyclopropylethyl, cyclopropylpropyl, cyclobutylmethyl, cyclobutylethyl, cyclobutylpropyl, cyclopentylmethyl, cyclopentylethyl, cyclopentylpropyl, cyclohexylmethyl, cyclohexylethyl, cyclohexylpropyl, and the like.

The term "alkenyl" as used herein denotes in each case a monounsaturated straight-chain or branched hydrocarbon radical having usually 2 to 8 ("C₂-C₈-alkenyl"), preferably 2 to 6 carbon atoms ("C₂-C₆-alkenyl"), in particular 2 to 4 carbon atoms ("C₂-C₄-alkenyl"), and a double bond in any position, for example C₂-C₄-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl; C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and the like, or C₂-C₈-alkenyl, such as the radicals mentioned for C₂-C₆-alkenyl and additionally 1-heptenyl, 2-heptenyl, 3-heptenyl, 1-octenyl, 2-octenyl, 3-octenyl, 4-octenyl and the positional isomers thereof.

The term "haloalkenyl" as used herein, which may also be expressed as "alkenyl which is substituted by halogen", and the haloalkenyl moieties in haloalkenyloxy and the like refers to unsaturated straight-chain or branched hydrocarbon radicals having 2 to 8 ("C₂-C₈-haloalkenyl") or 2 to 6 ("C₂-C₆-haloalkenyl") or 2 to 4 ("C₂-C₄-haloalkenyl") carbon atoms and a double bond in any position, where some or all of the hydrogen atoms in these groups are replaced by halogen atoms as mentioned above, in particular fluorine, chlorine and bromine, for example chlorovinyl, chloroallyl and the like.

The term "alkynyl" as used herein denotes unsaturated straight-chain or branched hydrocarbon radicals having usually 2 to 8 ("C₂-C₈-alkynyl"), frequently 2 to 6 ("C₂-C₆-alkynyl"), preferably 2 to 4 carbon atoms ("C₂-C₄-alkynyl") and a triple bond in any position, for example C₂-C₄-alkynyl, such as ethynyl, 1-propynyl, 2-propynyl, 1-butynyl, 2-butynyl, 3-butynyl, 1-methyl-2-propynyl and the like, C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl and the like.

The term "haloalkynyl" as used herein, which is also expressed as "alkynyl which is substituted by halogen", refers to unsaturated straight-chain or branched hydrocarbon radicals having usually 3 to 8 carbon atoms ("C₃-C₈-haloalkynyl"), frequently 3 to 6 ("C₃-C₆-haloalkynyl"), preferabyl 3 to 4 carbon atoms ("C₃-C₄-haloalkynyl"), and a triple bond in any position (as mentioned above), where some or all of the hydrogen atoms in these groups are replaced by halogen atoms as mentioned above, in particular fluorine, chlorine and bromine.

The term "alkoxy" as used herein denotes in each case a straight-chain or branched alkyl group usually having from 1 to 8 carbon atoms ("C₁-C₈-alkoxy"), frequently from 1 to 6 carbon atoms ("C₁-C₆-alkoxy"), preferably 1 to 4 carbon atoms ("C₁-C₄-alkoxy"), which is bound to the remainder of the molecule via an oxygen atom. C₁-C₂-Alkoxy is methoxy or ethoxy. C₁-C₄-Alkoxy is additionally, for example, n-propoxy, 1-methylethoxy (isopropoxy), butoxy, 1-methylpropoxy (sec-butoxy), 2-methylpropoxy (isobutoxy) or 1,1-dimethylethoxy (tert-butoxy). C₁-C₆-Alkoxy is additionally, for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy. C₁-C₈-Alkoxy is additionally, for example, heptyloxy, octyloxy, 2-ethylhexyloxy and positional isomers thereof.

The term "haloalkoxy" as used herein denotes in each case a straight-chain or branched alkoxy group, as defined above, having from 1 to 8 carbon atoms ("C₁-C₈-haloalkoxy"), frequently from 1 to 6 carbon atoms ("C₁-C₆-haloalkoxy"), preferably 1 to 4 carbon atoms ("C₁-C₄-haloalkoxy"), more preferably 1 to 3 carbon atoms ("C₁-C₃-haloalkoxy"), wherein the hydrogen atoms of this group are partially or totally replaced with halogen atoms, in particular fluorine atoms. C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F₅. C₁-C₄-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F5, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy. C₁-C₆-Haloalkoxy is additionally, for example, 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "alkoxyalkyl" as used herein denotes in each case alkyl usually comprising 1 to 6 carbon atoms, preferably 1 to 4 carbon atoms, wherein 1 carbon atom carries an alkoxy radical usually comprising 1 to 8, frequently 1 to 6, in particular 1 to 4, carbon atoms as defined above. "C₁-C₆-alkoxy-C₁-C₆-alkyl" is a C₁-C₆-alkyl group, as defined above, in which one hydrogen atom is replaced by a C₁-C₆-alkoxy group, as defined above. Examples are CH₂OCH₃, CH₂-OC₂H5, n-propoxymethyl, CH₂-OCH(CH₃)₂, n-butoxymethyl, (1-methylpropoxy)-methyl, (2-methylpropoxy)methyl, CH₂-OC(CH₃)₃, 2-(methoxy)ethyl, 2-(ethoxy)ethyl, 2-(n-propoxy)-ethyl, 2-(1-methylethoxy)-ethyl, 2-(n-butoxy)ethyl, 2-(1-methylpropoxy)-ethyl, 2-(2-methylpropoxy)-ethyl, 2-(1,1-dimethylethoxy)-ethyl, 2-(methoxy)-propyl, 2-(ethoxy)-propyl, 2-(n-propoxy)-propyl, 2-(1-methylethoxy)-propyl, 2-(n-butoxy)-propyl, 2-(1-methylpropoxy)-propyl, 2-(2-methylpropoxy)-propyl, 2-(1,1-dimethylethoxy)-propyl, 3-(methoxy)-propyl, 3-(ethoxy)-propyl, 3-(n-propoxy)-propyl, 3-(1-methylethoxy)-propyl, 3-(n-butoxy)-propyl, 3-(1-methylpropoxy)-propyl, 3-(2-methylpropoxy)-propyl, 3-(1,1-dimethylethoxy)-propyl, 2-(methoxy)-butyl, 2-(ethoxy)-butyl, 2-(n-propoxy)-butyl, 2-(1-methylethoxy)-butyl, 2-(n-butoxy)-butyl, 2-(1-methylpropoxy)-butyl, 2-(2-methyl-propoxy)-butyl, 2-(1,1-dimethylethoxy)-butyl, 3-(methoxy)-butyl, 3-(ethoxy)-butyl, 3-(n-propoxy)-butyl, 3-(1-methylethoxy)-butyl, 3-(n-butoxy)-butyl, 3-(1-methylpropoxy)-butyl, 3-(2-methylpropoxy)-butyl, 3-(1,1-dimethylethoxy)-butyl, 4-(methoxy)-butyl, 4-(ethoxy)-butyl, 4-(n-propoxy)-butyl, 4-(1-methylethoxy)-butyl, 4-(n-butoxy)-butyl, 4-(1-methylpropoxy)-butyl, 4-(2-methylpropoxy)-butyl, 4-(1,1-dimethylethoxy)-butyl and the like.

The term "haloalkoxy-alkyl" as used herein denotes in each case alkyl as defined above, usually comprising 1 to 6 carbon atoms, preferably 1 to 4 carbon atoms, wherein 1 carbon atom carries an haloalkoxy radical as defined above, usually comprising 1 to 8, frequently 1 to 6, in particular 1 to 4, carbon atoms as defined above. Examples are fluoromethoxymethyl, difluoromethoxymethyl, trifluoromethoxymethyl, 1-fluoroethoxymethyl, 2-fluoroethoxymethyl, 1,1-difluoroethoxymethyl, 1,2-difluoroethoxymethyl, 2,2-difluoroethoxymethyl, 1,1,2-trifluoroethoxymethyl, 1,2,2-trifluoroethoxymethyl, 2,2,2-trifluoroethoxymethyl, pentafluoroethoxymethyl, 1-fluoroethoxy-1-ethyl, 2-fluoroethoxy-1-ethyl, 1,1-difluoroethoxy-1-ethyl, 1,2-difluoroethoxy-1-ethyl, 2,2-difluoroethoxy-1-ethyl, 1,1,2-trifluoroethoxy-1-ethyl, 1,2,2-trifluoroethoxy-1-ethyl, 2,2,2-trifluoroethoxy-1-ethyl, pentafluoroethoxy-1-ethyl, 1-fluoroethoxy-2-ethyl, 2-fluoroethoxy-2-ethyl, 1,1-difluoroethoxy-2-ethyl, 1,2-difluoroethoxy-2-ethyl, 2,2-difluoroethoxy-2-ethyl, 1,1,2-trifluoroethoxy-2-ethyl, 1,2,2-trifluoroethoxy-2-ethyl, 2,2,2-trifluoroethoxy-2-ethyl, pentafluoroethoxy-2-ethyl, and the like.

The term "alkylthio" (also alkylsulfanyl, "alkyl-S" or "alkyl-S(O)ₖ" (wherein k is 0)) as used herein denotes in each case a straight-chain or branched saturated alkyl group as defined above, usually comprising 1 to 8 carbon atoms ("C₁-C₈-alkylthio"), frequently comprising 1 to 6 carbon atoms ("C₁-C₆-alkylthio"), preferably 1 to 4 carbon atoms ("C₁-C₄-alkylthio"), which is attached via a sulfur atom at any position in the alkyl group. C₁-C₂-Alkylthio is methylthio or ethylthio. C₁-C₄-Alkylthio is additionally, for example, n-propylthio, 1-methylethylthio (isopropylthio), butylthio, 1-methylpropylthio (sec-butylthio), 2-methylpropylthio (isobutylthio) or 1,1-dimethylethylthio (tert-butylthio). C₁-C₆-Alkylthio is additionally, for example, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio or 1-ethyl-2-methylpropylthio. C₁-C₈-Alkylthio is additionally, for example, heptylthio, octylthio, 2-ethylhexylthio and positional isomers thereof.

The term "haloalkylthio" as used herein refers to an alkylthio group as defined above wherein the hydrogen atoms are partially or completely substituted by fluorine, chlorine, bromine and/or iodine. C₁-C₂-Haloalkylthio is, for example, SCH₂F, SCHF₂, SCF₃, SCH₂Cl, SCHCl₂, SCCl₃, chlorofluoromethylthio, dichlorofluoromethylthio, chlorodifluoromethylthio, 2-fluoroethylthio, 2-chloroethylthio, 2-bromoethylthio, 2-iodoethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio or SC₂F₅. C₁-C₄-Haloalkylthio is additionally, for example, 2-fluoropropylthio, 3-fluoropropylthio, 2,2-difluoropropylthio, 2,3-difluoropropylthio, 2-chloropropylthio, 3-chloropropylthio, 2,3-dichloropropylthio, 2-bromopropylthio, 3-bromopropylthio, 3,3,3-trifluoropropylthio, 3,3,3-trichloropropylthio, SCH₂-C₂F₅, SCF₂-C₂F₅, 1-(CH₂F)-2-fluoroethylthio, 1-(CH₂Cl)-2-chloroethylthio, 1-(CH₂Br)-2-bromoethylthio, 4-fluorobutylthio, 4-chlorobutylthio, 4-bromobutylthio or nonafluorobutylthio. C₁-C₆-Haloalkylthio is additionally, for example, 5-fluoropentylthio, 5-chloropentylthio, 5-brompentylthio, 5-iodopentylthio, undecafluoropentylthio, 6-fluorohexylthio, 6-chlorohexylthio, 6-bromohexylthio, 6-iodohexylthio or dodecafluorohexylthio.

The terms "alkylsulfinyl" and "alkyl-S(O)ₖ" (wherein k is 1) are equivalent and, as used herein, denote an alkyl group, as defined above, attached via a sulfinyl [S(O)] group. For example, the term "C₁-C₂-alkylsulfinyl" refers to a C₁-C₂-alkyl group, as defined above, attached via a sulfinyl [S(O)] group. The term "C₁-C₄-alkylsulfinyl" refers to a C₁-C₄-alkyl group, as defined above, attached via a sulfinyl [S(O)] group. The term "C₁-C₆-alkylsulfinyl" refers to a C₁-C₆-alkyl group, as defined above, attached via a sulfinyl [S(O)] group. C₁-C₂-alkylsulfinyl is methylsulfinyl or ethylsulfinyl. C₁-C₄-alkylsulfinyl is additionally, for example, n-propylsulfinyl, 1-methylethylsulfinyl (isopropylsulfinyl), butylsulfinyl, 1-methylpropylsulfinyl (sec-butylsulfinyl), 2-methylpropylsulfinyl (isobutylsulfinyl) or 1,1-dimethylethylsulfinyl (tert-butylsulfinyl). C₁-C₆-alkylsulfinyl is additionally, for example, pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl or 1-ethyl-2-methylpropylsulfinyl.

The terms "alkylsulfonyl" and "alkyl-S(O)ₖ" (wherein k is 2) are equivalent and, as used herein, denote an alkyl group, as defined above, attached via a sulfonyl [S(O)₂] group. The term "C₁-C₂-alkylsulfonyl" refers to a C₁-C₂-alkyl group, as defined above, attached via a sulfonyl [S(O)₂] group. The term "C₁-C₄-alkylsulfonyl" refers to a C₁-C₄-alkyl group, as defined above, attached via a sulfonyl [S(O)₂] group. The term "C₁-C₆-alkylsulfonyl" refers to a C₁-C₆-alkyl group, as defined above, attached via a sulfonyl [S(O)₂] group. C₁-C₂-alkylsulfonyl is methylsulfonyl or ethylsulfonyl. C₁-C₄-alkylsulfonyl is additionally, for example, n-propylsulfonyl, 1-methylethylsulfonyl (isopropylsulfonyl), butylsulfonyl, 1-methylpropylsulfonyl (sec-butylsulfonyl), 2-methylpropylsulfonyl (isobutylsulfonyl) or 1,1-dimethylethylsulfonyl (tert-butylsulfonyl). C₁-C₆-alkylsulfonyl is additionally, for example, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1 ,2,2-trimethylpropylsulfonyl, 1-ethyl-1-methylpropylsulfonyl or 1-ethyl-2-methylpropylsulfonyl.

The term "alkylamino" as used herein denotes in each case a group R*HN-, wherein R* is a straight-chain or branched alkyl group usually having from 1 to 6 carbon atoms ("C₁-C₆-alkylamino"), preferably 1 to 4 carbon atoms("C₁-C₄-alkylamino"). Examples of C₁-C₆-alkylamino are methylamino, ethylamino, n-propylamino, isopropylamino, n-butylamino, 2-butylamino, isobutylamino, tert-butylamino, and the like.

The term "dialkylamino" as used herein denotes in each case a group R*R°N-, wherein R* and R°, independently of each other, are a straight-chain or branched alkyl group each usually having from 1 to 6 carbon atoms ("di-(C₁-C₆-alkyl)-amino"), preferably 1 to 4 carbon atoms ("di-(C₁-C₄-alkyl)-amino"). Examples of a di-(C₁-C₆-alkyl)-amino group are dimethylamino, diethylamino, dipropylamino, dibutylamino, methyl-ethyl-amino, methyl-propyl-amino, methyl-isopropylamino, methyl-butyl-amino, methyl-isobutyl-amino, ethyl-propyl-amino, ethyl-isopropylamino, ethyl-butyl-amino, ethyl-isobutyl-amino, and the like.

The suffix "-carbonyl" in a group denotes in each case that the group is bound to the remainder of the molecule via a carbonyl C=O group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl.

The term "aryl" as used herein refers to a mono-, bi- or tricyclic aromatic hydrocarbon radical such as phenyl or naphthyl, in particular phenyl.

The term "het(ero)aryl" as used herein refers to a mono-, bi- or tricyclic heteroaromatic hydrocarbon radical, preferably to a monocyclic heteroaromatic radical, such as pyridyl, pyrimidyl and the like.

The term "3-, 4-, 5- or 6-membered monocyclic or 8-, 9- or 10-membered bicyclic saturated, unsaturated or aromatic heterocycle containing 1, 2, 3 or 4 heteroatoms as ring members selected from the groups consisting of N, O and S" as used herein denotes monocyclic or bicyclic radicals, the monocyclic or bicyclic radicals being saturated, unsaturated or aromatic where N can optionally be oxidized, i.e. in the form of an N-oxide, and S can also optionally be oxidized to various oxidation states, i.e. as SO or SO₂. An unsaturated heterocycle contains at least one C-C and/or C-N and/or N-N double bond(s). A fully unsaturated heterocycle contains as many conjugated C-C and/or C-N and/or N-N double bonds as allowed by the size(s) of the ring(s). An aromatic monocyclic heterocycle is a fully unsaturated 5- or 6-membered monocyclic heterocycle. An aromatic bicyclic heterocycle is an 8-, 9- or 10-membered bicyclic heterocycle consisting of a 5- or 6-membered heteroaromatic ring which is fused to a phenyl ring or to another 5- or 6-membered heteroaromatic ring. The heterocycle may be attached to the remainder of the molecule via a carbon ring member or via a nitrogen ring member. As a matter of course, the heterocyclic ring contains at least one carbon ring atom. If the ring contains more than one O ring atom, these are not adjacent.

Examples of a 3-, 4-, 5- or 6-membered monocyclic saturated heterocycle include: oxirane-2-yl, aziridine-1-yl, aziridine-2-yl, oxetan-2-yl, azetidine-1-yl, azetidine-2-yl, azetidine-3-yl, thietane-1-yl, thietan-2-yl, thietane-3-yl, tetrahydrofuran-2-yl, tetrahydrofuran-3-yl, tetrahydrothien-2-yl, tetrahydrothien-3-yl, pyrrolidin-1-yl, pyrrolidin-2-yl, pyrrolidin-3-yl, pyrazolidin-1-yl, pyrazolidin-3-yl, pyrazolidin-4-yl, pyrazolidin-5-yl, imidazolidin-1-yl, imidazolidin-2-yl, imidazolidin-4-yl, oxazolidin-2-yl, oxazolidin-3-yl, oxazolidin-4-yl, oxazolidin-5-yl, isoxazolidin-2-yl, isoxazolidin-3-yl, isoxazolidin-4-yl, isoxazolidin-5-yl, thiazolidin-2-yl, thiazolidin-3-yl, thiazolidin-4-yl, thiazolidin-5-yl, isothiazolidin-2-yl, isothiazolidin-3-yl, isothiazolidin-4-yl, isothiazolidin-5-yl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-1-yl, 1,3,4-triazolidin-2-yl, 2-tetrahydropyranyl, 4-tetrahydropyranyl, 1,3-dioxan-5-yl, 1,4-dioxan-2-yl, piperidin-1-yl, piperidin-2-yl, piperidin-3-yl, piperidin-4-yl, hexahydropyridazin-3-yl, hexahydropyridazin-4-yl, hexahydropyrimidin-2-yl, hexahydropyrimidin-4-yl, hexahydropyrimidin-5-yl, piperazin-1-yl, piperazin-2-yl, 1,3,5-hexahydrotriazin-1-yl, 1,3,5-hexahydrotriazin-2-yl and 1,2,4-hexahydrotriazin-3-yl, morpholin-2-yl, morpholin-3-yl, morpholin-4-yl, thiomorpholin-2-yl, thiomorpholin-3-yl, thiomorpholin-4-yl, 1-oxothiomorpholin-2-yl, 1-oxothiomorpholin-3-yl, 1-oxothiomorpholin-4-yl, 1,1-dioxothiomorpholin-2-yl, 1,1-dioxothiomorpholin-3-yl, 1,1-dioxothiomorpholin-4-yl and the like.

Examples of a 5- or 6-membered monocyclic partially unsaturated heterocycle include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1 -yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl and 1,2,4-di- or tetrahydrotriazin-3-yl.

A 5- or 6-membered monocyclic fully unsaturated (including aromatic) heterocyclic ring is e.g. a 5- or 6-membered monocyclic fully unsaturated (including aromatic) heterocyclic ring. Examples are: 2-furyl, 3-furyl, 2-thienyl, 3-thienyl, 1-pyrrolyl, 2-pyrrolyl, 3-pyrrolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 1-imidazolyl, 2-imidazolyl, 4-imidazolyl, 1,3,4-triazol-1-yl, 1,3,4-triazol-2-yl, 2-pyridinyl, 3-pyridinyl, 4-pyridinyl, 1-oxopyridin-2-yl, 1-oxopyridin-3-yl, 1-oxopyridin-4-yl,3-pyridazinyl, 4-pyridazinyl, 2-pyrimidinyl, 4-pyrimidinyl, 5-pyrimidinyl and 2-pyrazinyl.

Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like.

If two radicals bound on the same nitrogen atom (for example R^{2c} and R^{2d} or R^{2e} and R^{2f} or R^{2g} and R^{2h}) together with the nitrogen atom, to which they are bound, form a 5-, 6 or 7-membered, saturated or unsaturated N-bound heterocyclic radical, which may carry as a ring member a further heteroatom selected from O, S and N, this is for example pyrrolidine-1-yl, pyrazolidin-1-yl, imidazolidin-1-yl, oxazolidin-3-yl, thiazolidin-3-yl, isoxazolidin-2-yl, isothiazolin-2-yl, [1,2,3]-triazolidin-1-yl, [1,2,3]-triazolidin-2-yl, [1,2,4]-triazolidin-1-yl, [1,2,4]-triazolidin-4-yl, [1,2,3]-oxadiazolidin-2-yl, [1,2,3]-oxadiazolidin-3-yl, [1,2,5]-oxadiazolidin-2-yl, [1,2,4]-oxadiazolidin-2-yl, [1,2,4]-oxadiazolidin-4-yl, [1,3,4]-oxadiazolidin-3-yl, [1,2,3]-thiadiazolidin-2-yl, [1,2,3]- thiadiazolidin-3-yl, [1,2,5]-thiadiazolidin-2-yl, [1,2,4]-thiadiazolidin-2-yl, [1,2,4]-thiadiazolidin-4-yl, [1,3,4]-thiadiazolidin-3-yl, piperdin-1-yl, piperazine-1-yl, morpholin-1-yl, thiomorpholin-1-yl, 1-oxothiomorpholin-1-yl, 1,1-dioxothiomorpholin-1-yl, azepan-1-yl, 1,4-diazepan-1-yl, pyrrolin-1-yl, pyrazolin-1-yl, imidazolin-1-yl, oxazolin-3-yl, isoxazolin-2-yl, thiazolin-3-yl, isothiazolin-1-yl, 1,2-dihydropyridin-1-yl, 1,2,3,4-tetrahydropyridin-1-yl, 1,2,5,6-tetrahydropyridin-1-yl, 1,2-dihydropyridazin, 1,6-dihydropyridazin, 1,2,3,4-tetrahydropyridazin-1-yl, 1,2,5,6-tetrahydropyridazin-1 -yl, 1,2-dihydropyrimidin, 1,6-dihydropyrimidin, 1,2,3,4-tetrahydropyrimidin-1-yl, 1,2,5,6-tetrahydropyrimidin-1-yl, 1,2-dihydropyrazin-1-yl, 1,2,3,4-tetrahydropyrazin-1-yl, 1,2,5,6-tetrahydropyrazin-1-yl, pyrrol-1-yl, pyrazol-1-yl, imidazol-1-yl, [1,2,3]-1H-triazol-1-yl, [1,2,3]-2H-triazol-2-yl, [1,2,4]-1H-triazol-1-yl and [1,2,4]-4H-triazol-4-yl.

The remarks made below as to preferred embodiments of the variables (substituents) of the compounds of formula I are valid on their own as well as preferably in combination with each other, as well as in combination with the stereoisomers, salts, or tautomers thereof.

The remarks made below concerning preferred embodiments of the variables further are valid on their own as well as preferably in combination with each other concerning the compounds of formulae I, where applicable, as well as concerning the uses and methods according to the invention and the composition according to the invention.

Preferred compounds according to the invention are compounds of formula I or a stereoisomer, or salt thereof, wherein the salt is an agriculturally suitable salt. Particularly preferred compounds according to the invention are compounds of formula I or a salt thereof, especially an agriculturally suitable salt thereof.

According to a first group 1 of embodiments of the invention the variable Q in the compounds of formula I is Q¹:

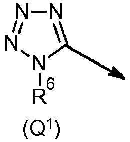

Herein, the arrow represents the binding site of the variable Q¹ conjugated to the remaining part of the compound of formula I. Compounds of formula I wherein Q is Q¹ have the following formula I.A, where the variables R¹, R², R³, R⁴ and R⁶ are as defined herein:

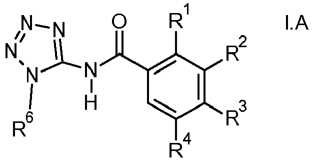

According to a second group 2 of embodiments of the invention the variable Q in the compounds of formula I is Q²:

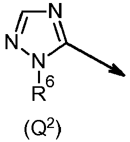

Herein, the arrow represents the binding site of the variable Q² conjugated to the remaining part of the compound of formula I. Compounds of formula I wherein Q is Q² have the following formula I.B, where the variables R¹, R², R³, R⁴ and R⁶ are as defined herein:

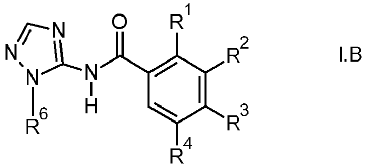

According to a third group 2 of embodiments of the invention the variable Q in the compounds of formula I is Q³:

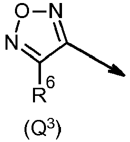

Herein, the arrow represents the binding site of the variable Q³ conjugated to the remaining part of the compound of formula I. Compounds of formula I wherein Q is Q³ have the following formula I.C, where the variables R¹, R², R³, R⁴ and R⁶ are as defined herein:

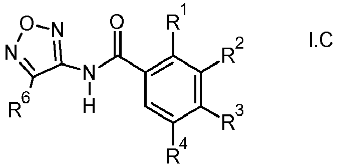

According to a fourth group 4 of embodiments of the invention the variable Q in the compounds of formula I is Q⁴:

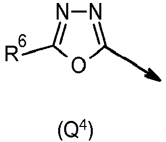

Herein, the arrow represents the binding site of the variable Q⁴ conjugated to the remaining part of the compound of formula I. Compounds of formula I wherein Q is Q⁴ have the following formula I.D, where the variables R¹, R², R³, R⁴ and R⁶ are as defined herein:

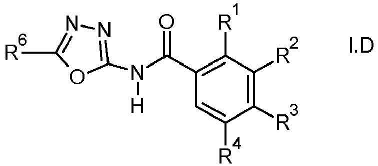

Preferred compounds according to the invention are compounds of formulae I, I.A, I.B, I.C and I.D and the agriculturally suitable salts thereof, wherein R¹ is selected from the group consisting of cyano, halogen, nitro, C₁-C₆-alkyl, C₁-C₆-haloalkyl, C₁-C₄-alkoxy-C₁-C₄-alkyl, C₁-C₄-haloalkoxy-C₁-C₄-alkyl, C₁-C₆-alkoxy, C₁-C₄-alkoxy-C₁-C₄-alkoxy-Z¹, R^{1b}-S(O)ₖ-Z¹, where k and Z¹ are as defined herein and where R^{1b} is as defined above and in particular selected from the group consisting of C₁-C₄-alkyl and C₁-C₄-haloalkyl. In this context Z¹ is in particular a covalent bond.

More preferably, R¹ is selected from halogen, nitro, C₁-C₄-alkyl, C₁-C₄-alkoxy-C₁-C₄-alkoxy-Z¹ and R^{1b}-S(O)ₖ-Z¹, where k and Z¹ are as defined herein and where R^{1b} is as defined above and in particular selected from the group consisting of C₁-C₄-alkyl and C₁-C₄-haloalkyl. In this context Z¹ is in particular a covalent bond.

In particular, R¹ is selected from the group consisting of halogen, nitro, C₁-C₄-alkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, with preference given to R¹ being selected from the group consisting of halogen and C₁-C₄-alkyl. Specifically R¹ is F, Cl, Br, I, nitro, CH₃, SCH₃, SCF₃, SO₂CH₃ or CH₂OCH₂CH₂OCH₃, and more specifically R¹ is F, Cl, Br, I, nitro, CH₃, SCH₃ or SO₂CH₃.

Especially, R¹ in formulae I, I.A, I.B, I.C and I.Di s CH₃ or Cl.

According to the present invention, R² is R^{2c}R^{2d}NC(O)NR²ⁿ-Z².

A particular group 5 of embodiments according to the invention are compounds of formula I and the agriculturally suitable salts thereof, and likeweise the compounds of formulae I.A, I.B, I.C and I.D and the agriculturally suitable salts thereof, wherein in R², Z² is C₁-C₄-alkanediyl; preferably, Z² is -CH₂- or -CH₂CH₂-, i.e. R² is R^{2c}R^{2d}NC(O)NR²ⁿCH₂- or R^{2c}R^{2d}NC(O)NR²"CH₂CH₂-;
According to another preferred embodiment, Z² is a covalent bond, i.e. R² is R^{2c}R^{2d}NC(O)NR²ⁿ-.

According to a preferred group 6 of embodiments, Z² in formulae I, I.A, I.B, I.C and I.D is a covalent bond and R²ⁿ is selected from the group consisting of hydrogen and C₁-C₆-alkyl.

According to an even more group 7 of embodiments, Z² in formulae I, I.A, I.B, I.C and I.D is a covalent bond and R²ⁿ is hydrogen, i.e. R² is R^{2c}R^{2d}NC(O)NH-.

In the context of R², and likewise in the context of groups 1, 2, 3, 4, 5, 6, and 7 of embodiments, R^{2c} is preferably selected from the group consisting of C₁-C₆-alkyl, C₃-C₇-CyCloalkyl, C₃-C₇-CyCloalkyl-C₁-C₄-alkyl, C₁-C₆-haloalkyl, C₁-C₆-alkoxy, phenyl, and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
More preferably, R^{2c} is selected from the group consisting of C₁-C₄-alkyl, C₃-C₇-CyCloalkyl, C₁-C₄-haloalkyl, C₁-C₄-alkoxy, and phenyl, where phenyl is unsubstituted or substituted by 1 or 2 groups, which are identical or different and selected from the group consisting of halogen and C₁-C₄-alkoxy;
Even more preferably, R^{2c} is selected from the group consisting of C₁-C₄-alkyl, C₃-C₇-cycloalkyl, and C₁-C₄-haloalkyl.

Herein, particularly preferably, R^{2c} is selected from the group consisting of methyl, ethyl, isopropyl (iPr), cyclopropyl (cPr), CHF₂CH₂-, CF₃CH₂-, CH₃O-, 4-Cl-phenyl, 4-CH₃O-phenyl, and phenyl, or R^{2c} is selected from the group consisting of methyl, ethyl, isopropyl (iPr), cyclopropyl (cPr), CHF₂CH₂- and CF₃CH₂-.

In the context of R², and likewise in the context of groups 1, 2, 3, 4, 5, 6, and 7 of embodiments, R^{2d} is preferably selected from the group consisting of C₁-C₆-alkyl, C₃-C₇-CyCloalkyl, C₃-C₇-cycloalkyl-C₁-C₄-alkyl, C₁-C₆-haloalkyl and phenyl where phenyl is unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
more preferably, R^{2d} is selected from the group consisting of C₁-C₆-alkyl, C₃-C₇-CyCloalkyl, C₃-C₇-cycloalkyl-C₁-C₂-alkyl and C₁-C₆-haloalkyl, in particular selected from the group consisting of C₁-C₄-alkyl, C₃-C₇-CyCloalkyl, and C₁-C₄-haloalkyl;
Herein, very particularly preferably, R^{2d} is methyl, ethyl, isopropyl (iPr), cyclopropyl (cPr), CHF₂CH₂-, or CF₃CH₂-.

In the context of R², and likewise in the context of groups 1, 2, 3, 4, 5, 6, and 7 of embodiments, the combination of R^{2c} and R^{2d} is preferably as follows:
R^{2c} is selected from the group consisting of C₁-C₄-alkyl, C₃-C₇-CyCloalkyl and C₁-C₄-haloalkyl;
R^{2d} is selected from the group consisting of C₁-C₆-alkyl, C₃-C₇-CyCloalkyl, C₃-C₇-CyCloalkyl-C₁-C₂-alkyl and C₁-C₆-haloalkyl.

In the context of R², and likewise in the context of groups 1, 2, 3, 4, 5, 6, and 7 of embodiments, the combination of R^{2c} and R^{2d} is even more preferably as follows:
R^{2c} is selected from the group consisting of C₁-C₄-alkyl, C₃-C₇-CyCloalkyl, and C₁-C₄-haloalkyl, with particular preference given to R^{2c} being methyl, ethyl, isopropyl (iPr), cyclopropyl (cPr), CHF₂CH₂-, or CF₃CH₂-; and
R^{2d} is selected from the group consisting of C₁-C₄-alkyl, C₃-C₇-CyCloalkyl and C₁-C₄-haloalkyl, with particular preference given to R^{2d} being methyl, ethyl, isopropyl (iPr), cyclopropyl (cPr), CHF₂CH₂-, or CF₃CH₂-.

In the context of R², and likewise in the context of groups 1 2, 3, 4, 5, 6, and 7 of embodiments, the combination of R^{2c} and R^{2d} is also preferably as follows:
R^{2c} is selected from the group consisting of C₁-C₄-alkyl, C₃-C₇-CyCloalkyl, and C₁-C₄-haloalkyl, with particular preference given to R^{2c} being methyl, ethyl, isopropyl (iPr), cyclopropyl (cPr), CHF₂CH₂-, or CF₃CH₂-; and
R^{2d} is selected from the group consisting of C₃-C₇-CyCloalkyl and C₁-C₄-haloalkyl, with particular preference given to R^{2d} being cyclopropyl (cPr), CHF₂CH₂-, or CF₃CH₂-.

In the context of R², and likewise in the context of groups 1, 2, 3, 4, 5, 6, and 7 of embodiments, R^{2c} and R^{2d} together with the nitrogen atom, to which they are bound may also form a 5- or 6- membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
preferably, R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl.

Preferred compounds according to the invention are compounds of formulae I, I.A, I.B, I.C and I.D and the agriculturally suitable salts thereof, wherein R³ is selected from the group consisting of hydrogen, cyano, halogen, C₁-C₄-alkyl, C₁-C₄-haloalkyl, C₁-C₄-alkoxy, C₁-C₄-haloalkoxy, C₂-C₄-alkenyl, C₂-C₄-alkynyl, C2-C4-alkenyloxy, C2-C4-alkynyloxy or R^{2b}-S(O)k, where the variables k and R^{2b} have one of the herein defined meanings; in particular, R³ is selected from the group consisting of hydrogen, halogen, C₁-C₄-alkyl, C₁-C₄-haloalkyl, C₁-C₄-haloalkoxy, or R^{2b}-S(O)ₖ, where the variables k and R^{2b} have one of the herein defined meanings.

More preferably, R³ is selected from the group consisting of hydrogen, halogen, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂.

In particular, R³ is selected from the group consisting of hydrogen, halogen, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂, specifically from H, Cl, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃, and more specifically from H, Cl, Br, I, CF₃, SCH₃, S(O)CH₃ and S(O)₂CH₃.

Especially, R³ is selected from the group consisting of hydrogen, halogen and C₁-C₂-haloalkyl, with particular preference given to those compounds, where R³ is selected from the group consisting of H, Cl, Br and CF₃, with even more preference given to compounds of the formulae I.A and I.D and the agriculturally acceptable salts thereof, where R³ is selected from the group consisting of H, Cl, Br and CF₃.

In special subgroups embodiments 1, 2, 3, 4, 5, 6, 7, R³ in formulae I, I.A, I.B, I.C and I.D is hydrogen. In other special subgroups embodiments 1, 2, 3, 4, 5, 6, 7, R³ in formulae I, I.A, I.B, I.C and I.D is different from hydrogen.

Preferred compounds according to the invention are compounds of formulae I, I.A, I.B, I.C and I.D and the agriculturally suitable salts thereof, wherein R⁴ is selected from the group consisting of cyano, halogen, nitro, C₁-C₂-alkyl, and C₁-C₂-haloalkyl. More preferably, R⁴ is selected from the group consisting of cyano, halogen, nitro, CH₃, CHF₂, and CF₃, in particular from cyano, chlorine, fluorine and CH₃. Most preferred compounds according to the invention are compounds of formula I, wherein R⁴ is chlorine or fluorine.

Preferred compounds according to the invention are compounds of formulae I, I.A, I.B, I.C and I.D and the agriculturally suitable salts thereof, wherein R⁶ is selected from the group consisting of C₁-C₆-alkyl, C₁-C₄-alkoxy-C₁-C₄-alkyl, and phenyl. In particular, R⁶ is selected from the group consisting of C₁-C₂-alkyl, C₁-C₂-alkoxy-C₁-C₂-alkyl, specifically from CH₃, CH₃CH₂, CH₃OCH₂CH₂ and CH₃OCH₂. Especially preferred compounds according to the invention are compounds of formulae I, I.A, I.B, I.C and I.D and the agriculturally suitable salts thereof, where R⁶ is CH₃.

In this context, the variables R²¹, Z¹, Z², Z^{2a}, R^{b}, R^{1b}, R^{2b}, R^{2c}, R^{2d}, R^{2e}, R^{2f}, R^{2g}, R^{2h}, R^{k}, n and k, independently of each other, preferably have one of the following meanings:
R²¹ is selected from halogen, C₁-C₄-alkyl, C₃-C₆-cycloalkyl, C₃-C₆-halocycloalkyl, C₁-C₄-haloalkyl, C₁-C₄-alkoxy, C₁-C₄-alkoxy-C₁-C₄-alkyl, C₁-C₄-alkylthio-C₁-C₄-alkyl, C₁-C₄-alkoxy-C₁-C₄-alkoxy and C₁-C₆-haloalkyloxy, more preferably from halogen, C₁-C₄-alkyl, C₃-C₆-cycloalkyl, C₁-C₄-haloalkyl and C₁-C₄-alkoxy.

More preferably R²¹ is selected from the group consisting of halogen, C₁-C₄-alkyl, C₃-C₆-cycloalkyl, C₁-C₄-haloalkyl, C₁-C₄-alkoxy, C₁-C₄-alkoxy-C₁-C₄-alkyl, C₁-C₄-alkylthio-C₁-C₄-alkyl and C₁-C₄-alkoxy-C₁-C₄-alkoxy; in particular from halogen, C₁-C₄-alkyl, C₁-C₄-alkoxy, C₁-C₄-haloalkyl, C₁-C₄-alkoxy-C₁-C₄-alkyl and C₁-C₄-alkoxy-C₁-C₄-alkoxy; and specifically from Cl, F, Br, methyl, ethyl, methoxy and trifluoromethyl.

Z¹, Z² independently of each other are selected from a covalent bond, methanediyl and ethanediyl, and in particular are a covalent bond.

Z^{2a} is selected from a covalent bond, C₁-C₂-alkanediyl, O-C₁-C₂-alkanediyl, C₁-C₂-alkanediyl-O and C₁-C₂-alkanediyl-O-C₁-C₂-alkanediyl; more preferably from a covalent bond, methanediyl, ethanediyl, O-methanediyl, O-ethanediyl, methanediyl-O, and ethanediyl-O; and in particular from a covalent bond, methanediyl and ethanediyl.

R^{b}, R^{1b}, R^{2b} independently of each other are selected from C₁-C₆-alkyl, C₃-C₇-CyCloalkyl, C₁-C₆-haloalkyl, C₂-C₆-alkenyl, C₂-C₆-haloalkenyl, C₂-C₆-alkynyl, C₂-C₆-haloalkynyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S, where phenyl and heterocyclyl are unsubstituted or substituted by 1, 2 or 3 groups, which are identical or different and selected from the group consisting of halogen, C₁-C₄-alkyl, C₁-C₂-haloalkyl and C₁-C₂-alkoxy.

More preferably R^{b}, R^{1b}, R^{2b} independently of each other are selected from the group consisting of C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S.

In particular, R^{b}, R^{1b}, R^{2b} independently of each other are selected from C₁-C₄-alkyl, C₁-C₄-haloalkyl, C₂-C₄-alkenyl, C₂-C₄-haloalkenyl, C₂-C₄-alkynyl, C₃-C₆-cycloalkyl, phenyl and heterocyclyl, where heterocyclyl is a 5- or 6-membered aromatic heterocyclic radical having 1 or 2 nitrogen atoms as ring members.

R^{2c}, R^{k} independently of each other are selected from hydrogen, C₁-C₆-alkyl, C₃-C₇-cycloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl, benzyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S, where phenyl, benzyl and heterocyclyl are unsubstituted or substituted by 1, 2 or 3 groups, which are identical or different and selected from the group consisting of halogen, C₁-C₄-alkyl, C₁-C₄-haloalkyl and C₁-C₄-alkoxy.

More preferably R^{2c}, R^{k} independently of each other are selected from hydrogen, C₁-C₄-alkyl, C₁-C₄-haloalkyl, C₂-C₄-alkenyl, C₂-C₄-haloalkenyl, C₂-C₄-alkynyl, C₃-C₆-cycloalkyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S.

In particular, R^{2c}, R^{k} independently of each other are selected from hydrogen, C₁-C₄-alkyl, C₁-C₄-haloalkyl, C₂-C₄-alkenyl, C₂-C₄-haloalkenyl, C₃-C₆-cycloalkyl, phenyl and heterocyclyl, where heterocyclyl is a 5- or 6-membered aromatic heterocyclic radical having 1 or 2 nitrogen atoms as ring members.

R^{2d} is selected from C₁-C₆-alkyl, C₃-C₇-CyCloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl.

More preferably R^{2d} is selected from C₁-C₆-alkyl, C₁-C₆-haloalkyl, C₂-C₆-alkenyl, C₂-C₆-haloalkenyl, C₂-C₆-alkynyl, C₁-C₄-alkoxy-C₁-C₄-alkyl and C₃-C₇-CyCloalkyl, which is unsubstituted or partly or completely halogenated, and in particular selected from C₁-C₄-alkyl, C₁-C₄-haloalkyl, C₂-C₄-alkenyl, C₂-C₄-haloalkenyl, C₂-C₄-alkynyl and C₃-C₆-cycloalkyl.

R^{2e}, R^{2f} independently of each other are selected from the group consisting of hydrogen, C₁-C₆-alkyl, C₃-C₇-CyCloalkyl, which is unsubstituted or partially or completely halogenated, C₁-C₆-haloalkyl, C₂-C₆-alkenyl, C₂-C₆-haloalkenyl, C₁-C₄-alkoxy-C₁-C₄-alkyl, phenyl and benzyl, where phenyl and benzyl are unsubstituted or substituted by 1, 2 or 3 groups, which are identical or different and selected from the group consisting of halogen, C₁-C₄-alkyl, C₁-C₄-haloalkyl and C₁-C₄-alkoxy, or R^{2e} and R^{2f} together with the nitrogen atom, to which they are bound may form a 5-, 6 or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of halogen, C₁-C₄-alkyl, C₁-C₄-haloalkyl and C₁-C₄-alkoxy.

More preferably R^{2e}, R^{2f} independently of each other are selected from hydrogen, C₁-C₆-alkyl, C₁-C₆-haloalkyl and benzyl, or R^{2e} and R^{2f} together with the nitrogen atom, to which they are bound may form a 5- or 6-membered, saturated or unsaturated heterocyclic radical, which may carry as a ring member a further heteroatom selected from O, S and N and which is unsubstituted or may carry 1, 2 or 3 groups, which are identical or different and selected from the group consisting of halogen, C₁-C₄-alkyl and C₁-C₄-haloalkyl.

In particular, R^{2e}, R^{2f} independently of each other are selected from hydrogen and C₁-C₄-alkyl, or R^{2e} and R^{2f} together with the nitrogen atom, to which they are bound may form a 5- or 6-membered, saturated heterocyclic radical, which may carry as a ring member a further heteroatom selected from O, S and N and which is unsubstituted or may carry 1, 2 or 3 methyl groups.

R^{2g} is selected from hydrogen, C₁-C₆-alkyl, C₃-C₇-CyCloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl.

More preferably R^{2g} is selected from hydrogen, C₁-C₆-alkyl, C₁-C₆-haloalkyl, C₂-C₆-alkenyl, C₂-C₆-haloalkenyl, benzyl, C₁-C₄-alkoxy-C₁-C₄-alkyl and C₃-C₇-CyCloalkyl, which is unsubstituted or partly or completely halogenated, and in particular selected from hydrogen, C₁-C₄-alkyl, C₁-C₄-haloalkyl, C₂-C₄-alkenyl, C₂-C₄-haloalkenyl, benzyl and C₃-C₆-cycloalkyl.

R^{2h} is selected from hydrogen, C₁-C₆-alkyl, C₃-C₇-CyCloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl, benzyl and a radical C(=O)-R^{k}, where R^{k} is H, C₁-C₄-alkyl, C₁-C₄-haloalkyl or phenyl.

More preferably R^{2h} is selected from hydrogen, C₁-C₆-alkyl, C₁-C₆-haloalkyl, C₂-C₆-alkenyl, C₂-C₆-haloalkenyl, benzyl, C₁-C₄-alkoxy-C₁-C₄-alkyl and C₃-C₇-CyCloalkyl, which is unsubstituted or partly or completely halogenated, and in particular selected from hydrogen, C₁-C₄-alkyl, C₁-C₄-haloalkyl, C₂-C₄-alkenyl, C₂-C₄-haloalkenyl, benzyl and C₃-C₆-cycloalkyl; or
R^{2g} and R^{2h} together with the nitrogen atom, to which they are bound may form a 5-, 6 or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl and C₁-C₄-haloalkyl and C₁-C₄-alkoxy;
more preferably R^{2g} and R^{2h} together with the nitrogen atom, to which they are bound may form a 5- or 6-membered, saturated or unsaturated heterocyclic radical, which may carry as a ring member a further heteroatom selected from O, S and N and which is unsubstituted or may carry 1, 2 or 3 groups, which are identical or different and selected from the group consisting of halogen, C₁-C₄-alkyl and C₁-C₄-haloalkyl;
and in particular, R^{2g} and R^{2h} together with the nitrogen atom, to which they are bound may form a 5- or 6-membered, saturated heterocyclic radical, which may carry as a ring member a further heteroatom selected from O, S and N and which is unsubstituted or may carry 1, 2 or 3 methyl groups.
n and k independently of each other are 0 or 2, and in particular 2.

Particularly preferred are compounds of formulae I, I.A, I.B, I.C and I.D and the agriculturally suitable salts thereof, wherein the variables R¹ and R³ have the following meanings:
R¹ is selected from the group consisting of halogen, nitro, C₁-C₄-alkyl, C₁-C₄-alkoxy-C₁-C₄-alkoxy-Z¹ and R^{1b}-S(O)ₖ-Z¹, where k and Z¹ are as defined herein and where R^{1b} is as defined above and in particular selected from the group consisting of C₁-C₄-alkyl and C₁-C₄-haloalkyl with preference given to R¹ being selected from the group consisting of halogen and C₁-C₄-alkyl. In this context Z¹ is in particular a covalent bond. In particular, R¹ is selected from F, CI, Br, I, nitro, CH₃, SCH₃, SCF₃, SO₂CH₃, and CH₂OCH₂CH₂OCH₃ with most preference given to CH₃ and Cl; and
R³ is selected from the group consisting of hydrogen, halogen, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂ with preference given to R³ being selected from the group consisting of hydrogen, halogen and C₁-C₂-haloalkyl.

In particular, R³ is selected from H, Cl, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃ with most preference given to H, Cl, Br and CF₃.

Also particularly preferred are compounds of formula I, wherein the variables R¹ and R³ have the following meanings:
R¹ is selected from the group consisting of halogen, nitro, C₁-C₄-alkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, with preference given to R¹ being selected from the group consisting of halogen and C₁-C₄-alkyl; in particular R¹ is selected from the group consisting of F, Cl, Br, I, nitro, CH₃, SCH₃ and SO₂CH₃ with most preference given to CH₃ and Cl; and
R³ is selected from the group consisting of hydrogen, halogen, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ with preference given to R³ being selected from the group consisting of hydrogen, halogen and C₁-C₂-haloalkyl, most particular from H, Cl, Br, I, CF₃, SCH₃, S(O)CH₃ and S(O)₂CH₃ with most preference given to H, Cl, Br and CF₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is selected from the group consisting of halogen, nitro, C₁-C₄-alkyl, C₁-C₄-alkoxy-C₁-C₄-alkoxy-Z¹ and R^{1b}-S(O)ₖ-Z¹, where k and Z¹ are as defined herein and where R^{1b} is as defined above and in particular selected from the group consisting of C₁-C₄-alkyl and C₁-C₄-haloalkyl with preference given to R¹ being selected from the group consisting of halogen and C₁-C₄-alkyl. In this context Z¹ is in particular a covalent bond; specifically R¹ is selected from F, Cl, Br, I, nitro, CH₃, SCH₃, SCF₃, SO₂CH₃, and CH₂OCH₂CH₂OCH₃ with most preference given to CH₃ and Cl;
R² is R^{2c}R^{2d}NC(O)NR²ⁿ-;
R²ⁿ is hydrogen;
R^{2c} is selected from C₁-C₄-alkyl, C₃-C₇-cycloalkyl, C₁-C₄-haloalkyl, C₁-C₄-alkoxy, and phenyl, where phenyl is unsubstituted or substituted by 1 or 2 groups, which are identical or different and selected from the group consisting of halogen and C₁-C₄-alkoxy; specifically, R^{2c} is selected from methyl, ethyl, isopropyl (iPr), cyclopropyl (cPr), CHF₂CH₂-, CF₃CH₂-, CH₃O-, 4-Cl-phenyl, 4-CH₃O-phenyl, or phenyl;
R^{2d} is selected from C₁-C₄-alkyl, C₃-C₇-cycloalkyl, and C₁-C₄-haloalkyl, preferably from methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of hydrogen, halogen, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂, with preference given to R³ being selected from the group consisting of hydrogen, halogen and C₁-C₂-haloalkyl, in particular from H, Cl, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃ with most preference given to H, Cl, Br and CF₃;
R⁴ is selected from the group consisting of cyano, halogen, nitro, C₁-C₂-alkyl, and C₁-C₂-haloalkyl with most preference given to F and Cl;
R⁶ is selected from the group consisting of C₁-C₆-alkyl, C₁-C₄-alkoxy-C₁-C₄-alkyl, and phenyl, in particular from CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is selected from the group consisting of halogen, nitro, C₁-C₄-alkyl, C₁-C₄-alkoxy-C₁-C₄-alkoxy-Z¹ and R^{1b}-S(O)ₖ-Z¹, where k and Z¹ are as defined herein and where R^{1b} is as defined above and in particular selected from the group consisting of C₁-C₄-alkyl and C₁-C₄-haloalkyl. In this context Z¹ is in particular a covalent bond with preference given to R¹ being selected from the group consisting of halogen and C₁-C₄-alkyl; specifically R¹ is selected from F, Cl, Br, I, nitro, CH₃, SCH₃, SCF₃, SO₂CH₃, and CH₂OCH₂CH₂OCH₃ with most preference given to CH₃ and Cl;
R² is R^{2c}R^{2d}NC(O)NR²ⁿ-;
R²ⁿ is C₁-C₆-alkyl, preferably methyl or ethyl;
R^{2c} is selected from C₁-C₄-alkyl, C₃-C₇-cycloalkyl, C₁-C₄-haloalkyl, C₁-C₄-alkoxy, and phenyl, where phenyl is unsubstituted or substituted by 1 or 2 groups, which are identical or different and selected from the group consisting of halogen and C₁-C₄-alkoxy; specifically, R^{2c} is selected from methyl, ethyl, isopropyl (iPr), cyclopropyl (cPr), CHF₂CH₂-, CF₃CH₂-, CH₃O-, 4-Cl-phenyl, 4-CH₃O-phenyl, or phenyl;
R^{2d} is selected from C₁-C₄-alkyl, C₃-C₇-cycloalkyl, and C₁-C₄-haloalkyl, preferably from methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of hydrogen, halogen, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂ with preference given to R³ being selected from the group consisting of hydrogen, halogen and C₁-C₂-haloalkyl, in particular from H, Cl, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃ with most preference given to H, Cl, Br and CF₃;
R⁴ is selected from the group consisting of cyano, halogen, nitro, C₁-C₂-alkyl, and C₁-C₂-haloalkyl with most preference given to F and Cl;
R⁶ is selected from the group consisting of C₁-C₆-alkyl, C₁-C₄-alkoxy-C₁-C₄-alkyl, and phenyl, in particular from CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is selected from the group consisting of halogen, nitro, C₁-C₄-alkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl with preference given to R¹ being selected from the group consisting of halogen and C₁-C₄-alkyl; specifically R¹ is F, Cl, Br, I, nitro, CH₃, SCH₃ or SO₂CH₃ with most preference given to CH₃ and Cl;
R² is R^{2c}R^{2d}NC(O)NR²ⁿ-;
R²ⁿ is hydrogen;
R^{2c} is selected from C₁-C₄-alkyl, C₃-C₇-cycloalkyl, C₁-C₄-haloalkyl, C₁-C₄-alkoxy, and phenyl, where phenyl is unsubstituted or substituted by 1 or 2 groups, which are identical or different and selected from the group consisting of halogen and C₁-C₄-alkoxy; specifically, R^{2c} is selected from methyl, ethyl, isopropyl (iPr), cyclopropyl (cPr), CHF₂CH₂-, CF₃CH₂-, CH₃O-, 4-Cl-phenyl, 4-CH₃O-phenyl, or phenyl;
R^{2d} is selected from C₁-C₄-alkyl, C₃-C₇-cycloalkyl, and C₁-C₄-haloalkyl, preferably from methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of hydrogen, halogen, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ with preference given to R³ being selected from the group consisting of hydrogen, halogen and C₁-C₂-haloalkyl, and more specifically from H, Cl, Br, I, CF₃, SCH₃, S(O)CH₃ and S(O)₂CH₃ with most preference given to H, Cl, Br and CF₃;
R⁴ is selected from the group consisting of cyano, halogen, nitro, CH₃, CHF₂, and CF₃ with most preference given to F and Cl.
R⁶ is selected from the group consisting of C₁-C₆-alkyl, C₁-C₄-alkoxy-C₁-C₄-alkyl, and phenyl, in particular from CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is selected from the group consisting of halogen, nitro, C₁-C₄-alkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl with preference given to R¹ being selected from the group consisting of halogen and C₁-C₄-alkyl; specifically R¹ is F, Cl, Br, I, nitro, CH₃, SCH₃ or SO₂CH₃ with most preference given to CH₃ and Cl;
R² is R^{2c}R^{2d}NC(O)NR²ⁿ-;
R²ⁿ is C₁-C₆-alkyl, preferably methyl or ethyl;
R^{2c} is selected from C₁-C₄-alkyl, C₃-C₇-cycloalkyl, C₁-C₄-haloalkyl, C₁-C₄-alkoxy, and phenyl, where phenyl is unsubstituted or substituted by 1 or 2 groups, which are identical or different and selected from the group consisting of halogen and C₁-C₄-alkoxy; specifically, R^{2c} is selected from methyl, ethyl, isopropyl (iPr), cyclopropyl (cPr), CHF₂CH₂-, CF₃CH₂-, CH₃O-, 4-Cl-phenyl, 4-CH₃O-phenyl, or phenyl;
R^{2d} is selected from C₁-C₄-alkyl, C₃-C₇-cycloalkyl, and C₁-C₄-haloalkyl, preferably from methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of hydrogen, halogen, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ with preference given to R³ being selected from the group consisting of hydrogen, halogen and C₁-C₂-haloalkyl, and more specifically from H, Cl, Br, I, CF₃, SCH₃, S(O)CH₃ and S(O)₂CH₃ with most preference given to H, Cl, Br and CF₃;
R⁴ is selected from the group consisting of cyano, halogen, nitro, CH₃, CHF₂, and CF₃ with most preference given to F and Cl.

R⁶ is selected from the group consisting of C₁-C₆-alkyl, C₁-C₄-alkoxy-C₁-C₄-alkyl, and phenyl, in particular from CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is F, Cl, Br, I, nitro, CH₃, SCH₃, SCF₃, SO₂CH₃, and CH₂OCH₂CH₂OCH₃ with most preference given to CH₃ and Cl;
R² is R^{2c}R^{2d}NC(O)NH-;
R^{2c} is selected from the group consisting of methyl, ethyl, isopropyl (iPr), cyclopropyl (cPr), CHF₂CH₂-, CF₃CH₂-, 4-Cl-phenyl, 4-CH₃O-phenyl, and phenyl;
R^{2d} is selected from from the group consisting of ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of H, Cl, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃ with most preference given to H, Cl, Br and CF₃;
R⁴ is Cl or F;
R⁶ is selected from the group consisting of CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is F, Cl, Br, I, nitro, CH₃, SCH₃, SCF₃, SO₂CH₃, and CH₂OCH₂CH₂OCH₃ with most preference given to H, Cl, Br and CF₃;
R² is R^{2c}R^{2d}NC(O)NR²ⁿ-;
R²ⁿ is C₁-C₆-alkyl, preferably methyl or ethyl;
R^{2c} is selected from the group consisting of methyl, ethyl, isopropyl (iPr), cyclopropyl (cPr), CHF₂CH₂-, CF₃CH₂-, CH₃O-, 4-Cl-phenyl, 4-CH₃O-phenyl, and phenyl;
R^{2d} is selected from from the group consisting of methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of H, Cl, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃ with most preference given to H, Cl, Br and CF₃;
R⁴ is chlorine or fluorine;
R⁶ is selected from the group consisting of CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is selected from the group consisting of halogen, nitro, C₁-C₄-alkyl, C₁-C₄-alkoxy-C₁-C₄-alkoxy-Z¹ and R^{1b}-S(O)ₖ-Z¹, where k and Z¹ are as defined herein and where R^{1b} is as defined above and in particular selected from the group consisting of C₁-C₄-alkyl and C₁-C₄-haloalkyl with preference given to R¹ being selected from the group consisting of halogen and C₁-C₄-alkyl. In this context Z¹ is in particular a covalent bond; specifically R¹ is selected from F, Cl, Br, I, nitro, CH₃, SCH₃, SCF₃, SO₂CH₃, and CH₂OCH₂CH₂OCH₃ with most preference given to CH₃ and Cl;
R² is R^{2c}R^{2d}NC(O)NR²ⁿ-Z²;
Z² is C₁-C₄-alkanediyl, preferably -CH₂- or -CH₂CH₂-;
R²ⁿ is selected from hydrogen and C₁-C₆-alkyl, preferably from hydrogen, methyl and ethyl;
R^{2c} is selected from C₁-C₄-alkyl, C₃-C₇-cycloalkyl, C₁-C₄-haloalkyl, C₁-C₄-alkoxy, and phenyl, where phenyl is unsubstituted or substituted by 1 or 2 groups, which are identical or different and selected from the group consisting of halogen and C₁-C₄-alkoxy; specifically, R^{2c} is selected from methyl, ethyl, isopropyl (iPr), cyclopropyl (cPr), CHF₂CH₂-, CF₃CH₂-, CH₃O-, 4-Cl-phenyl, 4-CH₃O-phenyl, or phenyl;
R^{2d} is selected from C₁-C₄-alkyl, C₃-C₇-cycloalkyl, and C₁-C₄-haloalkyl, preferably from methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of hydrogen, halogen, nitro, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂ with preference given to R³ being selected from the group consisting of hydrogen, halogen and C₁-C₂-haloalkyl; R³ is in particular selected from the group consisting of H, Cl, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃ with most preference given to H, Cl, Br and CF₃;
R⁴ is selected from the group consisting of cyano, halogen, nitro, C₁-C₂-alkyl, and C₁-C₂-haloalkyl with most preference given to F and Cl;
R⁶ is selected from the group consisting of C₁-C₆-alkyl, C₁-C₄-alkoxy-C₁-C₄-alkyl, and phenyl, in particular from CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is selected from the group consisting of halogen, nitro, C₁-C₄-alkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl with preference given to R¹ being selected from the group consisting of halogen and C₁-C₄-alkyl; specifically R¹ is F, Cl, Br, I, nitro, CH₃, SCH₃ or SO₂CH₃ with most preference given to CH₃ and Cl;
R² is R^{2c}R^{2d}NC(O)NR²ⁿ-Z²;
Z² is C₁-C₄-alkanediyl, preferably -CH₂- or -CH₂CH₂-;
R²ⁿ is selected from hydrogen and C₁-C₆-alkyl, preferably from hydrogen, methyl and ethyl;
R^{2c} is selected from C₁-C₄-alkyl, C₃-C₇-cycloalkyl, C₁-C₄-haloalkyl, C₁-C₄-alkoxy, and phenyl, where phenyl is unsubstituted or substituted by 1 or 2 groups, which are identical or different and selected from the group consisting of halogen and C₁-C₄-alkoxy; specifically, R^{2c} is selected from methyl, ethyl, isopropyl (iPr), cyclopropyl (cPr), CHF₂CH₂-, CF₃CH₂-, CH₃O-, 4-Cl-phenyl, 4-CH₃O-phenyl, or phenyl;
R^{2d} is selected from C₁-C₄-alkyl, C₃-C₇-cycloalkyl, and C₁-C₄-haloalkyl, preferably from methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of hydrogen, halogen, nitro, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ with preference given to R³ being selected from the group consisting of hydrogen, halogen and C₁-C₂-haloalkyl, and more specifically from Cl, Br, I, CF₃, SCH₃, S(O)CH₃ and S(O)₂CH₃ with most preference given to H, Cl, Br and CF₃;
R⁴ is selected from the group consisting of cyano, halogen, nitro, CH₃, CHF₂, and CF₃ with most preference given to F and Cl;
R⁶ is selected from the group consisting of C₁-C₆-alkyl, C₁-C₄-alkoxy-C₁-C₄-alkyl, and phenyl, in particular from CH₃, CH₃CH₂, CH₃OCH₂CH₂ and CH₃OCH₂ with most preference given to CH₃.

Especially preferred are compounds of formulae I, I.A, I.B, I.C and I.D and the agriculturally suitable salts thereof, wherein Q is Q¹, Q², Q³ or Q⁴ and the variables R¹, R², R³, R⁴ and R⁶ have the following meanings:
R¹ is F, Cl, Br, I, nitro, CH₃, SCH₃, SCF₃, SO₂CH₃, and CH₂OCH₂CH₂OCH₃ with most preference given to CH₃ and Cl;
R² is R^{2c}R^{2d}NC(O)NR²ⁿ-Z²;
Z² is C₁-C₄-alkanediyl, preferably -CH₂- or -CH₂CH₂-;
R²ⁿ is selected from hydrogen and C₁-C₆-alkyl, preferably from hydrogen, methyl and ethyl;
R^{2c} is selected from the group consisting of methyl, ethyl, isopropyl (iPr), cyclopropyl (cPr), CHF₂CH₂-, CF₃CH₂-, CH₃O-, 4-Cl-phenyl, 4-CH₃O-phenyl, and phenyl;
R^{2d} is selected from from the group consisting of methyl, ethyl, isopropyl (iPr), cyclopropyl (cPr), CHF₂CH₂-, and CF₃CH₂-; or
R^{2c} and R^{2d} together with the nitrogen atom, to which they are bound form N-morpholinyl, N-pyrrolidinyl or N-piperidinyl; N-morpholinyl, N-pyrrolidinyl and N-piperidinyl may carry 1, 2, 3 or 4 groups, which are identical or different and selected from the group consisting of C₁-C₄-alkyl and C₁-C₄-haloalkyl, preferably from methyl, ethyl and trifluoromethyl;
R³ is selected from the group consisting of CI, F, Br, I, NO₂, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃ with most preference given to H, Cl, Br and CF₃;
R⁴ is chlorine or fluorine;
R⁶ is selected from the group consisting of CH₃, CH₃CH₂, CH₃OCH₂CH₂ and CH₃OCH₂ with most preference given to CH₃.

Particularly preferred are compounds of the formulae I, I.A, I.B, I.C, I.D and the agriculturally suitable salts thereof, where
R¹ is selected from the group consisting of halogen and C₁-C₄-alkyl,
R² is R^{2c}R^{2d}NC(O)NH-;
R^{2c} is selected from the group consisting of C₁-C₄-alkyl, C₃-C₇-CyCloalkyl and C₁-C₄-haloalkyl,
R^{2d} is selected from the group consisting of C₁-C₆-alkyl, C₃-C₇-CyCloalkyl, C₃-C₇-cycloalkyl-Ci-C₂-alkyl and C₁-C₆-haloalkyl,
R³ is selected from the group consisting of hydrogen, halogen and C₁-C₂-haloalkyl.
R⁴ is selected from the group consisting of fluorine and chlorine.

Even more preferred are compounds of the formulae I, I.A, I.B, I.C, I.D and the agriculturally suitable salts thereof, where
R¹ is selected from the group consisting of CH₃ and Cl,
R² is R^{2c}R^{2d}NC(O)NH-;
R^{2c} is selected from the group consisting of C₁-C₄-alkyl, C₃-C₇-CyCloalkyl and C₁-C₄-haloalkyl,
R^{2d} is selected from the group consisting of C₁-C₆-alkyl, C₃-C₇-CyCloalkyl, C₃-C₇-cycloalkyl-C₁-C₂-alkyl and C₁-C₆-haloalkyl,
R³ is selected from the group consisting of H, Cl, Br and CF₃.
R⁴ is selected from the group consisting of fluorine and chlorine.

Especially preferred are compounds of the formulae I, I.A, I.B, I.C, I.D and the agriculturally suitable salts thereof, where
R¹ is selected from the group consisting of CH₃ and Cl,
R² is R^{2c}R^{2d}NC(O)NH-;
R^{2c} is selected from the group consisting of methyl, ethyl, isopropyl (iPr), cyclopropyl (cPr), CHF₂CH₂-, CF₃CH₂-,
R^{2d} is selected from the group consisting of methyl, ethyl, isopropyl (iPr), cyclopropyl (cPr), CHF₂CH₂-, and CF₃CH₂-,
R³ is selected from the group consisting of H, Cl, Br and CF₃.
R⁴ is selected from the group consisting of fluorine and chlorine.

Particularly preferred are compounds of the formulae I.A.1 and I.D.1 and the agriculturally suitable salts thereof, 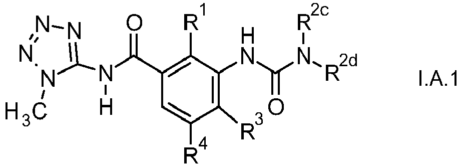 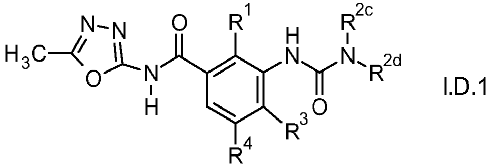 where
R¹ is selected from the group consisting of halogen and C₁-C₄-alkyl,
R^{2c} is selected from the group consisting of C₁-C₄-alkyl,C₃-C₇-CyCloalkyl and C₁-C₄-haloalkyl,
R^{2d} is selected from the group consisting of C₁-C₆-alkyl, C₃-C₇-CyCloalkyl, C₃-C₇-cycloalkyl-C₁-C₂-alkyl and C₁-C₆-haloalkyl,
R³ is selected from the group consisting of hydrogen, halogen and C₁-C₂-haloalkyl.
R⁴ is selected from the group consisting of fluorine and chlorine.

Even more preferred are compounds of the formulae I.A.1 and I.D.1 and the agriculturally suitable salts thereof, where R¹ is selected from the group consisting of CH₃ and Cl, R^{2c} is selected from the group consisting of C₁-C₄-alkyl,C₃-C₇-CyCloalkyl and C₁-C₄-haloalkyl,
R^{2d} is selected from the group consisting of C₁-C₆-alkyl, C₃-C₇-CyCloalkyl, C₃-C₇-cycloalkyl-C₁-C₂-alkyl and C₁-C₆-haloalkyl,
R³ is selected from the group consisting of H, Cl, Br and CF₃.
R⁴ is selected from the group consisting of fluorine and chlorine.

Especially preferred are compounds of the formulae I.A.1 and I.D.1 and the agriculturally suitable salts thereof, where
R¹ is selected from the group consisting of CH₃ and Cl,
R^{2c} is selected from the group consisting of methyl, ethyl, isopropyl (iPr), cyclopropyl (cPr), CHF₂CH₂-, CF₃CH₂-,
R^{2d} is selected from the group consisting of methyl, ethyl, isopropyl (iPr), cyclopropyl (cPr), CHF₂CH₂-, and CF₃CH₂-,
R³ is selected from the group consisting of H, Cl, Br and CF₃.
R⁴ is selected from the group consisting of fluorine and chlorine.

Examples of preferred compounds are the individual compounds compiled in the Tables below. Moreover, the meanings mentioned below for the individual variables in the Tables are per se, independently of the combination in which they are mentioned, a particularly preferred embodiment of the substituents in question.
Compounds I.A.I, wherein Q is Q¹, R⁴ is Cl, R⁶ is methyl, Z² in R² is a covalent bond and R²ⁿ in R² is H: 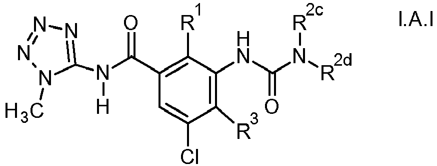 Table 1 Compounds of formula I (I.A.I.1 - I.A I.686) in which the combination of R¹, R^{2c}, R^{2d}, and R³ for a compound corresponds in each case to one row of Table A;
Compounds I.A.II, wherein Q is Q¹, R⁴ is Cl, R⁶ is ethyl, Z² in R² is a covalent bond and R²ⁿ in R² is H: 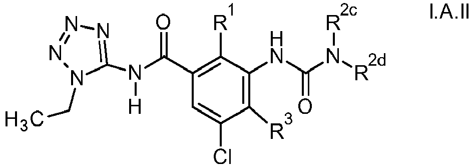 Table 2 Compounds of formula I (I.A.II.1 - I.A.II.686) in which the combination of R¹, R^{2c}, R^{2d} and R³ for a compound corresponds in each case to one row of Table A;
Compounds I.A.III, wherein Q is Q¹, R⁴ is Cl, R⁶ is methyl, Z² in R² is a covalent bond and R²ⁿ in R² is methyl: 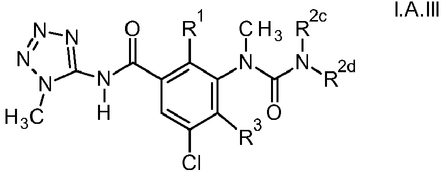 Table 3 Compounds of formula I (I.A.III.1 - I.A.III.686) in which the combination of R¹, R^{2c}, R^{2d} and R³ for a compound corresponds in each case to one row of Table A;
Compounds I.A.IV, wherein Q is Q¹, R⁴ is Cl, R⁶ is ethyl, Z² in R² is a covalent bond and R²ⁿ in R² is methyl: 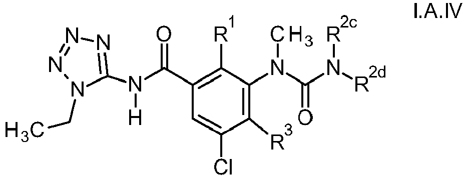 Table 4 Compounds of formula I (I.A.IV.1 - I.A.IV.686) in which the combination of R¹, R^{2c}, R^{2d} and R³ for a compound corresponds in each case to one row of Table A;
Compounds I.A.V, wherein Q is Q¹, R⁴ is Cl, R⁶ is methyl, Z² in R² is a covalent bond and R²ⁿ in R² is ethyl: 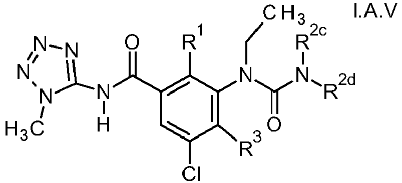 Table 5 Compounds of formula I (I.A.V.1 - I.A.V.686) in which the combination of R¹, R^{2c}, R^{2d} and R³ for a compound corresponds in each case to one row of Table A;
Compounds I.A.VI, wherein Q is Q¹, R⁴ is Cl, R⁶ is ethyl, Z² in R² is a covalent bond and R²ⁿ in R² is ethyl: 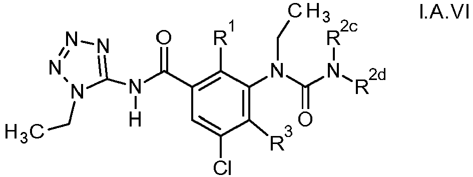 Table 6 Compounds of formula I (I.A.VI.1 - I.A.VI.686) in which the combination of R¹, R^{2c}, R^{2d} and R³ for a compound corresponds in each case to one row of Table A;
Compounds I.A.VII, wherein Q is Q¹, R⁴ is F, R⁶ is methyl, Z² in R² is a covalent bond and R²ⁿ in R² is H: 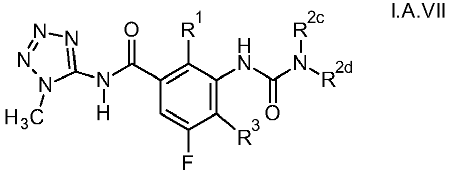 Table 7 Compounds of formula I (I.A.VII.1 - I.A.VII.686) in which the combination of R¹, R^{2c}, R^{2d}, and R³ for a compound corresponds in each case to one row of Table A;
Compounds I.A.VIII, wherein Q is Q¹, R⁴ is Fl, R⁶ is ethyl, Z² in R² is a covalent bond and R²ⁿ in R² is H: 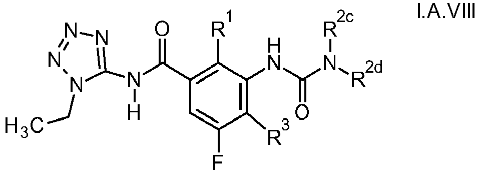 Table 8 Compounds of formula I (I.A.VIII.1 - I.A.VIII.686) in which the combination of R¹, R^{2c}, R^{2d} and R³ for a compound corresponds in each case to one row of Table A;
Compounds I.A.IX, wherein Q is Q¹, R⁴ is F, R⁶ is methyl, Z² in R² is a covalent bond and R²ⁿ in R² is methyl: 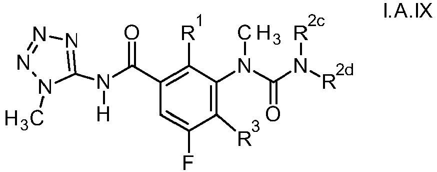 Table 9 Compounds of formula I (I.A.IX.1 - I.A.IX.686) in which the combination of R¹, R^{2c}, R^{2d} and R³ for a compound corresponds in each case to one row of Table A;
Compounds I.A.X, wherein Q is Q¹, R⁴ is F, R⁶ is ethyl, Z² in R² is a covalent bond and R²ⁿ in R² is methyl: 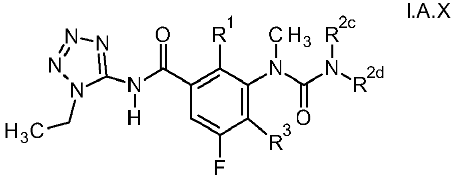 Table 10 Compounds of formula I (I.A.X.1 - I.A.X.686) in which the combination of R¹, R^{2c}, R^{2d} and R³ for a compound corresponds in each case to one row of Table A;
Compounds I.A.XI, wherein Q is Q¹, R⁴ is F, R⁶ is methyl, Z² in R² is a covalent bond and R²ⁿ in R² is ethyl: 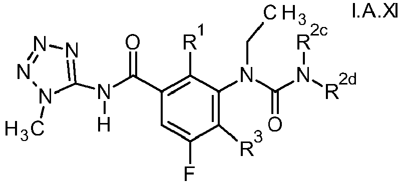 Table 11 Compounds of formula I (I.A.XI.1 - I.A.XI.686) in which the combination of R¹, R^{2c}, R^{2d} and R³ for a compound corresponds in each case to one row of Table A;
Compounds I.A.XII, wherein Q is Q¹, R⁴ is F, R⁶ is ethyl, Z² in R² is a covalent bond and R²ⁿ in R² is ethyl: 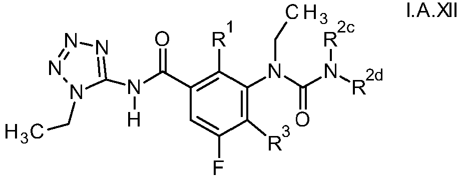 Table 12 Compounds of formula I (I.A.XII.1 - I.A.XII.686) in which the combination of R¹, R^{2c}, R^{2d} and R³ for a compound corresponds in each case to one row of Table A;
Compounds I.B.I, wherein Q is Q², R⁴ is Cl, R⁶ is methyl, Z² in R² is a covalent bond and R²ⁿ in R² is H: 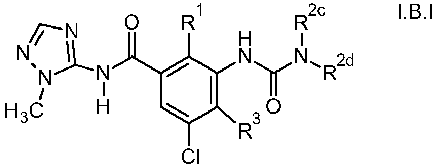 Table 13 Compounds of formula I (I.B.I.1 - I.B.I.686) in which the combination of R¹, R^{2c}, R^{2d} and R³ for a compound corresponds in each case to one row of Table A;
Compounds I.B.II, wherein Q is Q², R⁴ is Cl, R⁶ is ethyl, Z² in R² is a covalent bond and R²ⁿ in R² is H: 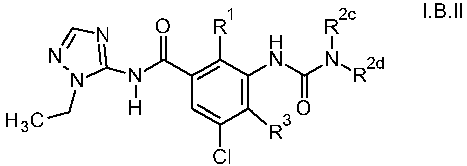 Table 14 Compounds of formula I (I.B.II.1 - I.B.II.686) in which the combination of R¹, R^{2c}, R^{2d} and R³ for a compound corresponds in each case to one row of Table A;
Compounds I.B.III, wherein Q is Q², R⁴ is Cl, R⁶ is methyl, Z² in R² is a covalent bond and R²ⁿ in R² is methyl: 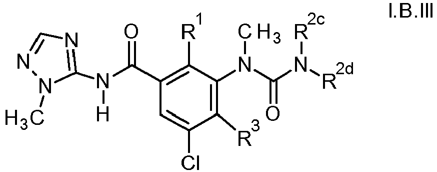 Table 15 Compounds of formula I (I.B.III.1 - I.B.III.686) in which the combination of R¹, R^{2c}, R^{2d} and R³ for a compound corresponds in each case to one row of Table A;
Compounds I.B.IV, wherein Q is Q², R⁴ is Cl, R⁶ is ethyl, Z² in R² is a covalent bond and R²ⁿ in R² is methyl: 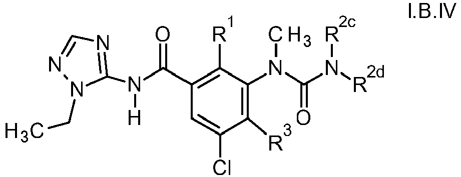 Table 16 Compounds of formula I (I.B.IV.1 - I.B.IV.686) in which the combination of R¹, R^{2c}, R^{2d} and R³ for a compound corresponds in each case to one row of Table A;
Compounds I.B.V, wherein Q is Q², R⁴ is Cl, R⁶ is methyl, Z² in R² is a covalent bond and R²ⁿ in R² is ethyl: 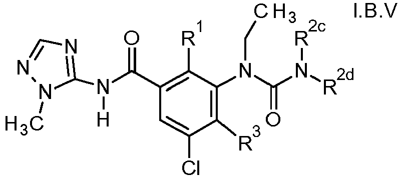 Table 17 Compounds of formula I (I.B.V.1 - I.B.V.686) in which the combination of R¹, R^{2c}, R^{2d} and R³ for a compound corresponds in each case to one row of Table A;
Compounds I.B.VI, wherein Q is Q², R⁴ is Cl, R⁶ is ethyl, Z² in R² is a covalent bond and R²ⁿ in R² is ethyl: 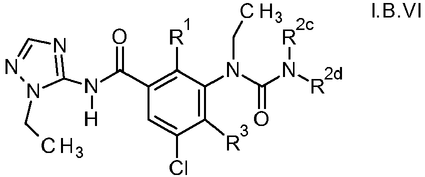 Table 18 Compounds of formula I (I.B.VI.1 - I.B.VI.686) in which the combination of R¹, R^{2c}, R^{2d} and R³ for a compound corresponds in each case to one row of Table A;
Compounds I.B.VII, wherein Q is Q², R⁴ is F, R⁶ is methyl, Z² in R² is a covalent bond and R²ⁿ in R² is H: 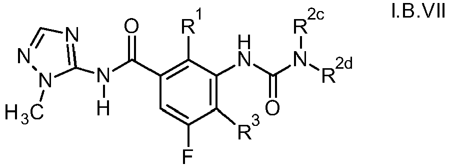 Table 19 Compounds of formula I (I.B.VII.1 - I.B.VII.686) in which the combination of R¹, R^{2c}, R^{2d} and R³ for a compound corresponds in each case to one row of Table A;
Compounds I.B.VIII, wherein Q is Q², R⁴ is F, R⁶ is ethyl, Z² in R² is a covalent bond and R²ⁿ in R² is H: 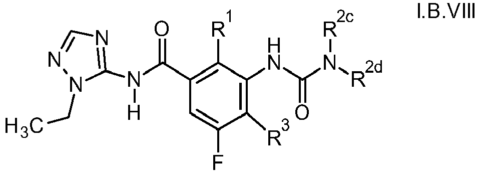 Table 20 Compounds of formula I (I.B.VIII.1 - I.B.VIII.686) in which the combination of R¹, R^{2c}, R^{2d} and R³ for a compound corresponds in each case to one row of Table A;
Compounds I.B.IX, wherein Q is Q², R⁴ is F, R⁶ is methyl, Z² in R² is a covalent bond and R²ⁿ in R² is methyl: 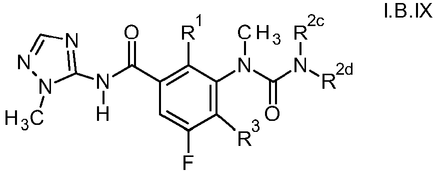 Table 21 Compounds of formula I (I.B.IX.1 - I.B.IX.686) in which the combination of R¹, R^{2c}, R^{2d} and R³ for a compound corresponds in each case to one row of Table A;
Compounds I.B.X, wherein Q is Q², R⁴ is F, R⁶ is ethyl, Z² in R² is a covalent bond and R²ⁿ in R² is methyl: 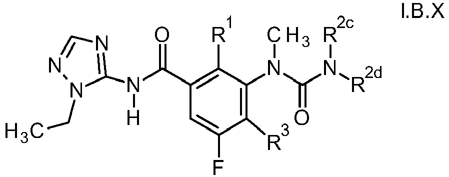 Table 22 Compounds of formula I (I.B.X.1 - I.B.X.686) in which the combination of R¹, R^{2c}, R^{2d} and R³ for a compound corresponds in each case to one row of Table A;
Compounds I.B.XI, wherein Q is Q², R⁴ is F, R⁶ is methyl, Z² in R² is a covalent bond and R²ⁿ in R² is ethyl: 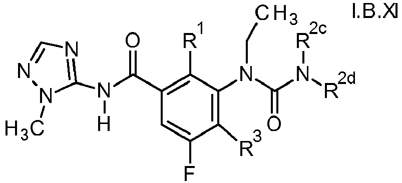 Table 23 Compounds of formula I (I.B.XI.1 - I.B.XI.686) in which the combination of R¹, R^{2c}, R^{2d} and R³ for a compound corresponds in each case to one row of Table A;
Compounds I.B.XII, wherein Q is Q², R⁴ is F, R⁶ is ethyl, Z² in R² is a covalent bond and R²ⁿ in R² is ethyl: 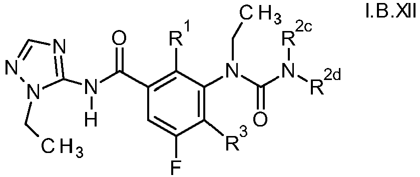 Table 24 Compounds of formula I (I.B.XII.1 - I.B.XII.686) in which the combination of R¹, R^{2c}, R^{2d} and R³ for a compound corresponds in each case to one row of Table A;
Compounds I.C.I, wherein Q is Q³, R⁴ is Cl, R⁶ is methyl, Z² in R² is a covalent bond and R²ⁿ in R² is H: 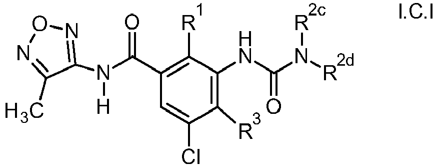 Table 25 Compounds of formula I (I.C.I.1 - I.C.I.686) in which the combination of R¹, R^{2c}, R^{2d} and R³ for a compound corresponds in each case to one row of Table A;
Compounds I.C.II, wherein Q is Q³, R⁴ is Cl, R⁶ is ethyl, Z² in R² is a covalent bond and R²ⁿ in R² is H: 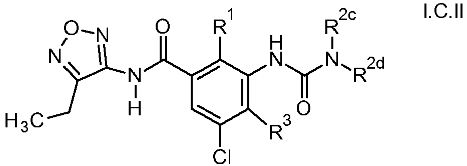 Table 26 Compounds of formula I (I.C.II.1 - I.C.II.686) in which the combination of R¹, R^{2c}, R^{2d} and R³ for a compound corresponds in each case to one row of Table A;
Compounds I.C.III, wherein Q is Q³, R⁴ is Cl, R⁶ is methyl, Z² in R² is a covalent bond and R²ⁿ in R² is methyl: 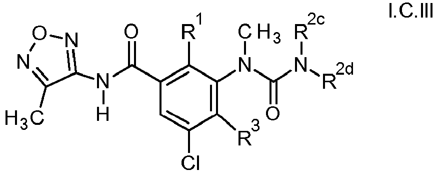 Table 27 Compounds of formula I (I.C.III.1 - I.C.III.686) in which the combination of R¹, R^{2c}, R^{2d} and R³ for a compound corresponds in each case to one row of Table A;
Compounds I.C.IV, wherein Q is Q³, R⁴ is Cl, R⁶ is ethyl, Z² in R² is a covalent bond and R²ⁿ in R² is methyl: 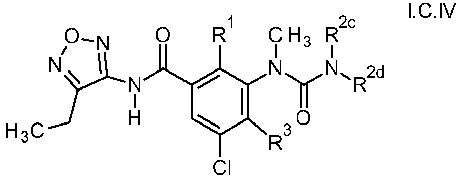 Table 28 Compounds of formula I (I.C.IV.1 - I.C.IV.686) in which the combination of R¹, R^{2c}, R^{2d} and R³ for a compound corresponds in each case to one row of Table A;
Compounds I.C.V, wherein Q is Q³, R⁴ is Cl, R⁶ is methyl, Z² in R² is a covalent bond and R²ⁿ in R² is ethyl: 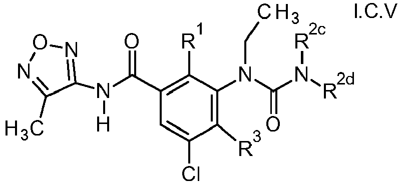 Table 29 Compounds of formula I (I.C.V.1 - I.C.V.686) in which the combination of R¹, R^{2c}, R^{2d} and R³ for a compound corresponds in each case to one row of Table A;
Compounds I.C.VI, wherein Q is Q³, R⁴ is Cl, R⁶ is ethyl, Z² in R² is a covalent bond and R²ⁿ in R² is ethyl: 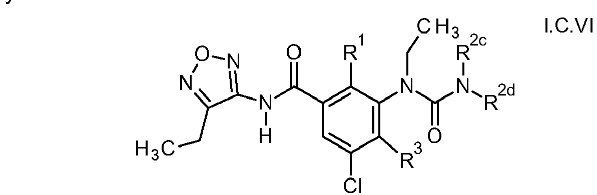 Table 30 Compounds of formula I (I.C.VI.1 - I.C.VI.686) in which the combination of R¹, R^{2c}, R^{2d} and R³ for a compound corresponds in each case to one row of Table A;
Compounds I.C.VII, wherein Q is Q³, R⁴ is F, R⁶ is methyl, Z² in R² is a covalent bond and R²ⁿ in R² is H: 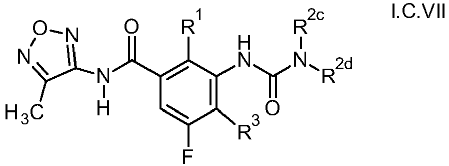 Table 31 Compounds of formula I (I.C.VII.1 - I.C.VII.686) in which the combination of R¹, R^{2c}, R^{2d} and R³ for a compound corresponds in each case to one row of Table A;
Compounds I.C.VIII, wherein Q is Q³, R⁴ is F, R⁶ is ethyl, Z² in R² is a covalent bond and R²ⁿ in R² is H: 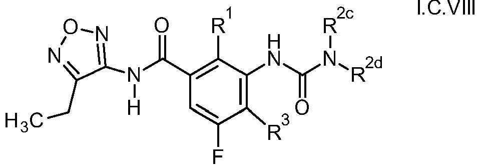 Table 32 Compounds of formula I (I.C.VIII.1 - I.C.VIII.686) in which the combination of R¹, R^{2c}, R^{2d} and R³ for a compound corresponds in each case to one row of Table A;
Compounds I.C.IX, wherein Q is Q³, R⁴ is F, R⁶ is methyl, Z² in R² is a covalent bond and R²ⁿ in R² is methyl: 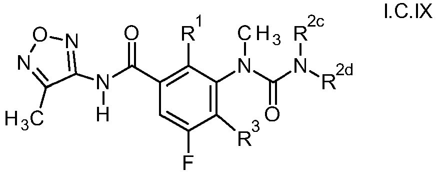 Table 33 Compounds of formula I (I.C.IX.1 - I.C.IX.686) in which the combination of R¹, R^{2c}, R^{2d} and R³ for a compound corresponds in each case to one row of Table A;
Compounds I.C.X, wherein Q is Q³, R⁴ is F, R⁶ is ethyl, Z² in R² is a covalent bond and R²ⁿ in R² is methyl: 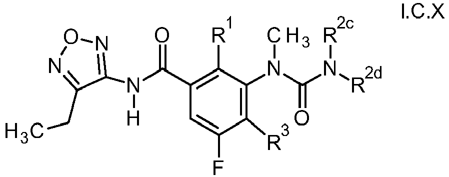 Table 34 Compounds of formula I (I.C.X.1 - I.C.X.686) in which the combination of R¹, R^{2c}, R^{2d} and R³ for a compound corresponds in each case to one row of Table A;
Compounds I.C.XI, wherein Q is Q³, R⁴ is F, R⁶ is methyl, Z² in R² is a covalent bond and R²ⁿ in R² is ethyl: 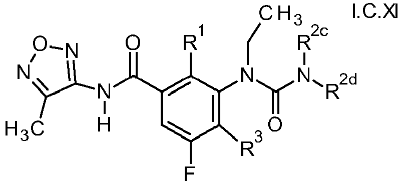 Table 35 Compounds of formula I (I.C.XI.1 - I.C.XI.686) in which the combination of R¹, R^{2c}, R^{2d} and R³ for a compound corresponds in each case to one row of Table A;
Compounds I.C.XII, wherein Q is Q³, R⁴ is F, R⁶ is ethyl, Z² in R² is a covalent bond and R²ⁿ in R² is ethyl: 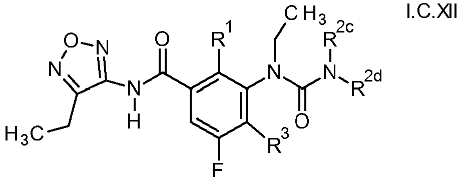 Table 36 Compounds of formula I (I.C.XII.1 - I.C.XII.686) in which the combination of R¹, R^{2c}, R^{2d} and R³ for a compound corresponds in each case to one row of Table A;
Compounds I.D.I, wherein Q is Q⁴, R⁴ is Cl, R⁶ is methyl, Z² in R² is a covalent bond and R²ⁿ in R² is H: 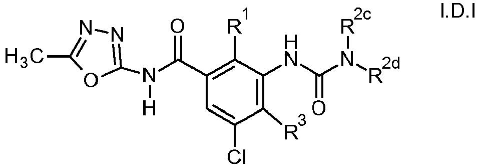 Table 37 Compounds of formula I (I.D.I.1 - I.D.I.686) in which the combination of R¹, R^{2c}, R^{2d} and R³ for a compound corresponds in each case to one row of Table A;
Compounds I.D.II, wherein Q is Q⁴, R⁴ is Cl, R⁶ is ethyl, Z² in R² is a covalent bond and R²ⁿ in R² is H: 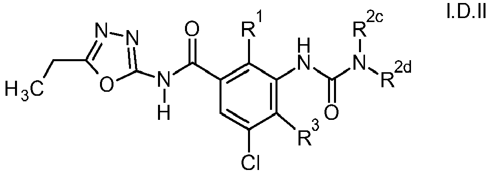 Table 38 Compounds of formula I (I.D.II.1 - I.D.II.686) in which the combination of R¹, R^{2c}, R^{2d} and R³ for a compound corresponds in each case to one row of Table A;
Compounds I.D.III, wherein Q is Q⁴, R⁴ is Cl, R⁶ is methyl, Z² in R² is a covalent bond and R²ⁿ in R² is methyl: 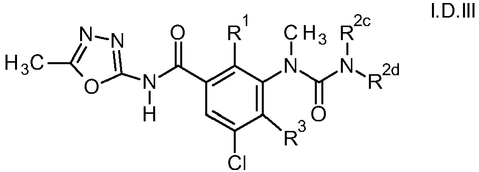 Table 39 Compounds of formula I (I.D.III.1 - 1.D.III.686) in which the combination of R¹, R^{2c}, R^{2d} and R³ for a compound corresponds in each case to one row of Table A;
Compounds I.D.IV, wherein Q is Q⁴, R⁴ is Cl, R⁶ is ethyl, Z² in R² is a covalent bond and R²ⁿ in R² is methyl: 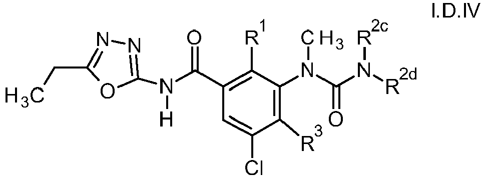 Table 40 Compounds of formula I (I.D.IV.1 - I.D.IV.686) in which the combination of R¹, R^{2c}, R^{2d} and R³ for a compound corresponds in each case to one row of Table A;
Compounds I.D.V, wherein Q is Q⁴, R⁴ is Cl, R⁶ is methyl, Z² in R² is a covalent bond and R²ⁿ in R² is ethyl: 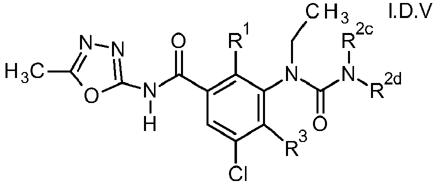 Table 41 Compounds of formula I (I.D.V.1 - I.D.V.686) in which the combination of R¹, R^{2c}, R^{2d} and R³ for a compound corresponds in each case to one row of Table A;
Compounds I.D.VI, wherein Q is Q⁴, R⁴ is Cl, R⁶ is ethyl, Z² in R² is a covalent bond and R²ⁿ in R² is ethyl: 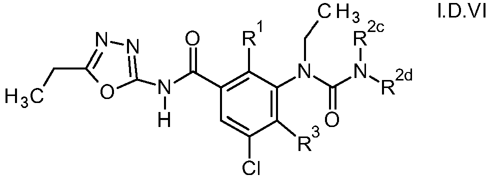 Table 42 Compounds of formula I (I.D.VI.1 - I.D.VI.686) in which the combination of R¹, R^{2c}, R^{2d} and R⁴ for a compound corresponds in each case to one row of Table A;
Compounds I.D.VII, wherein Q is Q⁴, R⁴ is F, R⁶ is methyl, Z² in R² is a covalent bond and R²ⁿ in R² is H: 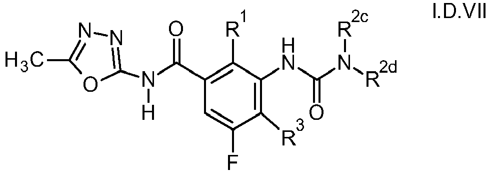 Table 43 Compounds of formula I (I.D.VII.1 - I.D.VII.686) in which the combination of R¹, R^{2c}, R^{2d} and R³ for a compound corresponds in each case to one row of Table A;
Compounds I.D.VIII, wherein Q is Q⁴, R⁴ is F, R⁶ is ethyl, Z² in R² is a covalent bond and R²ⁿ in R² is H: 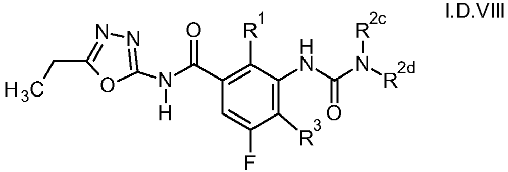 Table 44 Compounds of formula I (I.D.VIII.1 - I.D.VIII.686) in which the combination of R¹, R^{2c}, R^{2d} and R³ for a compound corresponds in each case to one row of Table A;
Compounds I.D.IX, wherein Q is Q⁴, R⁴ is F, R⁶ is methyl, Z² in R² is a covalent bond and R²ⁿ in R² is methyl: 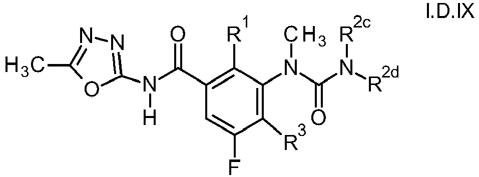 Table 45 Compounds of formula I (I.D.IX.1 - I.D.IX.686) in which the combination of R¹, R^{2c}, R^{2d} and R³ for a compound corresponds in each case to one row of Table A;
Compounds I.D.X, wherein Q is Q⁴, R⁴ is F, R⁶ is ethyl, Z² in R² is a covalent bond and R²ⁿ in R² is methyl: 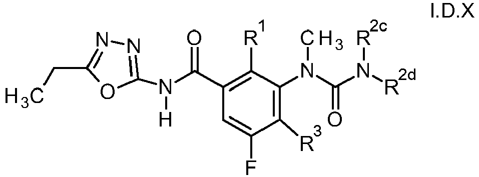 Table 46 Compounds of formula I (I.D.X.1 - I.D.X.686) in which the combination of R¹, R^{2c}, R^{2d} and R³ for a compound corresponds in each case to one row of Table A;
Compounds I.D.XI, wherein Q is Q⁴, R⁴ is F, R⁶ is methyl, Z² in R² is a covalent bond and R²ⁿ in R² is ethyl: 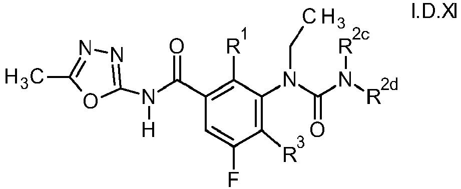 Table 47 Compounds of formula I (I.D.XI.1 - I.D.XI.686) in which the combination of R¹, R^{2c}, R^{2d} and R³ for a compound corresponds in each case to one row of Table A;
Compounds I.D.XII, wherein Q is Q⁴, R⁴ is F, R⁶ is ethyl, Z² in R² is a covalent bond and R²ⁿ in R² is ethyl: 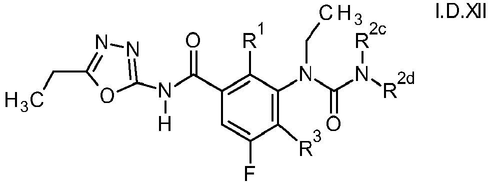 Table 48 Compounds of formula I (I.D.XII.1 - I.D.XII.686) in which the combination of R¹, R^{2c}, R^{2d} and R⁴ for a compound corresponds in each case to one row of Table A;

**Table A:**

| | **R¹** | **R^{2c}** | **R^{2d}** | **R³** |
|---|---|---|---|---|
| 1. | Cl | CH₃ | CH₃ | Cl |
| 2. | Cl | CH₃ | CH₃ | Br |
| 3. | Cl | CH₃ | CH₃ | I |
| 4. | Cl | CH₃ | CH₃ | CF₃ |
| 5. | Cl | CH₃ | CH₃ | CH₃S(O) |
| 6. | Cl | CH₃ | CH₃ | CH₃S(O)₂ |
| 7. | Cl | CH₃ | CH₃ | H |
| 8. | Cl | CH₃ | CH₃CH₂ | Cl |
| 9. | Cl | CH₃ | CH₃CH₂ | Br |
| 10. | Cl | CH₃ | CH₃CH₂ | I |
| 11. | Cl | CH₃ | CH₃CH₂ | CF₃ |
| 12. | Cl | CH₃ | CH₃CH₂ | CH₃S(O) |
| 13. | Cl | CH₃ | CH₃CH₂ | CH₃S(O)₂ |
| 14. | Cl | CH₃ | CH₃CH₂ | H |
| 15. | Cl | CH₃ | iPr | Cl |
| 16. | Cl | CH₃ | iPr | Br |
| 17. | Cl | CH₃ | iPr | I |
| 18. | Cl | CH₃ | iPr | CF₃ |
| 19. | Cl | CH₃ | iPr | CH₃S(O) |
| 20. | Cl | CH₃ | iPr | CH₃S(O)₂ |
| 21. | Cl | CH₃ | iPr | H |
| 22. | Cl | CH₃ | cPr | Cl |
| 23. | Cl | CH₃ | cPr | Br |
| 24. | Cl | CH₃ | cPr | I |
| 25. | Cl | CH₃ | cPr | CF₃ |
| 26. | Cl | CH₃ | cPr | CH₃S(O) |
| 27. | Cl | CH₃ | cPr | CH₃S(O)₂ |
| 28. | Cl | CH₃ | cPr | H |
| 29. | Cl | CH₃ | CHF₂CH₂ | Cl |
| 30. | Cl | CH₃ | CHF₂CH₂ | Br |
| 31. | Cl | CH₃ | CHF₂CH₂ | I |
| 32. | Cl | CH₃ | CHF₂CH₂ | CF₃ |
| 33. | Cl | CH₃ | CHF₂CH₂ | CH₃S(O) |
| 34. | Cl | CH₃ | CHF₂CH₂ | CH₃S(O)₂ |
| 35. | Cl | CH₃ | CHF₂CH₂ | H |
| 36. | Cl | CH₃ | CF₃CH₂ | Cl |
| 37. | Cl | CH₃ | CF₃CH₂ | Br |
| 38. | Cl | CH₃ | CF₃CH₂ | I |
| 39. | Cl | CH₃ | CF₃CH₂ | CF₃ |
| 40. | Cl | CH₃ | CF₃CH₂ | CH₃S(O) |
| 41. | Cl | CH₃ | CF₃CH₂ | CH₃S(O)₂ |
| 42. | Cl | CH₃ | CF₃CH₂ | H |
| 43. | Cl | CH₃CH₂ | CH₃CH₂ | Cl |
| 44. | Cl | CH₃CH₂ | CH₃CH₂ | Br |
| 45. | Cl | CH₃CH₂ | CH₃CH₂ | I |
| 46. | Cl | CH₃CH₂ | CH₃CH₂ | CF₃ |
| 47. | Cl | CH₃CH₂ | CH₃CH₂ | CH₃S(O) |
| 48. | Cl | CH₃CH₂ | CH₃CH₂ | CH₃S(O)₂ |
| 49. | Cl | CH₃CH₂ | CH₃CH₂ | H |
| 50. | Cl | CH₃CH₂ | iPr | Cl |
| 51. | Cl | CH₃CH₂ | iPr | Br |
| 52. | Cl | CH₃CH₂ | iPr | I |
| 53. | Cl | CH₃CH₂ | iPr | CF₃ |
| 54. | Cl | CH₃CH₂ | iPr | CH₃S(O) |
| 55. | Cl | CH₃CH₂ | iPr | CH₃S(O)₂ |
| 56. | Cl | CH₃CH₂ | iPr | H |
| 57. | Cl | CH₃CH₂ | cPr | Cl |
| 58. | Cl | CH₃CH₂ | cPr | Br |
| 59. | Cl | CH₃CH₂ | cPr | I |
| 60. | Cl | CH₃CH₂ | cPr | CF₃ |
| 61. | Cl | CH₃CH₂ | cPr | CH₃S(O) |
| 62. | Cl | CH₃CH₂ | cPr | CH₃S(O)₂ |
| 63. | Cl | CH₃CH₂ | cPr | H |
| 64. | Cl | CH₃CH₂ | CHF₂CH₂ | Cl |
| 65. | Cl | CH₃CH₂ | CHF₂CH₂ | Br |
| 66. | Cl | CH₃CH₂ | CHF₂CH₂ | I |
| 67. | Cl | CH₃CH₂ | CHF₂CH₂ | CF₃ |
| 68. | Cl | CH₃CH₂ | CHF₂CH₂ | CH₃S(O) |
| 69. | Cl | CH₃CH₂ | CHF₂CH₂ | CH₃S(O)₂ |
| 70. | Cl | CH₃CH₂ | CHF₂CH₂ | H |
| 71. | Cl | CH₃CH₂ | CF₃CH₂ | Cl |
| 72. | Cl | CH₃CH₂ | CF₃CH₂ | Br |
| 73. | Cl | CH₃CH₂ | CF₃CH₂ | I |
| 74. | Cl | CH₃CH₂ | CF₃CH₂ | CF₃ |
| 75. | Cl | CH₃CH₂ | CF₃CH₂ | CH₃S(O) |
| 76. | Cl | CH₃CH₂ | CF₃CH₂ | CH₃S(O)₂ |
| 77. | Cl | CH₃CH₂ | CF₃CH₂ | H |
| 78. | Cl | iPr | iPr | Cl |
| 79. | Cl | iPr | iPr | Br |
| 80. | Cl | iPr | iPr | I |
| 81. | Cl | iPr | iPr | CF₃ |
| 82. | Cl | iPr | iPr | CH₃S(O) |
| 83. | Cl | iPr | iPr | CH₃S(O)₂ |
| 84. | Cl | iPr | iPr | H |
| 85. | Cl | iPr | cPr | Cl |
| 86. | Cl | iPr | cPr | Br |
| 87. | Cl | iPr | cPr | I |
| 88. | Cl | iPr | cPr | CF₃ |
| 89. | Cl | iPr | cPr | CH₃S(O) |
| 90. | Cl | iPr | cPr | CH₃S(O)₂ |
| 91. | Cl | iPr | cPr | H |
| 92. | Cl | iPr | CHF₂CH₂ | Cl |
| 93. | Cl | iPr | CHF₂CH₂ | Br |
| 94. | Cl | iPr | CHF₂CH₂ | I |
| 95. | Cl | iPr | CHF₂CH₂ | CF₃ |
| 96. | Cl | iPr | CHF₂CH₂ | CH₃S(O) |
| 97. | Cl | iPr | CHF₂CH₂ | CH₃S(O)₂ |
| 98. | Cl | iPr | CHF₂CH₂ | H |
| 99. | Cl | iPr | CF₃CH₂ | Cl |
| 100. | Cl | iPr | CF₃CH₂ | Br |
| 101. | Cl | iPr | CF₃CH₂ | I |
| 102. | Cl | iPr | CF₃CH₂ | CF₃ |
| 103. | Cl | iPr | CF₃CH₂ | CH₃S(O) |
| 104. | Cl | iPr | CF₃CH₂ | CH₃S(O)₂ |
| 105. | Cl | iPr | CF₃CH₂ | H |
| 106. | Cl | cPr | cPr | Cl |
| 107. | Cl | cPr | cPr | Br |
| 108. | Cl | cPr | cPr | I |
| 109. | Cl | cPr | cPr | CF₃ |
| 110. | Cl | cPr | cPr | CH₃S(O) |
| 111. | Cl | cPr | cPr | CH₃S(O)₂ |
| 112. | Cl | cPr | cPr | H |
| 113. | Cl | cPr | CHF₂CH₂ | Cl |
| 114. | Cl | cPr | CHF₂CH₂ | Br |
| 115. | Cl | cPr | CHF₂CH₂ | I |
| 116. | Cl | cPr | CHF₂CH₂ | CF₃ |
| 117. | Cl | cPr | CHF₂CH₂ | CH₃S(O) |
| 118. | Cl | cPr | CHF₂CH₂ | CH₃S(O)₂ |
| 119. | Cl | cPr | CHF₂CH₂ | H |
| 120. | Cl | cPr | CF₃CH₂ | Cl |
| 121. | Cl | cPr | CF₃CH₂ | Br |
| 122. | Cl | cPr | CF₃CH₂ | I |
| 123. | Cl | cPr | CF₃CH₂ | CF₃ |
| 124. | Cl | cPr | CF₃CH₂ | CH₃S(O) |
| 125. | Cl | cPr | CF₃CH₂ | CH₃S(O)₂ |
| 126. | Cl | cPr | CF₃CH₂ | H |
| 127. | Cl | CHF₂CH₂ | CHF₂CH₂ | Cl |
| 128. | Cl | CHF₂CH₂ | CHF₂CH₂ | Br |
| 129. | Cl | CHF₂CH₂ | CHF₂CH₂ | I |
| 130. | Cl | CHF₂CH₂ | CHF₂CH₂ | CF₃ |
| 131. | Cl | CHF₂CH₂ | CHF₂CH₂ | CH₃S(O) |
| 132. | Cl | CHF₂CH₂ | CHF₂CH₂ | CH₃S(O)₂ |
| 133. | Cl | CHF₂CH₂ | CHF₂CH₂ | H |
| 134. | Cl | CHF₂CH₂ | CF₃CH₂ | Cl |
| 135. | Cl | CHF₂CH₂ | CF₃CH₂ | Br |
| 136. | Cl | CHF₂CH₂ | CF₃CH₂ | I |
| 137. | Cl | CHF₂CH₂ | CF₃CH₂ | CF₃ |
| 138. | Cl | CHF₂CH₂ | CF₃CH₂ | CH₃S(O) |
| 139. | Cl | CHF₂CH₂ | CF₃CH₂ | CH₃S(O)₂ |
| 140. | Cl | CHF₂CH₂ | CF₃CH₂ | H |
| 141. | Cl | CF₃CH₂ | CF₃CH₂ | Cl |
| 142. | Cl | CF₃CH₂ | CF₃CH₂ | Br |
| 143. | Cl | CF₃CH₂ | CF₃CH₂ | I |
| 144. | Cl | CF₃CH₂ | CF₃CH₂ | CF₃ |
| 145. | Cl | CF₃CH₂ | CF₃CH₂ | CH₃S(O) |
| 146. | Cl | CF₃CH₂ | CF₃CH₂ | CH₃S(O)₂ |
| 147. | Cl | CF₃CH₂ | CF₃CH₂ | H |
| 148. | Cl | CH₃O | CH₃ | Cl |
| 149. | Cl | CH₃O | CH₃ | Br |
| 150. | Cl | CH₃O | CH₃ | I |
| 151. | Cl | CH₃O | CH₃ | CF₃ |
| 152. | Cl | CH₃O | CH₃ | CH₃S(O) |
| 153. | Cl | CH₃O | CH₃ | CH₃S(O)₂ |
| 154. | Cl | CH₃O | CH₃ | H |
| 155. | Cl | CH₃O | CH₃CH₂ | Cl |
| 156. | Cl | CH₃O | CH₃CH₂ | Br |
| 157. | Cl | CH₃O | CH₃CH₂ | I |
| 158. | Cl | CH₃O | CH₃CH₂ | CF₃ |
| 159. | Cl | CH₃O | CH₃CH₂ | CH₃S(O) |
| 160. | Cl | CH₃O | CH₃CH₂ | CH₃S(O)₂ |
| 161. | Cl | CH₃O | CH₃CH₂ | H |
| 162. | Cl | CH₃O | iPr | Cl |
| 163. | Cl | CH₃O | iPr | Br |
| 164. | Cl | CH₃O | iPr | I |
| 165. | Cl | CH₃O | iPr | CF₃ |
| 166. | Cl | CH₃O | iPr | CH₃S(O) |
| 167. | Cl | CH₃O | iPr | CH₃S(O)₂ |
| 168. | Cl | CH₃O | iPr | H |
| 169. | Cl | CH₃O | cPr | Cl |
| 170. | Cl | CH₃O | cPr | Br |
| 171. | Cl | CH₃O | cPr | I |
| 172. | Cl | CH₃O | cPr | CF₃ |
| 173. | Cl | CH₃O | cPr | CH₃S(O) |
| 174. | Cl | CH₃O | cPr | CH₃S(O)₂ |
| 175. | Cl | CH₃O | cPr | H |
| 176. | Cl | CH₃O | CHF₂CH₂ | Cl |
| 177. | Cl | CH₃O | CHF₂CH₂ | Br |
| 178. | Cl | CH₃O | CHF₂CH₂ | I |
| 179. | Cl | CH₃O | CHF₂CH₂ | CF₃ |
| 180. | Cl | CH₃O | CHF₂CH₂ | CH₃S(O) |
| 181. | Cl | CH₃O | CHF₂CH₂ | CH₃S(O)₂ |
| 182. | Cl | CH₃O | CHF₂CH₂ | H |
| 183. | Cl | CH₃O | CF₃CH₂ | Cl |
| 184. | Cl | CH₃O | CF₃CH₂ | Br |
| 185. | Cl | CH₃O | CF₃CH₂ | I |
| 186. | Cl | CH₃O | CF₃CH₂ | CF₃ |
| 187. | Cl | CH₃O | CF₃CH₂ | CH₃S(O) |
| 188. | Cl | CH₃O | CF₃CH₂ | CH₃S(O)₂ |
| 189. | Cl | CH₃O | CF₃CH₂ | H |
| 190. | Cl | Ph | CH₃ | Cl |
| 191. | Cl | Ph | CH₃ | Br |
| 192. | Cl | Ph | CH₃ | I |
| 193. | Cl | Ph | CH₃ | CF₃ |
| 194. | Cl | Ph | CH₃ | CH₃S(O) |
| 195. | Cl | Ph | CH₃ | CH₃S(O)₂ |
| 196. | Cl | Ph | CH₃ | H |
| 197. | Cl | Ph | CH₃CH₂ | Cl |
| 198. | Cl | Ph | CH₃CH₂ | Br |
| 199. | Cl | Ph | CH₃CH₂ | I |
| 200. | Cl | Ph | CH₃CH₂ | CF₃ |
| 201. | Cl | Ph | CH₃CH₂ | CH₃S(O) |
| 202. | Cl | Ph | CH₃CH₂ | CH₃S(O)₂ |
| 203. | Cl | Ph | CH₃CH₂ | H |
| 204. | Cl | Ph | iPr | Cl |
| 205. | Cl | Ph | iPr | Br |
| 206. | Cl | Ph | iPr | I |
| 207. | Cl | Ph | iPr | CF₃ |
| 208. | Cl | Ph | iPr | CH₃S(O) |
| 209. | Cl | Ph | iPr | CH₃S(O)₂ |
| 210. | Cl | Ph | iPr | H |
| 211. | Cl | Ph | cPr | Cl |
| 212. | Cl | Ph | cPr | Br |
| 213. | Cl | Ph | cPr | I |
| 214. | Cl | Ph | cPr | CF₃ |
| 215. | Cl | Ph | cPr | CH₃S(O) |
| 216. | Cl | Ph | cPr | CH₃S(O)₂ |
| 217. | Cl | Ph | cPr | H |
| 218. | Cl | Ph | CHF₂CH₂ | Cl |
| 219. | Cl | Ph | CHF₂CH₂ | Br |
| 220. | Cl | Ph | CHF₂CH₂ | I |
| 221. | Cl | Ph | CHF₂CH₂ | CF₃ |
| 222. | Cl | Ph | CHF₂CH₂ | CH₃S(O) |
| 223. | Cl | Ph | CHF₂CH₂ | CH₃S(O)₂ |
| 224. | Cl | Ph | CHF₂CH₂ | H |
| 225. | Cl | Ph | CF₃CH₂ | Cl |
| 226. | Cl | Ph | CF₃CH₂ | Br |
| 227. | Cl | Ph | CF₃CH₂ | I |
| 228. | Cl | Ph | CF₃CH₂ | CF₃ |
| 229. | Cl | Ph | CF₃CH₂ | CH₃S(O) |
| 230. | Cl | Ph | CF₃CH₂ | CH₃S(O)₂ |
| 231. | Cl | Ph | CF₃CH₂ | H |
| 232. | Cl | 4-Cl-Ph | CH₃ | Cl |
| 233. | Cl | 4-Cl-Ph | CH₃ | Br |
| 234. | Cl | 4-Cl-Ph | CH₃ | I |
| 235. | Cl | 4-Cl-Ph | CH₃ | CF₃ |
| 236. | Cl | 4-Cl-Ph | CH₃ | CH₃S(O) |
| 237. | Cl | 4-Cl-Ph | CH₃ | CH₃S(O)₂ |
| 238. | Cl | 4-Cl-Ph | CH₃ | H |
| 239. | Cl | 4-Cl-Ph | CH₃CH₂ | Cl |
| 240. | Cl | 4-Cl-Ph | CH₃CH₂ | Br |
| 241. | Cl | 4-Cl-Ph | CH₃CH₂ | I |
| 242. | Cl | 4-Cl-Ph | CH₃CH₂ | CF₃ |
| 243. | Cl | 4-Cl-Ph | CH₃CH₂ | CH₃S(O) |
| 244. | Cl | 4-Cl-Ph | CH₃CH₂ | CH₃S(O)₂ |
| 245. | Cl | 4-Cl-Ph | CH₃CH₂ | H |
| 246. | Cl | 4-Cl-Ph | iPr | Cl |
| 247. | Cl | 4-Cl-Ph | iPr | Br |
| 248. | Cl | 4-Cl-Ph | iPr | I |
| 249. | Cl | 4-Cl-Ph | iPr | CF₃ |
| 250. | Cl | 4-Cl-Ph | iPr | CH₃S(O) |
| 251. | Cl | 4-Cl-Ph | iPr | CH₃S(O)₂ |
| 252. | Cl | 4-Cl-Ph | iPr | H |
| 253. | Cl | 4-Cl-Ph | cPr | Cl |
| 254. | Cl | 4-Cl-Ph | cPr | Br |
| 255. | Cl | 4-Cl-Ph | cPr | I |
| 256. | Cl | 4-Cl-Ph | cPr | CF₃ |
| 257. | Cl | 4-Cl-Ph | cPr | CH₃S(O) |
| 258. | Cl | 4-Cl-Ph | cPr | CH₃S(O)₂ |
| 259. | Cl | 4-Cl-Ph | cPr | H |
| 260. | Cl | 4-Cl-Ph | CHF₂CH₂ | Cl |
| 261. | Cl | 4-Cl-Ph | CHF₂CH₂ | Br |
| 262. | Cl | 4-Cl-Ph | CHF₂CH₂ | I |
| 263. | Cl | 4-Cl-Ph | CHF₂CH₂ | CF₃ |
| 264. | Cl | 4-Cl-Ph | CHF₂CH₂ | CH₃S(O) |
| 265. | Cl | 4-Cl-Ph | CHF₂CH₂ | CH₃S(O)₂ |
| 266. | Cl | 4-Cl-Ph | CHF₂CH₂ | H |
| 267. | Cl | 4-Cl-Ph | CF₃CH₂ | Cl |
| 268. | Cl | 4-Cl-Ph | CF₃CH₂ | Br |
| 269. | Cl | 4-Cl-Ph | CF₃CH₂ | I |
| 270. | Cl | 4-Cl-Ph | CF₃CH₂ | CF₃ |
| 271. | Cl | 4-Cl-Ph | CF₃CH₂ | CH₃S(O) |
| 272. | Cl | 4-Cl-Ph | CF₃CH₂ | CH₃S(O)₂ |
| 273. | Cl | 4-Cl-Ph | CF₃CH₂ | H |
| 274. | Cl | 4-CH₃O-Ph | CH₃ | Cl |
| 275. | Cl | 4-CH₃O-Ph | CH₃ | Br |
| 276. | Cl | 4-CH₃O-Ph | CH₃ | I |
| 277. | Cl | 4-CH₃O-Ph | CH₃ | CF₃ |
| 278. | Cl | 4-CH₃O-Ph | CH₃ | CH₃S(O) |
| 279. | Cl | 4-CH₃O-Ph | CH₃ | CH₃S(O)₂ |
| 280. | Cl | 4-CH₃O-Ph | CH₃ | H |
| 281. | Cl | 4-CH₃O-Ph | CH₃CH₂ | Cl |
| 282. | Cl | 4-CH₃O-Ph | CH₃CH₂ | Br |
| 283. | Cl | 4-CH₃O-Ph | CH₃CH₂ | I |
| 284. | Cl | 4-CH₃O-Ph | CH₃CH₂ | CF₃ |
| 285. | Cl | 4-CH₃O-Ph | CH₃CH₂ | CH₃S(O) |
| 286. | Cl | 4-CH₃O-Ph | CH₃CH₂ | CH₃S(O)₂ |
| 287. | Cl | 4-CH₃O-Ph | CH₃CH₂ | H |
| 288. | Cl | 4-CH₃O-Ph | iPr | Cl |
| 289. | Cl | 4-CH₃O-Ph | iPr | Br |
| 290. | Cl | 4-CH₃O-Ph | iPr | I |
| 291. | Cl | 4-CH₃O-Ph | iPr | CF₃ |
| 292. | Cl | 4-CH₃O-Ph | iPr | CH₃S(O) |
| 293. | Cl | 4-CH₃O-Ph | iPr | CH₃S(O)₂ |
| 294. | Cl | 4-CH₃O-Ph | iPr | H |
| 295. | Cl | 4-CH₃O-Ph | cPr | Cl |
| 296. | Cl | 4-CH₃O-Ph | cPr | Br |
| 297. | Cl | 4-CH₃O-Ph | cPr | I |
| 298. | Cl | 4-CH₃O-Ph | cPr | CF₃ |
| 299. | Cl | 4-CH₃O-Ph | cPr | CH₃S(O) |
| 300. | Cl | 4-CH₃O-Ph | cPr | CH₃S(O)₂ |
| 301. | Cl | 4-CH₃O-Ph | cPr | H |
| 302. | Cl | 4-CH₃O-Ph | CHF₂CH₂ | Cl |
| 303. | Cl | 4-CH₃O-Ph | CHF₂CH₂ | Br |
| 304. | Cl | 4-CH₃O-Ph | CHF₂CH₂ | I |
| 305. | Cl | 4-CH₃O-Ph | CHF₂CH₂ | CF₃ |
| 306. | Cl | 4-CH₃O-Ph | CHF₂CH₂ | CH₃S(O) |
| 307. | Cl | 4-CH₃O-Ph | CHF₂CH₂ | CH₃S(O)₂ |
| 308. | Cl | 4-CH₃O-Ph | CHF₂CH₂ | H |
| 309. | Cl | 4-CH₃O-Ph | CF₃CH₂ | Cl |
| 310. | Cl | 4-CH₃O-Ph | CF₃CH₂ | Br |
| 311. | Cl | 4-CH₃O-Ph | CF₃CH₂ | I |
| 312. | Cl | 4-CH₃O-Ph | CF₃CH₂ | CF₃ |
| 313. | Cl | 4-CH₃O-Ph | CF₃CH₂ | CH₃S(O) |
| 314. | Cl | 4-CH₃O-Ph | CF₃CH₂ | CH₃S(O)₂ |
| 315. | Cl | 4-CH₃O-Ph | CF₃CH₂ | H |
| 316. | Cl | 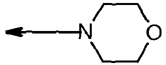 | | Cl |
| 317. | Cl | 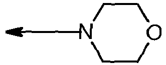 | | Br |
| 318. | Cl | 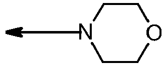 | | I |
| 319. | Cl | 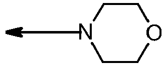 | | CF₃ |
| 320. | Cl | 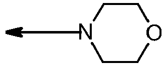 | | CH₃S(O) |
| 321. | Cl | 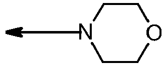 | | CH₃S(O)₂ |
| 322. | Cl | 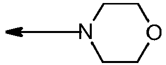 | | H |
| 323. | Cl | 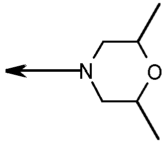 | | Cl |
| 324. | Cl | 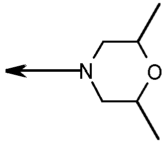 | | Br |
| 325. | Cl | 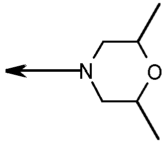 | | I |
| 326. | Cl | 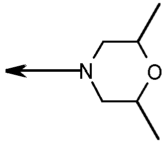 | | CF₃ |
| 327. | Cl | 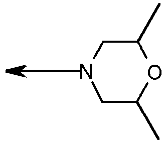 | | CH₃S(O) |
| 328. | Cl | 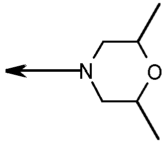 | | CH₃S(O)₂ |
| 329. | Cl | 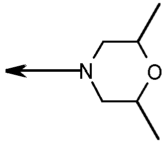 | | H |
| 330. | Cl | 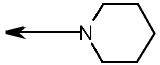 | | Cl |
| 331. | Cl | 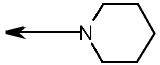 | | Br |
| 332. | Cl | 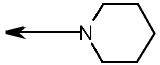 | | I |
| 333. | Cl | 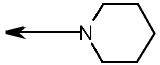 | | CF₃ |
| 334. | Cl | 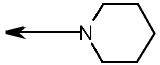 | | CH₃S(O) |
| 335. | Cl | 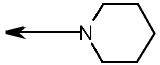 | | CH₃S(O)₂ |
| 336. | Cl | 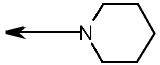 | | H |
| 337. | Cl | 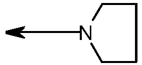 | | Cl |
| 338. | Cl | 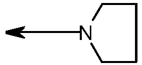 | | Br |
| 339. | Cl | 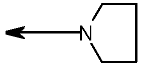 | | I |
| 340. | Cl | 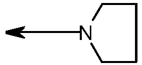 | | CF₃ |
| 341. | Cl | 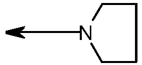 | | CH₃S(O) |
| 342. | Cl | 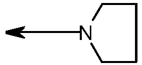 | | CH₃S(O)₂ |
| 343. | Cl | 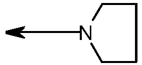 | | H |
| 344. | CH₃ | CH₃ | CH₃ | Cl |
| 345. | CH₃ | CH₃ | CH₃ | Br |
| 346. | CH₃ | CH₃ | CH₃ | I |
| 347. | CH₃ | CH₃ | CH₃ | CF₃ |
| 348. | CH₃ | CH₃ | CH₃ | CH₃S(O) |
| 349. | CH₃ | CH₃ | CH₃ | CH₃S(O)₂ |
| 350. | CH₃ | CH₃ | CH₃ | H |
| 351. | CH₃ | CH₃ | CH₃CH₂ | Cl |
| 352. | CH₃ | CH₃ | CH₃CH₂ | Br |
| 353. | CH₃ | CH₃ | CH₃CH₂ | I |
| 354. | CH₃ | CH₃ | CH₃CH₂ | CF₃ |
| 355. | CH₃ | CH₃ | CH₃CH₂ | CH₃S(O) |
| 356. | CH₃ | CH₃ | CH₃CH₂ | CH₃S(O)₂ |
| 357. | CH₃ | CH₃ | CH₃CH₂ | H |
| 358. | CH₃ | CH₃ | iPr | Cl |
| 359. | CH₃ | CH₃ | iPr | Br |
| 360. | CH₃ | CH₃ | iPr | I |
| 361. | CH₃ | CH₃ | iPr | CF₃ |
| 362. | CH₃ | CH₃ | iPr | CH₃S(O) |
| 363. | CH₃ | CH₃ | iPr | CH₃S(O)₂ |
| 364. | CH₃ | CH₃ | iPr | H |
| 365. | CH₃ | CH₃ | cPr | Cl |
| 366. | CH₃ | CH₃ | cPr | Br |
| 367. | CH₃ | CH₃ | cPr | I |
| 368. | CH₃ | CH₃ | cPr | CF₃ |
| 369. | CH₃ | CH₃ | cPr | CH₃S(O) |
| 370. | CH₃ | CH₃ | cPr | CH₃S(O)₂ |
| 371. | CH₃ | CH₃ | cPr | H |
| 372. | CH₃ | CH₃ | CHF₂CH₂ | Cl |
| 373. | CH₃ | CH₃ | CHF₂CH₂ | Br |
| 374. | CH₃ | CH₃ | CHF₂CH₂ | I |
| 375. | CH₃ | CH₃ | CHF₂CH₂ | CF₃ |
| 376. | CH₃ | CH₃ | CHF₂CH₂ | CH₃S(O) |
| 377. | CH₃ | CH₃ | CHF₂CH₂ | CH₃S(O)₂ |
| 378. | CH₃ | CH₃ | CHF₂CH₂ | H |
| 379. | CH₃ | CH₃ | CF₃CH₂ | Cl |
| 380. | CH₃ | CH₃ | CF₃CH₂ | Br |
| 381. | CH₃ | CH₃ | CF₃CH₂ | I |
| 382. | CH₃ | CH₃ | CF₃CH₂ | CF₃ |
| 383. | CH₃ | CH₃ | CF₃CH₂ | CH₃S(O) |
| 384. | CH₃ | CH₃ | CF₃CH₂ | CH₃S(O)₂ |
| 385. | CH₃ | CH₃ | CF₃CH₂ | H |
| 386. | CH₃ | CH₃CH₂ | CH₃CH₂ | Cl |
| 387. | CH₃ | CH₃CH₂ | CH₃CH₂ | Br |
| 388. | CH₃ | CH₃CH₂ | CH₃CH₂ | I |
| 389. | CH₃ | CH₃CH₂ | CH₃CH₂ | CF₃ |
| 390. | CH₃ | CH₃CH₂ | CH₃CH₂ | CH₃S(O) |
| 391. | CH₃ | CH₃CH₂ | CH₃CH₂ | CH₃S(O)₂ |
| 392. | CH₃ | CH₃CH₂ | CH₃CH₂ | H |
| 393. | CH₃ | CH₃CH₂ | iPr | Cl |
| 394. | CH₃ | CH₃CH₂ | iPr | Br |
| 395. | CH₃ | CH₃CH₂ | iPr | I |
| 396. | CH₃ | CH₃CH₂ | iPr | CF₃ |
| 397. | CH₃ | CH₃CH₂ | iPr | CH₃S(O) |
| 398. | CH₃ | CH₃CH₂ | iPr | CH₃S(O)₂ |
| 399. | CH₃ | CH₃CH₂ | iPr | H |
| 400. | CH₃ | CH₃CH₂ | cPr | Cl |
| 401. | CH₃ | CH₃CH₂ | cPr | Br |
| 402. | CH₃ | CH₃CH₂ | cPr | I |
| 403. | CH₃ | CH₃CH₂ | cPr | CF₃ |
| 404. | CH₃ | CH₃CH₂ | cPr | CH₃S(O) |
| 405. | CH₃ | CH₃CH₂ | cPr | CH₃S(O)₂ |
| 406. | CH₃ | CH₃CH₂ | cPr | H |
| 407. | CH₃ | CH₃CH₂ | CHF₂CH₂ | Cl |
| 408. | CH₃ | CH₃CH₂ | CHF₂CH₂ | Br |
| 409. | CH₃ | CH₃CH₂ | CHF₂CH₂ | I |
| 410. | CH₃ | CH₃CH₂ | CHF₂CH₂ | CF₃ |
| 411. | CH₃ | CH₃CH₂ | CHF₂CH₂ | CH₃S(O) |
| 412. | CH₃ | CH₃CH₂ | CHF₂CH₂ | CH₃S(O)₂ |
| 413. | CH₃ | CH₃CH₂ | CHF₂CH₂ | H |
| 414. | CH₃ | CH₃CH₂ | CF₃CH₂ | Cl |
| 415. | CH₃ | CH₃CH₂ | CF₃CH₂ | Br |
| 416. | CH₃ | CH₃CH₂ | CF₃CH₂ | I |
| 417. | CH₃ | CH₃CH₂ | CF₃CH₂ | CF₃ |
| 418. | CH₃ | CH₃CH₂ | CF₃CH₂ | CH₃S(O) |
| 419. | CH₃ | CH₃CH₂ | CF₃CH₂ | CH₃S(O)₂ |
| 420. | CH₃ | CH₃CH₂ | CF₃CH₂ | H |
| 421. | CH₃ | iPr | iPr | Cl |
| 422. | CH₃ | iPr | iPr | Br |
| 423. | CH₃ | iPr | iPr | I |
| 424. | CH₃ | iPr | iPr | CF₃ |
| 425. | CH₃ | iPr | iPr | CH₃S(O) |
| 426. | CH₃ | iPr | iPr | CH₃S(O)₂ |
| 427. | CH₃ | iPr | iPr | H |
| 428. | CH₃ | iPr | cPr | Cl |
| 429. | CH₃ | iPr | cPr | Br |
| 430. | CH₃ | iPr | cPr | I |
| 431. | CH₃ | iPr | cPr | CF₃ |
| 432. | CH₃ | iPr | cPr | CH₃S(O) |
| 433. | CH₃ | iPr | cPr | CH₃S(O)₂ |
| 434. | CH₃ | iPr | cPr | H |
| 435. | CH₃ | iPr | CHF₂CH₂ | Cl |
| 436. | CH₃ | iPr | CHF₂CH₂ | Br |
| 437. | CH₃ | iPr | CHF₂CH₂ | I |
| 438. | CH₃ | iPr | CHF₂CH₂ | CF₃ |
| 439. | CH₃ | iPr | CHF₂CH₂ | CH₃S(O) |
| 440. | CH₃ | iPr | CHF₂CH₂ | CH₃S(O)₂ |
| 441. | CH₃ | iPr | CHF₂CH₂ | H |
| 442. | CH₃ | iPr | CF₃CH₂ | Cl |
| 443. | CH₃ | iPr | CF₃CH₂ | Br |
| 444. | CH₃ | iPr | CF₃CH₂ | I |
| 445. | CH₃ | iPr | CF₃CH₂ | CF₃ |
| 446. | CH₃ | iPr | CF₃CH₂ | CH₃S(O) |
| 447. | CH₃ | iPr | CF₃CH₂ | CH₃S(O)₂ |
| 448. | CH₃ | iPr | CF₃CH₂ | H |
| 449. | CH₃ | cPr | cPr | Cl |
| 450. | CH₃ | cPr | cPr | Br |
| 451. | CH₃ | cPr | cPr | I |
| 452. | CH₃ | cPr | cPr | CF₃ |
| 453. | CH₃ | cPr | cPr | CH₃S(O) |
| 454. | CH₃ | cPr | cPr | CH₃S(O)₂ |
| 455. | CH₃ | cPr | cPr | H |
| 456. | CH₃ | cPr | CHF₂CH₂ | Cl |
| 457. | CH₃ | cPr | CHF₂CH₂ | Br |
| 458. | CH₃ | cPr | CHF₂CH₂ | I |
| 459. | CH₃ | cPr | CHF₂CH₂ | CF₃ |
| 460. | CH₃ | cPr | CHF₂CH₂ | CH₃S(O) |
| 461. | CH₃ | cPr | CHF₂CH₂ | CH₃S(O)₂ |
| 462. | CH₃ | cPr | CHF₂CH₂ | H |
| 463. | CH₃ | cPr | CF₃CH₂ | Cl |
| 464. | CH₃ | cPr | CF₃CH₂ | Br |
| 465. | CH₃ | cPr | CF₃CH₂ | I |
| 466. | CH₃ | cPr | CF₃CH₂ | CF₃ |
| 467. | CH₃ | cPr | CF₃CH₂ | CH₃S(O) |
| 468. | CH₃ | cPr | CF₃CH₂ | CH₃S(O)₂ |
| 469. | CH₃ | cPr | CF₃CH₂ | H |
| 470. | CH₃ | CHF₂CH₂ | CHF₂CH₂ | Cl |
| 471. | CH₃ | CHF₂CH₂ | CHF₂CH₂ | Br |
| 472. | CH₃ | CHF₂CH₂ | CHF₂CH₂ | I |
| 473. | CH₃ | CHF₂CH₂ | CHF₂CH₂ | CF₃ |
| 474. | CH₃ | CHF₂CH₂ | CHF₂CH₂ | CH₃S(O) |
| 475. | CH₃ | CHF₂CH₂ | CHF₂CH₂ | CH₃S(O)₂ |
| 476. | CH₃ | CHF₂CH₂ | CHF₂CH₂ | H |
| 477. | CH₃ | CHF₂CH₂ | CF₃CH₂ | Cl |
| 478. | CH₃ | CHF₂CH₂ | CF₃CH₂ | Br |
| 479. | CH₃ | CHF₂CH₂ | CF₃CH₂ | I |
| 480. | CH₃ | CHF₂CH₂ | CF₃CH₂ | CF₃ |
| 481. | CH₃ | CHF₂CH₂ | CF₃CH₂ | CH₃S(O) |
| 482. | CH₃ | CHF₂CH₂ | CF₃CH₂ | CH₃S(O)₂ |
| 483. | CH₃ | CHF₂CH₂ | CF₃CH₂ | H |
| 484. | CH₃ | CF₃CH₂ | CF₃CH₂ | Cl |
| 485. | CH₃ | CF₃CH₂ | CF₃CH₂ | Br |
| 486. | CH₃ | CF₃CH₂ | CF₃CH₂ | I |
| 487. | CH₃ | CF₃CH₂ | CF₃CH₂ | CF₃ |
| 488. | CH₃ | CF₃CH₂ | CF₃CH₂ | CH₃S(O) |
| 489. | CH₃ | CF₃CH₂ | CF₃CH₂ | CH₃S(O)₂ |
| 490. | CH₃ | CF₃CH₂ | CF₃CH₂ | H |
| 491. | CH₃ | CH₃O | CH₃ | Cl |
| 492. | CH₃ | CH₃O | CH₃ | Br |
| 493. | CH₃ | CH₃O | CH₃ | I |
| 494. | CH₃ | CH₃O | CH₃ | CF₃ |
| 495. | CH₃ | CH₃O | CH₃ | CH₃S(O) |
| 496. | CH₃ | CH₃O | CH₃ | CH₃S(O)₂ |
| 497. | CH₃ | CH₃O | CH₃ | H |
| 498. | CH₃ | CH₃O | CH₃CH₂ | Cl |
| 499. | CH₃ | CH₃O | CH₃CH₂ | Br |
| 500. | CH₃ | CH₃O | CH₃CH₂ | I |
| 501. | CH₃ | CH₃O | CH₃CH₂ | CF₃ |
| 502. | CH₃ | CH₃O | CH₃CH₂ | CH₃S(O) |
| 503. | CH₃ | CH₃O | CH₃CH₂ | CH₃S(O)₂ |
| 504. | CH₃ | CH₃O | CH₃CH₂ | H |
| 505. | CH₃ | CH₃O | iPr | Cl |
| 506. | CH₃ | CH₃O | iPr | Br |
| 507. | CH₃ | CH₃O | iPr | I |
| 508. | CH₃ | CH₃O | iPr | CF₃ |
| 509. | CH₃ | CH₃O | iPr | CH₃S(O) |
| 510. | CH₃ | CH₃O | iPr | CH₃S(O)₂ |
| 511. | CH₃ | CH₃O | iPr | H |
| 512. | CH₃ | CH₃O | cPr | Cl |
| 513. | CH₃ | CH₃O | cPr | Br |
| 514. | CH₃ | CH₃O | cPr | I |
| 515. | CH₃ | CH₃O | cPr | CF₃ |
| 516. | CH₃ | CH₃O | cPr | CH₃S(O) |
| 517. | CH₃ | CH₃O | cPr | CH₃S(O)₂ |
| 518. | CH₃ | CH₃O | cPr | H |
| 519. | CH₃ | CH₃O | CHF₂CH₂ | Cl |
| 520. | CH₃ | CH₃O | CHF₂CH₂ | Br |
| 521. | CH₃ | CH₃O | CHF₂CH₂ | I |
| 522. | CH₃ | CH₃O | CHF₂CH₂ | CF₃ |
| 523. | CH₃ | CH₃O | CHF₂CH₂ | CH₃S(O) |
| 524. | CH₃ | CH₃O | CHF₂CH₂ | CH₃S(O)₂ |
| 525. | CH₃ | CH₃O | CHF₂CH₂ | H |
| 526. | CH₃ | CH₃O | CF₃CH₂ | Cl |
| 527. | CH₃ | CH₃O | CF₃CH₂ | Br |
| 528. | CH₃ | CH₃O | CF₃CH₂ | I |
| 529. | CH₃ | CH₃O | CF₃CH₂ | CF₃ |
| 530. | CH₃ | CH₃O | CF₃CH₂ | CH₃S(O) |
| 531. | CH₃ | CH₃O | CF₃CH₂ | CH₃S(O)₂ |
| 532. | CH₃ | CH₃O | CF₃CH₂ | H |
| 533. | CH₃ | Ph | CH₃ | Cl |
| 534. | CH₃ | Ph | CH₃ | Br |
| 535. | CH₃ | Ph | CH₃ | I |
| 536. | CH₃ | Ph | CH₃ | CF₃ |
| 537. | CH₃ | Ph | CH₃ | CH₃S(O) |
| 538. | CH₃ | Ph | CH₃ | CH₃S(O)₂ |
| 539. | CH₃ | Ph | CH₃ | H |
| 540. | CH₃ | Ph | CH₃CH₂ | Cl |
| 541. | CH₃ | Ph | CH₃CH₂ | Br |
| 542. | CH₃ | Ph | CH₃CH₂ | I |
| 543. | CH₃ | Ph | CH₃CH₂ | CF₃ |
| 544. | CH₃ | Ph | CH₃CH₂ | CH₃S(O) |
| 545. | CH₃ | Ph | CH₃CH₂ | CH₃S(O)₂ |
| 546. | CH₃ | Ph | CH₃CH₂ | H |
| 547. | CH₃ | Ph | iPr | Cl |
| 548. | CH₃ | Ph | iPr | Br |
| 549. | CH₃ | Ph | iPr | I |
| 550. | CH₃ | Ph | iPr | CF₃ |
| 551. | CH₃ | Ph | iPr | CH₃S(O) |
| 552. | CH₃ | Ph | iPr | CH₃S(O)₂ |
| 553. | CH₃ | Ph | iPr | H |
| 554. | CH₃ | Ph | cPr | Cl |
| 555. | CH₃ | Ph | cPr | Br |
| 556. | CH₃ | Ph | cPr | I |
| 557. | CH₃ | Ph | cPr | CF₃ |
| 558. | CH₃ | Ph | cPr | CH₃S(O) |
| 559. | CH₃ | Ph | cPr | CH₃S(O)₂ |
| 560. | CH₃ | Ph | cPr | H |
| 561. | CH₃ | Ph | CHF₂CH₂ | Cl |
| 562. | CH₃ | Ph | CHF₂CH₂ | Br |
| 563. | CH₃ | Ph | CHF₂CH₂ | I |
| 564. | CH₃ | Ph | CHF₂CH₂ | CF₃ |
| 565. | CH₃ | Ph | CHF₂CH₂ | CH₃S(O) |
| 566. | CH₃ | Ph | CHF₂CH₂ | CH₃S(O)₂ |
| 567. | CH₃ | Ph | CHF₂CH₂ | H |
| 568. | CH₃ | Ph | CF₃CH₂ | Cl |
| 569. | CH₃ | Ph | CF₃CH₂ | Br |
| 570. | CH₃ | Ph | CF₃CH₂ | I |
| 571. | CH₃ | Ph | CF₃CH₂ | CF₃ |
| 572. | CH₃ | Ph | CF₃CH₂ | CH₃S(O) |
| 573. | CH₃ | Ph | CF₃CH₂ | CH₃S(O)₂ |
| 574. | CH₃ | Ph | CF₃CH₂ | H |
| 575. | CH₃ | 4-Cl-Ph | CH₃ | Cl |
| 576. | CH₃ | 4-Cl-Ph | CH₃ | Br |
| 577. | CH₃ | 4-Cl-Ph | CH₃ | I |
| 578. | CH₃ | 4-Cl-Ph | CH₃ | CF₃ |
| 579. | CH₃ | 4-Cl-Ph | CH₃ | CH₃S(O) |
| 580. | CH₃ | 4-Cl-Ph | CH₃ | CH₃S(O)₂ |
| 581. | CH₃ | 4-Cl-Ph | CH₃ | z |
| 582. | CH₃ | 4-Cl-Ph | CH₃CH₂ | Cl |
| 583. | CH₃ | 4-Cl-Ph | CH₃CH₂ | Br |
| 584. | CH₃ | 4-Cl-Ph | CH₃CH₂ | I |
| 585. | CH₃ | 4-Cl-Ph | CH₃CH₂ | CF₃ |
| 586. | CH₃ | 4-Cl-Ph | CH₃CH₂ | CH₃S(O) |
| 587. | CH₃ | 4-Cl-Ph | CH₃CH₂ | CH₃S(O)₂ |
| 588. | CH₃ | 4-Cl-Ph | CH₃CH₂ | H |
| 589. | CH₃ | 4-Cl-Ph | iPr | Cl |
| 590. | CH₃ | 4-Cl-Ph | iPr | Br |
| 591. | CH₃ | 4-Cl-Ph | iPr | I |
| 592. | CH₃ | 4-Cl-Ph | iPr | CF₃ |
| 593. | CH₃ | 4-Cl-Ph | iPr | CH₃S(O) |
| 594. | CH₃ | 4-Cl-Ph | iPr | CH₃S(O)₂ |
| 595. | CH₃ | 4-Cl-Ph | iPr | H |
| 596. | CH₃ | 4-Cl-Ph | cPr | Cl |
| 597. | CH₃ | 4-Cl-Ph | cPr | Br |
| 598. | CH₃ | 4-Cl-Ph | cPr | I |
| 599. | CH₃ | 4-Cl-Ph | cPr | CF₃ |
| 600. | CH₃ | 4-Cl-Ph | cPr | CH₃S(O) |
| 601. | CH₃ | 4-Cl-Ph | cPr | CH₃S(O)₂ |
| 602. | CH₃ | 4-Cl-Ph | cPr | H |
| 603. | CH₃ | 4-Cl-Ph | CHF₂CH₂ | Cl |
| 604. | CH₃ | 4-Cl-Ph | CHF₂CH₂ | Br |
| 605. | CH₃ | 4-Cl-Ph | CHF₂CH₂ | I |
| 606. | CH₃ | 4-Cl-Ph | CHF₂CH₂ | CF₃ |
| 607. | CH₃ | 4-Cl-Ph | CHF₂CH₂ | CH₃S(O) |
| 608. | CH₃ | 4-Cl-Ph | CHF₂CH₂ | CH₃S(O)₂ |
| 609. | CH₃ | 4-Cl-Ph | CHF₂CH₂ | H |
| 610. | CH₃ | 4-Cl-Ph | CF₃CH₂ | Cl |
| 611. | CH₃ | 4-Cl-Ph | CF₃CH₂ | Br |
| 612. | CH₃ | 4-Cl-Ph | CF₃CH₂ | I |
| 613. | CH₃ | 4-Cl-Ph | CF₃CH₂ | CF₃ |
| 614. | CH₃ | 4-Cl-Ph | CF₃CH₂ | CH₃S(O) |
| 615. | CH₃ | 4-Cl-Ph | CF₃CH₂ | CH₃S(O)₂ |
| 616. | CH₃ | 4-Cl-Ph | CF₃CH₂ | H |
| 617. | CH₃ | 4-CH₃O-Ph | CH₃ | Cl |
| 618. | CH₃ | 4-CH₃O-Ph | CH₃ | Br |
| 619. | CH₃ | 4-CH₃O-Ph | CH₃ | I |
| 620. | CH₃ | 4-CH₃O-Ph | CH₃ | CF₃ |
| 621. | CH₃ | 4-CH₃O-Ph | CH₃ | CH₃S(O) |
| 622. | CH₃ | 4-CH₃O-Ph | CH₃ | CH₃S(O)₂ |
| 623. | CH₃ | 4-CH₃O-Ph | CH₃ | H |
| 624. | CH₃ | 4-CH₃O-Ph | CH₃CH₂ | Cl |
| 625. | CH₃ | 4-CH₃O-Ph | CH₃CH₂ | Br |
| 626. | CH₃ | 4-CH₃O-Ph | CH₃CH₂ | I |
| 627. | CH₃ | 4-CH₃O-Ph | CH₃CH₂ | CF₃ |
| 628. | CH₃ | 4-CH₃O-Ph | CH₃CH₂ | CH₃S(O) |
| 629. | CH₃ | 4-CH₃O-Ph | CH₃CH₂ | CH₃S(O)₂ |
| 630. | CH₃ | 4-CH₃O-Ph | CH₃CH₂ | H |
| 631. | CH₃ | 4-CH₃O-Ph | iPr | Cl |
| 632. | CH₃ | 4-CH₃O-Ph | iPr | Br |
| 633. | CH₃ | 4-CH₃O-Ph | iPr | I |
| 634. | CH₃ | 4-CH₃O-Ph | iPr | CF₃ |
| 635. | CH₃ | 4-CH₃O-Ph | iPr | CH₃S(O) |
| 636. | CH₃ | 4-CH₃O-Ph | iPr | CH₃S(O)₂ |
| 637. | CH₃ | 4-CH₃O-Ph | iPr | H |
| 638. | CH₃ | 4-CH₃O-Ph | cPr | Cl |
| 639. | CH₃ | 4-CH₃O-Ph | cPr | Br |
| 640. | CH₃ | 4-CH₃O-Ph | cPr | I |
| 641. | CH₃ | 4-CH₃O-Ph | cPr | CF₃ |
| 642. | CH₃ | 4-CH₃O-Ph | cPr | CH₃S(O) |
| 643. | CH₃ | 4-CH₃O-Ph | cPr | CH₃S(O)₂ |
| 644. | CH₃ | 4-CH₃O-Ph | cPr | H |
| 645. | CH₃ | 4-CH₃O-Ph | CHF₂CH₂ | Cl |
| 646. | CH₃ | 4-CH₃O-Ph | CHF₂CH₂ | Br |
| 647. | CH₃ | 4-CH₃O-Ph | CHF₂CH₂ | I |
| 648. | CH₃ | 4-CH₃O-Ph | CHF₂CH₂ | CF₃ |
| 649. | CH₃ | 4-CH₃O-Ph | CHF₂CH₂ | CH₃S(O) |
| 650. | CH₃ | 4-CH₃O-Ph | CHF₂CH₂ | CH₃S(O)₂ |
| 651. | CH₃ | 4-CH₃O-Ph | CHF₂CH₂ | H |
| 652. | CH₃ | 4-CH₃O-Ph | CF₃CH₂ | Cl |
| 653. | CH₃ | 4-CH₃O-Ph | CF₃CH₂ | Br |
| 654. | CH₃ | 4-CH₃O-Ph | CF₃CH₂ | I |
| 655. | CH₃ | 4-CH₃O-Ph | CF₃CH₂ | CF₃ |
| 656. | CH₃ | 4-CH₃O-Ph | CF₃CH₂ | CH₃S(O) |
| 657. | CH₃ | 4-CH₃O-Ph | CF₃CH₂ | CH₃S(O)₂ |
| 658. | CH₃ | 4-CH₃O-Ph | CF₃CH₂ | H |
| 659. | CH₃ | 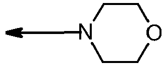 | | Cl |
| 660. | CH₃ | 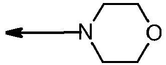 | | Br |
| 661. | CH₃ | 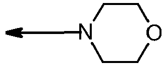 | | I |
| 662. | CH₃ | 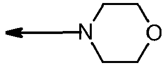 | | CF₃ |
| 663. | CH₃ | 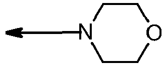 | | CH₃S(O) |
| 664. | CH₃ | 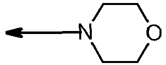 | | CH₃S(O)₂ |
| 665. | CH₃ | 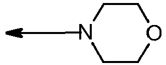 | | H |
| 666. | CH₃ | 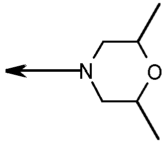 | | Cl |
| 667. | CH₃ | 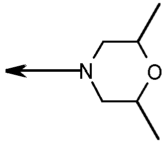 | | Br |
| 668. | CH₃ | 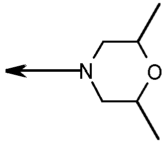 | | I |
| 669. | CH₃ | 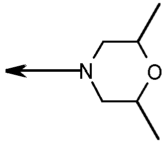 | | CF₃ |
| 670. | CH₃ | 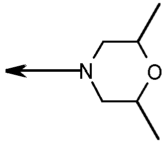 | | CH₃S(O) |
| 671. | CH₃ | 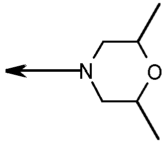 | | CH₃S(O)₂ |
| 672. | CH₃ | 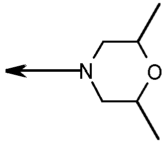 | | H |
| 673. | CH₃ | 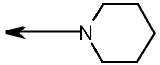 | | Cl |
| 674. | CH₃ | 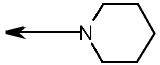 | | Br |
| 675. | CH₃ | 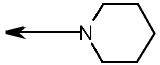 | | I |
| 676. | CH₃ | 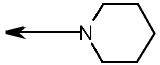 | | CF₃ |
| 677. | CH₃ | 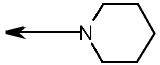 | | CH₃S(O) |
| 678. | CH₃ | 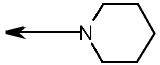 | | CH₃S(O)₂ |
| 679. | CH₃ | 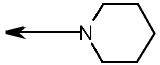 | | H |
| 680. | CH₃ | 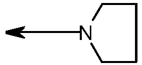 | | Cl |
| 681. | CH₃ | 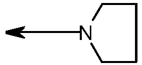 | | Br |
| 682. | CH₃ | 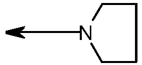 | | I |
| 683. | CH₃ | 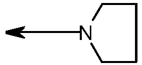 | | CF₃ |
| 684. | CH₃ | 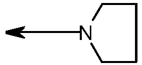 | | CH₃S(O) |
| 685. | CH₃ | 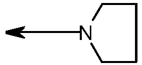 | | CH₃S(O)₂ |
| 686. | CH₃ | 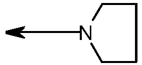 | | H |

In Table A, cPr means cyclopropyl and iPr means isopropyl (= 1-methylethyl).

It is apparent to a skilled person that in lines 316 to 343 and in lines 659 to 686 of table A the meanings given for the combination R^{2c}/R^{2d} relate to the moiety NR^{2c}R^{2d} in formulae I.A.I to I.D.VI.

Amongst the combinations of variables R¹, R^{2c}, R^{2d} and R³, those are preferred, where R¹ is CH₃ and where R³ is H, Cl, Br or CF₃.

Amongst the combinations of variables R¹, R^{2c}, R^{2d} and R³, those are preferred, where R¹ is Cl and where R³ is H, Cl, Br or CF₃.

Amongst the combinations of variables R¹, R^{2c}, R^{2d} and R³, those are preferred, where R¹ is Cl or CH₃, R^{2c} is CH₃ or C₂H₅, R^{2d} is CH₂CF₃ and where R³ is H, Cl, Br or CF₃.

Particular preference is given to compounds of the formula formulae I.A.I and I.A.VII, where R¹, R^{2c}, R^{2d} and R³ are as defined in Table A and where R¹, R^{2c}, R^{2d} and R³ are in particular as defined in the following tables A' and A".

Particular preference is also given to compounds of the formulae I.D.I and I.D.VII, where R¹, R^{2c}, R^{2d} and R³ are as defined in Table A and where R¹, R^{2c}, R^{2d} and R³ are in particular as defined in the following tables A' and A".

**Table A'**

| | **R¹** | **R^{2c}** | **R^{2d}** | **R³** |
|---|---|---|---|---|
| 687. | CH₃ | CH₃ | CH₃ | H |
| 688. | CH₃ | CH₃ | CH₂CH₃ | H |
| 689. | CH₃ | CH₃ | CH₂CH₃ | H |
| 690. | CH₃ | CH₂CH₃ | CH₂CH₃ | H |
| 691. | CH₃ | CH₃ | CH₂CF₃ | H |
| 692. | CH₃ | CH₂CH₃ | CH₂CF₃ | H |
| 693. | CH₃ | CH₃ | CH₂CHF₂ | H |
| 694. | CH₃ | CH₂CH₃ | CH₂CHF₂ | H |
| 695. | CH₃ | CH₃ | CH(CH₃)₂ | H |
| 696. | CH₃ | c-C₃H₅ | c-C₃H₅ | H |
| 697. | Cl | CH₃ | CH₃ | H |
| 698. | Cl | CH₃ | CH₂CH₃ | H |
| 699. | Cl | CH₂CH₃ | CH₂CH₃ | H |
| 700. | Cl | CH₃ | CH₂CF₃ | H |
| 701. | Cl | CH₂CH₃ | CH₂CF₃ | H |
| 702. | Cl | CH₃ | CH₂CHF₂ | H |
| 703. | Cl | CH₂CH₃ | CH₂CHF₂ | H |
| 704. | Cl | CH₃ | CH(CH₃)₂ | H |
| 705. | Cl | c-C₃H₅ | c-C₃H₅ | H |
| 706. | CH₃ | CH₃ | CH₃ | Cl |
| 707. | CH₃ | CH₃ | CH₂CH₃ | Cl |
| 708. | CH₃ | CH₃ | CH₂CH₃ | Cl |
| 709. | CH₃ | CH₂CH₃ | CH₂CH₃ | Cl |
| 710. | CH₃ | CH₃ | CH₂CF₃ | Cl |
| 711. | CH₃ | CH₂CH₃ | CH₂CF₃ | Cl |
| 712. | CH₃ | CH₃ | CH₂CHF₂ | Cl |
| 713. | CH₃ | CH₂CH₃ | CH₂CHF₂ | Cl |
| 714. | CH₃ | CH₃ | CH(CH₃)₂ | Cl |
| 715. | CH₃ | c-C₃H₅ | c-C₃H₅ | Cl |
| 716. | Cl | CH₃ | CH₃ | Cl |
| 717. | Cl | CH₃ | CH₂CH₃ | Cl |
| 718. | Cl | CH₂CH₃ | CH₂CH₃ | Cl |
| 719. | Cl | CH₃ | CH₂CF₃ | Cl |
| 720. | Cl | CH₂CH₃ | CH₂CF₃ | Cl |
| 721. | Cl | CH₃ | CH₂CHF₂ | Cl |
| 722. | Cl | CH₂CH₃ | CH₂CHF₂ | Cl |
| 723. | Cl | CH₃ | CH(CH₃)₂ | Cl |
| 724. | Cl | c-C₃H₅ | c-C₃H₅ | Cl |
| 725. | CH₃ | CH₃ | CH₃ | Br |
| 726. | CH₃ | CH₃ | CH₂CH₃ | Br |
| 727. | CH₃ | CH₂CH₃ | CH₂CH₃ | Br |
| 728. | CH₃ | CH₃ | CH₂CF₃ | Br |
| 729. | CH₃ | CH₂CH₃ | CH₂CF₃ | Br |
| 730. | CH₃ | CH₃ | CH₂CHF₂ | Br |
| 731. | CH₃ | CH₂CH₃ | CH₂CHF₂ | Br |
| 732. | CH₃ | CH₃ | CH(CH₃)₂ | Br |
| 733. | CH₃ | c-C₃H₅ | c-C₃H₅ | Br |
| 734. | Cl | CH₃ | CH₃ | Br |
| 735. | Cl | CH₃ | CH₂CH₃ | Br |
| 736. | Cl | CH₂CH₃ | CH₂CH₃ | Br |
| 737. | Cl | CH₃ | CH₂CF₃ | Br |
| 738. | Cl | CH₂CH₃ | CH₂CF₃ | Br |
| 739. | Cl | CH₃ | CH₂CHF₂ | Br |
| 740. | Cl | CH₂CH₃ | CH₂CHF₂ | Br |
| 741. | Cl | CH₃ | CH(CH₃)₂ | Br |
| 742. | Cl | c-C₃H₅ | c-C₃H₅ | Br |
| 743. | CH₃ | CH₃ | CH₃ | CF₃ |
| 744. | CH₃ | CH₃ | CH₂CH₃ | CF₃ |
| 745. | CH₃ | CH₂CH₃ | CH₂CH₃ | CF₃ |
| 746. | CH₃ | CH₃ | CH₂CF₃ | CF₃ |
| 747. | CH₃ | CH₂CH₃ | CH₂CF₃ | CF₃ |
| 748. | CH₃ | CH₃ | CH₂CHF₂ | CF₃ |
| 749. | CH₃ | CH₂CH₃ | CH₂CHF₂ | CF₃ |
| 750. | CH₃ | CH₃ | CH(CH₃)₂ | CF₃ |
| 751. | CH₃ | c-C₃H₅ | c-C₃H₅ | CF₃ |
| 752. | Cl | CH₃ | CH₃ | CF₃ |
| 753. | Cl | CH₃ | CH₂CH₃ | CF₃ |
| 754. | Cl | CH₂CH₃ | CH₂CH₃ | CF₃ |
| 755. | Cl | CH₃ | CH₂CF₃ | CF₃ |
| 756. | Cl | CH₂CH₃ | CH₂CF₃ | CF₃ |
| 757. | Cl | CH₃ | CH₂CHF₂ | CF₃ |
| 758. | Cl | CH₂CH₃ | CH₂CHF₂ | CF₃ |
| 759. | Cl | CH₃ | CH(CH₃)₂ | CF₃ |
| 760. | Cl | c-C₃H₅ | c-C₃H₅ | CF₃ |

**Table A'**

| | **R¹** | **NR^{2c}R^{2d}** | **R³** |
|---|---|---|---|
| 761. | CH₃ | pyrrolidin-1-yl | H |
| 762. | CH₃ | piperidin-1-yl | H |
| 763. | CH₃ | morpholin-4-yl | H |
| 764. | CH₃ | 2,6-dimethylmorpholin-4-yl | H |
| 765. | Cl | pyrrolidin-1-yl | H |
| 766. | Cl | piperidin-1-yl | H |
| 767. | Cl | morpholin-4-yl | H |
| 768. | Cl | 2,6-dimethylmorpholin-4-yl | H |
| 769. | CH₃ | pyrrolidin-1-yl | Cl |
| 770. | CH₃ | piperidin-1-yl | Cl |
| 771. | CH₃ | morpholin-4-yl | Cl |
| 772. | CH₃ | 2,6-dimethylmorpholin-4-yl | Cl |
| 773. | Cl | pyrrolidin-1-yl | Cl |
| 774. | Cl | piperidin-1-yl | Cl |
| 775. | Cl | morpholin-4-yl | Cl |
| 776. | Cl | 2,6-dimethylmorpholin-4-yl | Cl |
| 777. | CH₃ | pyrrolidin-1-yl | Br |
| 778. | CH₃ | piperidin-1-yl | Br |
| 779. | CH₃ | morpholin-4-yl | Br |
| 780. | CH₃ | 2,6-dimethylmorpholin-4-yl | Br |
| 781. | Cl | pyrrolidin-1-yl | Br |
| 782. | Cl | piperidin-1-yl | Br |
| 783. | Cl | morpholin-4-yl | Br |
| 784. | Cl | 2,6-dimethylmorpholin-4-yl | Br |
| 785. | CH₃ | pyrrolidin-1-yl | CF₃ |
| 786. | CH₃ | piperidin-1-yl | CF₃ |
| 787. | CH₃ | morpholin-4-yl | CF₃ |
| 788. | CH₃ | 2,6-dimethylmorpholin-4-yl | CF₃ |
| 789. | Cl | pyrrolidin-1-yl | CF₃ |
| 790. | Cl | piperidin-1-yl | CF₃ |
| 791. | Cl | morpholin-4-yl | CF₃ |
| 792. | Cl | 2,6-dimethylmorpholin-4-yl | CF₃ |

The compounds of formula I can be prepared by standard methods of organic chemistry, e.g. by the methods described in the schemes below. The substituents, variables and indices used in the schemes are as defined above for the compounds of formula I, if not specified otherwise.

The compounds of formula I.A can be prepared analogous to Scheme 1 below.

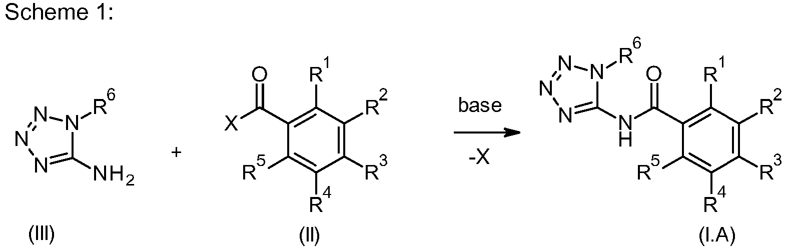

Likewise, the compounds of formula I.B can be prepared analogous to Scheme 2 below:

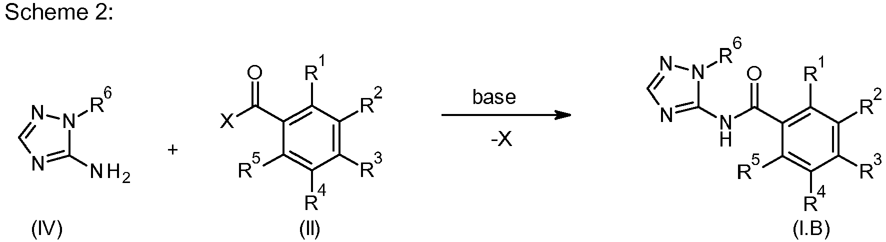

Likewise, the compounds of formula I.C can be prepared analogous to Scheme 3 below:

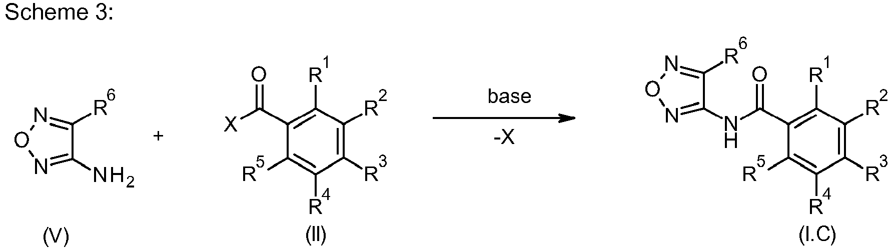

Likewise, the compounds of fromula I.D can be preparedd analogous to Scheme 4 below:

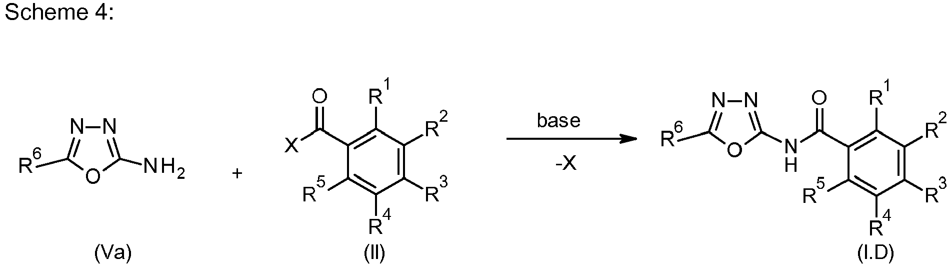

5-Amino-1-R-tetrazole compounds of formula III can be reacted with benzoyl derivatives of formula II to afford compounds of formula I.A. Likewise, 5-amino-1-R-1,2,4-triazole of formula IV can be reacted with benzoyl derivatives of formula II to afford compounds of formula I.B. Likewise, 4-amino-1,2,5-oxadiazole compounds of formula V can be reacted with benzoyl derivatives of formula II to afford compounds of the formula I.C. Likewise, 4-amino-1,3,4-oxadiazole compounds of formula Va can be reacted with benzoyl derivatives of formula II to afford compounds of the formula I.D. Herein, X is a leaving group, such as halogen, in particular Cl, an anhydride residue or an active ester residue, such as pentafluorophenoxy. Especially in case of X being halogen the reaction is suitably carried out in the presence of a base. Suitable bases are for example carbonates, such as lithium, sodium or potassium carbonates, amines, such as trimethylamine or triethylamine, and basic N-heterocycles, such as pyridine, 2,6-dimethylpyridine or 2,4,6-trimethylpyridine. Suitable solvents are in particular aprotic solvents such as pentane, hexane, heptane, octane, cyclohexane, dichloromethane, chloroform, 1,2-dichlorethane, benzene, chlorobenzene, toluene, the xylenes, dichlorobenzene, trimethylbenzene, pyridine, 2,6-dimethylpyridine, 2,4,6-trimethylpyridine, acetonitrile, diethyl ether, tetrahydrofuran, 2-methyl tetrahydrofuran, methyl tert-butylether, 1,4-dioxane, N,N-dimethyl formamide, N-methyl pyrrolidinone or mixtures thereof. The starting materials are generally reacted with one another in equimolar or nearly equimolar amounts at a reaction temperature usually in the range of -20°C to 100°C and preferably in the range of -5°C to 50°C.

Alternatively, compounds of formula I can also be prepared as shown in Schemes 5, 6, 7 and 8. Reaction of 5-amino-1-R-tetrazole of formula III with a benzoic acid derivative of formula VI yields compound I.A. Likewise, reaction of 5-amino-1-R-1,2,4-triazole of formula IV with a benzoic acid derivative of formula VI yields compound I.B. Likeweise, reaction of a 4-amino-1,2,5-oxadiazole compound V with a benzoic acid derivative of formula VI yields compound I.C. Likeweise, reaction of a 4-amino-1,3,4-oxadiazole compound Va with a benzoic acid derivative of formula VI yields compound I.D. The reaction is preferably carried out in the presence of a suitable activating agent, which converts the acid group of compound VI into an activated ester or amide. For this purpose activating agents known in the art, such as 1,1',carbonyldiimidazole (CDI), dicyclohexyl carbodiimide (DCC), 1-ethyl-3-(3-dimethylaminopropyl)carbodiimide (EDC) or 2,4,6-tripropyl-1,3,5,2,4,6-trioxatriphosphorinane-2,4,6-trioxide (T3P) can be employed. The activated ester or amide can be formed, depending in particular on the specific activating agent used, either in situ by contacting compound V with the activating agent in the presence of compound III or IV, or in a separate step prior to the reaction with compound III or IV. It may be advantageous, especially in cases where DCC or EDC are used as activating agent, to include further additives in the activating reaction, such as hydroxybenzotriazole (HOBt), nitrophenol, pentafluorophenol, 2,4,5-trichlorophenol or N-hydroxysuccinimide. It may further be advantageous to prepare the activated ester or amide in the presence of a base, for example a tertiary amine. The activated ester or amide is either in situ or subsequently reacted with the amine of formula III or IV to afford the amide of formula I. The reaction normally takes place in anhydrous inert solvents, such as chlorinated hydrocarbons, e.g. dichloromethane or dichloroethane, ethers, e.g. tetrahydrofuran or 1,4-dioxane or carboxamides, e.g. N,N-dimethylformamide, N,N-dimethylacetamide or N-methylpyrrolidone. The reaction is ordinarily carried out at temperatures in the range from -20°C to +25°C.

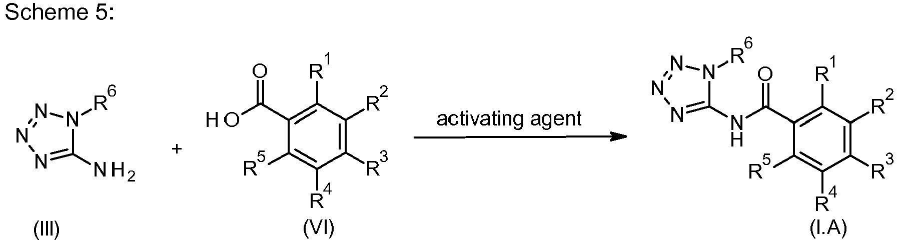

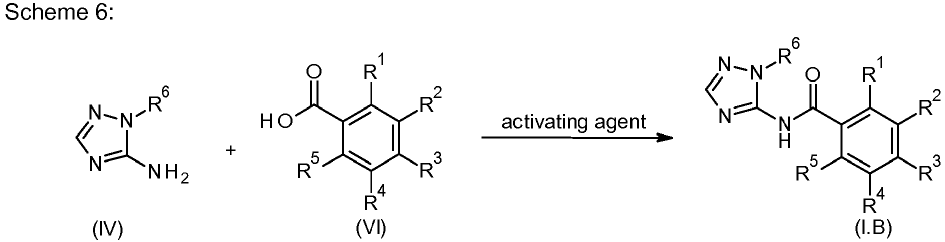

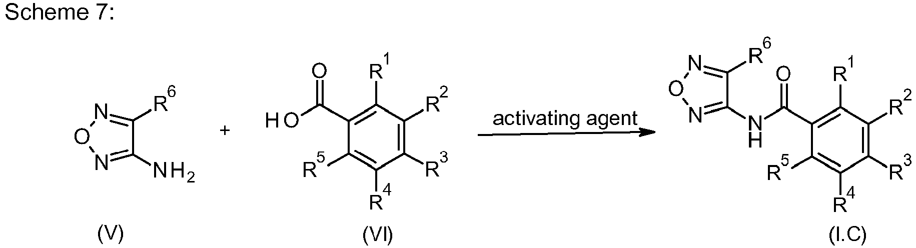

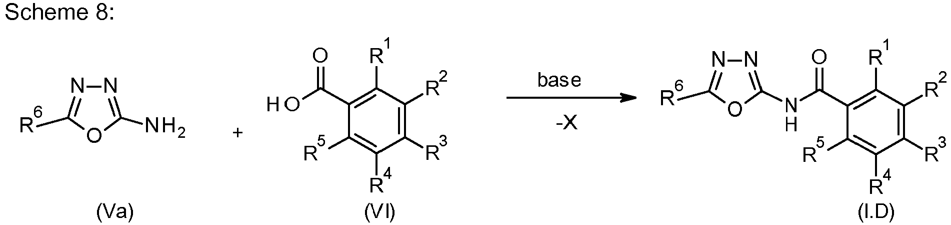

The 5-amino-1-R-tetrazoles of formula III, where R⁶ is, for example, hydrogen or an alkyl, are either commercially available or are obtainable according to methods known from the literature. For example, 5-amino-1-R-tetrazole can be prepared from 5-aminotetrazole according to the method described in the Journal of the American Chemical Society, 1954, 76, 923-924 (Scheme 9).

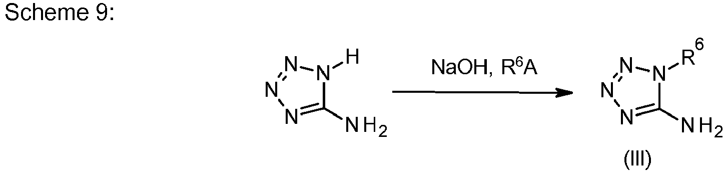

Alternatively, 5-amino-1-R-tetrazole compounds of formula III can be prepared according to the method described in the Journal of the American Chemical Society, 1954, 76, 88-89 (Scheme 10).

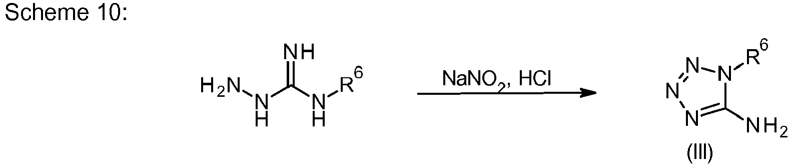

As shown in Scheme 11, 5-amino-1-R-triazoles of formula IV are either commercially available or are obtainable according to methods described in the literature. For example, 5-amino-1-R-triazole can be prepared from 5-aminotriazole according to the method described in Zeitschrift für Chemie, 1990, 30, 12, 436-437.

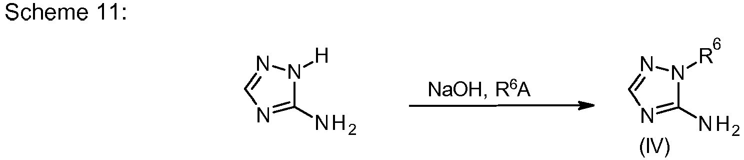

Alternatively, 5-amino-1-R-triazole compounds of formula IV, can also be prepared analogous to the synthesis described in Chemische Berichte, 1964, 97, 2, 396-404, as shown in Scheme 12.

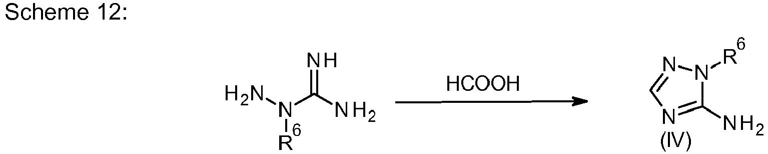

The compounds of formulae III, IV and V and the benzoic acid precursors of formulae II and V can be obtained by purchase or can be prepared by processes known in the art or disclosed in the literature, e.g. in WO 9746530, WO 9831676, WO 9831681, WO 2002/018352, WO 2000/003988, US 2007/0191335, US 6277847.

The 4-amino-1,2,5-oxadiazole compounds of the formula V are either commercially available or are obtainable according to methods known from the literature. For example, 3-alkyl-4-amino-1,2,5-oxadiazoles can be prepared from β-ketoesters pursuant to a procedure described in Russian Chemical Bulletin, Int. Ed., 54(4), 1032-1037 (2005), as depicted in Scheme 13.

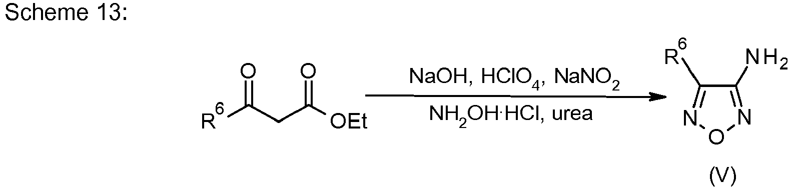

As shown in Scheme 14, the compounds of the formula V, where R⁶ is halogen, can be prepared from commercially available 3,4-diamino-1,2,5-oxadiazole according to procedures described in the literature, e.g. by the Sandmeyer-type reaction disclosed in Heteroatom Chemistry, 15(3), 199-207 (2004).

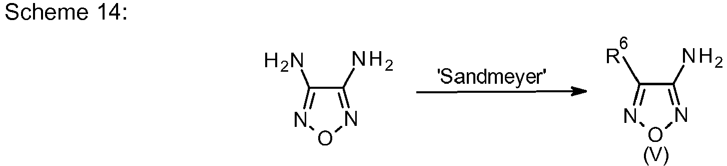

As shown in Scheme 15, the compounds of the formula V, where R⁶ is a nucleophilic residue, can be prepared by introducing the nucleophilic residue via the substitution of a leaving group L, e.g. halogene, in the 4-position of the 1,2,5-oxadiazoles compounds of formula IX in accordance to precedures disclosed, for example in Journal of Chemical Research, Synopses (6), 190 (1985), in Izvestiya Akademii Nauk SSSR, Seriya Khimicheskaya (9), 2086-8 (1986) or in Russian Chemical Bulletin (Translation of Izvestiya Akademii Nauk, Seriya Khimicheskaya), 53(3), 596-614 (2004).

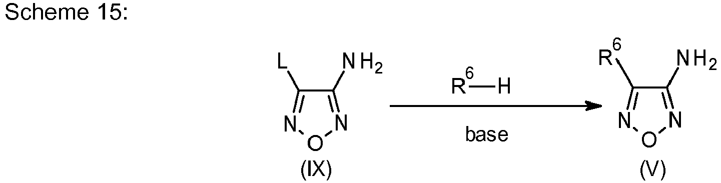

The compounds of the formula (VI), where R² is Z²-NH-C(O)-NR^{2c}R^{2d} can be prepared from the corresponding substituted 3-aminobenzoates of the formula (X), which comprises reacting the compound of formula (X) with phosgene or a phosgene equivalent (XI), such as diphosgene, i.e. trichloromethyl chloroformiate, or triphosgene, i.e. bis-trichloromethylcarbonate, and a secondary amine of the formula (XII), followed by subsequent hydrolysis as depicted in the following scheme 1. Instead of phosgene or the phosgene equivalent carbonyldiimidazole may be used.

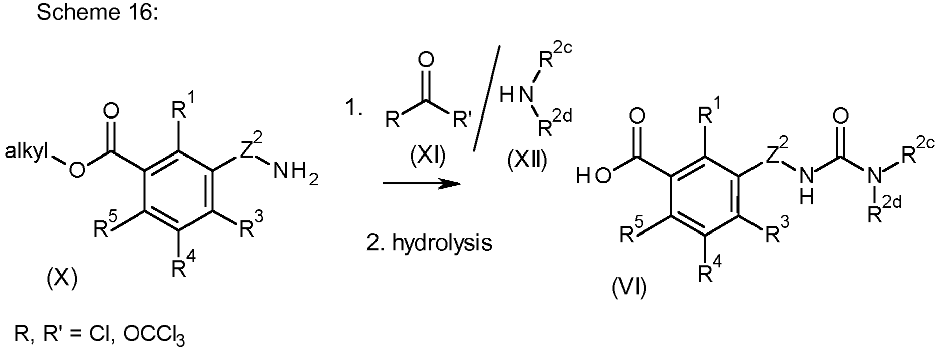

In scheme 16, alkyl means lower alkyl having 1 to 4 carbon atoms, in particular methyl or ethyl. The reaction of the compound of the formula (X) with phosgene or phosgene equivalent (XI) and the secondary amine of formula (XII) can be performed by analogy to the preparation of mixed ureas by reaction of two different amine with phosgene or phosgene equivalent. Preferably, the compound of the formula (X) is firstly reacted with phosgene or phosgene equivalent (XI) to obtain an intermediate compound or compound mixture, which is subsequently reacted with the secondary amine of the formulal (XII). The intermediate compound or compound mixture may be isolated from the reaction mixture. For economical reasons, the intermediate compound or compound mixture is usually not isolated but the reaction mixture obtained from the reaction of the compound (X) with the phosgene or phosgene equivalent (XI) is subjected to the reaction with the secondary amine of formula (XII). Compounds of formula (II) can be easily prepared from the compounds of formula (VI) by standard procedures.

As a rule, the compounds of formula I including their stereoisomers, salts, and tautomers, and their precursors in the synthesis process, can be prepared by the methods described above. If individual compounds can not be prepared via the above-described routes, they can be prepared by derivatization of other compounds I or the respective precursor or by customary modifications of the synthesis routes described. For example, in individual cases, certain compounds of formula I can advantageously be prepared from other compounds of formula I by derivatization, e.g. by ester hydrolysis, amidation, esterification, ether cleavage, olefination, reduction, oxidation and the like, or by customary modifications of the synthesis routes described.

The reaction mixtures are worked up in the customary manner, for example by mixing with water, separating the phases, and, if appropriate, purifying the crude products by chromatography, for example on alumina or on silica gel. Some of the intermediates and end products may be obtained in the form of colorless or pale brown viscous oils which are freed or purified from volatile components under reduced pressure and at moderately elevated temperature. If the intermediates and end products are obtained as solids, they may be purified by recrystallization or trituration.

The compounds of formula I and their agriculturally suitable salts are useful as herbicides. They are useful as such or as an appropriately formulated composition. The herbicidal compositions comprising the compound I, in particular the preferred aspects thereof, control vegetation on non-crop areas very efficiently, especially at high rates of application. They act against broad-leaved weeds and weed grasses in crops such as wheat, rice, corn, soybeans and cotton without causing any significant damage to the crop plants. This effect is mainly observed at low rates of application.

Depending on the application method in question, the compounds of formula I, in particular the preferred aspects thereof, or compositions comprising them can additionally be employed in a further number of crop plants for eliminating unwanted plants. Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

The compounds of the present invention are particularly suitable for use in crops from the family poaceae, in particular crops of the tribum triticeae, e.g. crops of the generae hordeum, sorghum, triticium and secale, and crops of the generae zea, e.g. zea mays and oryza, e.g. oryza sativa.

The term "crop plants" also includes plants which have been modified by breeding, mutagenesis or genetic engineering. Genetically modified plants are plants whose genetic material has been modified in a manner which does not occur under natural conditions by crossing, mutations or natural recombination (i.e. reassembly of the genetic information). Here, in general, one or more genes are integrated into the genetic material of the plant to improve the properties of the plant.

Accordingly, the term "crop plants" also includes plants which, by breeding and genetic engineering, have acquired tolerance to certain classes of herbicides, such as hydroxyphenylpyruvate dioxygenase (HPPD) inhibitors, acetolactate synthase (ALS) inhibitors, such as, for example, sulfonylureas (EP-A-0257993, US 5,013,659) or imidazolinones (see, for example, US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073), enolpyruvylshikimate 3-phosphate synthase (EPSPS) inhibitors, such as, for example, glyphosate (see, for example, WO 92/00377), glutamine synthetase (GS) inhibitors, such as, for example, glufosinate (see, for example, EP-A-0242236, EP-A-242246), or oxynil herbicides (see, for example, US 5,559,024).

In a preferred embodiment, the term "crop plants" refers to plants that comprise in their genomes a gene encoding a herbicide-tolerant wild-type or mutated HPPD protein. Such a gene may be an endogenous gene or a transgene, as described hereinafter.

By a "herbicide-tolerant" or "herbicide-resistant" plant, it is intended that a plant that is tolerant or resistant to at least one herbicide at a level that would normally kill, or inhibit the growth of, a normal or wild-type plant. By "herbicide-tolerant wild-type or mutated HPPD protein" or "herbicide -resistant wild-type or mutated HPPD protein", it is intended that such a HPPD protein displays higher HPPD activity, relative to the HPPD activity of a wild-type or reference HPPD protein, when in the presence of at least one herbicide that is known to interfere with HPPD activity and at a concentration or level of the herbicide that is known to inhibit the HPPD activity of the reference wild-type HPPD protein. Furthermore, the HPPD activity of such a herbicide-tolerant or herbicide-resistant HPPD protein may be referred to herein as "herbicide-tolerant" or "herbicide-resistant" HPPD activity.

The term "mutated HPPD nucleic acid" refers to an HPPD nucleic acid having a sequence that is mutated from a wild-type HPPD nucleic acid and that confers increased " HPPD-inhibiting herbicide" tolerance to a plant in which it is expressed. Furthermore, the term " mutated hydroxyphenyl pyruvate dioxygenase (mutated HPPD)" refers to the replacement of an amino acid of the wild-type primary sequences SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, a variant, a derivative, a homologue, an orthologue, or paralogue thereof, with another amino acid. The expression "mutated amino acid" will be used below to designate the amino acid which is replaced by another amino acid, thereby designating the site of the mutation in the primary sequence of the protein.

Several HPPDs and their primary sequences have been described in the state of the art, in particular the HPPDs of bacteria such as Pseudomonas (Ruetschi etal., Eur.J.Biochem., 205, 459-466, 1992, WO96/38567), of plants such as Arabidopsis (WO96/38567, Genebank AF047834) or of carrot (WO96/38567, Genebank 87257), of Coccicoides (Genebank COITRP), HPPDs of Brassica, cotton, Synechocystis, and tomato (US 7,297,541), of mammals such as the mouse or the pig. Furthermore, artificial HPPD sequences have been described, for example in US6,768,044; US6,268,549;
In a preferred embodiment, the nucleotide sequence of (i) comprises the sequence of SEQ ID NO: 1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69 or a variant or derivative thereof.

In a particularly preferred embodiment, the mutated HPPD nucleic acid useful for the present invention comprises a mutated nucleic acid sequence of SEQ ID NO: 1 or SEQ ID NO: 52, or a variant or derivative thereof.

Furthermore, it will be understood by the person skilled in the art that the nucleotide sequences of (i) or (ii) encompass homologues, paralogues and orthologues of SEQ ID NO: 1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69, as defined hereinafter.

The term "variant" with respect to a sequence (e.g., a polypeptide or nucleic acid sequence such as - for example - a transcription regulating nucleotide sequence of the invention) is intended to mean substantially similar sequences. For nucleotide sequences comprising an open reading frame, variants include those sequences that, because of the degeneracy of the genetic code, encode the identical amino acid sequence of the native protein. Naturally occurring allelic variants such as these can be identified with the use of well-known molecular biology techniques, as, for example, with polymerase chain reaction (PCR) and hybridization techniques. Variant nucleotide sequences also include synthetically derived nucleotide sequences, such as those generated, for example, by using site-directed mutagenesis and for open reading frames, encode the native protein, as well as those that encode a polypeptide having amino acid substitutions relative to the native protein. Generally, nucleotide sequence variants of the invention will have at least 30, 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide " sequence identity" to the nucleotide sequence of SEQ ID NO:1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69, 47, or 49. By "variant" polypeptide is intended a polypeptide derived from the protein of SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Such variants may result from, for example, genetic polymorphism or from human manipulation. Methods for such manipulations are generally known in the art.

In a preferred embodiment, variants of the polynucleotides useful for the present invention will have at least 30, 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide " sequence identity" to the nucleotide sequence of SEQ ID NO:1, 47, 49, or SEQ ID NO: 52.

It is recognized that the polynucleotide molecules and polypeptides of the invention encompass polynucleotide molecules and polypeptides comprising a nucleotide or an amino acid sequence that is sufficiently identical to nucleotide sequences set forth in SEQ ID NOs: 1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69, 47, or 49, or to the amino acid sequences set forth in SEQ ID NOs: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, 48, or 50 . The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity.

"Sequence identity" refers to the extent to which two optimally aligned DNA or amino acid sequences are invariant throughout a window of alignment of components, e.g., nucleotides or amino acids. An "identity fraction" for aligned segments of a test sequence and a reference sequence is the number of identical components that are shared by the two aligned sequences divided by the total number of components in reference sequence segment, i.e., the entire reference sequence or a smaller defined part of the reference sequence. "Percent identity" is the identity fraction times 100. Optimal alignment of sequences for aligning a comparison window are well known to those skilled in the art and may be conducted by tools such as the local homology algorithm of Smith and Waterman, the homology alignment algorithm of Needleman and Wunsch, the search for similarity method of Pearson and Lipman, and preferably by computerized implementations of these algorithms such as GAP, BESTFIT, FASTA, and TFASTA available as part of the GCG. Wisconsin Package. (Accelrys Inc. Burlington, Mass.)

The terms "polynucleotide(s)", "nucleic acid sequence(s)", "nucleotide sequence(s)", " nucleic acid(s)" , " nucleic acid molecule" are used interchangeably herein and refer to nucleotides, either ribonucleotides or deoxyribonucleotides or a combination of both, in a polymeric unbranched form of any length.

"Derivatives" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag• 100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α -helical structures or β -sheet structures). Amino acid substitutions are typically of single residues, but may be clustered depending upon functional constraints placed upon the polypeptide and may range from 1 to 10 amino acids; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds).

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuikChange Site Directed mutagenesis (Stratagene, San Diego, CA), PCR-mediated site-directed mutagenesis or other site-directed mutagenesis protocols.

"Derivatives" further include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

"Orthologues" and "paralogues" encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

It is well-known in the art that paralogues and orthologues may share distinct domains harboring suitable amino acid residues at given sites, such as binding pockets for particular substrates or binding motifs for interaction with other proteins.

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

By substituting one or more of the key amino acid residues, the herbicide tolerance or resistance of a plant to the herbicide as described herein could be remarkably increased as compared to the activity of the wild type HPPD enzymes with SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67. Preferred substitutions of mutated HPPD are those that increase the herbicide tolerance of the plant, but leave the biological activitiy of the dioxygenase activity substantially unaffected.

It will be understood by the person skilled in the art that amino acids located in a close proximity to the positions of amino acids mentioned below may also be substituted. Thus, in another embodiment the mutated HPPD useful for the present invention comprises a sequence of SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, or a variant, derivative, orthologue, paralogue or homologue thereof, wherein an amino acid ±3, ±2 or ±1 amino acid positions from a key amino acid is substituted by any other amino acid.

Based on techniques well-known in the art, a highly characteristic sequence pattern can be developed, by means of which further of mutated HPPD candidates with the desired activity may be searched.

Searching for further mutated HPPD candidates by applying a suitable sequence pattern would also be encompassed by the present invention. It will be understood by a skilled reader that the present sequence pattern is not limited by the exact distances between two adjacent amino acid residues of said pattern. Each of the distances between two neighbours in the above patterns may, for example, vary independently of each other by up to ±10, ± 5, ±3, ±2 or ±1 amino acid positions without substantially affecting the desired activity.

In line with said above functional and spatial analysis of individual amino acid residues based on the crystallographic data as obtained according to the present invention, unique partial amino acid sequences characteristic of potentially useful mutated HPPD candidates of the invention may be identified.

In a particularly preferred embodiment, the mutated HPPD refers to a variant or derivative of SEQ ID NO: 2 wherein the substitutions are selected from the following Table B.a.

**Table B.a: (Sequence ID No: 2): single amino acid substitutions**

| Key amino acid position | Substituents |
|---|---|
| Val212 | Ile, Leu |
| Val213 | Thr, Ala |
| Asn215 | Ala, His |
| Ala236 | Leu, Ser, Arg |
| Phe238 | Val, Ala |
| Leu250 | Val, Met |
| Ser252 | Thr |
| Pro265 | Ala |
| Asn267 | Tyr, Gln |
| Gln278 | His, Asn, Ser |
| Ile279 | Thr |
| Arg309 | Lys, Ala |
| Leu320 | Asn, Gln, His, Tyr, |
| Pro321 | Ala, Arg, Gly, Asn |
| Leu334 | Glu, Cys |
| Leu353 | Met, Tyr, Ala, Ser |
| Phe366 | Ile, Leu, Tyr |
| Gly371 | Ile, Phe |
| Thr375 | Pro |
| Phe377 | Ala, Leu, Ser |
| Gly403 | Arg |
| Phe404 | Leu, Pro |
| Lys406 | Thr |
| Gly407 | Cys, His |
| Phe409 | Ile, His |
| Glu411 | Thr |
| Leu412 | Met, Phe, Trp, Ala, Ser |
| lIe416 | Val, Phe |
| Ser410 | Gly |
| Val254 | Ala |

Furthermore, by substituting at least two of the key amino acid residues of SEQ ID NO: 2 with specific residues, the herbicide tolerance or resistance could be remarkably increased as compared to the activity of the wild type HPPD enzymes or HPPD enzymes in which only one amino acid residue had been substituted. Therefore, in another preferred embodiment, the variant or derivative of the mutated HPPD refers to a polypeptide of SEQ ID NO: 2, wherein two, three, four or five key amino acids are substituted by another amino acid residue. Particularly preferred double, triple, quadruple, or quintuple mutations are described in Table B.b.

**Table B.b: (with reference to Sequence ID No: 2): combined amino acid substitutions**

| Combination No | Key amino acid position and and its substitutents |
|---|---|
| 1 | A236L, E411T |
| 2 | L320H, P321A |
| 3 | L320H, P321R |
| 4 | L320N, P321A |
| 5 | L320N, P321R |
| 6 | L320Q, P321A |
| 7 | L320Q, P321R |
| 8 | L320Y, P321A |
| 9 | L320Y, P321R |
| 10 | L353M, P321R |
| 11 | L353M, P321R, A236L |
| 12 | L353M, P321R, A236L, E411T |
| 13 | L353M, P321R, E411T |
| 14 | L353M, P321R, L320H |
| 15 | L353M, P321R, L320N |
| 16 | L353M, P321R, L320Q |
| 17 | L353M, P321R, L320Y |
| 18 | L353M, P321R, V212I |
| 19 | L353M, P321R, V212I, L334E |
| 20 | L353M, P321R, V212L, L334E |
| 21 | L353M, P321R, V212L, L334E, A236L |
| 22 | L353M, P321R, V212L, L334E, A236L, E411T |
| 23 | L353M, P321R, V212L, L334E, E411T |
| 24 | L353M, P321R, V212L, L334E, L320H |
| 25 | L353M, P321R, V212L, L334E, L320N |
| 26 | L353M, P321R, V212L, L334E, L320Q |
| 27 | L353M, P321R, V212L, L334E, L320Y |
| 28 | L353M, V212I |

In a particularly preferred embodiment, the mutated HPPD enzyme comprising a polypeptide of SEQ ID NO: 2, a variant, derivative, homologue, paralogue or orthologue thereof, useful for the present invention comprises one or more of the following: the amino acid corresponding to or at position 320 is histidine, asparagine or glutamine; the amino acid position 334 is glutamic acid; the amino acid position 353 is methionine; the amino acid corresponding to or at position 321 alanine or arginine; the amino acid corresponding to or at position 212 is isoleucine.

In an especially particularly preferred embodiment, the mutated HPPD refers to a polypeptide comprising SEQ ID NO: 2, wherein the leucine corresponding to or at position 320 is substituted by a histidine, and the proline corresponding to or at position 321 is substituted by an alanine.

In another especially particularly preferred embodiment, the mutated HPPD refers to a polypeptide comprising SEQ ID NO: 2, wherein Leucine corresponding to or at position 353 is substituted by a Methionine, the Proline corresponding to or at position 321 is substituted by an Arginine, and the Leucine corresponding to or at position 320 is substituted by an Asparagine.

In another especially particularly preferred embodiment, the mutated HPPD refers to a polypeptide comprising SEQ ID NO: 2, wherein the Leucine corresponding to or at position 353 is substituted by a Methionine, the Proline corresponding to or at position 321 is substituted by an Arginine, and the Leucine corresponding to or at position 320 is substituted by a glutamine.

In another preferred embodiment, the mutated HPPD refers to a variant or derivative of SEQ ID NO: 53 wherein the substitutions are selected from the following Table B.c.

**Table B.c: (Sequence ID No: 53): single amino acid substitutions**

| Key amino acid position | Substituents | Preferred substituents |
|---|---|---|
| Val228 | Thr, Ala | Thr, Ala |
| Asn230 | Ala, His | Ala, His |
| Ala251 | Ser, Arg | Ser, Arg |
| Phe253 | Val, Ala | Val, Ala |
| Leu265 | Val, Met | Val, Met |
| Ser267 | Thr | Thr |
| Pro280 | Ala | Ala |
| Asn282 | Tyr, Gln | Tyr, Gln |
| Lys291 | Arg, Ala | Arg |
| Gln293 | Ala, Leu, Ile, Val, His, Asn, Ser | His, Asn, Ser |
| Ile294 | Thr | Thr |
| Arg324 | Lys, Ala | Lys, Ala |
| Met335 | Ala, Trp, Phe, Leu, Ile, Val, Asn, Gln, His, Tyr, Ser, Thr, Cys | Gln, Asn, His, Tyr |
| Pro336 | Ala, Arg, Gly, Asn | Ala, Gly |
| Ser337 | Ala, Pro, Thr | Pro, Thr |
| Pro339 | Deletion | Deletion |
| Pro340 | Gly | Gly |
| Glu363 | Gln | Gln |
| Leu368 | Met, Tyr, | Met |
| Phe381 | Ile, Leu, Tyr | Ile, Leu |
| Leu385 | Ala, Val, Gln, Asp | Val, Asp |
| Gly386 | Ile, Phe | Ile, Phe |
| Thr390 | Pro | Pro |
| Phe392 | Ala, Leu, Ser | Ala |
| Ile393 | Ala, Leu, Phe, Val | Leu |
| Phe419 | Leu, Pro | Leu, Pro |
| Lys421 | Thr | Thr |
| Gly422 | His, Met, Phe, Cys | His, Cys |
| Phe424 | Ile, His | Ile, His |
| Leu427 | Phe, Trp, Ala, Ser, Met | Phe |
| Ile431 | Val, Phe | Val, Phe |
| Ser425 | Gly | Gly |
| Val269 | Ala | Ala |

In another preferred embodiment, the variant or derivative of the mutated HPPD useful for the present invention refers to a polypeptide of SEQ ID NO: 53, a homologue, orthologue, or paralogue thereof, wherein two, three, four or five key amino acids are substituted by another amino acid residue. Particularly preferred double, triple, quadruple, or quintuple mutations are described in Table B.d.

**Table B.d: (reference to Sequence ID No: 53): combined amino acid substitutions**

| Combination No | Key amino acid position | Substituents | Preferred substituents |
|---|---|---|---|
| 1 | Pro336 | Ala, Arg | Ala |
| | Glu363 | Gln | Gln |
| 2 | Pro336 | Ala, Arg | Ala |
| | Glu363 | Gln | Gln |
| | Leu385 | Ala, Val | Val |
| 3 | Pro336 | Ala, Arg | Ala |
| | Glu363 | Gln | Gln |
| | Leu385 | Ala, Val | Val |
| | Ile393 | Ala, Leu | Leu |
| 4 | Leu385 | Ala, Val | Val |
| | Ile393 | Ala, Leu | Leu |
| 5 | Met335 | Ala, Trp, Phe, Leu, Ile, Val, Asn, Gln, His, Tyr, Ser, Thr, Cys | Gln, Asn, His, Tyr |
| | Pro336 | Ala, Arg, Gly | Ala, Gly |
| 6 | Met335 | Ala, Trp, Phe, Leu, Ile, Val, Asn, Gln, His, Tyr, Ser, Thr, Cys | Gln, Asn, His, Tyr |
| | Pro336 | Ala, Arg, Gly | Ala, Gly |
| | Glu363 | Gln | Gln |
| 7 | Met335 | Ala, Trp, Phe, Leu, Ile, Val, Asn, Gln, His, Tyr, Ser, Thr, Cys | Gln, Asn, His, Tyr, Leu |
| | Pro336 | Ala, Arg, Gly | Ala, Arg, Gly |
| | Ser337 | Ala, Pro, Thr | Pro, Thr |
| | Pro339 | Deletion | Deletion |
| | Pro340 | Gly | Gly |

Furthermore, by substituting the amino acids at some positions in the HPPD polypeptide sequences of Scenedesmus obliquus, the tolerance of crop plants as described herein towards the herbicides as described herein could be remarkably increased.

Thus, in a preferred embodiment,-the mutated HPPD of the present invention comprises a variant of the sequence of SEQ ID NO: 50, or a homologue or functional equivalent thereof, which comprises one or more of the following:
the amino acid corresponding to or at position 30 is other than proline, the amino acid corresponding to or at position 39 is other than Phe, the amino acid corresponding to or at position 54 is other than Gly, the amino acid corresponding to or at position 57 is other than Met, the amino acid corresponding to or at position 84 is other than Phe, the amino acid corresponding to or at position 210 is other than Val, the amino acid corresponding to or at position 212 is other than Asn, the amino acid corresponding to or at position 223 is other than Val, the amino acid corresponding to or at position 243 is other than Val, the amino acid corresponding to or at position 247 is other than Leu, the amino acid corresponding to or at position 249 is other than Ser, the amino acid corresponding to or at position 251 is other than Val, the amino acid corresponding to or at position 264 is other than Asn, the amino acid corresponding to or at position 291 is other than Leu, the amino acid corresponding to or at position 306 is other than His, the amino acid corresponding to or at position 317 is other than Gln, the amino acid corresponding to or at position 318 is other than Ala, the amino acid corresponding to or at position 319 is other than Ala, the amino acid corresponding to or at position 321 is other than Gly, the amino acid corresponding to or at position 326 is other than Lys, the amino acid corresponding to or at position 327 is other than Arg, the amino acid corresponding to or at position 331 is other than Lys, the amino acid corresponding to or at position 341 is other than Trp, the amino acid corresponding to or at position 342 is other than Ala, the amino acid corresponding to or at position 345 is other than Glu, the amino acid corresponding to or at position 350 is other than Leu, the amino acid corresponding to or at position 363 is other than Phe, the amino acid corresponding to or at position 367 is other than Leu, the amino acid corresponding to or at position 373 is other than Ile, the amino acid corresponding to or at position 374 is other than Phe, the amino acid corresponding to or at position 375 is other than Ile, the amino acid corresponding to or at position 379 is other than Glu, the amino acid corresponding to or at position 405 is other than Gly, the amino acid corresponding to or at position 407 is other than Phe, the amino acid corresponding to or at position 410 is other than Gly, the amino acid corresponding to or at position 412 is other than Phe, the amino acid corresponding to or at position 414 is other than Glu, the amino acid corresponding to or at position 419 is other than Ile, the amino acid corresponding to or at position 421 is other than Glu, the amino acid corresponding to or at position 422 is other than Tyr.

In another preferred embodiment, the mutated HPPD comprises a variant of the sequence of SEQ ID NO: 50, or a homologue or functional equivalent thereof, in which:
the amino acid corresponding to or at position 367 is Val, and the amino acid corresponding to or at position 375 is Leu.

In another preferred embodiment, the mutated HPPD comprises a variant of the sequence of SEQ ID NO: 50, or a homologue or functional equivalent thereof, in which:
the amino acid corresponding to or at position 367 is Val, and the amino acid corresponding to or at position 375 is Leu, and the amino acid corresponding to or at position 39 is Leu.

In another preferred embodiment, the mutated HPPD comprises a variant of the sequence of SEQ ID NO: 50, or a homologue or functional equivalent thereof, in which:
the amino acid corresponding to or at position 367 is Val, and the amino acid corresponding to or at position 375 is Leu, and the amino acid corresponding to or at position 39 is Trp.

In another preferred embodiment, the mutated HPPD comprises a variant of the sequence of SEQ ID NO: 50, or a homologue or functional equivalent thereof, in which:
the amino acid corresponding to or at position 345 is Ala, Arg, Asn, Asp, Cys, Gln, Gly, His, Ile, Leu, Lys, Met, Phe, Pro, Ser, Thr, Trp, Tyr, or Val, particularly preferred Gln

In another preferred embodiment, the mutated HPPD comprises a variant of the sequence of SEQ ID NO: 50, or a homologue or functional equivalent thereof, in which:
the amino acid corresponding to or at position 345 is Gln, and the amino acid corresponding to or at position 341 is Ile.

In another preferred embodiment, the mutated HPPD comprises a variant of the sequence of SEQ ID NO: 50, or a homologue or functional equivalent thereof, in which:
the amino acid corresponding to or at position 345 is Gln, and the amino acid corresponding to or at position 326 is Glu.

In another preferred embodiment, the mutated HPPD comprises a variant of the sequence of SEQ ID NO: 50, or a homologue or functional equivalent thereof, in which:
the amino acid corresponding to or at position 345 is Gln, and the amino acid corresponding to or at position 326 is Asp.

In another preferred embodiment, the mutated HPPD comprises a variant of the sequence of SEQ ID NO: 50, or a homologue or functional equivalent thereof, in which:
the amino acid corresponding to or at position 345 is Gln, and the amino acid corresponding to or at position 326 is Gln.

In another preferred embodiment, the mutated HPPD comprises a variant of the sequence of SEQ ID NO: 50, or a homologue or functional equivalent thereof, in which:
the amino acid corresponding to or at position 318 is Arg, Asn, Asp, Cys, Gln, Glu, Gly, His, Ile, Leu, Lys, Met, Phe, Pro, Ser, Thr, Trp, Tyr, or Val, particularly preferred Pro.

In another preferred embodiment, the mutated HPPD comprises a variant of the sequence of SEQ ID NO: 50, or a homologue or functional equivalent thereof, in which:
the amino acid corresponding to or at position 319 is Arg, Asn, Asp, Cys, Gln, Glu, Gly, His, Ile, Leu, Lys, Met, Phe, Pro, Ser, Thr, Trp, Tyr, Val, particularly preferred Pro.

In another preferred embodiment, the mutated HPPD comprises a variant of the sequence of SEQ ID NO: 50, or a homologue or functional equivalent thereof, in which:
the amino acid corresponding to or at position 318 is Pro, and the amino acid corresponding to or at position 319 is Pro.

In another preferred embodiment, the mutated HPPD comprises a variant of the sequence of SEQ ID NO: 50, or a homologue or functional equivalent thereof, in which:
the amino acid corresponding to or at position 321 is Ala, Arg, Asn, Asp, Cys, Gln, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 350 is Ala, Arg, Asn, Asp, Cys, Gln, Glu, Gly, His, Ile, Lys, Met, Phe, Pro, Ser, Thr, Trp, Tyr, or Val, particularly preferred Met.

In another preferred embodiment, the mutated HPPD comprises a variant of the sequence of SEQ ID NO: 50, or a homologue or functional equivalent thereof, in which:
the amino acid corresponding to or at position 405 is Ala, Arg, Asn, Asp, Cys, Gln, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 251 is Ala, Arg, Asn, Asp, Cys, Gln, Glu, Gly, His, Ile, Leu, Lys, Met, Phe, Pro, Ser, Thr, Trp, or Tyr, particularly preferred Ala.

In another preferred embodiment, the mutated HPPD comprises a variant of the sequence of SEQ ID NO: 50, or a homologue or functional equivalent thereof, in which:
the amino acid corresponding to or at position 317 is Ala, Arg, Asn, Asp, Cys, Glu, Gly, His, Ile, Leu, Lys, Met, Phe, Pro, Ser, Thr, Trp, Tyr, or Val, particularly preferred His or Met.

In another preferred embodiment, the mutated HPPD comprises a variant of the sequence of SEQ ID NO: 50, or a homologue or functional equivalent thereof, in which:
the amino acid corresponding to or at position 379 is Ala, Arg, Asn, Asp, Cys, Gln, Gly, His, Ile, Leu, Lys, Met, Phe, Pro, Ser, Thr, Trp, Tyr, or Val, particularly preferred Gln.

In another preferred embodiment, the mutated HPPD comprises a variant of the sequence of SEQ ID NO: 50, or a homologue or functional equivalent thereof, in which:
the amino acid corresponding to or at position 350 is Met, and the amino acid corresponding to or at position 318 is Arg.

In another preferred embodiment, the mutated HPPD comprises a variant of the sequence of SEQ ID NO: 50, or a homologue or functional equivalent thereof, in which:
the amino acid corresponding to or at position 350 is Met, and the amino acid corresponding to or at position 318 is Gly.

In another preferred embodiment, the mutated HPPD comprises a variant of the sequence of SEQ ID NO: 50, or a homologue or functional equivalent thereof, in which:
the amino acid corresponding to or at position 350 is Met, and the amino acid corresponding to or at position 318 is Arg, and the amino acid corresponding to or at position 317 is Asn.

In another preferred embodiment, the mutated HPPD comprises a variant of the sequence of SEQ ID NO: 50, or a homologue or functional equivalent thereof, in which:
the amino acid corresponding to or at position 210 is Ala, Arg, Asn, Asp, Cys, Gln, Glu, Gly, His, Ile, Leu, Lys, Met, Phe, Pro, Ser, Thr, Trp, or Tyr.

In another preferred embodiment, the mutated HPPD comprises a variant of the sequence of SEQ ID NO: 50, or a homologue or functional equivalent thereof, in which:
the amino acid corresponding to or at position 317 is His, and the amino acid corresponding to or at position 318 is Gly, and the amino acid corresponding to or at position 345 is Gln.

In another preferred embodiment, the mutated HPPD comprises a variant of the sequence of SEQ ID NO: 50, or a homologue or functional equivalent thereof, in which:
the amino acid corresponding to or at position 317 is Met, and the amino acid corresponding to or at position 318 is Gly, and the amino acid corresponding to or at position 345 is Gln.

In another preferred embodiment, the mutated HPPD comprises a variant of the sequence of SEQ ID NO: 50, or a homologue or functional equivalent thereof, in which:
the amino acid corresponding to or at position 363 is Ala, Arg, Asn, Asp, Cys, Gln, Glu, Gly, His, Ile, Leu, Lys, Met, Pro, Ser, Thr, Trp, Tyr, or Val, particularly preferred Ile.

In another preferred embodiment, the mutated HPPD comprises a variant of the sequence of SEQ ID NO: 50, or a homologue or functional equivalent thereof, in which:
the amino acid corresponding to or at position 419 is Ala, Arg, Asn, Asp, Cys, Gln, Glu, Gly, His, Leu, Lys, Met, Phe, Pro, Ser, Thr, Trp, Tyr, or Val, particularly preferred Val.

In another preferred embodiment, the mutated HPPD comprises a variant of the sequence of SEQ ID NO: 50, or a homologue or functional equivalent thereof, in which:
the amino acid corresponding to or at position 249 is Ala, Arg, Asn, Asp, Cys, Gln, Glu, Gly, His, Ile, Leu, Lys, Met, Phe, Pro, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 247 is Ala, Arg, Asn, Asp, Cys, Gln, Glu, Gly, His, Ile, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 407 is Ala, Arg, Asn, Asp, Cys, Gln, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 306 is Ala, Arg, Asn, Asp, Cys, Gln, Glu, Gly, Ile, Leu, Lys, Met, Phe, Pro, Ser, Thr, Trp, Tyr, or Val, particularly preferred Lys.

In another preferred embodiment, the mutated HPPD comprises a variant of the sequence of SEQ ID NO: 50, or a homologue or functional equivalent thereof, in which:
the amino acid corresponding to or at position 30 is Ala, Arg, Asn, Asp, Cys, Gln, Glu, Gly, His, Ile, Leu, Lys, Met, Phe, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 54 is Ala, Arg, Asn, Asp, Cys, Gln, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 57 is Ala, Arg, Asn, Asp, Cys, Gln, Glu, Gly, His, Ile, Leu, Lys, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 84 is Ala, Arg, Asn, Asp, Cys, Gln, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 212 is Ala, Arg, Asp, Cys, Gln, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 223 is Ala, Arg, Asn, Asp, Cys, Gln, Glu, Gly, His, Ile, Leu, Lys, Met, Phe, Pro, Ser, Thr, Trp, or Tyr.

In another preferred embodiment, the mutated HPPD comprises a variant of the sequence of SEQ ID NO: 50, or a homologue or functional equivalent thereof, in which:
the amino acid corresponding to or at position 243 is Ala, Arg, Asn, Asp, Cys, Gln, Glu, Gly, His, Ile, Leu, Lys, Met, Phe, Pro, Ser, Thr, Trp, or Tyr.

In another preferred embodiment, the mutated HPPD comprises a variant of the sequence of SEQ ID NO: 50, or a homologue or functional equivalent thereof, in which:
the amino acid corresponding to or at position 264 is Ala, Arg, Asp, Cys, Gln, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 291 is Ala, Arg, Asn, Asp, Cys, Gln, Glu, Gly, His, Ile, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 327 is Ala, Asn, Asp, Cys, Gln, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 331 is Ala, Arg, Asn, Asp, Cys, Gln, Glu, Gly, His, Ile, Leu, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 342 is Arg, Asn, Asp, Cys, Gln, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 373 is Ala, Arg, Asn, Asp, Cys, Gln, Glu, Gly, His, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 374 is Ala, Arg, Asn, Asp, Cys, Gln, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 410 is Ala, Arg, Asn, Asp, Cys, Gln, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 412 is Ala, Arg, Asn, Asp, Cys, Gln, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 414 is Ala, Arg, Asn, Asp, Cys, Gln, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 421 is Ala, Arg, Asn, Asp, Cys, Gln, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 422 is Ala, Arg, Asn, Asp, Cys, Gln, Glu, Gly, His, Ile, Leu, Lys, Met, Phe, Pro, Ser, Thr, Trp, or Val.

In another preferred embodiment, the mutated HPPD comprises a variant of the sequence of SEQ ID NO: 50, or a homologue or functional equivalent thereof, in which:
the amino acid corresponding to or at position 251 is Ala, and the amino acid corresponding to or at position 405 is Asp.

In another preferred embodiment, the mutated HPPD comprises a variant of the sequence of SEQ ID NO: 50, or a homologue or functional equivalent thereof, in which:
the amino acid corresponding to or at position 327 is Gly, and the amino acid corresponding to or at position 421 is Asp.

In another preferred embodiment, the mutated HPPD comprises a variant of the sequence of SEQ ID NO: 50, or a homologue or functional equivalent thereof, in which:
the amino acid corresponding to or at position 251 is Ala, and the amino acid corresponding to or at position 306 is Arg, and the amino acid corresponding to or at position 317 is Leu, and the amino acid corresponding to or at position 318 is Pro, and the amino acid corresponding to or at position 321 is Pro, and the amino acid corresponding to or at position 331 is Glu, and the amino acid corresponding to or at position 350 is Met.

In another preferred embodiment, the mutated HPPD comprises a variant of the sequence of SEQ ID NO: 50, or a homologue or functional equivalent thereof, in which:
the amino acid corresponding to or at position 407 is Ala, Arg, Asn, Asp, Cys, Gln, Glu, Gly, His, Ile, Leu, Lys, Met, Pro, Ser, Thr, Trp, Tyr, or Val.

Following mutagenesis of one of the sequences as shown herein, the encoded protein can be expressed recombinantly and the activity of the protein can be determined using, for example, assays described herein.

It will be within the knowledge of the skilled artisan to identify conserved regions and motifs shared between the homologues, orthologues and paralogues of of SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, and respectively SEQ ID NO: 48 or 50. Having identified such conserved regions that may represent suitable binding motifs, amino acids corresponding to the amino acids listed in Table B.a and B.b, B.c, and B.d can be chosen to be substituted by any other amino acid by conserved amino acids, and more preferably by the amino acids of tables B.a and B.b, B.c, and B.d.

Numerous crop plants, for example Clearfield® oilseed rape, tolerant to imidazolinones, for example imazamox, have been generated with the aid of classic breeding methods (mutagenesis). Crop plants such as soybeans, cotton, corn, beet and oilseed rape, resistant to glyphosate or glufosinate, which are available under the tradenames RoundupReady® (glyphosate) and Liberty Link® (glufosinate) have been generated with the aid of genetic engineering methods.

Accordingly, the term "crop plants" also includes plants which, with the aid of genetic engineering, produce one or more toxins, for example those of the bacterial strain *Bacillus* ssp. Toxins which are produced by such genetically modified plants include, for example, insecticidal proteins of *Bacillus* spp., in particular *B. thuringiensis,* such as the endotoxins Cry1Ab, Cry1Ac, Cry1F, Cry1Fa2, Cry2Ab, Cry3A, Cry3Bb1, Cry9c, Cry34Ab1 or Cry35Ab1; or vegetative insecticidal proteins (VIPs), for example VIP1, VIP2, VIP3, or VIP3A; insecticidal proteins of nematode-colonizing bacteria, for example *Photorhabdus* spp. or *Xenorhabdus* spp.; toxins of animal organisms, for example wasp, spider or scorpion toxins; fungal toxins, for example from Streptomycetes; plant lectins, for example from peas or barley; agglutinins; proteinase inhibitors, for example trypsin inhibitors, serine protease inhibitors, patatin, cystatin or papain inhibitors, ribosome-inactivating proteins (RIPs), for example ricin, corn-RIP, abrin, luffin, saporin or bryodin; steroid-metabolizing enzymes, for example 3-hydroxysteroid oxidase, ecdysteroid-IDP glycosyl transferase, cholesterol oxidase, ecdysone inhibitors, or HMG-CoA reductase; ion channel blockers, for example inhibitors of sodium channels or calcium channels; juvenile hormone esterase; receptors of the diuretic hormone (helicokinin receptors); stilbene synthase, bibenzyl synthase, chitinases and glucanases. In the plants, these toxins may also be produced as pre-toxins, hybrid proteins or truncated or otherwise modified proteins. Hybrid proteins are characterized by a novel combination of different protein domains (see, for example, WO 2002/015701). Further examples of such toxins or genetically modified plants which produce these toxins are disclosed in EP-A 374 753, WO 93/007278, WO 95/34656, EP-A 427 529, EP-A 451 878, WO 03/018810 and WO 03/052073. The methods for producing these genetically modified plants are known to the person skilled in the art and disclosed, for example, in the publications mentioned above. Numerous of the toxins mentioned above bestow, upon the plants by which they are produced, tolerance to pests from all taxonomic classes of arthropods, in particular to beetles (Coeleropta), dipterans (Diptera) and butterflies (Lepidoptera) and to nematodes (Nematoda).

Genetically modified plants which produce one or more genes coding for insecticidal toxins are described, for example, in the publications mentioned above, and some of them are commercially available, such as, for example, YieldGard® (corn varieties producing the toxin Cry1Ab), YieldGard® Plus (corn varieties which produce the toxins Cry1Ab and Cry3Bb1), Starlink® (corn varieties which produce the toxin Cry9c), Herculex® RW (corn varieties which produce the toxins Cry34Ab1, Cry35Ab1 and the enzyme phosphinothricin-N-acetyltransferase [PAT]); NuCOTN® 33B (cotton varieties which produce the toxin Cry1Ac), Bollgard® I (cotton varieties which produce the toxin Cry1Ac), Bollgard® II (cotton varieties which produce the toxins Cry1Ac and Cry2Ab2); VIPCOT® (cotton varieties which produce a VIP toxin); NewLeaf® (potato varieties which produce the toxin Cry3A); Bt-Xtra®, NatureGard®, KnockOut®, BiteGard®, Protecta®, Bt11 (for example Agrisure® CB) and Bt176 from Syngenta Seeds SAS, France (corn varieties which produce the toxin Cry1Ab and the PAT enyzme), MIR604 from Syngenta Seeds SAS, France (corn varieties which produce a modified version of the toxin Cry3A, see WO 03/018810), MON 863 from Monsanto Europe S.A., Belgium (corn varieties which produce the toxin Cry3Bb1), IPC 531 from Monsanto Europe S.A., Belgium (cotton varieties which produce a modified version of the toxin Cry1Ac) and 1507 from Pioneer Overseas Corporation, Belgium (corn varieties which produce the toxin Cry1F and the PAT enzyme). Accordingly, the term "crop plants" also includes plants which, with the aid of genetic engineering, produce one or more proteins which are more robust or have increased resistance to bacterial, viral or fungal pathogens, such as, for example, pathogenesis-related proteins (PR proteins, see EP-A 0 392 225), resistance proteins (for example potato varieties producing two resistance genes against *Phytophthora infestans* from the wild Mexican potato *Solanum bulbocastanum*) or T4 lysozyme (for example potato cultivars which, by producing this protein, are resistant to bacteria such as *Erwinia amylvora*)*.*

Accordingly, the term "crop plants" also includes plants whose productivity has been improved with the aid of genetic engineering methods, for example by enhancing the potential yield (for example biomass, grain yield, starch, oil or protein content), tolerance to drought, salt or other limiting environmental factors or resistance to pests and fungal, bacterial and viral pathogens.

The term "crop plants" also includes plants whose ingredients have been modified with the aid of genetic engineering methods in particular for improving human or animal diet, for example by oil plants producing health-promoting long-chain omega 3 fatty acids or monounsaturated omega 9 fatty acids (for example Nexera® oilseed rape).

The term "crop plants" also includes plants which have been modified with the aid of genetic engineering methods for improving the production of raw materials, for example by increasing the amylopectin content of potatoes (Amflora® potato).

Furthermore, it has been found that the compounds of formula I are also suitable for the defoliation and/or desiccation of plant parts, for which crop plants such as cotton, potato, oilseed rape, sunflower, soybean or field beans, in particular cotton, are suitable. In this regard, there have been found compositions for the desiccation and/or defoliation of plants, processes for preparing these compositions and methods for desiccating and/or defoliating plants using the compounds of formula I.

As desiccants, the compounds of formula I are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pomaceous fruit, stone fruit and nuts. The same mechanism, i.e. the promotion of the development of abscission tissue between fruit part or leaf part and shoot part of the plants is also essential for the readily controllable defoliation of useful plants, in particular cotton.

Moreover, a shortening of the time interval in which the individual cotton plants mature leads to an increased fiber quality after harvesting.

The compounds of formula I, or the herbicidal compositions comprising the compounds of formula I, can be used, for example, in the form of ready-to-spray aqueous solutions, powders, suspensions, also highly concentrated aqueous, oily or other suspensions or dispersions, emulsions, oil dispersions, pastes, dusts, materials for broadcasting, or granules, by means of spraying, atomizing, dusting, spreading, watering or treatment of the seed or mixing with the seed. The use forms depend on the intended purpose; in each case, they should ensure the finest possible distribution of the active ingredients according to the invention.

The herbicidal compositions comprise a herbicidally effective amount of at least one compound of the formula I or an agriculturally useful salt of I, and auxiliaries which are customary for the formulation of crop protection agents.

Examples of auxiliaries customary for the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, if appropriate colorants and, for seed formulations, adhesives.

Examples of thickeners (i.e. compounds which impart to the formulation modified flow properties, i.e. high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhardt).

Examples of antifoams are silicone emulsions (such as, for example, Silikon® SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulation. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants are both sparingly water-soluble pigments and water-soluble dyes. Examples which may be mentioned are the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following:
mineral oil fractions of medium to high boiling point, such as kerosene and diesel oil, furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example amines such as N-methylpyrrolidone, and water.

Solid carriers are mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF SE), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denatured proteins, polysaccharides (e.g. methylcellulose), hydrophobically modified starches, polyvinyl alcohol (Mowiol types Clariant), polycarboxylates (BASF SE, Sokalan types), polyalkoxylates, polyvinylamine (BASF SE, Lupamine types), polyethyleneimine (BASF SE, Lupasol types), polyvinylpyrrolidone and copolymers thereof.

Powders, materials for broadcasting and dusts can be prepared by mixing or grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water. To prepare emulsions, pastes or oil dispersions, the compounds of formula I or la, either as such or dissolved in an oil or solvent, can be homogenized in water by means of a wetting agent, tackifier, dispersant or emulsifier. Alternatively, it is also possible to prepare concentrates comprising active substance, wetting agent, tackifier, dispersant or emulsifier and, if desired, solvent or oil, which are suitable for dilution with water.

The concentrations of the compounds of formula I in the ready-to-use preparations can be varied within wide ranges. In general, the formulations comprise from 0.001 to 98% by weight, preferably 0.01 to 95% by weight of at least one active compound. The active compounds are employed in a purity of from 90% to 100%, preferably 95% to 100% (according to NMR spectrum).

The formulations or ready-to-use preparations may also comprise acids, bases or buffer systems, suitable examples being phosphoric acid or sulfuric acid, or urea or ammonia.

The compounds of formula I of the invention can for example be formulated as follows:
1. Products for dilution with water
   A. Water-soluble concentrates
      10 parts by weight of active compound are dissolved in 90 parts by weight of water or a water-soluble solvent. As an alternative, wetters or other adjuvants are added. The active compound dissolves upon dilution with water. This gives a formulation with an active compound content of 10% by weight.
   B. Dispersible concentrates
      20 parts by weight of active compound are dissolved in 70 parts by weight of cyclohexanone with addition of 10 parts by weight of a dispersant, for example polyvinylpyrrolidone. Dilution with water gives a dispersion. The active compound content is 20% by weight.
   C. Emulsifiable concentrates
      15 parts by weight of active compound are dissolved in 75 parts by weight of an organic solvent (e.g. alkylaromatics) with addition of calcium dodecylbenzenesulfonate and castor oil ethoxylate (in each case 5 parts by weight). Dilution with water gives an emulsion. The formulation has an active compound content of 15% by weight.
   D. Emulsions
      25 parts by weight of active compound are dissolved in 35 parts by weight of an organic solvent (e.g. alkylaromatics) with addition of calcium dodecylbenzenesulfonate and castor oil ethoxylate (in each case 5 parts by weight). This mixture is introduced into 30 parts by weight of water by means of an emulsifier (e.g. Ultraturrax) and made into a homogeneous emulsion. Dilution with water gives an emulsion. The formulation has an active compound content of 25% by weight.
   E. Suspensions
      In an agitated ball mill, 20 parts by weight of active compound are comminuted with addition of 10 parts by weight of dispersants and wetters and 70 parts by weight of water or an organic solvent to give a fine active compound suspension. Dilution with water gives a stable suspension of the active compound. The active compound content in the formulation is 20% by weight.
   F. Water-dispersible granules and water-soluble granules
      50 parts by weight of active compound are ground finely with addition of 50 parts by weight of dispersants and wetters and made into water-dispersible or water-soluble granules by means of technical appliances (for example extrusion, spray tower, fluidized bed). Dilution with water gives a stable dispersion or solution of the active compound. The formulation has an active compound content of 50% by weight.
   G. Water-dispersible powders and water-soluble powders
      75 parts by weight of active compound are ground in a rotor-stator mill with addition of 25 parts by weight of dispersants, wetters and silica gel. Dilution with water gives a stable dispersion or solution of the active compound. The active compound content of the formulation is 75% by weight.
   H. Gel formulations
      In a ball mill, 20 parts by weight of active compound, 10 parts by weight of dispersant, 1 part by weight of gelling agent and 70 parts by weight of water or of an organic solvent are ground to give a fine suspension. Dilution with water gives a stable suspension with active compound content of 20% by weight.
2. Products to be applied undiluted
   I. Dusts
      5 parts by weight of active compound are ground finely and mixed intimately with 95 parts by weight of finely divided kaolin. This gives a dusting powder with an active compound content of 5% by weight.
   J. Granules (GR, FG, GG, MG)
      0.5 parts by weight of active compound are ground finely and associated with 99.5 parts by weight of carriers. Current methods here are extrusion, spray-drying or the fluidized bed. This gives granules to be applied undiluted with an active compound content of 0.5% by weight.
   K. ULV solutions (UL)
      10 parts by weight of active compound are dissolved in 90 parts by weight of an organic solvent, for example xylene. This gives a product to be applied undiluted with an active compound content of 10% by weight.

The compounds of formula I or the herbicidal compositions comprising them can be applied pre- or post-emergence, or together with the seed of a crop plant. It is also possible to apply the herbicidal compositions or active compounds by applying seed, pretreated with the herbicidal compositions or active compounds, of a crop plant. If the active compounds are less well tolerated by certain crop plants, application techniques may be used in which the herbicidal compositions are sprayed, with the aid of the spraying equipment, in such a way that as far as possible they do not come into contact with the leaves of the sensitive crop plants, while the active compounds reach the leaves of undesirable plants growing underneath, or the bare soil surface (post-directed, lay-by).

In a further embodiment, the compounds of formula I or the herbicidal compositions can be applied by treating seed.

The treatment of seed comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula I according to the invention or the compositions prepared therefrom. Here, the herbicidal compositions can be applied diluted or undiluted.

The term seed comprises seed of all types, such as, for example, corns, seeds, fruits, tubers, cuttings and similar forms. Here, preferably, the term seed describes corns and seeds.

The seed used can be seed of the useful plants mentioned above, but also the seed of transgenic plants or plants obtained by customary breeding methods.

The rates of application of active compound are from 0.001 to 3.0, preferably 0.01 to 1.0, kg/ha of active substance (a.s.), depending on the control target, the season, the target plants and the growth stage. To treat the seed, the compounds of formula I are generally employed in amounts of from 0.001 to 10 kg per 100 kg of seed.

It may also be advantageous to use the compounds of formula I in combination with safeners, also termed herbicide safener. Safeners are chemical compounds which prevent or reduce damage to useful plants without substantially affecting the herbicidal action of the compounds of formula I on unwanted plants. They can be used both before sowing (for example in the treatment of seed, or on cuttings or seedlings) and before or after the emergence of the useful plant. The safeners and the compounds of formula I can be used simultaneously or in succession.

Suitable safeners are, for example, (quinolin-8-oxy)acetic acids, 1-phenyl-5-haloalkyl-1*H-*1,2,4-triazole-3-carboxylic acids, 1-phenyl-4,5-dihydro-5-alkyl-1*H*-pyrazole-3,5-dicarboxylic acids, 4,5-dihydro-5,5-diaryl-3-isoxazolecarboxylic acids, dichloroacetamides, alpha-oximinophenylacetonitriles, acetophenone oximes, 4,6-dihalo-2-phenylpyrimidines, N-[[4-(aminocarbonyl)phenyl]sulfonyl]-2-benzamides, 1,8-naphthalic anhydride, 2-halo-4-(haloalkyl)-5-thiazolecarboxylic acids, phosphorothiolates and O-phenyl N-alkylcarbamates and their agriculturally useful salts and, provided that they have an acid function, their agriculturally useful derivatives, such as amides, esters and thioesters.

To broaden the activity spectrum and to obtain synergistic effects, the compounds of the formula I can be mixed and jointly applied with numerous representatives of other compounds having herbicidal activity (herbicides B) or growth-regulating activitiy, optionally in combination with safeners. Suitable mixing partners are, for example, 1,2,4-thiadiazoles, 1,3,4-thiadiazoles, amides, aminophosphoric acid and its derivatives, aminotriazoles, anilides, aryloxy/heteroaryl-oxyalkanoic acids and their derivatives, benzoic acid and its derivatives, benzothiadiazinones, 2-(hetaroyl/aroyl)-1,3-cyclohexanediones, heteroaryl aryl ketones, benzylisoxazolidinones, meta-CF₃-phenyl derivatives, carbamates, quinoline carboxylic acid and its derivatives, chloroacetanilides, cyclohexenone oxime ether derivates, diazines, dichloropropionic acid and its derivatives, dihydrobenzofurans, dihydrofuran-3-ones, dinitroanilines, dinitrophenols, diphenyl ethers, dipyridyls, halocarboxylic acids and their derivatives, ureas, 3-phenyluracils, imidazoles, imidazolinones, N-phenyl-3,4,5,6-tetrahydrophthalimides, oxadiazoles, oxiranes, phenols, aryloxy- and heteroaryloxyphenoxypropionic esters, phenylacetic acid and its derivatives, 2-phenylpropionic acid and its derivatives, pyrazoles, phenylpyrazoles, pyridazines, pyridine-carboxylic acid and its derivatives, pyrimidyl ethers, sulfonamides, sulfonylureas, triazines, triazinones, triazolinones, triazolecarboxamides, uracils and also phenylpyrazolines and isoxazolines and their derivatives.

Moreover, it may be useful to apply the compounds of formula I alone or in combination with other herbicides B or else also mixed with further crop protection agents, jointly, for example with compositions for controlling pests or phytopathogenic fungi or bacteria. Also of interest is the miscibility with mineral salt solutions which are employed for alleviating nutritional and trace element deficiencies. Other additives such as nonphytotoxic oils and oil concentrates may also be added.

Examples of herbicides B which can be used in combination with the benzamide compounds of formula I according to the present invention are:
b1) from the group of the lipid biosynthesis inhibitors:
   alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro-4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5), benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) from the group of the ALS inhibitors:
   amidosulfuron, azimsulfuron, bensulfuron, bensulfuron-methyl, bispyribac, bispyribac-sodium, chlorimuron, chlorimuron-ethyl, chlorsulfuron, cinosulfuron, cloransulam, cloransulammethyl, cyclosulfamuron, diclosulam, ethametsulfuron, ethametsulfuron-methyl, ethoxysulfuron, flazasulfuron, florasulam, flucarbazone, flucarbazone-sodium, flucetosulfuron, flumetsulam, flupyrsulfuron, flupyrsulfuron-methyl-sodium, foramsulfuron, halosulfuron, halosulfuron-methyl, imazamethabenz, imazamethabenz-methyl, imazamox, imazapic, imazapyr, imazaquin, imazethapyr, imazosulfuron, iodosulfuron, iodosulfuron-methyl-sodium, mesosulfuron, metosulam, metsulfuron, metsulfuron-methyl, nicosulfuron, orthosulfamuron, oxasulfuron, penoxsulam, primisulfuron, primisulfuron-methyl, propoxycarbazone, propoxycarbazone-sodium, prosulfuron, pyrazosulfuron, pyrazosulfuron-ethyl, pyribenzoxim, pyrimisulfan, pyriftalid, pyriminobac, pyriminobac-methyl, pyrithiobac, pyrithiobac-sodium, pyroxsulam, rimsulfuron, sulfometuron, sulfometuron-methyl, sulfosulfuron, thiencarbazone, thiencarbazone-methyl, thifensulfuron, thifensulfuron-methyl, triasulfuron, tribenuron, tribenuron-methyl, trifloxysulfuron, triflusulfuron, triflusulfuron-methyl and tritosulfuron;
b3) from the group of the photosynthesis inhibitors:
   ametryn, amicarbazone, atrazine, bentazone, bentazone-sodium, bromacil, bromofenoxim, bromoxynil and its salts and esters, chlorobromuron, chloridazone, chlorotoluron, chloroxuron, cyanazine, desmedipham, desmetryn, dimefuron, dimethametryn, diquat, diquat-dibromide, diuron, fluometuron, hexazinone, ioxynil and its salts and esters, isoproturon, isouron, karbutilate, lenacil, linuron, metamitron, methabenzthiazuron, metobenzuron, metoxuron, metribuzin, monolinuron, neburon, paraquat, paraquat-dichloride, paraquat-dimetilsulfate, pentanochlor, phenmedipham, phenmedipham-ethyl, prometon, prometryn, propanil, propazine, pyridafol, pyridate, siduron, simazine, simetryn, tebuthiuron, terbacil, terbumeton, terbuthylazine, terbutryn, thidiazuron, trietazine, 1-(6-tert-butylpyrimidin-4-yl)-2-hydroxy-4-methoxy-3-methyl-2H-pyrrol-5-one (CAS 1654744-66-7), 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butyl-1-methyl-pyrazol-3-yl)-4-chloro-2-hydroxy-3-methyl-2H-pyrrol-5-one (CAS 1654057-29-0), 1-(5-tert-butyl-1-methyl-pyrazol-3-yl)-3-chloro-2-hydroxy-4-methyl-2H-pyrrol-5-one (CAS 1654747-80-4), 4-hydroxy-1-methoxy-5-methyl-3-[4-(trifluoromethyl)-2-pyridyl]imidazolidin-2-one; (CAS 2023785-78-4), 4-hydroxy-1,5-dimethyl-3-[4-(trifluoromethyl)-2-pyridyl]imidazolidin-2-one (CAS 2023785-79-5), 5-ethoxy-4-hydroxy-1-methyl-3-[4-(trifluoromethyl)-2-pyridyl]imidazolidin-2-one (CAS 1701416-69-4), 4-hydroxy-1-methyl-3-[4-(trifluoromethyl)-2-pyridyl]imidazolidin-2-one (CAS 1708087-22-2), 4-hydroxy-1,5-dimethyl-3-[1-methyl-5-(trifluoromethyl)pyrazol-3-yl]imidazolidin-2-one (CAS 2023785-80-8) and 1-(5-tert-butylisoxazol-3-yl)-4-ethoxy-5-hydroxy-3-methyl-imidazolidin-2-one (CAS 1844836-64-1);
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, 2-chloro-5-[3,6-dihydro-3-methyl-2,6-dioxo-4-(trifluoromethyl)-1(2*H*)-pyrimidinyl]-4-fluoro-N-[(isopropyl)methylsulfamoyl]benzamide (H-1; CAS 372137-35-4), ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (H-2; CAS 353292-31-6), N-ethyl-3-(2,6-dichloro-4-trifluoromethylphenoxy)-5-methyl-1*H*-pyrazole-1-carboxamide (H-3; CAS 452098-92-9), N-tetrahydrofurfuryl-3-(2,6-dichloro-4-trifluoromethylphenoxy)-5-methyl-1*H*-pyrazole-1-carboxamide (H-4; CAS 915396-43-9), N-ethyl-3-(2-chloro-6-fluoro-4-trifluoromethylphenoxy)-5-methyl-1*H*-pyrazole-1-carboxamide (H-5; CAS 452099-05-7), N-tetrahydrofurfuryl-3-(2-chloro-6-fluoro-4-trifluoromethylphenoxy)-5-methyl-1*H*-pyrazole-1-carboxamide (H-6; CAS 45100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione, 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione (trifludimoxazin), 2-(2,2,7-Trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-Methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione(CAS 1304113-05-0), methyl (*E*)-4-[2-chloro-5-[4-chloro-5-(difluoromethoxy)-1*H*-methyl-pyrazol-3-yl]-4-fluorophenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4);
b5) from the group of the bleacher herbicides:
   aclonifen, amitrol, beflubutamid, benzobicyclon, benzofenap, clomazone, diflufenican, fenquinotrione, flumeturon, fluridone, flurochloridone, flurtamone, isoxaflutole, mesotrione, norflurazon, oxotrione (CAS 1486617-21-3), picolinafen, pyrasulfotole, pyrazolynate, pyrazoxyfen, sulcotrione, tefuryltrione, tembotrione, tolpyralate, topramezone, 4-hydroxy-3-[[2-[(2-methoxyethoxy)methyl]-6-(trifluoromethyl)-3-pyridyl]carbonyl]bicyclo[3.2.1]oct-3-en-2-one (H-7; CAS 352010-68-5, bicyclopyrone), 4-(3-trifluoromethylphenoxy)-2-(4-trifluoromethylphenyl)pyrimidine (H-8; CAS 180608-33-7), 2-chloro-3-methylsulfanyl-N-(1-methyltetrazol-5-yl)-4-(trifluoromethyl)benzamide (CAS 1361139-71-0), 2-(2,4-dichlorophenyl)methyl-4,4-dimethyl-3-isoxazolidone (CAS 81777-95-9) and 2-(2,5-dichlorophenyl)methyl-4,4-dimethyl-3-isoxazolidinone (CAS 81778-66-7);
b6) from the group of the EPSP synthase inhibitors:
   glyphosate, glyphosate-isopropylammonium and glyphosate-trimesium (sulfosate);
b7) from the group of the glutamine synthase inhibitors:
   bilanaphos (bialaphos), bilanaphos-sodium, glufosinate and glufosinate-ammonium;
b8) from the group of the DHP synthase inhibitors:
   asulam;
b9) from the group of the mitose inhibitors:
   amiprophos, amiprophos-methyl, benfluralin, butamiphos, butralin, carbetamide, chlorpropham, chlorthal, chlorthal-dimethyl, dinitramine, dithiopyr, ethalfluralin, fluchloralin, oryzalin, pendimethalin, prodiamine, propham, propyzamide, tebutam, thiazopyr and trifluralin;
b10) from the group of the VLCFA inhibitors:
   acetochlor, alachlor, anilofos, butachlor, cafenstrole, dimethachlor, dimethanamid, dimethenamid-P, diphenamid, fentrazamide, flufenacet, mefenacet, metazachlor, metolachlor, metolachlor-S, naproanilide, napropamide, pethoxamid, piperophos, pretilachlor, propachlor, propisochlor, pyroxasulfone (KIH-485) and thenylchlor;
Compounds of the formula 2: 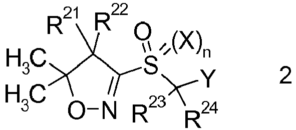 in which the variables have the following meanings:
Y is phenyl or 5- or 6-membered heteroaryl as defined at the outset, which radicals may be substituted by one to three groups R^{aa}; R²¹,R²²,R²³,R²⁴ are H, halogen or C₁-C₄-alkyl; X is O or
NH; N is 0 or 1.

Compounds of the formula 2 have in particular the following meanings:
Y is 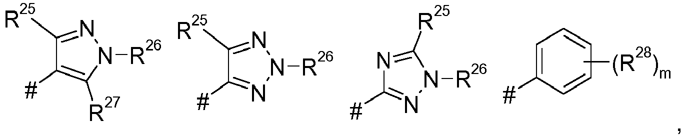 where # denotes the bond to the skeleton of the molecule; and
R²¹,R²²,R²³,R²⁴ are H, Cl, F or CH₃; R²⁵ is halogen, C₁-C₄-alkyl or C₁-C₄-haloalkyl; R²⁶ is C₁-C₄-alkyl; R²⁷ is halogen, C₁-C₄-alkoxy or C₁-C₄-haloalkoxy; R²⁸ is H, halogen, C₁-C₄-alkyl, C₁-C₄-haloalkyl or C₁-C₄-haloalkoxy; M is 0, 1, 2 or 3; X is oxygen; N is 0 or 1.

Preferred compounds of the formula 2 have the following meanings:
Y is 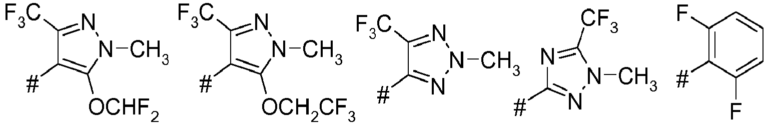 R²¹ is H; R²²,R²³ are F; R²⁴ is H or F; X is oxygen; N is 0 or 1.

Particularly preferred compounds of the formula 2 are:
3-[5-(2,2-difluoroethoxy)-1-methyl-3-trifluoromethyl-1H-pyrazol-4-ylmethane-sulfonyl]-4-fluoro-5,5-dimethyl-4,5-dihydroisoxazole (2-1); 3-{[5-(2,2-difluoroethoxy)-1-methyl-3-trifluoromethyl-1H-pyrazol-4-yl]fluoromethanesulfonyl}-5,5-dimethyl-4,5-dihydroisoxazole (2-2); 4-(4-fluoro-5,5-dimethyl-4,5-dihydroisoxazole-3-sulfonylmethyl)-2-methyl-5-trifluoromethyl-2H-[1,2,3]triazole (2-3); 4-[(5,5-dimethyl-4,5-dihydroisoxazole-3-sulfonyl)fluoromethyl]-2-methyl-5-trifluoromethyl-2H-[1,2,3]triazole (2-4); 4-(5,5-dimethyl-4,5-dihydroisoxazole-3-sulfonylmethyl)-2-methyl-5-trifluoromethyl-2H-[1,2,3]triazole (2-5); 3-{[5-(2,2-difluoroethoxy)-1-methyl-3-trifluoromethyl-1H-pyrazol-4-yl]difluoromethanesulfonyl}-5,5-dimethyl-4,5-dihydroisoxazole (2-6); 4-[(5,5-dimethyl-4,5-dihydroisoxazole-3-sulfonyl)difluoromethyl]-2-methyl-5-trifluoromethyl-2H-[1,2,3]triazole (2-7); 3-{[5-(2,2-difluoroethoxy)-1-methyl-3-trifluoromethyl-1H-pyrazol-4-yl]difluoromethanesulfonyl}-4-fluoro-5,5-dimethyl-4,5-dihydroisoxazole (2-8); 4-[difluoro-(4-fluoro-5,5-dimethyl-4,5-dihydroisoxazole-3-sulfonyl)methyl]-2-methyl-5-trifluoromethyl-2H-[1,2,3]triazole (2-9);
b11) from the group of the cellulose biosynthesis inhibitors:
   chlorthiamid, dichlobenil, flupoxam and isoxaben;
b12) from the group of the decoupler herbicides:
   dinoseb, dinoterb and DNOC and its salts;
b13) from the group of the auxin herbicides:
   2,4-D and its salts and esters, 2,4-DB and its salts and esters, aminopyralid and its salts such as aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, halauxifen and its salts and esters, MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, 5,6-dichloro-2-cyclopropyl-4-pyrimidinecarboxylic acid (H-9; CAS 858956-08-8) and its salts and esters, florpyrauxifen, florpyrauxifen-benzyl (CAS 1390661-72-9) and 4-amino-3-chloro-5-fluoro-6-(7-fluoro-1H-indol-6-yl)picolinic acid (CAS 1629965-65-6);
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, maleic hydrazide, mefluidide, metam, methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (H-10; CAS 499223-49-3) and its salts and esters.

Examples of preferred safeners C are benoxacor, cloquintocet, cyometrinil, cyprosulfamide, dichlormid, dicyclonone, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (H-11; MON4660, CAS 71526-07-3) and 2,2,5-trimethyl-3-(dichloroacetyl)-1,3-oxazolidine (H-12; R-29148, CAS 52836-31-4).

The active compounds of groups b1) to b15) and the safeners C are known herbicides and safeners, see, for example, The Compendium of Pesticide Common Names (http://www.alanwood.net/pesticides/); B. Hock, C. Fedtke, R. R. Schmidt, Herbizide [Herbicides], Georg Thieme Verlag, Stuttgart, 1995. Further herbicidally active compounds are known from WO 96/26202, WO 97/41116, WO 97/41117, WO 97/41118, WO 01/83459 and WO 2008/074991 and from W. Kramer et al. (ed.) "Modern Crop Protection Compounds", Vol. 1, Wiley VCH, 2007 and the literature quoted therein.

The invention also relates to combinations comprising at least one benzamide compound of the formula I and at least one further active compound, in particular a compound having herbicide activity (herbicide B) preferably selected from the active compounds of groups b1 to b15, and/or a safener C.

The invention also relates to compositions in the form of a crop protection composition formulated as a 1-component composition comprising an active compound combination comprising at least one benzamide compound of the formula I and at least one further active compound, in particular a compound having herbicide activity (herbicide B) preferably selected from the active compounds of groups b1 to b15, and at least one solid or liquid carrier and/or one or more surfactants and, if desired, one or more further auxiliaries customary for crop protection compositions.

The invention also relates to compositions in the form of a crop protection composition formulated as a 1-component composition comprising an active compound combination comprising at least one benzamide compound of the formula I and at least one safener C and at least one solid or liquid carrier and/or one or more surfactants and, if desired, one or more further auxiliaries customary for crop protection compositions.

The invention also relates to compositions in the form of a crop protection composition formulated as a 1-component composition comprising an active compound combination comprising at least one benzamide compound of the formula I and at least one further active compound, in particular a compound having herbicide activity (herbicide B) which is preferably selected from the active compounds of groups b1 to b15, a safener C and at least one solid or liquid carrier and/or one or more surfactants and, if desired, one or more further auxiliaries customary for crop protection compositions.

The invention also relates to compositions in the form of a crop protection composition formulated as a 2-component composition comprising a first component comprising at least one compound of the formula I, a solid or liquid carrier and/or one or more surfactants and a second component comprising at least one further active compound, in particular a compound having herbicide activity (herbicide B) which is preferably selected from the active compounds of groups b1 to b15, a solid or liquid carrier and/or one or more surfactants, where additionally both components may also comprise further auxiliaries customary for crop protection compositions.

The invention also relates to compositions in the form of a crop protection composition formulated as a 2-component composition comprising a first component comprising at least one compound of the formula I, a solid or liquid carrier and/or one or more surfactants and a second component comprising at least one further active compound, in particular a compound having herbicide activity (herbicide B) which is preferably selected from the active compounds of groups b1 to b15, a solid or liquid carrier and/or one or more surfactants, where additionally both components may also comprise further auxiliaries customary for crop protection compositions, where the first component or the second component further comprises a safener C.

In binary compositions comprising at least one compound of the formula I as component A and at least one herbicide B, the weight ratio of the active compounds A:B is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising at least one compound of the formula I as component A and at least one safener C, the weight ratio of the active compounds A:C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising both at least one compound of the formula I as component A, at least one herbicide B and at least one safener C, the relative parts by weight of the components A:B are generally in the range of from 1:1000 to 1000:1, preferably in the range of from 1:500 to 500:1, in particular in the range of from 1:250 to 250:1 and particularly preferably in the range of from 1:75 to 75:1; the weight ratio of the components A:C is generally in the range of from 1:1000 to 1000:1, preferably in the range of from 1:500 to 500:1, in particular in the range of from 1:250 to 250:1 and particularly preferably in the range of from 1:75 to 75:1; and the weight ratio of the components B:C is generally in the range of from 1:1000 to 1000:1, preferably in the range of from 1:500 to 500:1, in particular in the range of from 1:250 to 250:1 and particularly preferably in the range of from 1:75 to 75:1. Preferably, the weight ratio of the components A + B to the component C is in the range of from 1:500 to 500:1, in particular in the range of from 1:250 to 250:1 and particularly preferably in the range of from 1:75 to 75:1.

Examples of particularly preferred compositions according to the invention comprising in each case one individualized compound of the formula I and one mixing partner or a mixing partner combination are given in Table B below.

A further aspect of the invention relates to the combinations B-1 to B-1406 listed in Table B below, where in each case one row of Table B corresponds to a herbicidal composition comprising one of the compounds of formula I individualized in the above description (component 1) and the further active compound from groups b1) to b15) and/or safener C stated in each case in the row in question (component 2). The active compounds in the compositions described are in each case preferably present in synergistically effective amounts.

**Table B:**

| | **Herbicide(s) B** | **Safener C** |
|---|---|---|
| B-1 | clodinafop-propargyl | -- |
| B-2 | cycloxydim | -- |
| B-3 | cyhalofop-butyl | -- |
| B-4 | fenoxaprop-P-ethyl | -- |
| B-5 | pinoxaden | -- |
| B-6 | profoxydim | -- |
| B-7 | tepraloxydim | -- |
| B-8 | tralkoxydim | -- |
| B-9 | esprocarb | -- |
| B-10 | prosulfocarb | -- |
| B-11 | thiobencarb | -- |
| B-12 | triallate | -- |
| B-13 | bensulfuron-methyl | -- |
| B-14 | bispyribac-sodium | -- |
| B-15 | cyclosulfamuron | -- |
| B-16 | flumetsulam | -- |
| B-17 | flupyrsulfuron-methyl-sodium | -- |
| B-18 | foramsulfuron | -- |
| B-19 | imazamox | -- |
| B-20 | imazapic | -- |
| B-21 | imazapyr | -- |
| B-22 | imazaquin | -- |
| B-23 | imazethapyr | -- |
| B-24 | imazosulfuron | -- |
| B-25 | iodosulfuron-methyl-sodium | -- |
| B-26 | mesosulfuron | -- |
| B-27 | nicosulfuron | -- |
| B-28 | penoxsulam | -- |
| B-29 | propoxycarbazone-sodium | -- |
| B-30 | pyrazosulfuron-ethyl | -- |
| B-31 | pyroxsulam | -- |
| B-32 | rimsulfuron | -- |
| B-33 | sulfosulfuron | -- |
| B-34 | thiencarbazone-methyl | -- |
| B-35 | tritosulfuron | -- |
| B-36 | 2,4-D and its salts and esters | -- |
| B-37 | aminopyralid and its salts and esters | -- |
| B-38 | clopyralid and its salts and esters | -- |
| B-39 | dicamba and its salts and esters | -- |
| B-40 | fluroxypyr-meptyl | -- |
| B-41 | quinclorac | -- |
| B-42 | quinmerac | -- |
| B-43 | H-9 | -- |
| B-44 | diflufenzopyr | -- |
| B-45 | diflufenzopyr-sodium | -- |
| B-46 | clomazone | -- |
| B-47 | diflufenican | -- |
| B-48 | fluorochloridone | -- |
| B-49 | isoxaflutol | -- |
| B-50 | mesotrione | -- |
| B-51 | picolinafen | -- |
| B-52 | sulcotrione | -- |
| B-53 | tefuryltrione | -- |
| B-54 | tembotrione | -- |
| B-55 | topramezone | -- |
| B-56 | H-7 | -- |
| B-57 | atrazine | -- |
| B-58 | diuron | -- |
| B-59 | fluometuron | -- |
| B-60 | hexazinone | -- |
| B-61 | isoproturon | -- |
| B-62 | metribuzin | -- |
| B-63 | propanil | -- |
| B-64 | terbuthylazine | -- |
| B-65 | paraquat dichloride | -- |
| B-66 | flumioxazin | -- |
| B-67 | oxyfluorfen | -- |
| B-68 | saflufenacil | -- |
| B-69 | sulfentrazone | -- |
| B-70 | H-1 | -- |
| B-71 | H-2 | -- |
| B-72 | glyphosate | -- |
| B-73 | glyphosate-isopropylammonium | -- |
| B-74 | glyphosate-trimesium (sulfosate) | -- |
| B-75 | glufosinate | -- |
| B-76 | glufosinate-ammonium | -- |
| B-77 | pendimethalin | -- |
| B-78 | trifluralin | -- |
| B-79 | acetochlor | -- |
| B-80 | cafenstrole | -- |
| B-81 | dimethenamid-P | -- |
| B-82 | fentrazamide | -- |
| B-83 | flufenacet | -- |
| B-84 | mefenacet | -- |
| B-85 | metazachlor | -- |
| B-86 | metolachlor-S | -- |
| B-87 | pyroxasulfone | -- |
| B-88 | isoxaben | -- |
| B-89 | dymron | -- |
| B-90 | indanofan | -- |
| B-91 | oxaziclomefone | -- |
| B-92 | triaziflam | -- |
| B-93 | chlorotoluron | -- |
| B-94 | pinoxaden | -- |
| B-95 | sethoxydim | -- |
| B-96 | clethodim | -- |
| B-97 | diclofop | -- |
| B-98 | quizalofop | -- |
| B-99 | thifensulfuron | -- |
| B-100 | tribenuron | -- |
| B-101 | metsulfuron | -- |
| B-102 | foramsulfuron | -- |
| B-103 | chlorimuron | -- |
| B-104 | chlorsulfuron | -- |
| B-105 | flucarbazone-sodium | -- |
| B-106 | propoxycarbazone-sodium | -- |
| B-107 | ethalfluralin | -- |
| B-108 | halauxifen | -- |
| B-109 | MCPA | -- |
| B-110 | bromoxynil | -- |
| B-111 | bentazone | -- |
| B-112 | carfentrazone | -- |
| B-113 | trifludimoxazin | -- |
| B-114 | bicyclopyrone | -- |
| B-115 | benzobicyclon | -- |
| B-116 | pyrasulfotole | -- |
| B-117 | diquat | -- |
| B-118 | cinmethylin | -- |
| B-119 | acetochlor | -- |
| B-120 | naptalam | -- |
| B-121 | atrazine + H-1 | -- |
| B-122 | atrazine + glyphosate | -- |
| B-123 | atrazine + mesotrione | -- |
| B-124 | atrazine + nicosulfuron | -- |
| B-125 | atrazine + tembotrione | -- |
| B-126 | atrazine + topramezone | -- |
| B-127 | clomazone + glyphosate | -- |
| B-128 | diflufenican + clodinafop-propargyl | -- |
| B-129 | diflufenican + fenoxaprop-P-ethyl | -- |
| B-130 | diflufenican + flupyrsulfuron-methyl-sodium | -- |
| B-131 | diflufenican + glyphosate | -- |
| B-132 | diflufenican + mesosulfuron-methyl | -- |
| B-133 | diflufenican + pinoxaden | -- |
| B-134 | diflufenican + pyroxsulam | -- |
| B-135 | flumetsulam + glyphosate | -- |
| B-136 | flumioxazin + glyphosate | -- |
| B-137 | imazapic + glyphosate | -- |
| B-138 | imazethapyr + glyphosate | -- |
| B-139 | isoxaflutol + H-1 | -- |
| B-140 | isoxaflutol + glyphosate | -- |
| B-141 | metazachlor + H-1 | -- |
| B-142 | metazachlor + glyphosate | -- |
| B-143 | metazachlor + mesotrione | -- |
| B-144 | metazachlor + nicosulfuron | -- |
| B-145 | metazachlor + terbuthylazine | -- |
| B-146 | metazachlor + topramezone | -- |
| B-147 | metribuzin + glyphosate | -- |
| B-148 | pendimethalin + H-1 | -- |
| B-149 | pendimethalin + clodinafop-propargyl | -- |
| B-150 | pendimethalin + fenoxaprop-P-ethyl | -- |
| B-151 | pendimethalin + flupyrsulfuron-methyl-sodium | -- |
| B-152 | pendimethalin + glyphosate | -- |
| B-153 | pendimethalin + mesosulfuron-methyl | -- |
| B-154 | pendimethalin + mesotrione | -- |
| B-155 | pendimethalin + nicosulfuron | -- |
| B-156 | pendimethalin + pinoxaden | -- |
| B-157 | pendimethalin + pyroxsulam | -- |
| B-158 | pendimethalin + tembotrione | -- |
| B-159 | pendimethalin + topramezone | -- |
| B-160 | pyroxasulfone + tembotrione | -- |
| B-161 | pyroxasulfone + topramezone | -- |
| B-162 | sulfentrazone + glyphosate | -- |
| B-163 | terbuthylazine + H-1 | -- |
| B-164 | terbuthylazine + foramsulfuron | -- |
| B-165 | terbuthylazine + glyphosate | -- |
| B-166 | terbuthylazine + mesotrione | -- |
| B-167 | terbuthylazine + nicosulfuron | -- |
| B-168 | terbuthylazine + tembotrione | -- |
| B-169 | terbuthylazine + topramezone | -- |
| B-170 | trifluralin + glyphosate | -- |
| B-171 | -- | benoxacor |
| B-172 | -- | cloquintocet |
| B-173 | -- | cyprosulfamide |
| B-174 | -- | dichlormid |
| B-175 | -- | fenchlorazole |
| B-176 | -- | fenclorim |
| B-177 | -- | isoxadifen |
| B-178 | -- | mefenpyr |
| B-179 | -- | H-11 |
| B-180 | -- | H-12 |
| B-181 | clodinafop-propargyl | benoxacor |
| B-182 | cycloxydim | benoxacor |
| B-183 | cyhalofop-butyl | benoxacor |
| B-184 | fenoxaprop-P-ethyl | benoxacor |
| B-185 | pinoxaden | benoxacor |
| B-186 | profoxydim | benoxacor |
| B-187 | tepraloxydim | benoxacor |
| B-188 | tralkoxydim | benoxacor |
| B-189 | esprocarb | benoxacor |
| B-190 | prosulfocarb | benoxacor |
| B-191 | thiobencarb | benoxacor |
| B-192 | triallate | benoxacor |
| B-193 | bensulfuron-methyl | benoxacor |
| B-194 | bispyribac-sodium | benoxacor |
| B-195 | cyclosulfamuron | benoxacor |
| B-196 | flumetsulam | benoxacor |
| B-197 | flupyrsulfuron-methyl-sodium | benoxacor |
| B-198 | foramsulfuron | benoxacor |
| B-199 | imazamox | benoxacor |
| B-200 | imazapic | benoxacor |
| B-201 | imazapyr | benoxacor |
| B-202 | imazaquin | benoxacor |
| B-203 | imazethapyr | benoxacor |
| B-204 | imazosulfuron | benoxacor |
| B-205 | iodosulfuron-methyl-sodium | benoxacor |
| B-206 | mesosulfuron | benoxacor |
| B-207 | nicosulfuron | benoxacor |
| B-208 | penoxsulam | benoxacor |
| B-209 | propoxycarbazone-sodium | benoxacor |
| B-210 | pyrazosulfuron-ethyl | benoxacor |
| B-211 | pyroxsulam | benoxacor |
| B-212 | rimsulfuron | benoxacor |
| B-213 | sulfosulfuron | benoxacor |
| B-214 | thiencarbazone-methyl | benoxacor |
| B-215 | tritosulfuron | benoxacor |
| B-216 | 2,4-D and its salts and esters | benoxacor |
| B-217 | aminopyralid and its salts and esters | benoxacor |
| B-218 | clopyralid and its salts and esters | benoxacor |
| B-219 | dicamba and its salts and esters | benoxacor |
| B-220 | fluroxypyr-meptyl | benoxacor |
| B-221 | quinclorac | benoxacor |
| B-222 | quinmerac | benoxacor |
| B-223 | H-9 | benoxacor |
| B-224 | diflufenzopyr | benoxacor |
| B-225 | diflufenzopyr-sodium | benoxacor |
| B-226 | clomazone | benoxacor |
| B-227 | diflufenican | benoxacor |
| B-228 | fluorochloridone | benoxacor |
| B-229 | isoxaflutol | benoxacor |
| B-230 | mesotrione | benoxacor |
| B-231 | picolinafen | benoxacor |
| B-232 | sulcotrione | benoxacor |
| B-233 | tefuryltrione | benoxacor |
| B-234 | tembotrione | benoxacor |
| B-235 | topramezone | benoxacor |
| B-236 | H-7 | benoxacor |
| B-237 | atrazine | benoxacor |
| B-238 | diuron | benoxacor |
| B-239 | fluometuron | benoxacor |
| B-240 | hexazinone | benoxacor |
| B-241 | isoproturon | benoxacor |
| B-242 | metribuzin | benoxacor |
| B-243 | propanil | benoxacor |
| B-244 | terbuthylazine | benoxacor |
| B-245 | paraquat dichloride | benoxacor |
| B-246 | flumioxazin | benoxacor |
| B-247 | oxyfluorfen | benoxacor |
| B-248 | saflufenacil | benoxacor |
| B-249 | sulfentrazone | benoxacor |
| B-250 | H-1 | benoxacor |
| B-251 | H-2 | benoxacor |
| B-252 | glyphosate | benoxacor |
| B-253 | glyphosate-isopropylammonium | benoxacor |
| B-254 | glyphosate-trimesium (sulfosate) | benoxacor |
| B-255 | glufosinate | benoxacor |
| B-256 | glufosinate-ammonium | benoxacor |
| B-257 | pendimethalin | benoxacor |
| B-258 | trifluralin | benoxacor |
| B-259 | acetochlor | benoxacor |
| B-260 | cafenstrole | benoxacor |
| B-261 | dimethenamid-P | benoxacor |
| B-262 | fentrazamide | benoxacor |
| B-263 | flufenacet | benoxacor |
| B-264 | mefenacet | benoxacor |
| B-265 | metazachlor | benoxacor |
| B-266 | metolachlor-S | benoxacor |
| B-267 | pyroxasulfone | benoxacor |
| B-268 | isoxaben | benoxacor |
| B-269 | dymron | benoxacor |
| B-270 | indanofan | benoxacor |
| B-271 | oxaziclomefone | benoxacor |
| B-272 | triaziflam | benoxacor |
| B-273 | atrazine + H-1 | benoxacor |
| B-274 | atrazine + glyphosate | benoxacor |
| B-275 | atrazine + mesotrione | benoxacor |
| B-276 | atrazine + nicosulfuron | benoxacor |
| B-277 | atrazine + tembotrione | benoxacor |
| B-278 | atrazine + topramezone | benoxacor |
| B-279 | clomazone + glyphosate | benoxacor |
| B-280 | diflufenican + clodinafop-propargyl | benoxacor |
| B-281 | diflufenican + fenoxaprop-P-ethyl | benoxacor |
| B-282 | diflufenican + flupyrsulfuron-methyl-sodium | benoxacor |
| B-283 | diflufenican + glyphosate | benoxacor |
| B-284 | diflufenican + mesosulfuron-methyl | benoxacor |
| B-285 | diflufenican + pinoxaden | benoxacor |
| B-286 | diflufenican + pyroxsulam | benoxacor |
| B-287 | flumetsulam + glyphosate | benoxacor |
| B-288 | flumioxazin + glyphosate | benoxacor |
| B-289 | imazapic + glyphosate | benoxacor |
| B-290 | imazethapyr + glyphosate | benoxacor |
| B-291 | isoxaflutol + H-1 | benoxacor |
| B-292 | isoxaflutol + glyphosate | benoxacor |
| B-293 | metazachlor + H-1 | benoxacor |
| B-294 | metazachlor + glyphosate | benoxacor |
| B-295 | metazachlor + mesotrione | benoxacor |
| B-296 | metazachlor + nicosulfuron | benoxacor |
| B-297 | metazachlor + terbuthylazine | benoxacor |
| B-298 | metazachlor + topramezone | benoxacor |
| B-299 | metribuzin + glyphosate | benoxacor |
| B-300 | pendimethalin + H-1 | benoxacor |
| B-301 | pendimethalin + clodinafop-propargyl | benoxacor |
| B-302 | pendimethalin + fenoxaprop-P-ethyl | benoxacor |
| B-303 | pendimethalin + flupyrsulfuron-methyl-sodium | benoxacor |
| B-304 | pendimethalin + glyphosate | benoxacor |
| B-305 | pendimethalin + mesosulfuron-methyl | benoxacor |
| B-306 | pendimethalin + mesotrione | benoxacor |
| B-307 | pendimethalin + nicosulfuron | benoxacor |
| B-308 | pendimethalin + pinoxaden | benoxacor |
| B-309 | pendimethalin + pyroxsulam | benoxacor |
| B-310 | pendimethalin + tembotrione | benoxacor |
| B-311 | pendimethalin + topramezone | benoxacor |
| B-312 | pyroxasulfone + tembotrione | benoxacor |
| B-313 | pyroxasulfone + topramezone | benoxacor |
| B-314 | sulfentrazone + glyphosate | benoxacor |
| B-315 | terbuthylazine + H-1 | benoxacor |
| B-316 | terbuthylazine + foramsulfuron | benoxacor |
| B-317 | terbuthylazine + glyphosate | benoxacor |
| B-318 | terbuthylazine + mesotrione | benoxacor |
| B-319 | terbuthylazine + nicosulfuron | benoxacor |
| B-320 | terbuthylazine + tembotrione | benoxacor |
| B-321 | terbuthylazine + topramezone | benoxacor |
| B-322 | trifluralin + glyphosate | benoxacor |
| B-323 | clodinafop-propargyl | cloquintocet |
| B-324 | cycloxydim | cloquintocet |
| B-325 | cyhalofop-butyl | cloquintocet |
| B-326 | fenoxaprop-P-ethyl | cloquintocet |
| B-327 | pinoxaden | cloquintocet |
| B-328 | profoxydim | cloquintocet |
| B-329 | tepraloxydim | cloquintocet |
| B-330 | tralkoxydim | cloquintocet |
| B-331 | esprocarb | cloquintocet |
| B-332 | prosulfocarb | cloquintocet |
| B-333 | thiobencarb | cloquintocet |
| B-334 | triallate | cloquintocet |
| B-335 | bensulfuron-methyl | cloquintocet |
| B-336 | bispyribac-sodium | cloquintocet |
| B-337 | cyclosulfamuron | cloquintocet |
| B-338 | flumetsulam | cloquintocet |
| B-339 | flupyrsulfuron-methyl-sodium | cloquintocet |
| B-340 | foramsulfuron | cloquintocet |
| B-341 | imazamox | cloquintocet |
| B-342 | imazapic | cloquintocet |
| B-343 | imazapyr | cloquintocet |
| B-344 | imazaquin | cloquintocet |
| B-345 | imazethapyr | cloquintocet |
| B-346 | imazosulfuron | cloquintocet |
| B-347 | iodosulfuron-methyl-sodium | cloquintocet |
| B-348 | mesosulfuron | cloquintocet |
| B-349 | nicosulfuron | cloquintocet |
| B-350 | penoxsulam | cloquintocet |
| B-351 | propoxycarbazone-sodium | cloquintocet |
| B-352 | pyrazosulfuron-ethyl | cloquintocet |
| B-353 | pyroxsulam | cloquintocet |
| B-354 | rimsulfuron | cloquintocet |
| B-355 | sulfosulfuron | cloquintocet |
| B-356 | thiencarbazone-methyl | cloquintocet |
| B-357 | tritosulfuron | cloquintocet |
| B-358 | 2,4-D and its salts and esters | cloquintocet |
| B-359 | aminopyralid and its salts and esters | cloquintocet |
| B-360 | clopyralid and its salts and esters | cloquintocet |
| B-361 | dicamba and its salts and esters | cloquintocet |
| B-362 | fluroxypyr-meptyl | cloquintocet |
| B-363 | quinclorac | cloquintocet |
| B-364 | quinmerac | cloquintocet |
| B-365 | H-9 | cloquintocet |
| B-366 | diflufenzopyr | cloquintocet |
| B-367 | diflufenzopyr-sodium | cloquintocet |
| B-368 | clomazone | cloquintocet |
| B-369 | diflufenican | cloquintocet |
| B-370 | fluorochloridone | cloquintocet |
| B-371 | isoxaflutol | cloquintocet |
| B-372 | mesotrione | cloquintocet |
| B-373 | picolinafen | cloquintocet |
| B-374 | sulcotrione | cloquintocet |
| B-375 | tefuryltrione | cloquintocet |
| B-376 | tembotrione | cloquintocet |
| B-377 | topramezone | cloquintocet |
| B-378 | H-7 | cloquintocet |
| B-379 | atrazine | cloquintocet |
| B-380 | diuron | cloquintocet |
| B-381 | fluometuron | cloquintocet |
| B-382 | hexazinone | cloquintocet |
| B-383 | isoproturon | cloquintocet |
| B-384 | metribuzin | cloquintocet |
| B-385 | propanil | cloquintocet |
| B-386 | terbuthylazine | cloquintocet |
| B-387 | paraquat dichloride | cloquintocet |
| B-388 | flumioxazin | cloquintocet |
| B-389 | oxyfluorfen | cloquintocet |
| B-390 | saflufenacil | cloquintocet |
| B-391 | sulfentrazone | cloquintocet |
| B-392 | H-1 | cloquintocet |
| B-393 | H-2 | cloquintocet |
| B-394 | glyphosate | cloquintocet |
| B-395 | glyphosate-isopropylammonium | cloquintocet |
| B-396 | glyphosate-trimesium (sulfosate) | cloquintocet |
| B-397 | glufosinate | cloquintocet |
| B-398 | glufosinate-ammonium | cloquintocet |
| B-399 | pendimethalin | cloquintocet |
| B-400 | trifluralin | cloquintocet |
| B-401 | acetochlor | cloquintocet |
| B-402 | cafenstrole | cloquintocet |
| B-403 | dimethenamid-P | cloquintocet |
| B-404 | fentrazamide | cloquintocet |
| B-405 | flufenacet | cloquintocet |
| B-406 | mefenacet | cloquintocet |
| B-407 | metazachlor | cloquintocet |
| B-408 | metolachlor-S | cloquintocet |
| B-409 | pyroxasulfone | cloquintocet |
| B-410 | isoxaben | cloquintocet |
| B-411 | dymron | cloquintocet |
| B-412 | indanofan | cloquintocet |
| B-413 | oxaziclomefone | cloquintocet |
| B-414 | triaziflam | cloquintocet |
| B-415 | atrazine + H-1 | cloquintocet |
| B-416 | atrazine + glyphosate | cloquintocet |
| B-417 | atrazine + mesotrione | cloquintocet |
| B-418 | atrazine + nicosulfuron | cloquintocet |
| B-419 | atrazine + tembotrione | cloquintocet |
| B-420 | atrazine + topramezone | cloquintocet |
| B-421 | clomazone + glyphosate | cloquintocet |
| B-422 | diflufenican + clodinafop-propargyl | cloquintocet |
| B-423 | diflufenican + fenoxaprop-p-ethyl | cloquintocet |
| B-424 | diflufenican + flupyrsulfuron-methyl-sodium | cloquintocet |
| B-425 | diflufenican + glyphosate | cloquintocet |
| B-426 | diflufenican + mesosulfuron-methyl | cloquintocet |
| B-427 | diflufenican + pinoxaden | cloquintocet |
| B-428 | diflufenican + pyroxsulam | cloquintocet |
| B-429 | flumetsulam + glyphosate | cloquintocet |
| B-430 | flumioxazin + glyphosate | cloquintocet |
| B-431 | imazapic + glyphosate | cloquintocet |
| B-432 | imazethapyr + glyphosate | cloquintocet |
| B-433 | isoxaflutol + H-1 | cloquintocet |
| B-434 | isoxaflutol + glyphosate | cloquintocet |
| B-435 | metazachlor + H-1 | cloquintocet |
| B-436 | metazachlor + glyphosate | cloquintocet |
| B-437 | metazachlor + mesotrione | cloquintocet |
| B-438 | metazachlor + nicosulfuron | cloquintocet |
| B-439 | metazachlor + terbuthylazine | cloquintocet |
| B-440 | metazachlor + topramezone | cloquintocet |
| B-441 | metribuzin + glyphosate | cloquintocet |
| B-442 | pendimethalin + H-1 | cloquintocet |
| B-443 | pendimethalin + clodinafop-propargyl | cloquintocet |
| B-444 | pendimethalin + fenoxaprop-P-ethyl | cloquintocet |
| B-445 | pendimethalin + flupyrsulfuron-methyl-sodium | cloquintocet |
| B-446 | pendimethalin + glyphosate | cloquintocet |
| B-447 | pendimethalin + mesosulfuron-methyl | cloquintocet |
| B-448 | pendimethalin + mesotrione | cloquintocet |
| B-449 | pendimethalin + nicosulfuron | cloquintocet |
| B-450 | pendimethalin + pinoxaden | cloquintocet |
| B-451 | pendimethalin + pyroxsulam | cloquintocet |
| B-452 | pendimethalin + tembotrione | cloquintocet |
| B-453 | pendimethalin + topramezone | cloquintocet |
| B-454 | pyroxasulfone + tembotrione | cloquintocet |
| B-455 | pyroxasulfone + topramezone | cloquintocet |
| B-456 | sulfentrazone + glyphosate | cloquintocet |
| B-457 | terbuthylazine + H-1 | cloquintocet |
| B-458 | terbuthylazine + foramsulfuron | cloquintocet |
| B-459 | terbuthylazine + glyphosate | cloquintocet |
| B-460 | terbuthylazine + mesotrione | cloquintocet |
| B-461 | terbuthylazine + nicosulfuron | cloquintocet |
| B-462 | terbuthylazine + tembotrione | cloquintocet |
| B-463 | terbuthylazine + topramezone | cloquintocet |
| B-464 | trifluralin + glyphosate | cloquintocet |
| B-465 | clodinafop-propargyl | dichlormid |
| B-466 | cycloxydim | dichlormid |
| B-467 | cyhalofop-butyl | dichlormid |
| B-468 | fenoxaprop-P-ethyl | dichlormid |
| B-469 | pinoxaden | dichlormid |
| B-470 | profoxydim | dichlormid |
| B-471 | tepraloxydim | dichlormid |
| B-472 | tralkoxydim | dichlormid |
| B-473 | esprocarb | dichlormid |
| B-474 | prosulfocarb | dichlormid |
| B-475 | thiobencarb | dichlormid |
| B-476 | triallate | dichlormid |
| B-477 | bensulfuron-methyl | dichlormid |
| B-478 | bispyribac-sodium | dichlormid |
| B-479 | cyclosulfamuron | dichlormid |
| B-480 | flumetsulam | dichlormid |
| B-481 | flupyrsulfuron-methyl-sodium | dichlormid |
| B-482 | foramsulfuron | dichlormid |
| B-483 | imazamox | dichlormid |
| B-484 | imazapic | dichlormid |
| B-485 | imazapyr | dichlormid |
| B-486 | imazaquin | dichlormid |
| B-487 | imazethapyr | dichlormid |
| B-488 | imazosulfuron | dichlormid |
| B-489 | iodosulfuron-methyl-sodium | dichlormid |
| B-490 | mesosulfuron | dichlormid |
| B-491 | nicosulfuron | dichlormid |
| B-492 | penoxsulam | dichlormid |
| B-493 | propoxycarbazone-sodium | dichlormid |
| B-494 | pyrazosulfuron-ethyl | dichlormid |
| B-495 | pyroxsulam | dichlormid |
| B-496 | rimsulfuron | dichlormid |
| B-497 | sulfosulfuron | dichlormid |
| B-498 | thiencarbazone-methyl | dichlormid |
| B-499 | tritosulfuron | dichlormid |
| B-500 | 2,4-D and its salts and esters | dichlormid |
| B-501 | aminopyralid and its salts and esters | dichlormid |
| B-502 | clopyralid and its salts and esters | dichlormid |
| B-503 | dicamba and its salts and esters | dichlormid |
| B-504 | fluroxypyr-meptyl | dichlormid |
| B-505 | quinclorac | dichlormid |
| B-506 | quinmerac | dichlormid |
| B-507 | H-9 | dichlormid |
| B-508 | diflufenzopyr | dichlormid |
| B-509 | diflufenzopyr-sodium | dichlormid |
| B-510 | clomazone | dichlormid |
| B-511 | diflufenican | dichlormid |
| B-512 | fluorochloridone | dichlormid |
| B-513 | isoxaflutol | dichlormid |
| B-514 | mesotrione | dichlormid |
| B-515 | picolinafen | dichlormid |
| B-516 | sulcotrione | dichlormid |
| B-517 | tefuryltrione | dichlormid |
| B-518 | tembotrione | dichlormid |
| B-519 | topramezone | dichlormid |
| B-520 | H-7 | dichlormid |
| B-521 | atrazine | dichlormid |
| B-522 | diuron | dichlormid |
| B-523 | fluometuron | dichlormid |
| B-524 | hexazinone | dichlormid |
| B-525 | isoproturon | dichlormid |
| B-526 | metribuzin | dichlormid |
| B-527 | propanil | dichlormid |
| B-528 | terbuthylazine | dichlormid |
| B-529 | paraquat dichloride | dichlormid |
| B-530 | flumioxazin | dichlormid |
| B-531 | oxyfluorfen | dichlormid |
| B-532 | saflufenacil | dichlormid |
| B-533 | sulfentrazone | dichlormid |
| B-534 | H-1 | dichlormid |
| B-535 | H-2 | dichlormid |
| B-536 | glyphosate | dichlormid |
| B-537 | glyphosate-isopropylammonium | dichlormid |
| B-538 | glyphosate-trimesium (sulfosate) | dichlormid |
| B-539 | glufosinate | dichlormid |
| B-540 | glufosinate-ammonium | dichlormid |
| B-541 | pendimethalin | dichlormid |
| B-542 | trifluralin | dichlormid |
| B-543 | acetochlor | dichlormid |
| B-544 | cafenstrole | dichlormid |
| B-545 | dimethenamid-P | dichlormid |
| B-546 | fentrazamide | dichlormid |
| B-547 | flufenacet | dichlormid |
| B-548 | mefenacet | dichlormid |
| B-549 | metazachlor | dichlormid |
| B-550 | metolachlor-S | dichlormid |
| B-551 | pyroxasulfone | dichlormid |
| B-552 | isoxaben | dichlormid |
| B-553 | dymron | dichlormid |
| B-554 | indanofan | dichlormid |
| B-555 | oxaziclomefone | dichlormid |
| B-556 | triaziflam | dichlormid |
| B-557 | atrazine + H-1 | dichlormid |
| B-558 | atrazine + glyphosate | dichlormid |
| B-559 | atrazine + mesotrione | dichlormid |
| B-560 | atrazine + nicosulfuron | dichlormid |
| B-561 | atrazine + tembotrione | dichlormid |
| B-562 | atrazine + topramezone | dichlormid |
| B-563 | clomazone + glyphosate | dichlormid |
| B-564 | diflufenican + clodinafop-propargyl | dichlormid |
| B-565 | diflufenican + fenoxaprop-p-ethyl | dichlormid |
| B-566 | diflufenican + flupyrsulfuron-methyl-sodium | dichlormid |
| B-567 | diflufenican + glyphosate | dichlormid |
| B-568 | diflufenican + mesosulfuron-methyl | dichlormid |
| B-569 | diflufenican + pinoxaden | dichlormid |
| B-570 | diflufenican + pyroxsulam | dichlormid |
| B-571 | flumetsulam + glyphosate | dichlormid |
| B-572 | flumioxazin + glyphosate | dichlormid |
| B-573 | imazapic + glyphosate | dichlormid |
| B-574 | imazethapyr + glyphosate | dichlormid |
| B-575 | isoxaflutol + H-1 | dichlormid |
| B-576 | isoxaflutol + glyphosate | dichlormid |
| B-577 | metazachlor + H-1 | dichlormid |
| B-578 | metazachlor + glyphosate | dichlormid |
| B-579 | metazachlor + mesotrione | dichlormid |
| B-580 | metazachlor + nicosulfuron | dichlormid |
| B-581 | metazachlor + terbuthylazine | dichlormid |
| B-582 | metazachlor + topramezone | dichlormid |
| B-583 | metribuzin + glyphosate | dichlormid |
| B-584 | pendimethalin + H-1 | dichlormid |
| B-585 | pendimethalin + clodinafop-propargyl | dichlormid |
| B-586 | pendimethalin + fenoxaprop-P-ethyl | dichlormid |
| B-587 | pendimethalin + flupyrsulfuron-methyl-sodium | dichlormid |
| B-588 | pendimethalin + glyphosate | dichlormid |
| B-589 | pendimethalin + mesosulfuron-methyl | dichlormid |
| B-590 | pendimethalin + mesotrione | dichlormid |
| B-591 | pendimethalin + nicosulfuron | dichlormid |
| B-592 | pendimethalin + pinoxaden | dichlormid |
| B-593 | pendimethalin + pyroxsulam | dichlormid |
| B-594 | pendimethalin + tembotrione | dichlormid |
| B-595 | pendimethalin + topramezone | dichlormid |
| B-596 | pyroxasulfone + tembotrione | dichlormid |
| B-597 | pyroxasulfone + topramezone | dichlormid |
| B-598 | sulfentrazone + glyphosate | dichlormid |
| B-599 | terbuthylazine + H-1 | dichlormid |
| B-600 | terbuthylazine + foramsulfuron | dichlormid |
| B-601 | terbuthylazine + glyphosate | dichlormid |
| B-602 | terbuthylazine + mesotrione | dichlormid |
| B-603 | terbuthylazine + nicosulfuron | dichlormid |
| B-604 | terbuthylazine + tembotrione | dichlormid |
| B-605 | terbuthylazine + topramezone | dichlormid |
| B-606 | trifluralin + glyphosate | dichlormid |
| B-607 | clodinafop-propargyl | fenchlorazole |
| B-608 | cycloxydim | fenchlorazole |
| B-609 | cyhalofop-butyl | fenchlorazole |
| B-610 | fenoxaprop-P-ethyl | fenchlorazole |
| B-611 | pinoxaden | fenchlorazole |
| B-612 | profoxydim | fenchlorazole |
| B-613 | tepraloxydim | fenchlorazole |
| B-614 | tralkoxydim | fenchlorazole |
| B-615 | esprocarb | fenchlorazole |
| B-616 | prosulfocarb | fenchlorazole |
| B-617 | thiobencarb | fenchlorazole |
| B-618 | triallate | fenchlorazole |
| B-619 | bensulfuron-methyl | fenchlorazole |
| B-620 | bispyribac-sodium | fenchlorazole |
| B-621 | cyclosulfamuron | fenchlorazole |
| B-622 | flumetsulam | fenchlorazole |
| B-623 | flupyrsulfuron-methyl-sodium | fenchlorazole |
| B-624 | foramsulfuron | fenchlorazole |
| B-625 | imazamox | fenchlorazole |
| B-626 | imazapic | fenchlorazole |
| B-627 | imazapyr | fenchlorazole |
| B-628 | imazaquin | fenchlorazole |
| B-629 | imazethapyr | fenchlorazole |
| B-630 | imazosulfuron | fenchlorazole |
| B-631 | iodosulfuron-methyl-sodium | fenchlorazole |
| B-632 | mesosulfuron | fenchlorazole |
| B-633 | nicosulfuron | fenchlorazole |
| B-634 | penoxsulam | fenchlorazole |
| B-635 | propoxycarbazone-sodium | fenchlorazole |
| B-636 | pyrazosulfuron-ethyl | fenchlorazole |
| B-637 | pyroxsulam | fenchlorazole |
| B-638 | rimsulfuron | fenchlorazole |
| B-639 | sulfosulfuron | fenchlorazole |
| B-640 | thiencarbazone-methyl | fenchlorazole |
| B-641 | tritosulfuron | fenchlorazole |
| B-642 | 2,4-D and its salts and esters | fenchlorazole |
| B-643 | aminopyralid and its salts and esters | fenchlorazole |
| B-644 | clopyralid and its salts and esters | fenchlorazole |
| B-645 | dicamba and its salts and esters | fenchlorazole |
| B-646 | fluroxypyr-meptyl | fenchlorazole |
| B-647 | quinclorac | fenchlorazole |
| B-648 | quinmerac | fenchlorazole |
| B-649 | H-9 | fenchlorazole |
| B-650 | diflufenzopyr | fenchlorazole |
| B-651 | diflufenzopyr-sodium | fenchlorazole |
| B-652 | clomazone | fenchlorazole |
| B-653 | diflufenican | fenchlorazole |
| B-654 | fluorochloridone | fenchlorazole |
| B-655 | isoxaflutol | fenchlorazole |
| B-656 | mesotrione | fenchlorazole |
| B-657 | picolinafen | fenchlorazole |
| B-658 | sulcotrione | fenchlorazole |
| B-659 | tefuryltrione | fenchlorazole |
| B-660 | tembotrione | fenchlorazole |
| B-661 | topramezone | fenchlorazole |
| B-662 | H-7 | fenchlorazole |
| B-663 | atrazine | fenchlorazole |
| B-664 | diuron | fenchlorazole |
| B-665 | fluometuron | fenchlorazole |
| B-666 | hexazinone | fenchlorazole |
| B-667 | isoproturon | fenchlorazole |
| B-668 | metribuzin | fenchlorazole |
| B-669 | propanil | fenchlorazole |
| B-670 | terbuthylazine | fenchlorazole |
| B-671 | paraquat dichloride | fenchlorazole |
| B-672 | flumioxazin | fenchlorazole |
| B-673 | oxyfluorfen | fenchlorazole |
| B-674 | saflufenacil | fenchlorazole |
| B-675 | sulfentrazone | fenchlorazole |
| B-676 | H-1 | fenchlorazole |
| B-677 | H-2 | fenchlorazole |
| B-678 | glyphosate | fenchlorazole |
| B-679 | glyphosate-isopropylammonium | fenchlorazole |
| B-680 | glyphosate-trimesium (sulfosate) | fenchlorazole |
| B-681 | glufosinate | fenchlorazole |
| B-682 | glufosinate-ammonium | fenchlorazole |
| B-683 | pendimethalin | fenchlorazole |
| B-684 | trifluralin | fenchlorazole |
| B-685 | acetochlor | fenchlorazole |
| B-686 | cafenstrole | fenchlorazole |
| B-687 | dimethenamid-P | fenchlorazole |
| B-688 | fentrazamide | fenchlorazole |
| B-689 | flufenacet | fenchlorazole |
| B-690 | mefenacet | fenchlorazole |
| B-691 | metazachlor | fenchlorazole |
| B-692 | metolachlor-S | fenchlorazole |
| B-693 | pyroxasulfone | fenchlorazole |
| B-694 | isoxaben | fenchlorazole |
| B-695 | dymron | fenchlorazole |
| B-696 | indanofan | fenchlorazole |
| B-697 | oxaziclomefone | fenchlorazole |
| B-698 | triaziflam | fenchlorazole |
| B-699 | atrazine + H-1 | fenchlorazole |
| B-700 | atrazine + glyphosate | fenchlorazole |
| B-701 | atrazine + mesotrione | fenchlorazole |
| B-702 | atrazine + nicosulfuron | fenchlorazole |
| B-703 | atrazine + tembotrione | fenchlorazole |
| B-704 | atrazine + topramezone | fenchlorazole |
| B-705 | clomazone + glyphosate | fenchlorazole |
| B-706 | diflufenican + clodinafop-propargyl | fenchlorazole |
| B-707 | diflufenican + fenoxaprop-P-ethyl | fenchlorazole |
| B-708 | diflufenican + flupyrsulfuron-methyl-sodium | fenchlorazole |
| B-709 | diflufenican + glyphosate | fenchlorazole |
| B-710 | diflufenican + mesosulfuron-methyl | fenchlorazole |
| B-711 | diflufenican + pinoxaden | fenchlorazole |
| B-712 | diflufenican + pyroxsulam | fenchlorazole |
| B-713 | flumetsulam + glyphosate | fenchlorazole |
| B-714 | flumioxazin + glyphosate | fenchlorazole |
| B-715 | imazapic + glyphosate | fenchlorazole |
| B-716 | imazethapyr + glyphosate | fenchlorazole |
| B-717 | isoxaflutol + H-1 | fenchlorazole |
| B-718 | isoxaflutol + glyphosate | fenchlorazole |
| B-719 | metazachlor + H-1 | fenchlorazole |
| B-720 | metazachlor + glyphosate | fenchlorazole |
| B-721 | metazachlor + mesotrione | fenchlorazole |
| B-722 | metazachlor + nicosulfuron | fenchlorazole |
| B-723 | metazachlor + terbuthylazine | fenchlorazole |
| B-724 | metazachlor + topramezone | fenchlorazole |
| B-725 | metribuzin + glyphosate | fenchlorazole |
| B-726 | pendimethalin + H-1 | fenchlorazole |
| B-727 | pendimethalin + clodinafop-propargyl | fenchlorazole |
| B-728 | pendimethalin + fenoxaprop-P-ethyl | fenchlorazole |
| B-729 | pendimethalin + flupyrsulfuron-methyl-sodium | fenchlorazole |
| B-730 | pendimethalin + glyphosate | fenchlorazole |
| B-731 | pendimethalin + mesosulfuron-methyl | fenchlorazole |
| B-732 | pendimethalin + mesotrione | fenchlorazole |
| B-733 | pendimethalin + nicosulfuron | fenchlorazole |
| B-734 | pendimethalin + pinoxaden | fenchlorazole |
| B-735 | pendimethalin + pyroxsulam | fenchlorazole |
| B-736 | pendimethalin + tembotrione | fenchlorazole |
| B-737 | pendimethalin + topramezone | fenchlorazole |
| B-738 | pyroxasulfone + tembotrione | fenchlorazole |
| B-739 | pyroxasulfone + topramezone | fenchlorazole |
| B-740 | sulfentrazone + glyphosate | fenchlorazole |
| B-741 | terbuthylazine + H-1 | fenchlorazole |
| B-742 | terbuthylazine + foramsulfuron | fenchlorazole |
| B-743 | terbuthylazine + glyphosate | fenchlorazole |
| B-744 | terbuthylazine + mesotrione | fenchlorazole |
| B-745 | terbuthylazine + nicosulfuron | fenchlorazole |
| B-746 | terbuthylazine + tembotrione | fenchlorazole |
| B-747 | terbuthylazine + topramezone | fenchlorazole |
| B-748 | trifluralin + glyphosate | fenchlorazole |
| B-749 | clodinafop-propargyl | fenclorim |
| B-750 | cycloxydim | fenclorim |
| B-751 | cyhalofop-butyl | fenclorim |
| B-752 | fenoxaprop-P-ethyl | fenclorim |
| B-753 | pinoxaden | fenclorim |
| B-754 | profoxydim | fenclorim |
| B-755 | tepraloxydim | fenclorim |
| B-756 | tralkoxydim | fenclorim |
| B-757 | esprocarb | fenclorim |
| B-758 | prosulfocarb | fenclorim |
| B-759 | thiobencarb | fenclorim |
| B-760 | triallate | fenclorim |
| B-761 | bensulfuron-methyl | fenclorim |
| B-762 | bispyribac-sodium | fenclorim |
| B-763 | cyclosulfamuron | fenclorim |
| B-764 | flumetsulam | fenclorim |
| B-765 | flupyrsulfuron-methyl-sodium | fenclorim |
| B-766 | foramsulfuron | fenclorim |
| B-767 | imazamox | fenclorim |
| B-768 | imazapic | fenclorim |
| B-769 | imazapyr | fenclorim |
| B-770 | imazaquin | fenclorim |
| B-771 | imazethapyr | fenclorim |
| B-772 | imazosulfuron | fenclorim |
| B-773 | iodosulfuron-methyl-sodium | fenclorim |
| B-774 | mesosulfuron | fenclorim |
| B-775 | nicosulfuron | fenclorim |
| B-776 | penoxsulam | fenclorim |
| B-777 | propoxycarbazone-sodium | fenclorim |
| B-778 | pyrazosulfuron-ethyl | fenclorim |
| B-779 | pyroxsulam | fenclorim |
| B-780 | rimsulfuron | fenclorim |
| B-781 | sulfosulfuron | fenclorim |
| B-782 | thiencarbazone-methyl | fenclorim |
| B-783 | tritosulfuron | fenclorim |
| B-784 | 2,4-D and its salts and esters | fenclorim |
| B-785 | aminopyralid and its salts and esters | fenclorim |
| B-786 | clopyralid and its salts and esters | fenclorim |
| B-787 | dicamba and its salts and esters | fenclorim |
| B-788 | fluroxypyr-meptyl | fenclorim |
| B-789 | quinclorac | fenclorim |
| B-790 | quinmerac | fenclorim |
| B-791 | H-9 | fenclorim |
| B-792 | diflufenzopyr | fenclorim |
| B-793 | diflufenzopyr-sodium | fenclorim |
| B-794 | clomazone | fenclorim |
| B-795 | diflufenican | fenclorim |
| B-796 | fluorochloridone | fenclorim |
| B-797 | isoxaflutol | fenclorim |
| B-798 | mesotrione | fenclorim |
| B-799 | picolinafen | fenclorim |
| B-800 | sulcotrione | fenclorim |
| B-801 | tefuryltrione | fenclorim |
| B-802 | tembotrione | fenclorim |
| B-803 | topramezone | fenclorim |
| B-804 | H-7 | fenclorim |
| B-805 | atrazine | fenclorim |
| B-806 | diuron | fenclorim |
| B-807 | fluometuron | fenclorim |
| B-808 | hexazinone | fenclorim |
| B-809 | isoproturon | fenclorim |
| B-810 | metribuzin | fenclorim |
| B-811 | propanil | fenclorim |
| B-812 | terbuthylazine | fenclorim |
| B-813 | paraquat dichloride | fenclorim |
| B-814 | flumioxazin | fenclorim |
| B-815 | oxyfluorfen | fenclorim |
| B-816 | saflufenacil | fenclorim |
| B-817 | sulfentrazone | fenclorim |
| B-818 | H-1 | fenclorim |
| B-819 | H-2 | fenclorim |
| B-820 | glyphosate | fenclorim |
| B-821 | glyphosate-isopropylammonium | fenclorim |
| B-822 | glyphosate-trimesium (sulfosate) | fenclorim |
| B-823 | glufosinate | fenclorim |
| B-824 | glufosinate-ammonium | fenclorim |
| B-825 | pendimethalin | fenclorim |
| B-826 | trifluralin | fenclorim |
| B-827 | acetochlor | fenclorim |
| B-828 | cafenstrole | fenclorim |
| B-829 | dimethenamid-P | fenclorim |
| B-830 | fentrazamide | fenclorim |
| B-831 | flufenacet | fenclorim |
| B-832 | mefenacet | fenclorim |
| B-833 | metazachlor | fenclorim |
| B-834 | metolachlor-S | fenclorim |
| B-835 | pyroxasulfone | fenclorim |
| B-836 | isoxaben | fenclorim |
| B-837 | dymron | fenclorim |
| B-838 | indanofan | fenclorim |
| B-839 | oxaziclomefone | fenclorim |
| B-840 | triaziflam | fenclorim |
| B-841 | atrazine + H-1 | fenclorim |
| B-842 | atrazine + glyphosate | fenclorim |
| B-843 | atrazine + mesotrione | fenclorim |
| B-844 | atrazine + nicosulfuron | fenclorim |
| B-845 | atrazine + tembotrione | fenclorim |
| B-846 | atrazine + topramezone | fenclorim |
| B-847 | clomazone + glyphosate | fenclorim |
| B-848 | diflufenican + clodinafop-propargyl | fenclorim |
| B-849 | diflufenican + fenoxaprop-P-ethyl | fenclorim |
| B-850 | diflufenican + flupyrsulfuron-methyl-sodium | fenclorim |
| B-851 | diflufenican + glyphosate | fenclorim |
| B-852 | diflufenican + mesosulfuron-methyl | fenclorim |
| B-853 | diflufenican + pinoxaden | fenclorim |
| B-854 | diflufenican + pyroxsulam | fenclorim |
| B-855 | flumetsulam + glyphosate | fenclorim |
| B-856 | flumioxazin + glyphosate | fenclorim |
| B-857 | imazapic + glyphosate | fenclorim |
| B-858 | imazethapyr + glyphosate | fenclorim |
| B-859 | isoxaflutol + H-1 | fenclorim |
| B-860 | isoxaflutol + glyphosate | fenclorim |
| B-861 | metazachlor + H-1 | fenclorim |
| B-862 | metazachlor + glyphosate | fenclorim |
| B-863 | metazachlor + mesotrione | fenclorim |
| B-864 | metazachlor + nicosulfuron | fenclorim |
| B-865 | metazachlor + terbuthylazine | fenclorim |
| B-866 | metazachlor + topramezone | fenclorim |
| B-867 | metribuzin + glyphosate | fenclorim |
| B-868 | pendimethalin + H-1 | fenclorim |
| B-869 | pendimethalin + clodinafop-propargyl | fenclorim |
| B-870 | pendimethalin + fenoxaprop-P-ethyl | fenclorim |
| B-871 | pendimethalin + flupyrsulfuron-methyl-sodium | fenclorim |
| B-872 | pendimethalin + glyphosate | fenclorim |
| B-873 | pendimethalin + mesosulfuron-methyl | fenclorim |
| B-874 | pendimethalin + mesotrione | fenclorim |
| B-875 | pendimethalin + nicosulfuron | fenclorim |
| B-876 | pendimethalin + pinoxaden | fenclorim |
| B-877 | pendimethalin + pyroxsulam | fenclorim |
| B-878 | pendimethalin + tembotrione | fenclorim |
| B-879 | pendimethalin + topramezone | fenclorim |
| B-880 | pyroxasulfone + tembotrione | fenclorim |
| B-881 | pyroxasulfone + topramezone | fenclorim |
| B-882 | sulfentrazone + glyphosate | fenclorim |
| B-883 | terbuthylazine + H-1 | fenclorim |
| B-884 | terbuthylazine + foramsulfuron | fenclorim |
| B-885 | terbuthylazine + glyphosate | fenclorim |
| B-886 | terbuthylazine + mesotrione | fenclorim |
| B-887 | terbuthylazine + nicosulfuron | fenclorim |
| B-888 | terbuthylazine + tembotrione | fenclorim |
| B-889 | terbuthylazine + topramezone | fenclorim |
| B-890 | trifluralin + glyphosate | fenclorim |
| B-891 | clodinafop-propargyl | isoxadifen |
| B-892 | cycloxydim | isoxadifen |
| B-893 | cyhalofop-butyl | isoxadifen |
| B-894 | fenoxaprop-P-ethyl | isoxadifen |
| B-895 | pinoxaden | isoxadifen |
| B-896 | profoxydim | isoxadifen |
| B-897 | tepraloxydim | isoxadifen |
| B-898 | tralkoxydim | isoxadifen |
| B-899 | esprocarb | isoxadifen |
| B-900 | prosulfocarb | isoxadifen |
| B-901 | thiobencarb | isoxadifen |
| B-902 | triallate | isoxadifen |
| B-903 | bensulfuron-methyl | isoxadifen |
| B-904 | bispyribac-sodium | isoxadifen |
| B-905 | cyclosulfamuron | isoxadifen |
| B-906 | flumetsulam | isoxadifen |
| B-907 | flupyrsulfuron-methyl-sodium | isoxadifen |
| B-908 | foramsulfuron | isoxadifen |
| B-909 | imazamox | isoxadifen |
| B-910 | imazapic | isoxadifen |
| B-911 | imazapyr | isoxadifen |
| B-912 | imazaquin | isoxadifen |
| B-913 | imazethapyr | isoxadifen |
| B-914 | imazosulfuron | isoxadifen |
| B-915 | iodosulfuron-methyl-sodium | isoxadifen |
| B-916 | mesosulfuron | isoxadifen |
| B-917 | nicosulfuron | isoxadifen |
| B-918 | penoxsulam | isoxadifen |
| B-919 | propoxycarbazone-sodium | isoxadifen |
| B-920 | pyrazosulfuron-ethyl | isoxadifen |
| B-921 | pyroxsulam | isoxadifen |
| B-922 | rimsulfuron | isoxadifen |
| B-923 | sulfosulfuron | isoxadifen |
| B-924 | thiencarbazone-methyl | isoxadifen |
| B-925 | tritosulfuron | isoxadifen |
| B-926 | 2,4-D and its salts and esters | isoxadifen |
| B-927 | aminopyralid and its salts and esters | isoxadifen |
| B-928 | clopyralid and its salts and esters | isoxadifen |
| B-929 | dicamba and its salts and esters | isoxadifen |
| B-930 | fluroxypyr-meptyl | isoxadifen |
| B-931 | quinclorac | isoxadifen |
| B-932 | quinmerac | isoxadifen |
| B-933 | H-9 | isoxadifen |
| B-934 | diflufenzopyr | isoxadifen |
| B-935 | diflufenzopyr-sodium | isoxadifen |
| B-936 | clomazone | isoxadifen |
| B-937 | diflufenican | isoxadifen |
| B-938 | fluorochloridone | isoxadifen |
| B-939 | isoxaflutol | isoxadifen |
| B-940 | mesotrione | isoxadifen |
| B-941 | picolinafen | isoxadifen |
| B-942 | sulcotrione | isoxadifen |
| B-943 | tefuryltrione | isoxadifen |
| B-944 | tembotrione | isoxadifen |
| B-945 | topramezone | isoxadifen |
| B-946 | H-7 | isoxadifen |
| B-947 | atrazine | isoxadifen |
| B-948 | diuron | isoxadifen |
| B-949 | fluometuron | isoxadifen |
| B-950 | hexazinone | isoxadifen |
| B-951 | isoproturon | isoxadifen |
| B-952 | metribuzin | isoxadifen |
| B-953 | propanil | isoxadifen |
| B-954 | terbuthylazine | isoxadifen |
| B-955 | paraquat dichloride | isoxadifen |
| B-956 | flumioxazin | isoxadifen |
| B-957 | oxyfluorfen | isoxadifen |
| B-958 | saflufenacil | isoxadifen |
| B-959 | sulfentrazone | isoxadifen |
| B-960 | H-1 | isoxadifen |
| B-961 | H-2 | isoxadifen |
| B-962 | glyphosate | isoxadifen |
| B-963 | glyphosate-isopropylammonium | isoxadifen |
| B-964 | glyphosate-trimesium (sulfosate) | isoxadifen |
| B-965 | glufosinate | isoxadifen |
| B-966 | glufosinate-ammonium | isoxadifen |
| B-967 | pendimethalin | isoxadifen |
| B-968 | trifluralin | isoxadifen |
| B-969 | acetochlor | isoxadifen |
| B-970 | cafenstrole | isoxadifen |
| B-971 | dimethenamid-P | isoxadifen |
| B-972 | fentrazamide | isoxadifen |
| B-973 | flufenacet | isoxadifen |
| B-974 | mefenacet | isoxadifen |
| B-975 | metazachlor | isoxadifen |
| B-976 | metolachlor-S | isoxadifen |
| B-977 | pyroxasulfone | isoxadifen |
| B-978 | isoxaben | isoxadifen |
| B-979 | dymron | isoxadifen |
| B-980 | indanofan | isoxadifen |
| B-981 | oxaziclomefone | isoxadifen |
| B-982 | triaziflam | isoxadifen |
| B-983 | atrazine + H-1 | isoxadifen |
| B-984 | atrazine + glyphosate | isoxadifen |
| B-985 | atrazine + mesotrione | isoxadifen |
| B-986 | atrazine + nicosulfuron | isoxadifen |
| B-987 | atrazine + tembotrione | isoxadifen |
| B-988 | atrazine + topramezone | isoxadifen |
| B-989 | clomazone + glyphosate | isoxadifen |
| B-990 | diflufenican + clodinafop-propargyl | isoxadifen |
| B-991 | diflufenican + fenoxaprop-P-ethyl | isoxadifen |
| B-992 | diflufenican + flupyrsulfuron-methyl-sodium | isoxadifen |
| B-993 | diflufenican + glyphosate | isoxadifen |
| B-994 | diflufenican + mesosulfuron-methyl | isoxadifen |
| B-995 | diflufenican + pinoxaden | isoxadifen |
| B-996 | diflufenican + pyroxsulam | isoxadifen |
| B-997 | flumetsulam + glyphosate | isoxadifen |
| B-998 | flumioxazin + glyphosate | isoxadifen |
| B-999 | imazapic + glyphosate | isoxadifen |
| B-1000 | imazethapyr + glyphosate | isoxadifen |
| B-1001 | isoxaflutol + H-1 | isoxadifen |
| B-1002 | isoxaflutol + glyphosate | isoxadifen |
| B-1003 | metazachlor + H-1 | isoxadifen |
| B-1004 | metazachlor + glyphosate | isoxadifen |
| B-1005 | metazachlor + mesotrione | isoxadifen |
| B-1006 | metazachlor + nicosulfuron | isoxadifen |
| B-1007 | metazachlor + terbuthylazine | isoxadifen |
| B-1008 | metazachlor + topramezone | isoxadifen |
| B-1009 | metribuzin + glyphosate | isoxadifen |
| B-1010 | pendimethalin + H-1 | isoxadifen |
| B-1011 | pendimethalin + clodinafop-propargyl | isoxadifen |
| B-1012 | pendimethalin + fenoxaprop-P-ethyl | isoxadifen |
| B-1013 | pendimethalin + flupyrsulfuron-methyl-sodium | isoxadifen |
| B-1014 | pendimethalin + glyphosate | isoxadifen |
| B-1015 | pendimethalin + mesosulfuron-methyl | isoxadifen |
| B-1016 | pendimethalin + mesotrione | isoxadifen |
| B-1017 | pendimethalin + nicosulfuron | isoxadifen |
| B-1018 | pendimethalin + pinoxaden | isoxadifen |
| B-1019 | pendimethalin + pyroxsulam | isoxadifen |
| B-1020 | pendimethalin + tembotrione | isoxadifen |
| B-1021 | pendimethalin + topramezone | isoxadifen |
| B-1022 | pyroxasulfone + tembotrione | isoxadifen |
| B-1023 | pyroxasulfone + topramezone | isoxadifen |
| B-1024 | sulfentrazone + glyphosate | isoxadifen |
| B-1025 | terbuthylazine + H-1 | isoxadifen |
| B-1026 | terbuthylazine + foramsulfuron | isoxadifen |
| B-1027 | terbuthylazine + glyphosate | isoxadifen |
| B-1028 | terbuthylazine + mesotrione | isoxadifen |
| B-1029 | terbuthylazine + nicosulfuron | isoxadifen |
| B-1030 | terbuthylazine + tembotrione | isoxadifen |
| B-1031 | terbuthylazine + topramezone | isoxadifen |
| B-1032 | trifluralin + glyphosate | isoxadifen |
| B-1033 | clodinafop-propargyl | mefenpyr |
| B-1034 | cycloxydim | mefenpyr |
| B-1035 | cyhalofop-butyl | mefenpyr |
| B-1036 | fenoxaprop-P-ethyl | mefenpyr |
| B-1037 | pinoxaden | mefenpyr |
| B-1038 | profoxydim | mefenpyr |
| B-1039 | tepraloxydim | mefenpyr |
| B-1040 | tralkoxydim | mefenpyr |
| B-1041 | esprocarb | mefenpyr |
| B-1042 | prosulfocarb | mefenpyr |
| B-1043 | thiobencarb | mefenpyr |
| B-1044 | triallate | mefenpyr |
| B-1045 | bensulfuron-methyl | mefenpyr |
| B-1046 | bispyribac-sodium | mefenpyr |
| B-1047 | cyclosulfamuron | mefenpyr |
| B-1048 | flumetsulam | mefenpyr |
| B-1049 | flupyrsulfuron-methyl-sodium | mefenpyr |
| B-1050 | foramsulfuron | mefenpyr |
| B-1051 | imazamox | mefenpyr |
| B-1052 | imazapic | mefenpyr |
| B-1053 | imazapyr | mefenpyr |
| B-1054 | imazaquin | mefenpyr |
| B-1055 | imazethapyr | mefenpyr |
| B-1056 | imazosulfuron | mefenpyr |
| B-1057 | iodosulfuron-methyl-sodium | mefenpyr |
| B-1058 | mesosulfuron | mefenpyr |
| B-1059 | nicosulfuron | mefenpyr |
| B-1060 | penoxsulam | mefenpyr |
| B-1061 | propoxycarbazone-sodium | mefenpyr |
| B-1062 | pyrazosulfuron-ethyl | mefenpyr |
| B-1063 | pyroxsulam | mefenpyr |
| B-1064 | rimsulfuron | mefenpyr |
| B-1065 | sulfosulfuron | mefenpyr |
| B-1066 | thiencarbazone-methyl | mefenpyr |
| B-1067 | tritosulfuron | mefenpyr |
| B-1068 | 2,4-D and its salts and esters | mefenpyr |
| B-1069 | aminopyralid and its salts and esters | mefenpyr |
| B-1070 | clopyralid and its salts and esters | mefenpyr |
| B-1071 | dicamba and its salts and esters | mefenpyr |
| B-1072 | fluroxypyr-meptyl | mefenpyr |
| B-1073 | quinclorac | mefenpyr |
| B-1074 | quinmerac | mefenpyr |
| B-1075 | H-9 | mefenpyr |
| B-1076 | diflufenzopyr | mefenpyr |
| B-1077 | diflufenzopyr-sodium | mefenpyr |
| B-1078 | clomazone | mefenpyr |
| B-1079 | diflufenican | mefenpyr |
| B-1080 | fluorochloridone | mefenpyr |
| B-1081 | isoxaflutol | mefenpyr |
| B-1082 | mesotrione | mefenpyr |
| B-1083 | picolinafen | mefenpyr |
| B-1084 | sulcotrione | mefenpyr |
| B-1085 | tefuryltrione | mefenpyr |
| B-1086 | tembotrione | mefenpyr |
| B-1087 | topramezone | mefenpyr |
| B-1088 | H-7 | mefenpyr |
| B-1089 | atrazine | mefenpyr |
| B-1090 | diuron | mefenpyr |
| B-1091 | fluometuron | mefenpyr |
| B-1092 | hexazinone | mefenpyr |
| B-1093 | isoproturon | mefenpyr |
| B-1094 | metribuzin | mefenpyr |
| B-1095 | propanil | mefenpyr |
| B-1096 | terbuthylazine | mefenpyr |
| B-1097 | paraquat dichloride | mefenpyr |
| B-1098 | flumioxazin | mefenpyr |
| B-1099 | oxyfluorfen | mefenpyr |
| B-1100 | saflufenacil | mefenpyr |
| B-1101 | sulfentrazone | mefenpyr |
| B-1102 | H-1 | mefenpyr |
| B-1103 | H-2 | mefenpyr |
| B-1104 | glyphosate | mefenpyr |
| B-1105 | glyphosate-isopropylammonium | mefenpyr |
| B-1106 | glyphosate-trimesium (sulfosate) | mefenpyr |
| B-1107 | glufosinate | mefenpyr |
| B-1108 | glufosinate-ammonium | mefenpyr |
| B-1109 | pendimethalin | mefenpyr |
| B-1110 | trifluralin | mefenpyr |
| B-1111 | acetochlor | mefenpyr |
| B-1112 | cafenstrole | mefenpyr |
| B-1113 | dimethenamid-P | mefenpyr |
| B-1114 | fentrazamide | mefenpyr |
| B-1115 | flufenacet | mefenpyr |
| B-1116 | mefenacet | mefenpyr |
| B-1117 | metazachlor | mefenpyr |
| B-1118 | metolachlor-S | mefenpyr |
| B-1119 | pyroxasulfone | mefenpyr |
| B-1120 | isoxaben | mefenpyr |
| B-1121 | dymron | mefenpyr |
| B-1122 | indanofan | mefenpyr |
| B-1123 | oxaziclomefone | mefenpyr |
| B-1124 | triaziflam | mefenpyr |
| B-1125 | atrazine + H-1 | mefenpyr |
| B-1126 | atrazine + glyphosate | mefenpyr |
| B-1127 | atrazine + mesotrione | mefenpyr |
| B-1128 | atrazine + nicosulfuron | mefenpyr |
| B-1129 | atrazine + tembotrione | mefenpyr |
| B-1130 | atrazine + topramezone | mefenpyr |
| B-1131 | clomazone + glyphosate | mefenpyr |
| B-1132 | diflufenican + clodinafop-propargyl | mefenpyr |
| B-1133 | diflufenican + fenoxaprop-P-ethyl | mefenpyr |
| B-1134 | diflufenican + flupyrsulfuron-methyl-sodium | mefenpyr |
| B-1135 | diflufenican + glyphosate | mefenpyr |
| B-1136 | diflufenican + mesosulfuron-methyl | mefenpyr |
| B-1137 | diflufenican + pinoxaden | mefenpyr |
| B-1138 | diflufenican + pyroxsulam | mefenpyr |
| B-1139 | flumetsulam + glyphosate | mefenpyr |
| B-1140 | flumioxazin + glyphosate | mefenpyr |
| B-1141 | imazapic + glyphosate | mefenpyr |
| B-1142 | imazethapyr + glyphosate | mefenpyr |
| B-1143 | isoxaflutol + H-1 | mefenpyr |
| B-1144 | isoxaflutol + glyphosate | mefenpyr |
| B-1145 | metazachlor + H-1 | mefenpyr |
| B-1146 | metazachlor + glyphosate | mefenpyr |
| B-1147 | metazachlor + mesotrione | mefenpyr |
| B-1148 | metazachlor + nicosulfuron | mefenpyr |
| B-1149 | metazachlor + terbuthylazine | mefenpyr |
| B-1150 | metazachlor + topramezone | mefenpyr |
| B-1151 | metribuzin + glyphosate | mefenpyr |
| B-1152 | pendimethalin + H-1 | mefenpyr |
| B-1153 | pendimethalin + clodinafop-propargyl | mefenpyr |
| B-1154 | pendimethalin + fenoxaprop-P-ethyl | mefenpyr |
| B-1155 | pendimethalin + flupyrsulfuron-methyl-sodium | mefenpyr |
| B-1156 | pendimethalin + glyphosate | mefenpyr |
| B-1157 | pendimethalin + mesosulfuron-methyl | mefenpyr |
| B-1158 | pendimethalin + mesotrione | mefenpyr |
| B-1159 | pendimethalin + nicosulfuron | mefenpyr |
| B-1160 | pendimethalin + pinoxaden | mefenpyr |
| B-1161 | pendimethalin + pyroxsulam | mefenpyr |
| B-1162 | pendimethalin + tembotrione | mefenpyr |
| B-1163 | pendimethalin + topramezone | mefenpyr |
| B-1164 | pyroxasulfone + tembotrione | mefenpyr |
| B-1165 | pyroxasulfone + topramezone | mefenpyr |
| B-1166 | sulfentrazone + glyphosate | mefenpyr |
| B-1167 | terbuthylazine + H-1 | mefenpyr |
| B-1168 | terbuthylazine + foramsulfuron | mefenpyr |
| B-1169 | terbuthylazine + glyphosate | mefenpyr |
| B-1170 | terbuthylazine + mesotrione | mefenpyr |
| B-1171 | terbuthylazine + nicosulfuron | mefenpyr |
| B-1172 | terbuthylazine + tembotrione | mefenpyr |
| B-1173 | terbuthylazine + topramezone | mefenpyr |
| B-1174 | trifluralin + glyphosate | mefenpyr |
| B-1175 | clodinafop-propargyl | H-12 |
| B-1176 | cycloxydim | H-12 |
| B-1177 | cyhalofop-butyl | H-12 |
| B-1178 | fenoxaprop-P-ethyl | H-12 |
| B-1179 | pinoxaden | H-12 |
| B-1180 | profoxydim | H-12 |
| B-1181 | tepraloxydim | H-12 |
| B-1182 | tralkoxydim | H-12 |
| B-1183 | esprocarb | H-12 |
| B-1184 | prosulfocarb | H-12 |
| B-1185 | thiobencarb | H-12 |
| B-1186 | triallate | H-12 |
| B-1187 | bensulfuron-methyl | H-12 |
| B-1188 | bispyribac-sodium | H-12 |
| B-1189 | cyclosulfamuron | H-12 |
| B-1190 | flumetsulam | H-12 |
| B-1191 | flupyrsulfuron-methyl-sodium | H-12 |
| B-1192 | foramsulfuron | H-12 |
| B-1193 | imazamox | H-12 |
| B-1194 | imazapic | H-12 |
| B-1195 | imazapyr | H-12 |
| B-1196 | imazaquin | H-12 |
| B-1197 | imazethapyr | H-12 |
| B-1198 | imazosulfuron | H-12 |
| B-1199 | iodosulfuron-methyl-sodium | H-12 |
| B-1200 | mesosulfuron | H-12 |
| B-1201 | nicosulfuron | H-12 |
| B-1202 | penoxsulam | H-12 |
| B-1203 | propoxycarbazone-sodium | H-12 |
| B-1204 | pyrazosulfuron-ethyl | H-12 |
| B-1205 | pyroxsulam | H-12 |
| B-1206 | rimsulfuron | H-12 |
| B-1207 | sulfosulfuron | H-12 |
| B-1208 | thiencarbazone-methyl | H-12 |
| B-1209 | tritosulfuron | H-12 |
| B-1210 | 2,4-D and its salts and esters | H-12 |
| B-1211 | aminopyralid and its salts and esters | H-12 |
| B-1212 | clopyralid and its salts and esters | H-12 |
| B-1213 | dicamba and its salts and esters | H-12 |
| B-1214 | fluroxypyr-meptyl | H-12 |
| B-1215 | quinclorac | H-12 |
| B-1216 | quinmerac | H-12 |
| B-1217 | B-9 | H-12 |
| B-1218 | diflufenzopyr | H-12 |
| B-1219 | diflufenzopyr-sodium | H-12 |
| B-1220 | clomazone | H-12 |
| B-1221 | diflufenican | H-12 |
| B-1222 | fluorochloridone | H-12 |
| B-1223 | isoxaflutol | H-12 |
| B-1224 | mesotrione | H-12 |
| B-1225 | picolinafen | H-12 |
| B-1226 | sulcotrione | H-12 |
| B-1227 | tefuryltrione | H-12 |
| B-1228 | tembotrione | H-12 |
| B-1229 | topramezone | H-12 |
| B-1230 | H-7 | H-12 |
| B-1231 | atrazine | H-12 |
| B-1232 | diuron | H-12 |
| B-1233 | fluometuron | H-12 |
| B-1234 | hexazinone | H-12 |
| B-1235 | isoproturon | H-12 |
| B-1236 | metribuzin | H-12 |
| B-1237 | propanil | H-12 |
| B-1238 | terbuthylazine | H-12 |
| B-1239 | paraquat dichloride | H-12 |
| B-1240 | flumioxazin | H-12 |
| B-1241 | oxyfluorfen | H-12 |
| B-1242 | saflufenacil | H-12 |
| B-1243 | sulfentrazone | H-12 |
| B-1244 | H-1 | H-12 |
| B-1245 | H-2 | H-12 |
| B-1246 | glyphosate | H-12 |
| B-1247 | glyphosate-isopropylammonium | H-12 |
| B-1248 | glyphosate-trimesium (sulfosate) | H-12 |
| B-1249 | glufosinate | H-12 |
| B-1250 | glufosinate-ammonium | H-12 |
| B-1251 | pendimethalin | H-12 |
| B-1252 | trifluralin | H-12 |
| B-1253 | acetochlor | H-12 |
| B-1254 | cafenstrole | H-12 |
| B-1255 | dimethenamid-P | H-12 |
| B-1256 | fentrazamide | H-12 |
| B-1257 | flufenacet | H-12 |
| B-1258 | mefenacet | H-12 |
| B-1259 | metazachlor | H-12 |
| B-1260 | metolachlor-S | H-12 |
| B-1261 | pyroxasulfone | H-12 |
| B-1262 | isoxaben | H-12 |
| B-1263 | dymron | H-12 |
| B-1264 | indanofan | H-12 |
| B-1265 | oxaziclomefone | H-12 |
| B-1266 | triaziflam | H-12 |
| B-1267 | atrazine + H-1 | H-12 |
| B-1268 | atrazine + glyphosate | H-12 |
| B-1269 | atrazine + mesotrione | H-12 |
| B-1270 | atrazine + nicosulfuron | H-12 |
| B-1271 | atrazine + tembotrione | H-12 |
| B-1272 | atrazine + topramezone | H-12 |
| B-1273 | clomazone + glyphosate | H-12 |
| B-1274 | diflufenican + clodinafop-propargyl | H-12 |
| B-1275 | diflufenican + fenoxaprop-P-ethyl | H-12 |
| B-1276 | diflufenican + flupyrsulfuron-methyl-sodium | H-12 |
| B-1277 | diflufenican + glyphosate | H-12 |
| B-1278 | diflufenican + mesosulfuron-methyl | H-12 |
| B-1279 | diflufenican + pinoxaden | H-12 |
| B-1280 | diflufenican + pyroxsulam | H-12 |
| B-1281 | flumetsulam + glyphosate | H-12 |
| B-1282 | flumioxazin + glyphosate | H-12 |
| B-1283 | imazapic + glyphosate | H-12 |
| B-1284 | imazethapyr + glyphosate | H-12 |
| B-1285 | isoxaflutol + H-1 | H-12 |
| B-1286 | isoxaflutol + glyphosate | H-12 |
| B-1287 | metazachlor + H-1 | H-12 |
| B-1288 | metazachlor + glyphosate | H-12 |
| B-1289 | metazachlor + mesotrione | H-12 |
| B-1290 | metazachlor + nicosulfuron | H-12 |
| B-1291 | metazachlor + terbuthylazine | H-12 |
| B-1292 | metazachlor + topramezone | H-12 |
| B-1293 | metribuzin + glyphosate | H-12 |
| B-1294 | pendimethalin + H-1 | H-12 |
| B-1295 | pendimethalin + clodinafop-propargyl | H-12 |
| B-1296 | pendimethalin + fenoxaprop-P-ethyl | H-12 |
| B-1297 | pendimethalin + flupyrsulfuron-methyl-sodium | H-12 |
| B-1298 | pendimethalin + glyphosate | H-12 |
| B-1299 | pendimethalin + mesosulfuron-methyl | H-12 |
| B-1300 | pendimethalin + mesotrione | H-12 |
| B-1301 | pendimethalin + nicosulfuron | H-12 |
| B-1302 | pendimethalin + pinoxaden | H-12 |
| B-1303 | pendimethalin + pyroxsulam | H-12 |
| B-1304 | pendimethalin + tembotrione | H-12 |
| B-1305 | pendimethalin + topramezone | H-12 |
| B-1306 | pyroxasulfone + tembotrione | H-12 |
| B-1307 | pyroxasulfone + topramezone | H-12 |
| B-1308 | sulfentrazone + glyphosate | H-12 |
| B-1309 | terbuthylazine + H-1 | H-12 |
| B-1310 | terbuthylazine + foramsulfuron | H-12 |
| B-1311 | terbuthylazine + glyphosate | H-12 |
| B-1312 | terbuthylazine + mesotrione | H-12 |
| B-1313 | terbuthylazine + nicosulfuron | H-12 |
| B-1314 | terbuthylazine + tembotrione | H-12 |
| B-1315 | terbuthylazine + topramezone | H-12 |
| B-1316 | trifluralin + glyphosate | H-12 |
| B-1317 | 2-1 | -- |
| B-1318 | 2-2 | -- |
| B-1319 | 2-3 | -- |
| B-1320 | 2-4 | -- |
| B-1321 | 2-5 | -- |
| B-1322 | 2-6 | -- |
| B-1323 | 2-7 | -- |
| B-1324 | 2-8 | -- |
| B-1325 | 2-9 | -- |
| B-1326 | 2-1 | benoxacor |
| B-1327 | 2-2 | benoxacor |
| B-1328 | 2-3 | benoxacor |
| B-1329 | 2-4 | benoxacor |
| B-1330 | 2-5 | benoxacor |
| B-1331 | 2-6 | benoxacor |
| B-1332 | 2-7 | benoxacor |
| B-1333 | 2-8 | benoxacor |
| B-1334 | 2-9 | benoxacor |
| B-1335 | 2-1 | cloquintocet |
| B-1336 | 2-2 | cloquintocet |
| B-1337 | 2-3 | cloquintocet |
| B-1338 | 2-4 | cloquintocet |
| B-1339 | 2-5 | cloquintocet |
| B-1340 | 2-6 | cloquintocet |
| B-1341 | 2-7 | cloquintocet |
| B-1342 | 2-8 | cloquintocet |
| B-1343 | 2-9 | cloquintocet |
| B-1344 | 2-1 | cyprosulfamide |
| B-1345 | 2-2 | cyprosulfamide |
| B-1346 | 2-3 | cyprosulfamide |
| B-1347 | 2-4 | cyprosulfamide |
| B-1348 | 2-5 | cyprosulfamide |
| B-1349 | 2-6 | cyprosulfamide |
| B-1350 | 2-7 | cyprosulfamide |
| B-1351 | 2-8 | cyprosulfamide |
| B-1352 | 2-9 | cyprosulfamide |
| B-1353 | 2-1 | dichlormid |
| B-1354 | 2-2 | dichlormid |
| B-1355 | 2-3 | dichlormid |
| B-1356 | 2-4 | dichlormid |
| B-1357 | 2-5 | dichlormid |
| B-1358 | 2-6 | dichlormid |
| B-1359 | 2-7 | dichlormid |
| B-1360 | 2-8 | dichlormid |
| B-1361 | 2-9 | dichlormid |
| B-1362 | 2-1 | fenchlorazole |
| B-1363 | 2-2 | fenchlorazole |
| B-1364 | 2-3 | fenchlorazole |
| B-1365 | 2-4 | fenchlorazole |
| B-1366 | 2-5 | fenchlorazole |
| B-1367 | 2-6 | fenchlorazole |
| B-1368 | 2-7 | fenchlorazole |
| B-1369 | 2-8 | fenchlorazole |
| B-1370 | 2-9 | fenchlorazole |
| B-1371 | 2-1 | isoxadifen |
| B-1372 | 2-2 | isoxadifen |
| B-1373 | 2-3 | isoxadifen |
| B-1374 | 2-4 | isoxadifen |
| B-1375 | 2-5 | isoxadifen |
| B-1376 | 2-6 | isoxadifen |
| B-1377 | 2-7 | isoxadifen |
| B-1378 | 2-8 | isoxadifen |
| B-1379 | 2-9 | isoxadifen |
| B-1380 | 2-1 | mefenpyr |
| B-1381 | 2-2 | mefenpyr |
| B-1382 | 2-3 | mefenpyr |
| B-1383 | 2-4 | mefenpyr |
| B-1384 | 2-5 | mefenpyr |
| B-1385 | 2-6 | mefenpyr |
| B-1386 | 2-7 | mefenpyr |
| B-1387 | 2-8 | mefenpyr |
| B-1388 | 2-9 | mefenpyr |
| B-1389 | 2-1 | H-11 |
| B-1390 | 2-2 | H-11 |
| B-1391 | 2-3 | H-11 |
| B-1392 | 2-4 | H-11 |
| B-1393 | 2-5 | H-11 |
| B-1394 | 2-6 | H-11 |
| B-1395 | 2-7 | H-11 |
| B-1396 | 2-8 | H-11 |
| B-1397 | 2-9 | H-11 |
| B-1398 | 2-1 | H-12 |
| B-1399 | 2-2 | H-12 |
| B-1400 | 2-3 | H-12 |
| B-1401 | 2-4 | H-12 |
| B-1402 | 2-5 | H-12 |
| B-1403 | 2-6 | H-12 |
| B-1404 | 2-7 | H-12 |
| B-1405 | 2-8 | H-12 |
| B-1406 | 2-9 | H-12 |

The compounds of formula I and the compositions according to the invention may also have a plant-strengthening action. Accordingly, they are suitable for mobilizing the defense system of the plants against attack by unwanted microorganisms, such as harmful fungi, but also viruses and bacteria. Plant-strengthening (resistance-inducing) substances are to be understood as meaning, in the present context, those substances which are capable of stimulating the defense system of treated plants in such a way that, when subsequently inoculated by unwanted microorganisms, the treated plants display a substantial degree of resistance to these microorganisms.

The compounds of formula I can be employed for protecting plants against attack by unwanted microorganisms within a certain period of time after the treatment. The period of time within which their protection is effected generally extends from 1 to 28 days, preferably from 1 to 14 days, after the treatment of the plants with the compounds of formula I, or, after treatment of the seed, for up to 9 months after sowing.

The compounds of formula I and the compositions according to the invention are also suitable for increasing the harvest yield.

Moreover, they have reduced toxicity and are tolerated well by the crop plants.

The following examples will further illustrate the invention:
With appropriate modification of the starting materials, the procedures given in the synthesis examples below were used to obtain further compounds I. The compounds obtained in this manner are listed in the tables that follow, together with physical data. The products shown below were characterized by determination of the melting point, NMR spectroscopy or the masses ([m/z]) determined by HPLC-MS spectrometry.
HPLC-MS = high performance liquid chromatography coupled with mass spectrometry;
HPLC column: RP-18 column (Chromolith Speed ROD from Merck KgaA, Germany), 50*4.6 mm; mobile phase: acetonitrile + 0.1 % trifluoroacetic acid (TFA)/water + 0.1% TFA, using a gradient from 5:95 to 100:0 over 5 minutes at 40 °C, flow rate 1.8 mL/min.
MS: quadrupole electrospray ionization, 80 V (positive mode).
HPLC column: Luna-C18(2) 5 µ m column (Phenomenex), 2.0*50 mm; mobile phase: acetonitrile + 0.0625% trifluoroacetic acid (TFA)/water + 0.0675% TFA, using a gradient from 10:90 to 80:20 over 4.0 minutes at 40 °C, flow rate 0.8 mL/min.
   MS: quadrupole electrospray ionization, 70 V (positive mode).
DCM: dichloromethane
DIPEA: diisopropylethylamine
DIC (*N,N'* -diisopropyl carbodiimide
DMAP: Dimethylaminopyridine
DMF: *N*,*N*-dimethylformamide
MS: Mass spectrometry
EtOAc: acetic acid ethyl ester
THF: tetrahydrofuran
TFA: trifluoroacetic acid

Example 1: Preparation of: 2,5-dichloro-3-(dicyclopropylcarbamoylamino)-N-(1-methyltetrazol-5-yl)benzamide

### Step 1: Methyl 2,5-dichloro-3-(dicyclopropylcarbamoylamino)-benzoate

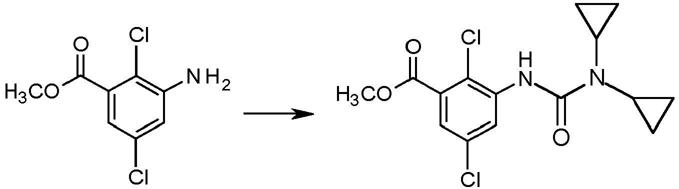

To a solution of triphosgene (640 mg, 2.19 mmol) in DCM (8 mL) a solution of commercially available methyl 3-amino-2,5-dichloro-benzoate (800 mg, 3.65 mol) in DCM (8 mL) was added. Then DIPEA (diisopropylethylamine, 844 mg, 6.57 mmol) was added to the mixture at 0 °C under N₂ atmosphere. The mixture was stirred at 0 °C for 3 h. N-cyclopropylcyclopropanamine hydrochloride (960 mg, 7.3 mmol) and triethylamine (1.45 g, 14.6 mmol) was added and the mixture was stirred for further 3 h at 15 °C. To the reaction mixture 20 mL H₂O was added. The layers were separated and andn the aqueous layer was washed with DCM. The combined organic layers were washed with brine and dried over Na₂SO₄. After concentration in vacuo the compound was purified by column chromatography to give the title compound (1.642 g, crude) as a light yellow solid.
¹H NMR (400 MHz, CDCl₃), δ 8.74 (d, 1H), 8.22 (s, 1H), 7.43 (d, 1H), 3.96 - 3.91 (m, 3H), 2.63 - 2.55 (m, 2H), 1.01 - 0.95 (m, 4H), 0.92 - 0.87 (m, 4H)

### Step 2: 2,5-dichloro-3-(dicyclopropylcarbamoylamino)-benzoic acid

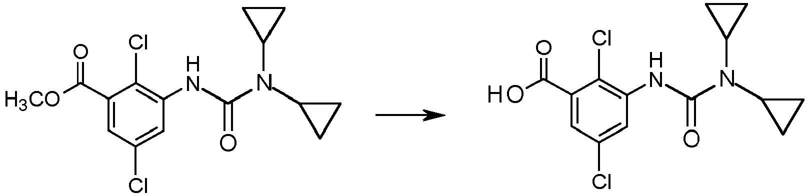

To a solution of methyl 2,5-dichloro-3-(dicyclopropylcarbamoylamino) benzoate (1 g, crude) in THF/H₂O (10 mL/2.5 mL) LiOH (420 mg, 17.5 mmol) was added at 0 °C under N2 atmosphere. It was stirred at 15 °C for 2 days. The reaction mixture was adjusted to pH 3 with aqueous 1 N HCl solution and the mixture was extracted with EtOAc. The combined organic layers were washed with brine, dried over Na₂SO₄ and concentrated in vacuo to give 2,5-dichloro-3-(dicyclopropylcarbamoylamino)benzoic acid as a crude solid which was used without further purification for the next step.
¹H NMR (400 MHz, CDCl₃), δ 8.50 - 8.36 (m, 1H), 8.25 (s, 1H), 7.41 (br d, 1H), 2.71 - 2.57 (m, 2H), 0.87 (br d, *J*=5.5 Hz, 4H), 0.84 - 0.76 (m, 4H)

### Step 3: pentafluorophenyl 2,5-dichloro-3-(dicyclopropylcarbamoylamino)-benzoate

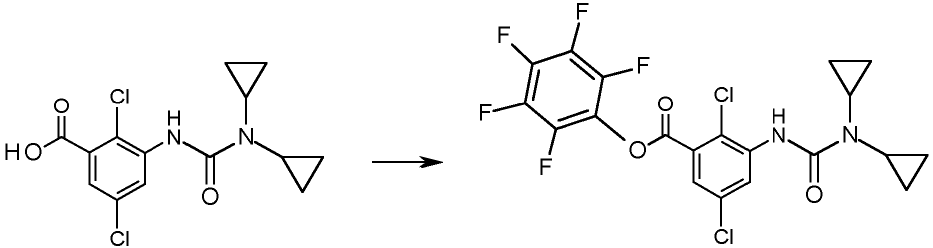

To a solution of 2,5-dichloro-3-(dicyclopropylcarbamoylamino) benzoic acid (1 g, 2.9 mmol), 2,3,4,5,6-pentafluorophenol (810 mg, 4.4 mmol) and DMAP (35 mg, 0.29 mmol) in DMF (10 mL) DIC (554 mg, 4.4 mmol) was added at 0 °C. The mixture was stirred at 10 °C for 10 h. The reaction mixture was poured into 20 mL lithium chloride solution (water, 4%), extracted with EtOAc and the organic layers were washed with brine, dried over Na₂SO₄, concentrated in vacuo and purified by column chromatography to give the title compound (1.1 g, crude) as a white solid.
¹H NMR (400 MHz, CDCl₃), δ 8.89 (d, 1H), 8.29 (s, 1H), 7.72 (d, 1H), 2.62 (m_{c}, 2H), 1.05 - 0.97 (m, 4H), 0.96 - 0.89 (m, 4H)

### Step 4: 2,5-dichloro-3-(dicyclopropylcarbamoylamino)-N-(1-methyltetrazol-5-yl)benzamide

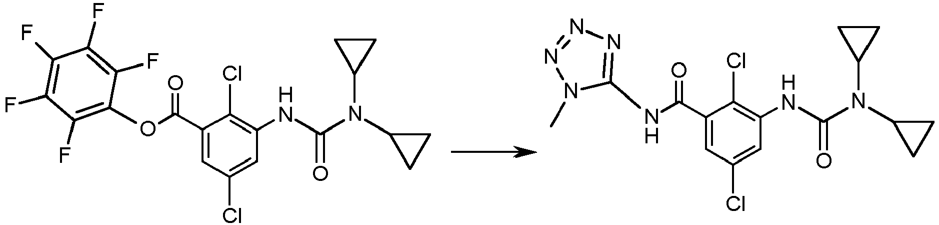

To a solution of 1-methyltetrazol-5-amine (481 mg, 4.86 mmol) in NMP (16 mL) NaH (60%, 162 mg, 4.05 mmol) was added at 0 °C and the mixture was stirred for 0.5 h. Then, 2,3,4,5,6-pentafluorophenyl 2,5-dichloro-3-(dicyclopropylcarbamoylamino) benzoate (800 mg, 1.62 mmol) in NMP (4 mL) was added, and the mixture was stirred at 15 °C for another 8 h. The reaction mixture was poured into lithium chloride solution (4%, 30 mL). The the mixture was adjusted to pH 6 -7 with 1 N HCl, extracted with EtOAc, the combined organic layers were washed with brine, dried over Na₂SO₄, concentrated in vacuo and purified by chromatography to give the title compound (220 mg) as a white solid.
¹H NMR (400 MHz, d6-DMSO), δ 8.34 (d, 1H), 8.22 (s, 1H), 7.42 (d, 1H), 3.91 (s, 3H), 2.66 - 2.60 (m, 2H), 0.92 - 0.85 (m, 4H), 0.81 (q, 4H).

### Example 2: Preparation of: 2,5-dichloro-3-[(2,2,2-trifluoroethyl)(methyl)carbamoylamino]-N-(1-methyltetrazol-5-yl)benzamide

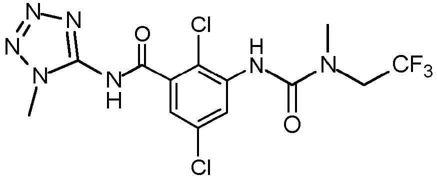

The title compound was prepared by analogy to the method described in Example 1.
HPLC-MS: m/z 425.8 (M+H)⁺

### Use examples

The herbicidal activity of the compounds of formula (I) was demonstrated by the following greenhouse experiments:
The culture containers used were plastic flowerpots containing loamy sand with approximately 3.0% of humus as the substrate. The seeds of the test plants were sown separately for each species.
For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this had been impaired by the active ingredients.
For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment. Depending on the species, the plants were kept at 10 - 25°C or 20 - 25°C, respectively.
The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A good herbicidal activity is given at values of at least 70 and a very good herbicidal activity is given at values of at least 85.

At an application rate of 250g/ha the following compounds were tested in post-emergence tests against:
ECHCG (Echinocloa crus-galli)
AMARE (Amaranthus retroflexus)
CHEAL (Chenopodium album)

| **Example** | ECHCG | AMARE | CHEAL |
|---|---|---|---|
| 1 | > 80 | > 80 | > 80 |
| 2 | > 80 | > 80 | > 80 |

## Claims

1. A compound of formula I, 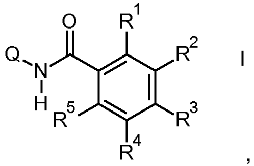 wherein
Q is Q¹ or Q² or Q³ or Q⁴, 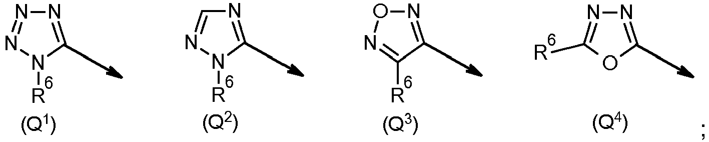
R¹ is selected from the group consisting of cyano, halogen, nitro, C₁-C₈-alkyl, C₁-C₈-haloalkyl, C₁-C₄-alkoxy-C₁-C₄-alkyl, C₁-C₄-haloalkoxy-C₁-C₄-alkyl, C₁-C₈-alkoxy, C₁-C₄-alkoxy-C₁-C₄-alkoxy-Z¹, C₁-C₆-haloalkoxy, R^{1b}-S(O)ₖ-Z¹;
R² is R^{2c}R^{2d}NC(O)NR²ⁿ-Z²;
R³ is selected from the group consisting of hydrogen, cyano, thiocyanato, halogen, hydroxy-Z², C₁-C₆-alkyl, C₂-C₈-alkenyl, C₂-C₈-alkynyl, C₃-C₁₀-cycloalkyl-Z², C₃-C₆-cycloalkenyl-Z², C₃-C₁₀-cycloalkoxy-Z², C₃-C₁₀-cycloalkyl-C₁-C₂-alkoxy, where the cyclic groups of the four aforementioned radicals are unsubstituted or partially or completely halogenated, C₁-C₄-cyanoalkyl, C₁-C₈-haloalkyl, C₂-C₈-haloalkenyl, C₃-C₈-haloalkynyl, C₁-C₈-alkoxy-Z², C₁-C₈-haloalkoxy-Z², C₁-C₄-alkoxy-C₁-C₄-alkoxy-Z², C₁-C₄-haloalkoxy-C₁-C₄-alkoxy-Z², C₂-C₃-alkenyloxy-Z², C₂-C₈-alkynyloxy-Z², C₂-C₈-haloalkenyloxy-Z², C₃-C₈-haloalkynyloxy-Z², R^{2b}-S(O)ₖ-Z², R^{2c}-C(O)-Z², R^{2d}O-C(O)-Z², R^{2d}O-N=CH-Z², R^{ze}R^{2f}N-C(O)-Z², R²⁹R^{2h}N-Z², R²²C(O)O-Z², R²⁵OC(O)O-Z², (R²²)₂NC(O)O-Z², R²⁵S(O)₂O-Z², R²²OS(O)₂-Z², (R²²)₂NS(O)₂-Z², R²⁵OC(O)N(R²²)-Z², (R²²)₂NC(O)N(R²²)-Z², (R²²)₂NS(O)₂N(R²²)-Z², (OH)₂P(O)-Z², (C₁-C₄-alkoxy)₂P (O)-Z², phenyl-Z^{2a}, heterocyclyl-Z^{2a}, where heterocyclyl is a 3-, 4-, 5- or 6-membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where cyclic groups in phenyl-Z^{2a} and heterocyclyl-Z^{2a} are unsubstituted or substituted by 1, 2, 3 or 4 groups R²¹, which are identical or different;
R⁴ is selected from the group consisting of halogen, C₁-C₈-alkyl, cyano-Z¹, nitro, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₂-C₈-alkenyl, C₂-C₈-alkynyl, C₁-C₈-haloalkyl, C₁-C₃-alkylamino, C₁-C₃-dialkylamino, C₁-C₃-alkylamino-S(O)ₖ, C₁-C₃-alkylcarbonyl, C₁-C₈-alkoxy, C₁-C₄-alkoxy-C₁-C₄-alkyl, C₁-C₄-alkoxy-C₁-C₄-alkoxy-Z¹, C₁-C₄-alkylthio-C₁-C₄-alkyl, C₁-C₄-alkylthio-C₁-C₄-alkylthio-Z¹, C₂-C₆-alkenyloxy, C₂-C₆-alkynyloxy, C₁-C₆-haloalkoxy, C₁-C₄-haloalkoxy-C₁-C₄-alkyl, C₁-C₄-haloalkoxy-C₁-C₄-alkoxy-Z¹, R^{1b}-S(O)ₖ-Z¹, phenoxy-Z¹ and heterocyclyloxy-Z¹, where heterocyclyloxy is an oxygen bound 5- or 6- membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where the cyclic groups in phenoxy and heterocyclyloxy are unsubstituted or substituted by 1, 2, 3 or 4 groups R¹¹, which are identical or different;
R⁵ is hydrogen;
R⁶ is selected from the group consisting of cyano, C₁-C₆-alkyl, C₃-C₇-cycloalkyl, C₃-C₇-cycloalkyl-C₁-C₄-alkyl, where the C₃-C₇-cycloalkyl groups of the two aforementioned radicals are unsubstituted or partially or completely halogenated, C₁-C₆-haloalkyl, C₂-C₆-alkenyl, C₂-C₆-alkynyl, C₂-C₆-haloalkenyl, C₃-C₆-haloalkynyl, C₁-C₄-alkoxy-C₁-C₄-alkyl, C₁-C₄-haloalkoxy-C₁-C₄-alkyl, R^{b}-S(O)ₙ-C₁-C₃-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R²¹ is selected from the group consisting of cyano, halogen, nitro, C₁-C₆-alkyl, C₃-C₇-cycloalkyl, C₃-C₇-halocycloalkyl, C₁-C₆-haloalkyl, C₂-C₆-alkenyl, C₂-C₆-haloalkenyl, C₂-C₆-alkynyl, C₃-C₆-haloalkynyl, C₁-C₆-alkoxy, C₁-C₄-alkoxy-C₁-C₄-alkyl, C₁-C₄-alkylthio-C₁-C₄-alkyl, C₁-C₄-haloalkoxy-C₁-C₄-alkyl, C₁-C₄-alkoxy-C₁-C₄-alkoxy, C₃-C₇-cycloalkoxy and C₁-C₆-haloalkoxy, or two radicals R²¹ bound to the same carbon atom together may form a group =O;
Z¹, Z² independently of each other are selected from the group consisting of a covalent bond and C₁-C₄-alkanediyl;
Z^{2a} is selected from the group consisting of a covalent bond, C₁-C₄-alkanediyl, O-C₁-C₄-alkanediyl, C₁-C₄-alkanediyl-O and C₁-C₄-alkanediyl-O-C₁-C₄-alkanediyl;
R^{b}, R^{1b}, R^{2b} independently of each other are selected from the group consisting of C₁-C₆-alkyl, C₃-C₇-cycloalkyl, C₁-C₆-haloalkyl, C₂-C₆-alkenyl, C₂-C₆-haloalkenyl, C₂-C₆-alkynyl, C₃-C₆-haloalkynyl, phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{2c} is selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl, benzyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{2d} is selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
R^{2c}, R^{2d} together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
R²ⁿ is selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-cyanoalkyl, C₁-C₆-haloalkyl, C₂-C₆-alkenyl, C₂-C₆-haloalkenyl, C₂-C₆-alkynyl, C₁-C₄-alkoxy-C₁-C₄-alkyl;
R^{2e}, R^{2f} independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
R^{2e}, R^{2f} together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
R^{2g} is selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylsulfonyl, C₁-C₄-alkylcarbonyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{2h} is selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylsulfonyl, C₁-C₄-alkylcarbonyl, a radical C(O)R^{k}, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
R^{2g}, R^{2h} together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
R²² is selected from the group consisting of hydrogen, C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-cycloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl, C₁-C₆-haloalkyl, C₃-C₆-cycloalkyl, C(O)OR²³, C(O)N(R²³)₂, OR²³, N(R²³)₂, S(O),R²⁴, S(O)₂OR²³, S(O)₂N(R²³)₂, and R²³O-C₁-C₆-alkyl, and where heterocyclyl bears 0, 1 or 2 oxo groups;
R²³ is selected from the group consisting of hydrogen, C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
R²⁴ is selected from the group consisting of C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
R²⁵ is selected from the group consisting of C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-cycloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl, C₁-C₆-haloalkyl, C₃-C₆-cycloalkyl, C(O)OR²³, C(O)N(R²³)₂, OR²³, N(R²³)₂, S(O)ₙR²⁴, S(O)₂OR²³, S(O)₂N(R²³)₂, and R²³O-C₁-C₆-alkyl, and where heterocyclyl bears 0, 1 or 2 oxo groups;
k is 0, 1 or 2;
n is 0, 1 or 2;
R^{k} has the meanings of R^{2c};
or an agriculturally suitable salt thereof.

2. The compound as claimed in claim 1, or an agriculturally suitable salt thereof, where R¹ is selected from the group consisting of halogen, nitro, C₁-C₄-alkyl, C₁-C₄-alkoxy-C₁-C₄-alkoxy-Z¹, R^{1b}-S(O)ₖ-Z¹, where in particular R¹ is selected from the group consisting of halogen and C₁-C₄-alkyl.

3. The compound as claimed in any one of claims 1 or 2, or an agriculturally suitable salt thereof, where R² is R^{2c}R^{2d}NC(O)NR²ⁿ-Z², where R²ⁿ is selected from the group consisting of hydrogen and C₁-C₆-alkyl.

4. The compound as claimed in any one of claims 1 to 3, or an agriculturally suitable salt thereof, where Z² in R² is a covalent bond and R^{2c} and R^{2d} independently of each other are selected from the group consisting of C₁-C₆-alkyl, C₃-C₇-cycloalkyl, C₃-C₇-cycloalkyl-C₁-C₄-alkyl, C₁-C₆-haloalkyl, phenyl, and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy.

5. The compound as claimed in claim 4, or an agriculturally suitable salt thereof, where Z² in R² is a covalent bond R^{2c} is selected from the group consisting of C₁-C₄-alkyl, C₃-C₇-cycloalkyl and C₁-C₄-haloalkyl and R^{2d} is selected from the group consisting of C₁-C₆-alkyl, C₃-C₇-cycloalkyl, C₃-C₇-Cycloalkyl-C₁-C₂-alkyl and C₁-C₆-haloalkyl.

6. The compound as claimed in any one of claims 1 to 3, or an agriculturally suitable salt thereof, where Z² in R² is a covalent bond and R^{2c} and R^{2d} together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy.

7. The compound as claimed in any one of claims 1 to 6, or an agriculturally suitable salt thereof, where R³ is selected from the group consisting of hydrogen, halogen, C₁-C₄-alkyl, C₁-C₄-haloalkyl, C₁-C₄-haloalkoxy, and R^{2b}-S(O)ₖ; where in particular R³ is selected from the group consisting of hydrogen, halogen and C₁-C₂-haloalkyl.

8. The compound as claimed in any one of claims 1 to 7, or an agriculturally suitable salt thereof, where R⁴ is selected from the group consisting cyano, halogen, nitro, C₁-C₂-alkyl, and C₁-C₂-haloalkyl.

9. The compound as claimed in claim 8, or an agriculturally suitable salt thereof, where R⁴ is selected from the group consisting of fluorine and chlorine.

10. The compound as claimed in any one of claims 1 to 9, or an agriculturally suitable salt thereof, where R⁶ is selected from the group consisting of C₁-C₆-alkyl, C₁-C₄-alkoxy-C₁-C₄-alkyl, and phenyl.

11. The compound as claimed in claim 1, or an agriculturally suitable salt thereof, which is selected from the compounds of the following formulae I.A.I and I.D.I, 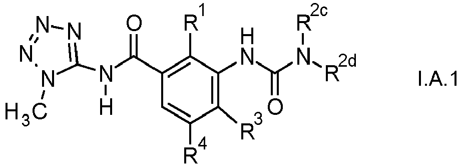 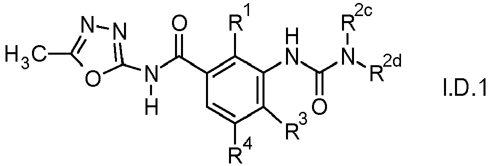 where
R¹ is selected from the group consisting of halogen and C₁-C₄-alkyl,
R^{2c} is selected from the group consisting of C₁-C₄-alkyl, C₃-C₇-cycloalkyl and C₁-C₄-haloalkyl,
R^{2d} is selected from the group consisting of C₁-C₆-alkyl, C₃-C₇-cycloalkyl, C₃-C₇-cycloalkyl-C₁-C₂-alkyl and C₁-C₆-haloalkyl,
R³ is selected from the group consisting of hydrogen, halogen and C₁-C₂-haloalkyl.
R⁴ is selected from the group consisting of fluorine and chlorine.

12. The compound as claimed in claim 11, or an agriculturally suitable salt thereof, where, R¹, R^{2c}, R^{2d}, R³ and R⁴ are as defned in the following tables:
| | R¹ | R^{2c} | R^{2d} | R³ | R⁴ |
|---|---|---|---|---|---|
| 1. | CH₃ | CH₃ | CH₃ | H | Cl |
| 2. | CH₃ | CH₃ | CH₂CH₃ | H | Cl |
| 3. | CH₃ | CH₃ | CH₂CH₃ | H | Cl |
| 4. | CH₃ | CH₂CH₃ | CH₂CH₃ | H | Cl |
| 5. | CH₃ | CH₃ | CH₂CF₃ | H | Cl |
| 6. | CH₃ | CH₂CH₃ | CH₂CF₃ | H | Cl |
| 7. | CH₃ | CH₃ | CH₂CHF₂ | H | Cl |
| 8. | CH₃ | CH₂CH₃ | CH₂CHF₂ | H | Cl |
| 9. | CH₃ | CH₃ | CH(CH₃)₂ | H | Cl |
| 10. | CH₃ | c-C₃H₅ | c-C₃H₅ | H | Cl |
| 11. | Cl | CH₃ | CH₃ | H | Cl |
| 12. | Cl | CH₃ | CH₂CH₃ | H | Cl |
| 13. | Cl | CH₂CH₃ | CH₂CH₃ | H | Cl |
| 14. | Cl | CH₃ | CH₂CF₃ | H | Cl |
| 15. | Cl | CH₂CH₃ | CH₂CF₃ | H | Cl |
| 16. | Cl | CH₃ | CH₂CHF₂ | H | Cl |
| 17. | Cl | CH₂CH₃ | CH₂CHF₂ | H | Cl |
| 18. | Cl | CH₃ | CH(CH₃)₂ | H | Cl |
| 19. | Cl | c-C₃H₅ | c-C₃H₅ | H | Cl |
| 20. | CH₃ | CH₃ | CH₃ | Cl | Cl |
| 21. | CH₃ | CH₃ | CH₂CH₃ | Cl | Cl |
| 22. | CH₃ | CH₃ | CH₂CH₃ | Cl | Cl |
| 23. | CH₃ | CH₂CH₃ | CH₂CH₃ | Cl | Cl |
| 24. | CH₃ | CH₃ | CH₂CF₃ | Cl | Cl |
| 25. | CH₃ | CH₂CH₃ | CH₂CF₃ | Cl | Cl |
| 26. | CH₃ | CH₃ | CH₂CHF₂ | Cl | Cl |
| 27. | CH₃ | CH₂CH₃ | CH₂CHF₂ | Cl | Cl |
| 28. | CH₃ | CH₃ | CH(CH₃)₂ | Cl | Cl |
| 29. | CH₃ | c-C₃H₅ | c-C₃H₅ | Cl | Cl |
| 30. | Cl | CH₃ | CH₃ | Cl | Cl |
| 31. | Cl | CH₃ | CH₂CH₃ | Cl | Cl |
| 32. | Cl | CH₂CH₃ | CH₂CH₃ | Cl | Cl |
| 33. | Cl | CH₃ | CH₂CF₃ | Cl | Cl |
| 34. | Cl | CH₂CH₃ | CH₂CF₃ | Cl | Cl |
| 35. | Cl | CH₃ | CH₂CHF₂ | Cl | Cl |
| 36. | Cl | CH₂CH₃ | CH₂CHF₂ | Cl | Cl |
| 37. | Cl | CH₃ | CH(CH₃)₂ | Cl | Cl |
| 38. | Cl | c-C₃H₅ | c-C₃H₅ | Cl | Cl |
| 39. | CH₃ | CH₃ | CH₃ | Br | Cl |
| 40. | CH₃ | CH₃ | CH₂CH₃ | Br | Cl |
| 41. | CH₃ | CH₂CH₃ | CH₂CH₃ | Br | Cl |
| 42. | CH₃ | CH₃ | CH₂CF₃ | Br | Cl |
| 43. | CH₃ | CH₂CH₃ | CH₂CF₃ | Br | Cl |
| 44. | CH₃ | CH₃ | CH₂CHF₂ | Br | Cl |
| 45. | CH₃ | CH₂CH₃ | CH₂CHF₂ | Br | Cl |
| 46. | CH₃ | CH₃ | CH(CH₃)₂ | Br | Cl |
| 47. | CH₃ | c-C₃H₅ | c-C₃H₅ | Br | Cl |
| 48. | Cl | CH₃ | CH₃ | Br | Cl |
| 49. | Cl | CH₃ | CH₂CH₃ | Br | Cl |
| 50. | Cl | CH₂CH₃ | CH₂CH₃ | Br | Cl |
| 51. | Cl | CH₃ | CH₂CF₃ | Br | Cl |
| 52. | Cl | CH₂CH₃ | CH₂CF₃ | Br | Cl |
| 53. | Cl | CH₃ | CH₂CHF₂ | Br | Cl |
| 54. | Cl | CH₂CH₃ | CH₂CHF₂ | Br | Cl |
| 55. | Cl | CH₃ | CH(CH₃)₂ | Br | Cl |
| 56. | Cl | c-C₃H₅ | c-C₃H₅ | Br | Cl |
| 57. | CH₃ | CH₃ | CH₃ | CF₃ | Cl |
| 58. | CH₃ | CH₃ | CH₂CH₃ | CF₃ | Cl |
| 59. | CH₃ | CH₂CH₃ | CH₂CH₃ | CF₃ | Cl |
| 60. | CH₃ | CH₃ | CH₂CF₃ | CF₃ | Cl |
| 61. | CH₃ | CH₂CH₃ | CH₂CF₃ | CF₃ | Cl |
| 62. | CH₃ | CH₃ | CH₂CHF₂ | CF₃ | Cl |
| 63. | CH₃ | CH₂CH₃ | CH₂CHF₂ | CF₃ | Cl |
| 64. | CH₃ | CH₃ | CH(CH₃)₂ | CF₃ | Cl |
| 65. | CH₃ | c-C₃H₅ | c-C₃H₅ | CF₃ | Cl |
| 66. | Cl | CH₃ | CH₃ | CF₃ | Cl |
| 67. | Cl | CH₃ | CH₂CH₃ | CF₃ | Cl |
| 68. | Cl | CH₂CH₃ | CH₂CH₃ | CF₃ | Cl |
| 69. | Cl | CH₃ | CH₂CF₃ | CF₃ | Cl |
| 70. | Cl | CH₂CH₃ | CH₂CF₃ | CF₃ | Cl |
| 71. | Cl | CH₃ | CH₂CHF₂ | CF₃ | Cl |
| 72. | Cl | CH₂CH₃ | CH₂CHF₂ | CF₃ | Cl |
| 73. | Cl | CH₃ | CH(CH₃)₂ | CF₃ | Cl |
| 74. | Cl | c-C₃H₅ | c-C₃H₅ | CF₃ | Cl |
| 75. | CH₃ | CH₃ | CH₃ | H | F |
| 76. | CH₃ | CH₃ | CH₂CH₃ | H | F |
| 77. | CH₃ | CH₃ | CH₂CH₃ | H | F |
| 78. | CH₃ | CH₂CH₃ | CH₂CH₃ | H | F |
| 79. | CH₃ | CH₃ | CH₂CF₃ | H | F |
| 80. | CH₃ | CH₂CH₃ | CH₂CF₃ | H | F |
| 81. | CH₃ | CH₃ | CH₂CHF₂ | H | F |
| 82. | CH₃ | CH₂CH₃ | CH₂CHF₂ | H | F |
| 83. | CH₃ | CH₃ | CH(CH₃)₂ | H | F |
| 84. | CH₃ | c-C₃H₅ | c-C₃H₅ | H | F |
| 85. | Cl | CH₃ | CH₃ | H | F |
| 86. | Cl | CH₃ | CH₂CH₃ | H | F |
| 87. | Cl | CH₂CH₃ | CH₂CH₃ | H | F |
| 88. | Cl | CH₃ | CH₂CF₃ | H | F |
| 89. | Cl | CH₂CH₃ | CH₂CF₃ | H | F |
| 90. | Cl | CH₃ | CH₂CHF₂ | H | F |
| 91. | Cl | CH₂CH₃ | CH₂CHF₂ | H | F |
| 92. | Cl | CH₃ | CH(CH₃)₂ | H | F |
| 93. | Cl | c-C₃H₅ | c-C₃H₅ | H | F |
| 94. | CH₃ | CH₃ | CH₃ | Cl | F |
| 95. | CH₃ | CH₃ | CH₂CH₃ | Cl | F |
| 96. | CH₃ | CH₃ | CH₂CH₃ | Cl | F |
| 97. | CH₃ | CH₂CH₃ | CH₂CH₃ | Cl | F |
| 98. | CH₃ | CH₃ | CH₂CF₃ | Cl | F |
| 99. | CH₃ | CH₂CH₃ | CH₂CF₃ | Cl | F |
| 100. | CH₃ | CH₃ | CH₂CHF₂ | Cl | F |
| 101. | CH₃ | CH₂CH₃ | CH₂CHF₂ | Cl | F |
| 102. | CH₃ | CH₃ | CH(CH₃)₂ | Cl | F |
| 103. | CH₃ | c-C₃H₅ | c-C₃H₅ | Cl | F |
| 104. | Cl | CH₃ | CH₃ | Cl | F |
| 105. | Cl | CH₃ | CH₂CH₃ | Cl | F |
| 106. | Cl | CH₂CH₃ | CH₂CH₃ | Cl | F |
| 107. | Cl | CH₃ | CH₂CF₃ | Cl | F |
| 108. | Cl | CH₂CH₃ | CH₂CF₃ | Cl | F |
| 109. | Cl | CH₃ | CH₂CHF₂ | Cl | F |
| 110. | Cl | CH₂CH₃ | CH₂CHF₂ | Cl | F |
| 111. | Cl | CH₃ | CH(CH₃)₂ | Cl | F |
| 112. | Cl | c-C₃H₅ | c-C₃H₅ | Cl | F |
| 113. | CH₃ | CH₃ | CH₃ | Br | F |
| 114. | CH₃ | CH₃ | CH₂CH₃ | Br | F |
| 115. | CH₃ | CH₂CH₃ | CH₂CH₃ | Br | F |
| 116. | CH₃ | CH₃ | CH₂CF₃ | Br | F |
| 117. | CH₃ | CH₂CH₃ | CH₂CF₃ | Br | F |
| 118. | CH₃ | CH₃ | CH₂CHF₂ | Br | F |
| 119. | CH₃ | CH₂CH₃ | CH₂CHF₂ | Br | F |
| 120. | CH₃ | CH₃ | CH(CH₃)₂ | Br | F |
| 121. | CH₃ | c-C₃H₅ | c-C₃H₅ | Br | F |
| 122. | Cl | CH₃ | CH₃ | Br | F |
| 123. | Cl | CH₃ | CH₂CH₃ | Br | F |
| 124. | Cl | CH₂CH₃ | CH₂CH₃ | Br | F |
| 125. | Cl | CH₃ | CH₂CF₃ | Br | F |
| 126. | Cl | CH₂CH₃ | CH₂CF₃ | Br | F |
| 127. | Cl | CH₃ | CH₂CHF₂ | Br | F |
| 128. | Cl | CH₂CH₃ | CH₂CHF₂ | Br | F |
| 129. | Cl | CH₃ | CH(CH₃)₂ | Br | F |
| 130. | Cl | c-C₃H₅ | c-C₃H₅ | Br | F |
| 131. | CH₃ | CH₃ | CH₃ | CF₃ | F |
| 132. | CH₃ | CH₃ | CH₂CH₃ | CF₃ | F |
| 133. | CH₃ | CH₂CH₃ | CH₂CH₃ | CF₃ | F |
| 134. | CH₃ | CH₃ | CH₂CF₃ | CF₃ | F |
| 135. | CH₃ | CH₂CH₃ | CH₂CF₃ | CF₃ | F |
| 136. | CH₃ | CH₃ | CH₂CHF₂ | CF₃ | F |
| 137. | CH₃ | CH₂CH₃ | CH₂CHF₂ | CF₃ | F |
| 138. | CH₃ | CH₃ | CH(CH₃)₂ | CF₃ | F |
| 139. | CH₃ | c-C₃H₅ | c-C₃H₅ | CF₃ | F |
| 140. | Cl | CH₃ | CH₃ | CF₃ | F |
| 141. | Cl | CH₃ | CH₂CH₃ | CF₃ | F |
| 142. | Cl | CH₂CH₃ | CH₂CH₃ | CF₃ | F |
| 143. | Cl | CH₃ | CH₂CF₃ | CF₃ | F |
| 144. | Cl | CH₂CH₃ | CH₂CF₃ | CF₃ | F |
| 145. | Cl | CH₃ | CH₂CHF₂ | CF₃ | F |
| 146. | Cl | CH₂CH₃ | CH₂CHF₂ | CF₃ | F |
| 147. | Cl | CH₃ | CH(CH₃)₂ | CF₃ | F |
| 148. | Cl | c-C₃H₅ | c-C₃H₅ | CF₃ | F |
where c-C₃H₅ is cyclopropyl.
| | **R¹** | **NR^{2c}R^{2d}** | **R³** | **R³** |
|---|---|---|---|---|
| 149. | CH₃ | pyrrolidin-1-yl | H | Cl |
| 150. | CH₃ | piperidin-1-yl | H | Cl |
| 151. | CH₃ | morpholin-4-yl | H | Cl |
| 152. | CH₃ | 2,6-dimethylmorpholin-4-yl | H | Cl |
| 153. | Cl | pyrrolidin-1-yl | H | Cl |
| 154. | Cl | piperidin-1-yl | H | Cl |
| 155. | Cl | morpholin-4-yl | H | Cl |
| 156. | Cl | 2,6-dimethylmorpholin-4-yl | H | Cl |
| 157. | CH₃ | pyrrolidin-1-yl | Cl | Cl |
| 158. | CH₃ | piperidin-1-yl | Cl | Cl |
| 159. | CH₃ | morpholin-4-yl | Cl | Cl |
| 160. | CH₃ | 2,6-dimethylmorpholin-4-yl | Cl | Cl |
| 161. | Cl | pyrrolidin-1-yl | Cl | Cl |
| 162. | Cl | piperidin-1-yl | Cl | Cl |
| 163. | Cl | morpholin-4-yl | Cl | Cl |
| 164. | Cl | 2,6-dimethylmorpholin-4-yl | Cl | Cl |
| 165. | CH₃ | pyrrolidin-1-yl | Br | Cl |
| 166. | CH₃ | piperidin-1-yl | Br | Cl |
| 167. | CH₃ | morpholin-4-yl | Br | Cl |
| 168. | CH₃ | 2,6-dimethylmorpholin-4-yl | Br | Cl |
| 169. | Cl | pyrrolidin-1-yl | Br | Cl |
| 170. | Cl | piperidin-1-yl | Br | Cl |
| 171. | Cl | morpholin-4-yl | Br | Cl |
| 172. | Cl | 2,6-dimethylmorpholin-4-yl | Br | Cl |
| 173. | CH₃ | pyrrolidin-1-yl | CF₃ | Cl |
| 174. | CH₃ | piperidin-1-yl | CF₃ | Cl |
| 175. | CH₃ | morpholin-4-yl | CF₃ | Cl |
| 176. | CH₃ | 2,6-dimethylmorpholin-4-yl | CF₃ | Cl |
| 177. | Cl | pyrrolidin-1-yl | CF₃ | Cl |
| 178. | Cl | piperidin-1-yl | CF₃ | Cl |
| 179. | Cl | morpholin-4-yl | CF₃ | Cl |
| 180. | Cl | 2,6-dimethylmorpholin-4-yl | CF₃ | Cl |
| 181. | CH₃ | pyrrolidin-1-yl | H | F |
| 182. | CH₃ | piperidin-1-yl | H | F |
| 183. | CH₃ | morpholin-4-yl | H | F |
| 184. | CH₃ | 2,6-dimethylmorpholin-4-yl | H | F |
| 185. | Cl | pyrrolidin-1-yl | H | F |
| 186. | Cl | piperidin-1-yl | H | F |
| 187. | Cl | morpholin-4-yl | H | F |
| 188. | Cl | 2,6-dimethylmorpholin-4-yl | H | F |
| 189. | CH₃ | pyrrolidin-1-yl | Cl | F |
| 190. | CH₃ | piperidin-1-yl | Cl | F |
| 191. | CH₃ | morpholin-4-yl | Cl | F |
| 192. | CH₃ | 2,6-dimethylmorpholin-4-yl | Cl | F |
| 193. | Cl | pyrrolidin-1-yl | Cl | F |
| 194. | Cl | piperidin-1-yl | Cl | F |
| 195. | Cl | morpholin-4-yl | Cl | F |
| 196. | Cl | 2,6-dimethylmorpholin-4-yl | Cl | F |
| 197. | CH₃ | pyrrolidin-1-yl | Br | F |
| 198. | CH₃ | piperidin-1-yl | Br | F |
| 199. | CH₃ | morpholin-4-yl | Br | F |
| 200. | CH₃ | 2,6-dimethylmorpholin-4-yl | Br | F |
| 201. | Cl | pyrrolidin-1-yl | Br | F |
| 202. | Cl | piperidin-1-yl | Br | F |
| 203. | Cl | morpholin-4-yl | Br | F |
| 204. | Cl | 2,6-dimethylmorpholin-4-yl | Br | F |
| 205. | CH₃ | pyrrolidin-1-yl | CF₃ | F |
| 206. | CH₃ | piperidin-1-yl | CF₃ | F |
| 207. | CH₃ | morpholin-4-yl | CF₃ | F |
| 208. | CH₃ | 2,6-dimethylmorpholin-4-yl | CF₃ | F |
| 209. | Cl | pyrrolidin-1-yl | CF₃ | F |
| 210. | Cl | piperidin-1-yl | CF₃ | F |
| 211. | Cl | morpholin-4-yl | CF₃ | F |
| 212. | Cl | 2,6-dimethylmorpholin-4-yl | CF₃ | F |

13. A composition comprising at least one compound as claimed in any one of claims 1 to 12, or an agriculturally suitable salt thereof, and at least one auxiliary, which is customary for formulating crop protection compounds.

14. The use of a compound as claimed in any one of claims 1 to 12, or an agriculturally suitable salt thereof, or of the composition of claim 13 for controlling unwanted vegetation.

15. A method for controlling unwanted vegetation which comprises allowing a herbicidally effective amount of at least one compound as claimed in any one of claims 1 to 12, or an agriculturally suitable salt thereof, or of the composition of claim 13 to act on plants, their seed and/or their habitat.

## Patentansprüche

1. Verbindung der Formel I 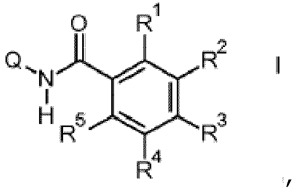 wobei
Q für Q¹ oder Q² oder Q³ oder Q⁴ steht 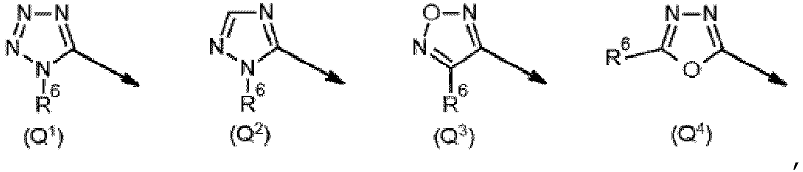
R¹ aus der aus Cyano, Halogen, Nitro, C₁-C₈-Alkyl, C₁-C₈-Halogenalkyl, C₁-C₄-Alkoxy-C₁-C₄-alkyl, C₁-C₄-Halogenalkoxy-C₁-C₄alkyl, C₁-C₈-Alkoxy, C₁-C₄-Alkoxy-C₁-C₄-alkoxy-Z¹, C₁-C₆-Halogenalkoxy und R^{1b}S(O)ₖ-Z¹ bestehenden Gruppe ausgewählt ist,
R² für R^{2c}R^{2d}NC(O)NR²ⁿ-Z² steht,
R³ aus der aus Wasserstoff, Cyano, Thiocyanato, Halogen, Hydroxy-Z², C₁-C₆-Alkyl, C₂-C₈-Alkenyl, C₂-C₈-Alkinyl, C₃-C₁₀-Cycloalkyl-Z², C₃-C₆-Cycloalkenyl-Z², C₃-C₁₀-Cycloalkoxy-Z², C₃-C₁₀-Cycloalkyl-C₁-C₂-alkoxy, wobei die cyclischen Gruppen der vier oben erwähnten Reste unsubstituiert oder teilweise oder vollständig halogeniert sind, C₁-C₄-Cyanoalkyl, C₁-C₈-Halogenalkyl, C₂-C₈-Halogenalkenyl, C₃-C₈-Halogenalkinyl, C₁-C₈-Alkoxy-Z², C₁-C₈-Halogenalkoxy-Z², C₁-C₄-Alkoxy-C₁-C₄-alkoxy-Z², C₁-C₄-Halogenalkoxy-C₁-C₄-alkoxy-Z², C₂-C₈-Alkenyloxy-Z², C₂-C₈-Alkinyloxy-Z², C₂-C₈-Halogenalkenyloxy-Z², C₃-C₈-Halogenalkinyloxy-Z², R^{2b}-S(O)ₖ-Z², R^{2c}-C(O)-Z², R^{2d}O-C(O)-Z², R^{2d}O-N=CH-Z², R^{2e}R^{2f}N-C(O)-Z², R²⁹R^{2h}N-Z², R²²C(O)O-Z², R²⁵OC(O)-Z², (R²²)₂NC(O)O-Z², R²⁵S(O)₂O-Z², R²²OS(O)₂-Z², (R²²)₂NS(O)₂-Z², R²⁵OC(O)N(R²²)-Z², (R²²)₂NC(O)N(R²²)-Z², (R²²)₂NS(O)₂N(R²²)-Z², (OH)₂P(O)-Z², (C₁-C₄-Alkoxy)₂P(O)-Z², Phenyl-Z^{2a} und Heterocyclyl-Z^{2a} bestehenden Gruppe ausgewählt ist, wobei Heterocyclyl für einen 3-, 4-, 5- oder 6-gliedrigen monocyclischen oder 8-, 9- oder 10-gliedrigen bicyclischen gesättigten, teilweise ungesättigten oder aromatischen Heterocyclus mit 1, 2, 3 oder 4 aus der aus O, N und S bestehenden Gruppe ausgewählten Heteroatomen als Ringgliedern steht, wobei cyclische Gruppen in Phenyl-Z^{2a} und Heterocyclyl-Z^{2a} unsubstituiert oder durch 1, 2, 3 oder 4 gleiche oder verschiedene Gruppen R²¹ substituiert sind,
R⁴ aus der aus Halogen, C₁-C₈-Alkyl, Cyano-Z¹, Nitro, C₃-C₇-Cycloalkyl, C₃-C₇-Cycloalkyl-C₁-C₄-alkyl, wobei die C₃-C₇-Cycloalkylgruppen in den beiden oben erwähnten Resten unsubstituiert oder teilweise oder vollständig halogeniert sind, C₂-C₈-Alkenyl, C₂-C₈-Alkinyl, C₁-C₈-Halogenalkyl, C₁-C₃-Alkylamino, C₁-C₃-Dialkylamino, C₁-C₃-Alkylamino-S(O)ₖ, C₁-C₃-Alkylcarbonyl, C₁-C₈-Alkoxy, C₁-C₄-Alkoxy-C₁-C₄-alkyl, C₁-C₄-Alkoxy-C₁-C₄-alkoxy-Z¹, C₁-C₄-Alkylthio-C₁-C₄-alkyl, C₁-C₄-Alkylthio-C₁-C₄-alkylthio-Z¹, C₂-C₆-Alkenyloxy, C₂-C₆-Alkinyloxy, C₁-C₆-Halogenalkoxy, C₁-C₄-Halogenalkoxy-C₁-C₄-alkyl, C₁-C₄-Halogenalkoxy-C₁-C₄-alkoxy-Z¹, R^{1b}-S(O)ₖ-Z¹, Phenoxy-Z¹ und Heterocyclyloxy-Z¹ bestehenden Gruppe ausgewählt ist, wobei Heterocyclyloxy für einen an Sauerstoff gebundenen 5- oder 6-gliedrigen monocyclischen oder 8-, 9- oder 10-gliedrigen bicyclischen gesättigten, teilweise ungesättigten oder aromatischen Heterocyclus mit 1, 2, 3 oder 4 aus der aus O, N und S bestehenden Gruppe ausgewählten Heteroatomen als Ringgliedern steht, wobei die cyclischen Gruppen in Phenoxy und Heterocyclyloxy unsubstituiert oder durch 1, 2, 3 oder 4 gleiche oder verschiedene Gruppen R¹¹ substituiert sind,
R⁵ für Wasserstoff steht,
R⁶ aus der aus Cyano, C₁-C₆-Alkyl, C₃-C₇-Cycloalkyl, C₃-C₇-Cycloalkyl-C₁-C₄-alkyl, wobei die C₃-C₇-Cycloalkylgruppen der beiden oben erwähnten Reste unsubstituiert oder teilweise oder vollständig halogeniert sind, C₁-C₆-Halogenalkyl, C₂-C₆-Alkenyl, C₂-C₆-Alkinyl, C₂-C₆-Halogenalkenyl, C₃-C₆-Halogenalkinyl, C₁-C₄-Alkoxy-C₁-C₄-alkyl, C₁-C₄-Halogenalkoxy-C₁-C₄-alkyl, R^{b}-S(O)ₙ-C₁-C₃-alkyl, Phenyl und Benzyl bestehenden Gruppe ausgewählt ist, wobei Phenyl und Benzyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind,
R²¹ aus der aus Cyano, Halogen, Nitro, C₁-C₆-Alkyl, C₃-C₇-Cycloalkyl, C₃-C₇-Halogencycloalkyl, C₁-C₆-Halogenalkyl, C₂-C₆-Alkenyl, C₂-C₆-Halogenalkenyl, C₂-C₆-Alkinyl, C₃-C₆-Halogenalkinyl, C₁-C₆-Alkoxy, C₁-C₄-Alkoxy-C₁-C₄-alkyl, C₁-C₄-Alkylthio-C₁-C₄-alkyl, C₁-C₄-Halogenalkoxy-C₁-C₄-alkyl, C₁-C₄-Alkoxy-C₁-C₄-alkoxy, C₃-C₇-Cycloalkoxy und C₁-C₆-Halogenalkoxy bestehenden Gruppe ausgewählt ist oder zwei an das gleiche Kohlenstoffatom gebundene Reste R²¹ zusammen eine Gruppe =O bilden können,
Z¹, Z² unabhängig voneinander aus der aus einer kovalenten Bindung und C₁-C₄-Alkandiyl bestehenden Gruppe ausgewählt sind,
Z^{2a} aus der aus einer kovalenten Bindung, C₁-C₄-Alkandiyl, O-C₁-C₄-Alkandiyl, C₁-C₄-Alkandiyl-O und C₁-C₄-Alkandiyl-O-C₁-C₄-alkandiyl bestehenden Gruppe ausgewählt ist,
R^{b}, R^{1b}, R^{2b} unabhängig voneinander aus der aus C₁-C₆-Alkyl, C₃-C₇-Cycloalkyl, C₁-C₆-Halogenalkyl, C₂-C₆-Alkenyl, C₂-C₆-Halogenalkenyl, C₂-C₆-Alkinyl, C₃-C₆-Halogenalkinyl, Phenyl und Hetercyclyl bestehenden Gruppe ausgewählt sind, wobei Heterocyclyl für einen 5- oder 6-gliedrigen monocyclischen gesättigten, teilweise ungesättigten oder aromatischen Heterocyclus mit 1, 2, 3 oder 4 aus der aus O, N und S bestehenden Gruppe ausgewählten Heteroatomen als Ringgliedern steht, wobei Phenyl und Heterocyclyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind,
R^{2c} aus der aus Wasserstoff, C₁-C₆-Alkyl, C₃-C₇-Cycloalkyl, C₃-C₇-Cycloalkyl-C₁-C₄-alkyl, wobei die C₃-C₇-Cycloalkylgruppen in den beiden oben erwähnten Resten unsubstituiert oder teilweise oder vollständig halogeniert sind, C₁-C₆-Halogenalkyl, C₁-C₆-Alkoxy, C₂-C₆-Alkenyl, C₁-C₄-Alkyl-C₂-C₆-alkenyl, C₂-C₆-Halogenalkenyl, C₂-C₆-Alkinyl, C₃-C₆-Halogenalkinyl, C₁-C₄-Alkoxy-C₁-C₄-alkyl, C₁-C₄-Alkyl-S(O)ₙ-C₁-C₄-alkyl, C₁-C₄-Alkylamino-C₁-C₄-alkyl, C₁-C₄-Dialkylamino-C₁-C₄-alkyl, C₁-C₆-Cyanoalkyl, Phenyl, Benzyl und Heterocylyl bestehenden Gruppe ausgewählt ist, wobei Heterocyclyl für einen 5- oder 6-gliedrigen monocyclischen gesättigten, teilweise ungesättigten oder aromatischen Heterocyclus mit 1, 2, 3 oder 4 aus der aus O, N und S bestehenden Gruppe ausgewählten Heteroatomen als Ringgliedern steht, wobei Phenyl, Benzyl und Heterocyclyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind,
R^{2d} aus der aus Wasserstoff, C₁-C₆-Alkyl, C₃-C₇-Cycloalkyl, C₃-C₇-Cycloalkyl-C₁-C₄-alkyl, wobei die C₃-C₇-Cycloalkylgruppen in den beiden oben erwähnten Resten unsubstituiert oder teilweise oder vollständig halogeniert sind, C₁-C₆-Halogenalkyl, C₁-C₆-Alkoxy, C₂-C₆-Alkenyl, C₁-C₄-Alkyl-C₂-C₆-alkenyl, C₂-C₆-Halogenalkenyl, C₂-C₆-Alkinyl, C₃-C₆-Halogenalkinyl, C₁-C₄-Alkoxy-C₁-C₄-alkyl, C₁-C₄-Alkyl-S(O)ₙ-C₁-C₄-alkyl, C₁-C₄-Alkylamino-C₁-C₄-alkyl, C₁-C₄-Dialkylamino-C₁-C₄-alkyl, C₁-C₆-Cyanoalkyl, Phenyl und Benzyl bestehenden Gruppe ausgewählt ist, wobei Phenyl und Benzyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind, oder
R^{2c}, R^{2d} zusammen mit dem Stickstoffatom, an das sie gebunden sind, einen 4-, 5-, 6- oder 7-gliedrigen, gesättigten oder ungesättigten heterocyclischen Rest bilden können, der als ein Ringglied ein weiteres aus O, S und N ausgewähltes Heteroatom tragen kann und der unsubstituiert ist oder 1, 2, 3 oder 4 gleiche oder verschiedene und aus der aus =O, Halogen, C₁-C₄-Alkyl, C₁-C₄-Halogenalkyl, C₁-C₄-Alkoxy und C₁-C₄-Halogenalkoxy bestehenden Gruppe ausgewählte Gruppen tragen kann,
R²ⁿ aus der aus Wasserstoff, C₁-C₆-Alkyl, C₃-C₇-Cycloalkyl, C₃-C₇-Cycloalkyl-C₁-C₄-alkyl, wobei die C₃-C₇-Cycloalkylgruppen in den beiden oben erwähnten Resten unsubstituiert oder teilweise oder vollständig halogeniert sind, C₁-C₆-Cyanoalkyl, C₁-C₆-Halogenalkyl, C₂-C₆-Alkenyl, C₂-C₆-Halogenalkenyl, C₂-C₆-Alkinyl und C₁-C₄-Alkoxy-C₁-C₄-alkyl bestehenden Gruppe ausgewählt ist, R^{2e}, R^{2f} unabhängig voneinander aus der aus Wasserstoff, C₁-C₆-Alkyl, C₃-C₇-Cycloalkyl, C₃-C₇-Cycloalkyl-C₁-C₄-alkyl, wobei die C₃-C₇-Cycloalkylgruppen in den beiden oben erwähnten Resten unsubstituiert oder teilweise oder vollständig halogeniert sind, C₁-C₆-Halogenalkyl, C₂-C₆-Alkenyl, C₂-C₆-Halogenalkenyl, C₂-C₆-Alkinyl, C₃-C₆-Halogenalkinyl, C₁-C₄-Alkoxy-C₁-C₄-alkyl, Phenyl und Benzyl bestehenden Gruppe ausgewählt sind, wobei Phenyl und Benzyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind, oder
R^{2e}, R^{2f} zusammen mit dem Stickstoffatom, an das sie gebunden sind, einen 4-, 5-, 6- oder 7-gliedrigen, gesättigten oder ungesättigten heterocyclischen Rest bilden können, der ein weiteres aus O, S und N ausgewähltes Heteroatom als ein Ringglied tragen kann und der unsubstituiert ist oder 1, 2, 3 oder 4 gleiche oder verschiedene und aus der aus =O, Halogen, C₁-C₄-Alkyl, C₁-C₄-Halogenalkyl, C₁-C₄-Alkoxy und C₁-C₄-Halogenalkoxy bestehenden Gruppe ausgewählte Gruppen tragen kann,
R^{2g} aus der aus Wasserstoff, C₁-C₆-Alkyl, C₃-C₇-Cycloalkyl, C₃-C₇-Cycloalkyl-C₁-C₄-alkyl, wobei die C₃-C₇-Cycloalkylgruppen in den beiden oben erwähnten Resten unsubstituiert oder teilweise oder vollständig halogeniert sind, C₁-C₆-Halogenalkyl, C₂-C₆-Alkenyl, C₂-C₆-Halogenalkenyl, C₂-C₆-Alkinyl, C₃-C₆-Halogenalkinyl, C₁-C₄-Alkoxy-C₁-C₄-alkyl, C₁-C₄-Alkylsulfonyl, C₁-C₄-Alkylcarbonyl, Phenyl und Benzyl bestehenden Gruppe ausgewählt ist, wobei Phenyl und Benzyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind,
R^{2h} aus der aus Wasserstoff, C₁-C₆-Alkyl, C₃-C₇-Cycloalkyl, C₃-C₇-Cycloalkyl-C₁-C₄-alkyl, wobei die C₃-C₇-Cycloalkylgruppen in den beiden oben erwähnten Resten unsubstituiert oder teilweise oder vollständig halogeniert sind, C₁-C₆-Halogenalkyl, C₂-C₆-Alkenyl, C₂-C₆-Halogenalkenyl, C₂-C₆-Alkinyl, C₃-C₆-Halogenalkinyl, C₁-C₄-Alkoxy-C₁-C₄-alkyl, C₁-C₄-Alkylsulfonyl, C₁-C₄-Alkylcarbonyl, einem Rest C(O)R^{k}, Phenyl und Benzyl bestehenden Gruppe ausgewählt ist, wobei Phenyl und Benzyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind, oder
R², R^{2h} zusammen mit dem Stickstoffatom, an das sie gebunden sind, einen 4-, 5-, 6- oder 7-gliedrigen, gesättigten oder ungesättigten heterocyclischen Rest bilden können, der ein weiteres aus O, S und N ausgewähltes Heteroatom als ein Ringglied tragen kann und der unsubstituiert ist oder 1, 2, 3 oder 4 gleiche oder verschiedene und aus der aus =O, Halogen, C₁-C₄-Alkyl, C₁-C₄-Halogenalkyl, C₁-C₄-Alkoxy und C₁-C₄-Halogenalkoxy bestehenden Gruppe ausgewählte Gruppen tragen kann,
R²² aus der aus Wasserstoff, C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₃-C₆-Cycloalkyl, C₃-C₆-Cycloalkenyl, C₃-C₆-Halogencycloalkyl, C₃-C₆-Cycloalkyl-C₁-C₆-alkyl, C₁-C₆-Alkoxy-C₁-C₆-alkyl, C₃-C₆-Cycloalkyl-C₁-C₆-alkoxy-C₁-C₆-alkyl, Phenyl-Z¹, Phenyl-O-C₁-C₆-alkyl, Phenyl-N (R²³)-C₁-C₆-alkyl, Phenyl-S(O)ₙ-C₁-C₆-alkyl, Heterocyclyl-Z¹, Heterocyclyl-N(R²³)-C₁-C₆-alkyl, Heterocyclyl-O-C₁-C₆-alkyl, und Heterocyclyl-S(O)ₙ-C₁-C₆-alkyl bestehenden Gruppe ausgewählt ist, wobei Heterocyclyl für einen 5- oder 6-gliedrigen monocyclischen gesättigten, teilweise ungesättigten oder aromatischen Heterocyclus mit 1, 2, 3 oder 4 aus der aus O, N und S bestehenden Gruppe ausgewählten Heteroatomen als Ringgliedern steht, wobei Phenyl und Heterocyclyl unsubstituiert oder durch 1, 2, 3 oder 4 gleiche oder verschiedene und aus der aus Cyano, Halogen, Nitro, Thiocyanato, C₁-C₆-Alkyl, C₁-C₆-Halogenalkyl, C₃-C₆-Cycloalkyl, C(O)OR²³, C(O)N(R²³)₂, OR²³, N(R²³)₂, S(O)ₙR²⁴, S(O)₂OR²³, S(O)₂N(R²³)₂ und R²³O-C₁-C₆-Alkyl bestehenden Gruppe ausgewählte Gruppen substituiert sind und wobei Heterocyclyl 0, 1 oder 2 Oxogruppen trägt,
R²³ aus der aus Wasserstoff, C₁-C₆-Alkyl, C₁-C₆-Halogenalkyl, C₂-C₆-Alkenyl, C₂-C₆-Alkinyl, C₃-C₆-Cycloalkyl, C₃-C₆-Cycloalkyl-C₁-C₆-alkyl und Phenyl bestehenden Gruppe ausgewählt ist,
R²⁴ aus der aus C₁-C₆-Alkyl, C₁-C₆-Halogenalkyl, C₂-C₆-Alkenyl, C₂-C₆-Alkinyl, C₃-C₆-Cycloalkyl, C₃-C₆-Cycloalkyl-C₁-C₆-alkyl und Phenyl bestehenden Gruppe ausgewählt ist,
R²⁵ aus der aus C₁-C₆-Alkyl, C₁-C₆-Halogenalkyl, C₂-C₆-Alkenyl, C₂-C₆-Halogenalkenyl, C₂-C₆-Alkinyl, C₃-C₆-Halogenalkinyl, C₃-C₆-Cycloalkyl, C₃-C₆-Cycloalkenyl, C₃-C₆-Halogencycloalkyl, C₃-C₆-Cycloalkyl-C₁-C₆-alkyl, C₁-C₆-Alkoxy-C₁-C₆-alkyl, C₃-C₆-Cycloalkyl-C₁-C₆-alkoxy-C₁-C₆-alkyl, Phenyl-Z¹, Phenyl-O-C₁-C₆-alkyl, Phenyl-N(R²³)-C₁-C₆-alkyl, Phenyl-S(O)ₙ-C₁-C₆-alkyl, Heterocyclyl-Z¹, Heterocyclyl-N(R²³)-C₁-C₆-alkyl, Heterocyclyl-O-C₁-C₆-alkyl und Heterocyclyl-S(O)ₙ-C₁-C₆-alkyl bestehenden Gruppe ausgewählt ist, wobei Heterocyclyl für einen 5- oder 6-gliedrigen monocyclischen gesättigten, teilweise ungesättigten oder aromatischen Heterocyclus mit 1, 2, 3 oder 4 aus der aus O, N und S bestehenden Gruppe ausgewählten Heteroatomen als Ringgliedern steht, wobei Phenyl und Heterocyclyl unsubstituiert oder durch 1, 2, 3 oder 4 gleiche oder verschiedene und aus der aus Cyano, Halogen, Nitro, Thiocyanato, C₁-C₆-Alkyl, C₁-C₆-Halogenalkyl, C₃-C₆-Cycloalkyl, C(O)OR²³, C(O)N(R²³)₂, OR²³, N(R²³)₂, S(O)ₙR²⁴, S(O)₂OR²³, S(O)₂N(R²³)₂ und R²³O-C₁-C₆-Alkyl bestehenden Gruppe ausgewählte Gruppen substituiert sind und wobei Heterocyclyl 0, 1 oder 2 Oxogruppen trägt,
k für 0, 1 oder 2 steht,
n für 0, 1 oder 2 steht,
R^{k} die Bedeutungen von R^{2c} hat,
oder ein landwirtschaftlich geeignetes Salz davon.

2. Verbindung nach Anspruch 1 oder ein landwirtschaftlich geeignetes Salz davon, wobei R¹ aus der aus Halogen, Nitro, C₁-C₄-Alkyl, C₁-C₄-Alkoxy-C₁-C₄-alkoxy-Z¹ und R^{1b}-S(O)ₖ-Z¹ bestehenden Gruppe ausgewählt ist, wobei R¹ insbesondere aus der aus Halogen und C₁-C₄-Alkyl bestehenden Gruppe ausgewählt ist.

3. Verbindung nach Anspruch 1 oder 2 oder landwirtschaftlich geeignetes Salz davon, wobei R² für R^{2c}R^{2d}NC(O)NR²ⁿ-Z² steht, wobei R²ⁿ aus der aus Wasserstoff und C₁-C₆-Alkyl bestehenden Gruppe ausgewählt ist.

4. Verbindung nach einem der Ansprüche 1 bis 3 oder landwirtschafltich geeignetes Salz davon, wobei Z² in R² für eine kovalente Bindung steht und R^{2c} und R^{2d} unabhängig voneinander aus der aus C₁-C₆-Alkyl, C₃-C₇-Cycloalkyl, C₃-C₇-Cycloalkyl-C₁-C₄-alkyl, C₁-C₆-Halogenalkyl, Phenyl und Heterocyclyl bestehenden Gruppe ausgewählt sind, wobei Heterocyclyl für einen 5- oder 6-gliedrigen monocyclischen gesättigten, teilweise ungesättigten oder aromatischen Heterocycylus mit 1, 2, 3 oder 4 aus der aus O, N und S bestehenden Gruppe ausgewählten Heteroatomen als Ringgliedern steht, wobei Phenyl und Heterocyclyl unsubstituiert oder durch 1, 2, 3 oder 4 gleiche oder verschiedene und aus der aus Halogen, C₁-C₄-Alkyl, C₁-C₄-Halogenalkyl, C₁-C₄-Alkoxy und C₁-C₄-Halogenalkoxy bestehenden Gruppe ausgewählte Gruppen substituiert sind.

5. Verbindung nach Anspruch 4 oder ein landwirtschaftlich geeignetes Salz davon, wobei Z² in R² für eine kovalente Bindung steht, R^{2c} aus der aus C₁-C₄-Alkyl, C₃-C₇-Cycloalkyl und C₁-C₄-Halogenalkyl bestehenden Gruppe ausgewählt ist und R^{2d} aus der aus C₁-C₆-Alkyl, C₃-C₇-Cycloalkyl, C₃-C₇-Cycloalkyl-C₁-C₂- alkyl und C₁-C₆-Halogenalkyl bestehenden Gruppe ausgewählt ist.

6. Verbindung nach einem der Ansprüche 1 bis 3 oder ein landwirtschaftlich geeignetes Salz davon, wobei Z² in R² für eine kovalente Bindung steht und R^{2c} und R^{2d} zusammen mit dem Stickstoffatom, an das sie gebunden sind, einen 4-, 5-, 6- oder 7-gliedrigen, gesättigten oder ungesättigten heterocyclischen Rest bilden können, der ein weiteres aus O, S und N ausgewähltes Heteroatom als ein Ringglied tragen kann und der unsubstituiert ist oder 1, 2, 3 oder 4 gleiche oder verschiedene und aus der aus =O, Halogen, C₁-C₄-Alkyl, C₁-C₄-Halogenalkyl, C₁-C₄-Alkoxy und C₁-C₄-Halogenalkoxy bestehenden Gruppe ausgewählte Gruppen tragen kann.

7. Verbindung nach einem der Ansprüche 1 bis 6 oder ein landwirtschaftlich geeignetes Salz davon, wobei R³ aus der aus Wasserstoff, Halogen, C₁-C₄-Alkyl, C₁-C₄-Halogenalkyl, C₁-C₄-Halogenalkoxy und R^{2b}-S(O)ₖ bestehenden Gruppe ausgewählt ist, wobei R³ insbesondere aus der aus Wasserstoff, Halogen und C₁-C₂-Halogenalkyl bestehenden Gruppe ausgewählt ist.

8. Verbindung nach einem der Ansprüche 1 bis 7 oder ein landwirtschaftlich geeignetes Salz davon, wobei R⁴ aus der aus Cyano, Halogen, Nitro, C₁-C₂-Alkyl und C₁-C₂-Halogenalkyl bestehenden Gruppe ausgewählt ist.

9. Verbindung nach Anspruch 8 oder ein landwirtschaftlich geeignetes Salz davon, wobei R⁴ aus der aus Fluor und Chlor bestehenden Gruppe ausgewählt ist.

10. Verbindung nach einem der Ansprüche 1 bis 9 oder ein landwirtschaftlich geeignetes Salz davon, wobei R⁶ aus der aus C₁-C₆-Alkyl, C₁-C₄-Alkoxy-C₁-C₄-alkyl und Phenyl bestehenden Gruppe ausgewählt ist.

11. Verbindung nach Anspruch 1 oder ein landwirtschaftlich geeignetes Salz davon, ausgewählt aus den Verbindungen der folgenden Formeln I.A.I und I.D.I 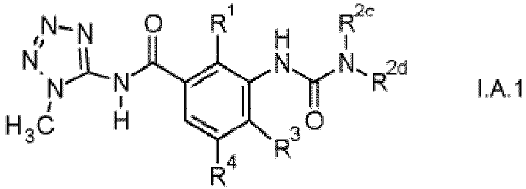 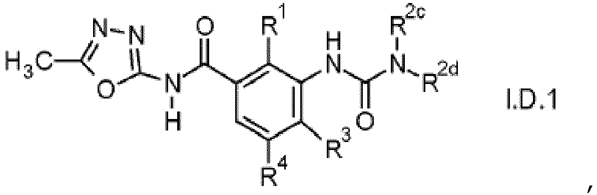 wobei
R¹ aus der aus Halogen und C₁-C₄-Alkyl bestehenden Gruppe ausgewählt ist,
R^{2c} aus der aus C₁-C₄-Alkyl, C₃-C₇-Cycloalkyl und C₁-C₄-Halogenalkyl bestehenden Gruppe ausgewählt ist,
R^{2d} aus der aus C₁-C₆-Alkyl, C₃-C₇-Cycloalkyl, C₃-C₇-Cycloalkyl-C₁-C₂-alkyl und C₁-C₆-Halogenalkyl bestehenden Gruppe ausgewählt ist,
R³ aus der aus Wasserstoff, Halogen und C₁-C₂-Halogenalkyl bestehenden Gruppe ausgewählt ist,
R⁴ aus der aus Fluor und Chlor bestehenden Gruppe ausgewählt ist.

12. Verbindung nach Anspruch 11 oder ein landwirtschaftlich geeignetes Salz davon, wobei R¹, R^{2c}, R^{2d}, R³ und R⁴ wie in den folgenden Tabellen definiert sind:
| | **R¹** | **R^{2c}** | **R^{2d}** | **R³** | **R⁴** |
|---|---|---|---|---|---|
| 1. | CH₃ | CH₃ | CH₃ | H | Cl |
| 2. | CH₃ | CH₃ | CH₂CH₃ | H | Cl |
| 3. | CH₃ | CH₃ | CH₂CH₃ | H | Cl |
| 4. | CH₃ | CH₂CH₃ | CH₃CH₃ | H | Cl |
| 5. | CH₃ | CH₃ | CH₂CF₃ | H | Cl |
| 6. | CH₃ | CH₂CH₃ | CH₂CF₃ | H | Cl |
| 7. | CH₃ | CH₃ | CH₂CHF₂ | H | Cl |
| 8. | CH₃ | CH₂CH₃ | CH₂CHF₂ | H | Cl |
| 9. | CH₃ | CH₃ | CH(CH₃)₂ | H | Cl |
| 10. | CH₃ | c-C₃H₅ | c-C₃H₅ | H | Cl |
| 11. | Cl | CH₃ | CH₃ | H | Cl |
| 12. | Cl | CH₃ | CH₂CH₃ | H | Cl |
| 13. | Cl | CH₂CH₃ | CH₂CH₃ | H | Cl |
| 14. | Cl | CH₃ | CH₂CF₃ | H | Cl |
| 15. | Cl | CH₂CH₃ | CH₂CF₃ | H | Cl |
| 16. | Cl | CH₃ | CH₂CHF₉ | H | Cl |
| 17. | Cl | CH₂CH₃ | CH₂CHF₂ | H | Cl |
| 18. | Cl | CH₃ | CH(CH₃)₂ | H | Cl |
| 19. | Cl | c-C₃H₅ | c-C₃H₅ | H | Cl |
| 20. | CH₃ | CH₃ | CH₃ | Cl | Cl |
| 21. | CH₃ | CH₃ | CH₂CH₃ | Cl | Cl |
| 22. | CH₃ | CH₃ | CH₂CH₃ | Cl | Cl |
| 23. | CH₃ | CH₂CH₃ | CH₂CH₃ | Cl | Cl |
| 24. | CH₃ | CH₃ | CH₂CF₃ | Cl | Cl |
| 25. | CH₃ | CH₂CH₃ | CH₂CF₃ | Cl | Cl |
| 26. | CH₃ | CH₃ | CH₂CHF₂ | Cl | Cl |
| 27. | CH₃ | CH₂CH₃ | CH₂CHF₂ | Cl | Cl |
| 28. | CH₃ | CH₃ | CH(CH₃)₂ | Cl | Cl |
| 29. | CH₃ | c-C₃H₅ | c-C₃H₅ | Cl | Cl |
| 30. | Cl | CH₃ | CH₃ | Cl | Cl |
| 31. | Cl | CH₃ | CH₂CH₃ | Cl | Cl |
| 32. | Cl | CH₂CH₃ | CH₂CH₃ | Cl | Cl |
| 33. | Cl | CH₃ | CH₂CF₃ | Cl | Cl |
| 34. | Cl | CH₂CH₃ | CH₂CF₃ | Cl | Cl |
| 35. | Cl | CH₃ | CH₂CHF₂ | Cl | Cl |
| 36. | Cl | CH₂CH₃ | CH₂CHF₂ | Cl | Cl |
| 37. | Cl | CH₃ | CH(CH₃)₂ | Cl | Cl |
| 38. | Cl | c-C₃H₅ | c-C₃H₅ | Cl | Cl |
| 39. | CH₃ | CH₃ | CH₃ | Br | Cl |
| 40. | CH₃ | CH₃ | CH₂CH₃ | Br | Cl |
| 41. | CH₃ | CH₂CH₃ | CH₂CH₃ | Br | Cl |
| 42. | CH₃ | CH₃ | CH₂CF₃ | Br | Cl |
| 43. | CH₃ | CH₂CH₃ | CH₂CF₃ | Br | Cl |
| 44. | CH₃ | CH₃ | CH₂CHF₂ | Br | Cl |
| 45. | CH₃ | CH₂CH₃ | CH₂CHF₂ | Br | Cl |
| 46. | CH₃ | CH₃ | CH(CH₃)₂ | Br | Cl |
| 47. | CH₃ | c-C₃H₅ | c-C₃H₅ | Br | Cl |
| 48. | Cl | CH₃ | CH₃ | Br | Cl |
| 49. | Cl | CH₃ | CH₂CH₃ | Br | Cl |
| 50. | Cl | CH₂CH₃ | CH₂CH₃ | Br | Cl |
| 51. | Cl | CH₃ | CH₂CF₃ | Br | Cl |
| 52. | Cl | CH₂CH₃ | CH₂CF₃ | Br | Cl |
| 53. | Cl | CH₃ | CH₂CHF₂ | Br | Cl |
| 54. | Cl | CH₂CH₃ | CH₂CHF₂ | Br | Cl |
| 55. | Cl | CH₃ | CH(CH₃)₂ | Br | Cl |
| 56. | Cl | c-C₃H₅ | c-C₃H₅ | Br | Cl |
| 57. | CH₃ | CH₃ | CH₃ | CF₃ | Cl |
| 58. | CH₃ | CH₃ | CH₂CH₃ | CF₃ | Cl |
| 59. | CH₃ | CH₂CH₃ | CH₂CH₃ | CF₃ | Cl |
| 60. | CH₃ | CH₃ | CH₂CF₃ | CF₃ | Cl |
| 61. | CH₃ | CH₂CH₃ | CH₂CF₃ | CF₃ | Cl |
| 62. | CH₃ | CH₃ | CH₂CHF₂ | CF₃ | Cl |
| 63. | CH₃ | CH₂CH₃ | CH₂CHF₂ | CF₃ | Cl |
| 64. | CH₃ | CH₃ | CH(CH₃)₂ | CF₃ | Cl |
| 65. | CH₃ | c-C₃H₅ | c-C₃H₅ | CF₃ | Cl |
| 66. | Cl | CH₃ | CH₃ | CF₃ | Cl |
| 67. | Cl | CH₃ | CH₂CH₃ | CF₃ | Cl |
| 68. | Cl | CH₂CH₃ | CH₂CH₃ | CF₃ | Cl |
| 69. | Cl | CH₃ | CH₂CF₃ | CF₃ | Cl |
| 70. | Cl | CH₂CH₃ | CH₂CF₃ | CF₃ | Cl |
| 71. | Cl | CH₃ | CH₂CHF₂ | CF₃ | Cl |
| 72. | Cl | CH₂CH₃ | CH₂CHF₂ | CF₃ | Cl |
| 73. | Cl | CH₃ | CH(CH₃)₂ | CF₃ | Cl |
| 74. | Cl | c-C₃H₅ | c-C₃H₅ | CF₃ | Cl |
| 75. | CH₃ | CH₃ | CH₃ | H | F |
| 76. | CH₃ | CH₃ | CH₂CH₃ | H | F |
| 77. | CH₃ | CH₃ | CH₂CH₃ | H | F |
| 78. | CH₃ | CH₂CH₃ | CH₂CH₃ | H | F |
| 79. | CH₃ | CH₃ | CH₂CF₃ | H | F |
| 80. | CH₃ | CH₂CH₃ | CH₂CF₃ | H | F |
| 81. | CH₃ | CH₃ | CH₂CHF₂ | H | F |
| 82. | CH₃ | CH₂CH₃ | CH₂CHF₂ | H | F |
| 83. | CH₃ | CH₃ | CH(CH₃)₂ | H | F |
| 84. | CH₃ | c-C₃H₅ | c-C₃H₅ | H | F |
| 85. | Cl | CH₃ | CH₃ | H | F |
| 86. | Cl | CH₃ | CH₂CH₃ | H | F |
| 87. | Cl | CH₂CH₃ | CH₂CH₃ | H | F |
| 88. | Cl | CH₃ | CH₂CF₃ | H | F |
| 89. | Cl | CH₂CH₃ | CH₂CF₃ | H | F |
| 90. | Cl | CH₃ | CH₂CHF₂ | H | F |
| 91. | Cl | CH₂CH₃ | CH₂CHF₂ | H | F |
| 92. | Cl | CH₃ | CH(CH₃)₂ | H | F |
| 93. | Cl | c-C₃H₅ | c-C₃H₅ | H | F |
| 94. | CH₃ | CH₃ | CH₃ | Cl | F |
| 95. | CH₃ | CH₃ | CH₂CH₃ | Cl | F |
| 96. | CH₃ | CH₃ | CH₂CH₃ | Cl | F |
| 97. | CH₃ | CH₂CH₃ | CH₂CH₃ | Cl | F |
| 98. | CH₃ | CH₃ | CH₂CF₃ | Cl | F |
| 99. | CH₃ | CH₂CH₃ | CH₂CF₃ | Cl | F |
| 100. | CH₃ | CH₃ | CH₂CHF₂ | Cl | F |
| 101. | CH₃ | CH₂CH₃ | CH₂CHF₂ | Cl | F |
| 102. | CH₃ | CH₃ | CH(CH₃)₂ | Cl | F |
| 103. | CH₃ | c-C₃H₅ | c-C₃H₅ | Cl | F |
| 104. | Cl | CH₃ | CH₃ | Cl | F |
| 105. | Cl | CH₃ | CH₂CH₃ | Cl | F |
| 106. | Cl | CH₂CH₃ | CH₂CH₃ | Cl | F |
| 107. | Cl | CH₃ | CH₂CF₃ | Cl | F |
| 108. | Cl | CH₂CH₃ | CH₂CF₃ | Cl | F |
| 109. | Cl | CH₃ | CH₂CHF₂ | Cl | F |
| 110. | Cl | CH₂CH₃ | CH₂CHF₂ | Cl | F |
| 111. | Cl | CH₃ | CH(CH₃)₂ | Cl | F |
| 112. | Cl | c-C₃H₅ | c-C₃H₅ | Cl | F |
| 113. | CH₃ | CH₃ | CH₃ | Br | F |
| 114. | CH₃ | CH₃ | CH₂CH₃ | Br | F |
| 115. | CH₃ | CH₂CH₃ | CH₂CH₃ | Br | F |
| 116. | CH₃ | CH₃ | CH₂CF₃ | Br | F |
| 117. | CH₃ | CH₂CH₃ | CH₂CF₃ | Br | F |
| 118. | CH₃ | CH₃ | CH₂CHF₂ | Br | F |
| 119. | CH₃ | CH₂CH₃ | CH₂CHF₂ | Br | F |
| 120. | CH₃ | CH₃ | CH(CH₃)₂ | Br | F |
| 121. | CH₃ | c-C₃H₅ | c-C₃H₅ | Br | F |
| 122. | Cl | CH₃ | CH₃ | Br | F |
| 123. | Cl | CH₃ | CH₂CH₃ | Br | F |
| 124. | Cl | CH₂CH₃ | CH₂CH₃ | Br | F |
| 125. | Cl | CH₃ | CH₂CF₃ | Br | F |
| 126. | Cl | CH₂CH₃ | CH₂CF₃ | Br | F |
| 127. | Cl | CH₃ | CH₂CHF₂ | Br | F |
| 128. | Cl | CH₂CH₃ | CH₂CHF₂ | Br | F |
| 129. | Cl | CH₃ | CH(CH₃)₂ | Br | F |
| 130. | Cl | c-C₃H₅ | c-C₃H₅ | Br | F |
| 131. | CH₃ | CH₃ | CH₃ | CF₃ | F |
| 132. | CH₃ | CH₃ | CH₂CH₃ | CF₃ | F |
| 133. | CH₃ | CH₂CH₃ | CH₂CH₃ | CF₃ | F |
| 134. | CH₃ | CH₃ | CH₂CF₃ | CF₃ | F |
| 135. | CH₃ | CH₂CH₃ | CH₂CF₃ | CF₃ | F |
| 136. | CH₃ | CH₃ | CH₂CHF₂ | CF₃ | F |
| 137. | CH₃ | CH₂CH₃ | CH₂CHF₂ | CF₃ | F |
| 138. | CH₃ | CH₃ | CH(CH₃)₂ | CF₃ | F |
| 139. | CH₃ | c-C₃H₅ | c-C₃H₅ | CF₃ | F |
| 140. | Cl | CH₃ | CH₃ | CF₃ | F |
| 141. | Cl | CH₃ | CH₂CH₃ | CF₃ | F |
| 142. | Cl | CH₂CH₃ | CH₂CH₃ | CF₃ | F |
| 143. | Cl | CH₃ | CH₂CF₃ | CF₃ | F |
| 144. | Cl | CH₂CH₃ | CH₂CF₃ | CF₃ | F |
| 145. | Cl | CH₃ | CH₂CHF₂ | CF₃ | F |
| 146. | Cl | CH₂CH₃ | CH₂CHF₂ | CF₃ | F |
| 147. | Cl | CH₃ | CH(CH₃)₂ | CF₃ | F |
| 148. | Cl | c-C₃H₅ | c-C₃H₅ | CF₃ | F |
wobei c-C₃H₅ für Cyclopropyl steht.
| | **R¹** | **NR^{2c}R^{2d}** | **R³** | **R³** |
|---|---|---|---|---|
| 149. | CH₃ | Pyrrolidin-1-yl | H | Cl |
| 150. | CH₃ | Piperidin-1-yl | H | Cl |
| 151. | CH₃ | Morpholin-4-yl | H | Cl |
| 152. | CH₃ | 2,6-Dimethylmorpholin-4-yl | H | Cl |
| 153. | Cl | pyrrolidin-1-yl | H | Cl |
| 154. | Cl | Piperidin-1-yl | H | Cl |
| 155. | Cl | Morpholin-4-yl | H | Cl |
| 156. | Cl | 2,6-Dimethylmorpholin-4-yl | H | Cl |
| 157. | CH₃ | Pyrrolidin-1-yl | Cl | Cl |
| 158. | CH₃ | Piperidin-1-yl | Cl | Cl |
| 159. | CH₃ | Morpholin-4-yl | Cl | Cl |
| 160. | CH₃ | 2,6-Dimethylmorpholin-4-yl | Cl | Cl |
| 161. | Cl | Pyrrolidin-1-yl | Cl | Cl |
| 162. | Cl | Piperidin-1-yl | Cl | Cl |
| 163. | Cl | Morpholin-4-yl | Cl | Cl |
| 164. | Cl | 2,6-Dimethylmorpholin-4-yl | Cl | Cl |
| 165. | CH₃ | Pyrrolidin-1-yl | Br | Cl |
| 166. | CH₃ | Piperidin-1-yl | Br | Cl |
| 167. | CH₃ | Morpholin-4-yl | Br | Cl |
| 168. | CH₃ | 2,6-Dimethylmorpholin-4-yl | Br | Cl |
| 169. | Cl | Pyrrolidin-1-yl | Br | Cl |
| 170. | Cl | Piperidin-1-yl | Br | Cl |
| 171. | Cl | Morpholin-4-yl | Br | Cl |
| 172. | Cl | 2,6-Dimethylmorpholin-4-yl | Br | Cl |
| 173. | CH₃ | Pyrrolidin-1-yl | CF₃ | Cl |
| 174. | CH₃ | Piperidin-1-yl | CF₃ | Cl |
| 175. | CH₃ | Morpholin-4-yl | CF₃ | Cl |
| 176. | CH₃ | 2,6-Dimethylmorpholin-4-yl | CF₃ | Cl |
| 177. | Cl | Pyrrolidin-1-yl | CF₃ | Cl |
| 178. | Cl | Piperidin-1-yl | CF₃ | Cl |
| 179. | Cl | Morpholin-4-yl | CF₃ | Cl |
| 180. | Cl | 2,6-Dimethylmorpholin-4-yl | CF₃ | Cl |
| 181. | CH₃ | Pyrrolidin-1-yl | H | F |
| 182. | CH₃ | Piperidin-1-yl | H | F |
| 183. | CH₃ | Morpholin-4-yl | H | F |
| 184. | CH₃ | 2,6-Dimethylmorpholin-4-yl | H | F |
| 185. | Cl | Pyrrolidin-1-yl | H | F |
| 186. | Cl | Piperidin-1-yl | H | F |
| 187. | Cl | Morpholin-4-yl | H | F |
| 188. | Cl | 2,6-Dimethylmorpholin-4-yl | H | F |
| 189. | CH₃ | Pyrrolidin-1-yl | Cl | F |
| 190. | CH₃ | Piperidin-1-yl | Cl | F |
| 191. | CH₃ | Morpholin-4-yl | Cl | F |
| 192. | CH₃ | 2,6-Dimethylmorpholin-4-yl | Cl | F |
| 193. | Cl | Pyrrolidin-1-yl | Cl | F |
| 194. | Cl | Piperidin-1-yl | Cl | F |
| 195. | Cl | M orpholin-4-yl | Cl | F |
| 196. | Cl | 2,6-Dimethylmorpholin-4-yl | Cl | F |
| 197. | CH₃ | Pyrrolidin-1-yl | Br | F |
| 198. | CH₃ | Piperidin-1-yl | Br | F |
| 199. | CH₃ | Morpholin-4-yl | Br | F |
| 200. | CH₃ | 2,6-Dimethylmorpholin-4-yl | Br | F |
| 201. | Cl | Pyrrolidin-1-yl | Br | F |
| 202. | Cl | Piperidin-1-yl | Br | F |
| 203. | Cl | Morpholin-4-yl | Br | F |
| 204. | Cl | 2,6-Dimethylmorpholin-4-yl | Br | F |
| 205. | CH₃ | Pyrrolidin-1-yl | CF₃ | F |
| 206. | CH₃ | Piperidin-1-yl | CF₃ | F |
| 207. | CH₃ | Morpholin-4-yl | CF₃ | F |
| 208. | CH₃ | 2,6-Dimethylmorpholin-4-yl | CF₃ | F |
| 209. | Cl | Pyrrolidin-1-yl | CF₃ | F |
| 210. | Cl | Piperidin-1-yl | CF₃ | F |
| 211. | Cl | Morpholin-4-yl | CF₃ | F |
| 212. | Cl | 2,6-Dimethylmorpholin-4-yl | CF₃ | F |

13. Zusammensetzung, umfassend mindestens eine Verbindung nach einem der Ansprüche 1 bis 12 oder ein landwirtschaftlich geeignetes Salz davon und mindestens einen für die Formulierung von Plfanzenschutzverbindungen üblichen Hilfsstoff.

14. Verwendung einer Verbindung nach einem der Ansprüche 1 bis 12 oder eines landwirtschaftlich geeigneten Salzes davon oder der Zusammensetzung nach Anspruch 13 zur Bekämpfung von unerwünschtem Pflanzenwuchs.

15. Verfahren zur Bekämpfung von unerwünschtem Pflanzenwuchs, bei dem man eine herbizid wirksame Menge mindestens einer Verbindung nach einem der Ansprüche 1 bis 12 oder eines landwirtschaftlich geeigneten Salzes davon oder der Zusammensetzung nach Anspruch 13 auf Pflanzen, ihr Saatgut und/oder ihren Lebensraum einwirken lässt.

## Revendications

1. Composé de formule I, 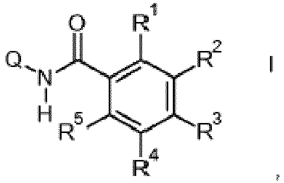
Q étant Q¹ ou Q² ou Q³ ou Q⁴, 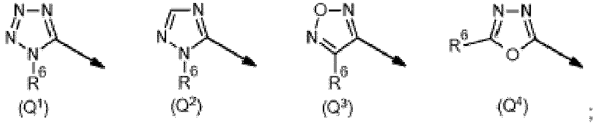
R¹ étant choisi dans le groupe constitué par cyano, halogène, nitro, C₁-C₈-alkyle, C₁-C₈-halogénoalkyle, C₁-C₄-alcoxy-C₁-C₄-alkyle, C₁-C₄-halogénoalcoxy-C₁-C₄-alkyle, C₁-C₈-alcoxy, C₁-C₄-alcoxy-C₁-C₄-alcoxy-Z¹, C₁-C₆-halogénoalcoxy, R^{1b}-S(O)ₖ-Z¹ ;
R² étant R^{2c}R^{2d}NC(O)NR²ⁿ-Z² ;
R³ étant choisi dans le groupe constitué par hydrogène, cyano, thiocyanato, halogène, hydroxy-Z², C₁-C₆-alkyle, C₂-C₈-alcényle, C₂-C₈-alcynyle, C₃-C₁₀-cycloalkyl-Z², C₃-C₆-cycloalcényl-Z², C₃-C₁₀-cycloalcoxy-Z², C₃-C₁₀-cycloalkyl-C₁-C₂-alcoxy, où les groupes cycliques des quatre radicaux mentionnés en dernier sont non substitués ou partiellement ou complètement halogénés, C₁-C₄-cyanoalkyle, C₁-C₈-halogénoalkyle, C₂-C₈-halogénoalcényle, C₃-C₈-halogénoalcynyle, C₁-C₈-alcoxy-Z², C₁-C₈-halogénoalcoxy-Z², C₁-C₄-alcoxy-C₁-C₄-alcoxy-Z², C₁-C₄-halogénoalcoxy-C₁-C₄-alcoxy-Z², C₂-C₈-alcényloxy-Z², C₂-C₈-alcynyloxy-Z², C₂-C₈-halogénoalcényloxy-Z², C₃-C₈-halogénoalcynyloxy-Z², R^{2b}-S(O)ₖ-Z², R^{2c}-C(O)-Z², R^{2d}O-C(O)-Z², R^{2d}O-N=CH-Z², R^{2e}R^{2f}N-C(O)-Z², R^{2g}R^{2h}N-Z², R²²C(O)O-Z², R²⁵OC(O)O-Z², (R²²)₂NC(O)O-Z², R²⁵S(O)₂O-Z², R²²OS(O)₂-Z², (R²²)₂NS(O)₂-Z², R²⁵OC(O)N(R²²)-Z², (R²²)₂NC(O)N(R²²)-Z², (R²²)₂NS(O)₂N(R²²)-Z², (OH)₂P(O)-Z², (C₁-C₄-alcoxy)₂P(O)-Z², phényl-Z^{2a}, hétérocyclyl-Z^{2a}, où hétérocyclyle est un hétérocycle monocyclique à 3, 4, 5 ou 6 chaînons ou bicyclique à 8, 9 ou 10 chaînons, saturé, partiellement insaturé ou aromatique, qui contient 1, 2, 3 ou 4 hétéroatomes en tant qu'éléments de cycle, qui sont choisis dans le groupe constitué par O, N et S, où des groupes cycliques dans phényl-Z^{2a} et hétérocyclyl-Z^{2a} sont non substitués ou substitués par 1, 2, 3 ou 4 groupes R²¹, qui sont identiques ou différents ;
R⁴ étant choisi dans le groupe constitué par halogène, C₁-C₈-alkyle, cyano-Z¹, nitro, C₃-C₇-cycloalkyle, C₃-C₇-cycloalkyl-C₁-C₄-alkyle, où les groupes C₃-C₇-cycloalkyle dans les deux radicaux mentionnés en dernier sont non substitués ou partiellement ou complètement halogénés, C₂-C₈-alcényle, C₂-C₈-alcynyle, C₁-C₈-halogénoalkyle, C₁-C₈-alkylamino, C₁-C₃-dialkylamino, C₁-C₃-alkylamino-S(O)ₖ, C₁-C₃-alkylcarbonyle, C₁-C₈-alcoxy, C₁-C₄-alcoxy-C₁-C₄-alkyle, C₁-C₄-alcoxy-C₁-C₄-alcoxy-Z¹, C₁-C₄-alkylthio-C₁-C₄-alkyle, C₁-C₄-alkylthio-C₁-C₄-alkylthio-Z¹, C₂-C₆-alcényloxy, C₂-C₆-alcynyloxy, C₁-C₆-halogénoalcoxy, C₁-C₄-halogénoalcoxy-C₁-C₄-alkyle, C₁-C₄-halogénoalcoxy-C₁-C₄-alcoxy-Z¹, R^{1b}-S(O)ₖ-Z¹, phénoxy-Z¹ et hétérocyclyloxy-Z¹, où hétérocyclyloxy est un hétérocycle lié par oxygène, monocyclique à 5 ou 6 chaînons ou bicyclique à 8, 9 ou 10 chaînons, saturé, partiellement insaturé ou aromatique, qui contient 1, 2, 3 ou 4 hétéroatomes en tant qu'éléments de cycle, qui sont choisis dans le groupe constitué par O, N et S, où les groupes cycliques dans phénoxy et hétérocyclyloxy sont non substitués ou substitués par 1, 2, 3 ou 4 groupes R¹¹, qui sont identiques ou différents ;
R⁵ étant hydrogène ;
R⁶ étant choisi dans le groupe constitué par cyano, C₁-C₆-alkyle, C₃-C₇-cycloalkyle, C₃-C₇-cycloalkyl-C₁-C₄-alkyle, où les groupes C₃-C₇-cycloalkyle des deux radicaux mentionnés en dernier sont non substitués ou partiellement ou complètement halogénés, C₁-C₆-halogénoalkyle, C₂-C₆-alcényle, C₂-C₆-alcynyle, C₂-C₆-halogénoalcényle, C₃-C₆-halogénoalcynyle, C₁-C₄-alcoxy-C₁-C₄-alkyle, C₁-C₄-halogénoalcoxy-C₁-C₄-alkyle, R^{b}-S(O)ₙ-C₁-C₃-alkyle, phényle et benzyle, où phényle et benzyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy ;
R²¹ étant choisi dans le groupe constitué par cyano, halogène, nitro, C₁-C₆-alkyle, C₃-C₇-cycloalkyle, C₃-C₇-halocycloalkyle, C₁-C₆-halogénoalkyle, C₂-C₆-alcényle, C₂-C₆-halogénoalcényle, C₂-C₆-alcynyle, C₃-C₆-halogénoalcynyle, C₁-C₆-alcoxy, C₁-C₄-alcoxy-C₁-C₄-alkyle, C₁-C₄-alkylthio-C₁-C₄-alkyle, C₁-C₄-halogénoalcoxy-C₁-C₄-alkyle, C₁-C₄-alcoxy-C₁-C₄-alcoxy, C₃-C₇-cycloalcoxy et C₁-C₆-halogénoalcoxy, ou deux radicaux R²¹ liés au même atome de carbone pouvant former ensemble un groupe =O ;
Z¹, Z² indépendamment l'un de l'autre sont choisis dans le groupe constitué par une liaison covalente et C₁-C₄-alcanediyle ;
Z^{2a} étant choisi dans le groupe constitué par une liaison covalente, C₁-C₄-alcanediyle, O-C₁-C₄-alcanediyle, C₁-C₄-alcanediyl-O et C₁-C₄-alcanediyl-O-C₁-C₄-alcanediyle ;
R^{b}, R^{1b}, R^{2b} indépendamment les uns des autres étant choisis dans le groupe constitué par C₁-C₆-alkyle, C₃-C₇-cycloalkyle, C₁-C₆-halogénoalkyle, C₂-C₆-alcényle, C₂-C₆-halogénoalcényle, C₂-C₆-alcynyle, C₃-C₆-halogénoalcynyle, phényle et hétérocyclyle, où hétérocyclyle est un hétérocycle monocyclique à 5 ou 6 chaînons, saturé, partiellement insaturé ou aromatique, qui contient 1, 2, 3 ou 4 hétéroatomes en tant qu'éléments de cycle, qui sont choisis dans le groupe constitué par O, N et S, où phényle et hétérocyclyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy ;
R^{2c} étant choisi dans le groupe constitué par hydrogène, C₁-C₆-alkyle, C₃-C₇-cycloalkyle, C₃-C₇-cycloalkyl-C₁-C₄-alkyle, où les groupes C₃-C₇-cycloalkyle dans les deux radicaux mentionnés en dernier sont non substitués ou partiellement ou complètement halogénés, C₁-C₆-halogénoalkyle, C₁-C₆-alcoxy, C₂-C₆-alcényle, C₁-C₄-alkyl-C₂-C₆-alcényle, C₂-C₆-halogénoalcényle, C₂-C₆-alcynyle, C₃-C₆-halogénoalcynyle, C₁-C₄-alcoxy-C₁-C₄-alkyle, C₁-C₄-alkyl-S(O)ₙ-C₁-C₄-alkyle, C₁-C₄-alkylamino-C₁-C₄-alkyle, C₁-C₄-dialkylamino-C₁-C₄-alkyle, C₁-C₆-cyanoalkyle, phényle, benzyle et hétérocyclyle, où hétérocyclyle est un hétérocycle monocyclique à 5 ou 6 chaînons, saturé, partiellement insaturé ou aromatique, qui contient 1, 2, 3 ou 4 hétéroatomes en tant qu'éléments de cycle, qui sont choisis dans le groupe constitué par O, N et S, où phényle, benzyle et hétérocyclyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy ;
R^{2d} étant choisi dans le groupe constitué par hydrogène, C₁-C₆-alkyle, C₃-C₇-cycloalkyle, C₃-C₇-cycloalkyl-C₁-C₄-alkyle, où les groupes C₃-C₇-cycloalkyle dans les deux radicaux mentionnés en dernier sont non substitués ou partiellement ou complètement halogénés, C₁-C₆-halogénoalkyle, C₁-C₆-alcoxy, C₂-C₆-alcényle, C₁-C₄-alkyl-C₂-C₆-alcényle, C₂-C₆-halogénoalcényle, C₂-C₆-alcynyle, C₃-C₆-halogénoalcynyle, C₁-C₄-alcoxy-C₁-C₄-alkyle, C₁-C₄-alkyl-S(O)ₙ-C₁-C₄-alkyle, C₁-C₄-alkylamino-C₁-C₄-alkyle, C₁-C₄-dialkylamino-C₁-C₄-alkyle, C₁-C₆-cyanoalkyle, phényle et benzyle, où phényle et benzyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy ; ou
R^{2c}, R^{2d} conjointement avec l'atome d'azote, auquel ils sont liés, pouvant former un radical hétérocyclique à 4, 5, 6 ou 7 chaînons, saturé ou insaturé, qui peut porter en tant qu'élément de cycle un hétéroatome supplémentaire choisi parmi O, S et N et qui est non substitué ou peut porter 1, 2, 3 ou 4 groupes, qui sont identiques ou différents et choisis dans le groupe constitué par =O, halogène, C₁-C₄-alkyle, C₁-C₄-halogénoalkyle, C₁-C₄-alcoxy et C₁-C₄-halogénoalcoxy ;
R²ⁿ étant choisi dans le groupe constitué par hydrogène, C₁-C₆-alkyle, C₃-C₇-cycloalkyle, C₃-C₇-cycloalkyl-C₁-C₄-alkyle, où les groupes C₃-C₇-cycloalkyle dans les deux radicaux mentionnés en dernier sont non substitués ou partiellement ou complètement halogénés, C₁-C₆-cyanoalkyle, C₁-C₆-halogénoalkyle, C₂-C₆-alcényle, C₂-C₆-halogénoalcényle, C₂-C₆-alcynyle, C₁-C₄-alcoxy-C₁-C₄-alkyle ;
R^{2e}, R^{2f} indépendamment les uns des autres étant choisis dans le groupe constitué par hydrogène, C₁-C₆-alkyle, C₃-C₇-cycloalkyle, C₃-C₇-cycloalkyl-C₁-C₄-alkyle, où les groupes C₃-C₇-cycloalkyle dans les deux radicaux mentionnés en dernier sont non substitués ou partiellement ou complètement halogénés, C₁-C₆-halogénoalkyle, C₂-C₆-alcényle, C₂-C₆-halogénoalcényle, C₂-C₆-alcynyle, C₃-C₆-halogénoalcynyle, C₁-C₄-alcoxy-C₁-C₄-alkyle, phényle et benzyle, où phényle et benzyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy ; ou
R^{2e}, R^{2f} conjointement avec l'atome d'azote, auquel ils sont liés, pouvant former un radical hétérocyclique à 4, 5, 6 ou 7 chaînons, saturé ou insaturé, qui peut porter en tant qu'élément de cycle un hétéroatome supplémentaire choisi parmi O, S et N et qui est non substitué ou peut porter 1, 2, 3 ou 4 groupes, qui sont identiques ou différents et choisis dans le groupe constitué par =O, halogène, C₁-C₄-alkyle, C₁-C₄-halogénoalkyle, C₁-C₄-alcoxy et C₁-C₄-halogénoalcoxy ;
R^{2g} étant choisi dans le groupe constitué par hydrogène, C₁-C₆-alkyle, C₃-C₇-cycloalkyle, C₃-C₇-cycloalkyl-C₁-C₄-alkyle, où les groupes C₃-C₇-cycloalkyle dans les deux radicaux mentionnés en dernier sont non substitués ou partiellement ou complètement halogénés, C₁-C₆-halogénoalkyle, C₂-C₆-alcényle, C₂-C₆-halogénoalcényle, C₂-C₆-alcynyle, C₃-C₆-halogénoalcynyle, C₁-C₄-alcoxy-C₁-C₄-alkyle, C₁-C₄-alkylsulfonyle, C₁-C₄-alkylcarbonyle, phényle et benzyle, où phényle et benzyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy ;
R^{2h} étant choisi dans le groupe constitué par hydrogène, C₁-C₆-alkyle, C₃-C₇-cycloalkyle, C₃-C₇-cycloalkyl-C₁-C₄-alkyle, où les groupes C₃-C₇-cycloalkyle dans les deux radicaux mentionnés en dernier sont non substitués ou partiellement ou complètement halogénés, C₁-C₆-halogénoalkyle, C₂-C₆-alcényle, C₂-C₆-halogénoalcényle, C₂-C₆-alcynyle, C₃-C₆-halogénoalcynyle, C₁-C₄-alcoxy-C₁-C₄-alkyle, C₁-C₄-alkylsulfonyle, C₁-C₄-alkylcarbonyle, un radical C(O)R^{k}, phényle et benzyle, où phényle et benzyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy ; ou
R^{2g}, R^{2h} conjointement avec l'atome d'azote, auquel ils sont liés, pouvant former un radical hétérocyclique à 4, 5, 6 ou 7 chaînons, saturé ou insaturé, qui peut porter en tant qu'élément de cycle un hétéroatome supplémentaire choisi parmi O, S et N et qui est non substitué ou peut porter 1, 2, 3 ou 4 groupes, qui sont identiques ou différents et choisis dans le groupe constitué par =O, halogène, C₁-C₄-alkyle, C₁-C₄-halogénoalkyle, C₁-C₄-alcoxy et C₁-C₄-halogénoalcoxy ;
R²² étant choisi dans le groupe constitué par hydrogène, C₁-C₆-alkyle, C₁-C₆-halogénoalkyle, C₂-C₆-alcényle, C₂-C₆-halogénoalcényle, C₂-C₆-alcynyle, C₃-C₆-halogénoalcynyle, C₃-C₆-cycloalkyle, C₃-C₆-cycloalcényle, C₃-C₆-halocycloalkyle, C₃-C₆-cycloalkyl-C₁-C₆-alkyle, C₁-C₆-alcoxy-C₁-C₆-alkyle, C₃-C₆-cycloalkyl-C₁-C₆-alcoxy-C₁-C₆-alkyle, phényl-Z¹, phényl-O-C₁-C₆-alkyle, phényl-N(R²³) -C₁-C₆-alkyle, phényl-S(O)ₙ-C₁-C₆-alkyle, hétérocyclyl-Z¹, hétérocyclyl-N(R²³) -C₁-C₆-alkyle, hétérocyclyl-O-C₁-C₆-alkyle, hétérocyclyl-S(O)ₙ-C₁-C₆-alkyle, où hétérocyclyle est un hétérocycle monocyclique à 5 ou 6 chaînons, saturé, partiellement insaturé ou aromatique, qui contient 1, 2, 3 ou 4 hétéroatomes en tant qu'éléments de cycle, qui sont choisis dans le groupe constitué par O, N et S, où phényle, hétérocyclyle sont non substitués ou substitués par 1, 2, 3 ou 4 groupes, qui sont identiques ou différents et choisis dans le groupe constitué par cyano, halogène, nitro, thiocyanato, C₁-C₆-alkyle, C₁-C₆-halogénoalkyle, C₃-C₆-cycloalkyle, C(O)OR²³, C(O)N(R²³)₂, OR²³, N(R²³)₂, S(O)ₙR²⁴, S(O)₂OR²³, S(O)₂N(R²³)₂, et R²³O-C₁-C₆-alkyle, et où hétérocyclyle porte 0, 1 ou 2 groupes oxo ;
R²³ étant choisi dans le groupe constitué par hydrogène, C₁-C₆-alkyle, C₁-C₆-halogénoalkyle, C₂-C₆-alcényle, C₂-C₆-alcynyle, C₃-C₆-cycloalkyle, C₃-C₆-cycloalkyl-C₁-C₆-alkyle, et phényle ;
R²⁴ étant choisi dans le groupe constitué par C₁-C₆-alkyle, C₁-C₆-halogénoalkyle, C₂-C₆-alcényle, C₂-C₆-alcynyle, C₃-C₆-cycloalkyle, C₃-C₆-cycloalkyl-C₁-C₆-alkyle, et phényle ;
R²⁵ étant choisi dans le groupe constitué par C₁-C₆-alkyle, C₁-C₆-halogénoalkyle, C₂-C₆-alcényle, C₂-C₆-halogénoalcényle, C₂-C₆-alcynyle, C₃-C₆-halogénoalcynyle, C₃-C₆-cycloalkyle, C₃-C₆-cycloalcényle, C₃-C₆-halocycloalkyle, C₃-C₆-cycloalkyl-C₁-C₆-alkyle, C₁-C₆-alcoxy-C₁-C₆-alkyle, C₃-C₆-cycloalkyl-C₁-C₆-alcoxy-C₁-C₆-alkyle, phényl-Z¹, phényl-O-C₁-C₆-alkyle, phényl-N (R²³)-C₁-C₆-alkyle, phényl-S(O)ₙ-C₁-C₆-alkyle, hétérocyclyl-Z¹, hétérocyclyl-N(R²³)-C₁-C₆-alkyle, hétérocyclyl-O-C₁-C₆-alkyle, hétérocyclyl-S(O)ₙ-C₁-C₆-alkyle, où hétérocyclyle est un hétérocycle monocyclique à 5 ou 6 chaînons, saturé, partiellement insaturé ou aromatique, qui contient 1, 2, 3 ou 4 hétéroatomes en tant qu'éléments de cycle, qui sont choisis dans le groupe constitué par O, N et S, où phényle, hétérocyclyle sont non substitués ou substitués par 1, 2, 3 ou 4 groupes, qui sont identiques ou différents et choisis dans le groupe constitué par cyano, halogène, nitro, thiocyanato, C₁-C₆-alkyle, C₁-C₆-halogénoalkyle, C₃-C₆-cycloalkyle, C(O)OR²³, C(O)N(R²³)₂, OR²³, N(R²³)₂, S(O)ₙR²⁴, S(O)₂OR²³, S(O)₂N(R²³)₂, et R²³O-C₁-C₆-alkyle, et où hétérocyclyle porte 0, 1 ou 2 groupes oxo ;
k étant 0, 1 ou 2 ;
n étant 0, 1 ou 2 ;
R^{k} possédant les significations de R^{2c} ;
ou sel approprié sur le plan agricole correspondant.

2. Composé selon la revendication 1, ou sel approprié sur le plan agricole correspondant, dans lequel R¹ est choisi dans le groupe constitué par halogène, nitro, C₁-C₄-alkyle, C₁-C₄-alcoxy-C₁-C₄-alcoxy-Z¹, R^{1b}-S(O)ₖ-Z¹, dans lequel en particulier R¹ est choisi dans le groupe constitué par halogène et C₁-C₄-alkyle.

3. Composé selon l'une quelconque des revendications 1 et 2, ou sel approprié sur le plan agricole correspondant, où R² est R^{2c}R^{2d}NC(O)NR²ⁿ-Z², où R²ⁿ est choisi dans le groupe constitué par hydrogène et C₁-C₆-alkyle.

4. Composé selon l'une quelconque des revendications 1 à 3, ou sel approprié sur le plan agricole correspondant, où Z² dans R² est une liaison covalente et R^{2c} et R^{2d} indépendamment les uns des autres sont choisis dans le groupe constitué par C₁-C₆-alkyle, C₃-C₇-cycloalkyle, C₃-C₇-cycloalkyl-C₁-C₄-alkyle, C₁-C₆-halogénoalkyle, phényle, et hétérocyclyle, où hétérocyclyle est un hétérocycle monocyclique à 5 ou 6 chaînons, saturé, partiellement insaturé ou aromatique, qui contient 1, 2, 3 ou 4 hétéroatomes en tant qu'éléments de cycle, qui sont choisis dans le groupe constitué par O, N et S, où phényle et hétérocyclyle sont non substitués ou substitués par 1, 2, 3 ou 4 groupes, qui sont identiques ou différents et choisis dans le groupe constitué par halogène, C₁-C₄-alkyle, C₁-C₄-halogénoalkyle, C₁-C₄-alcoxy et C₁-C₄-halogénoalcoxy.

5. Composé selon la revendication 4, ou sel approprié sur le plan agricole correspondant, où Z² dans R² est une liaison covalente, R^{2c} est choisi dans le groupe constitué par C₁-C₄-alkyle, C₃-C₇-cycloalkyle et C₁-C₄-halogénoalkyle et R^{2d} est choisi dans le groupe constitué par C₁-C₆-alkyle, C₃-C₇-cycloalkyle, C₃-C₇-cycloalkyl-C₁-C₂-alkyle et C₁-C₆-halogénoalkyle.

6. Composé selon l'une quelconque des revendications 1 à 3, ou sel approprié sur le plan agricole correspondant, où Z² dans R² est une liaison covalente et R^{2c} et R^{2d} conjointement avec l'atome d'azote auquel ils sont liés peuvent former un radical hétérocyclique à 4, 5, 6 ou 7 chaînons, saturé ou insaturé, qui peut porter en tant qu'élément de cycle un hétéroatome supplémentaire choisi parmi O, S et N et qui est non substitué ou peut porter 1, 2, 3 ou 4 groupes, qui sont identiques ou différents et choisis dans le groupe constitué par =O, halogène, C₁-C₄-alkyle, C₁-C₄-halogénoalkyle, C₁-C₄-alcoxy et C₁-C₄-halogénoalcoxy.

7. Composé selon l'une quelconque des revendications 1 à 6, ou sel approprié sur le plan agricole correspondant, où R³ est choisi dans le groupe constitué par hydrogène, halogène, C₁-C₄-alkyle, C₁-C₄-halogénoalkyle, C₁-C₄-halogénoalcoxy, et R^{2b}-S(O)ₖ ; dans lequel en particulier R³ est choisi dans le groupe constitué par hydrogène, halogène et C₁-C₂-halogénoalkyle.

8. Composé selon l'une quelconque des revendications 1 à 7, ou sel approprié sur le plan agricole correspondant, où R⁴ est choisi dans le groupe constitué par cyano, halogène, nitro, C₁-C₂-alkyle, et C₁-C₂-halogénoalkyle.

9. Composé selon la revendication 8, ou sel approprié sur le plan agricole correspondant, dans lequel R⁴ est choisi dans le groupe constitué par fluor et chlore.

10. Composé selon l'une quelconque des revendications 1 à 9, ou sel approprié sur le plan agricole correspondant, dans lequel R⁶ est choisi dans le groupe constitué par C₁-C₆-alkyle, C₁-C₄-alcoxy-C₁-C₄-alkyle, et phényle.

11. Composé selon la revendication 1, ou sel approprié sur le plan agricole correspondant, qui est choisi parmi les composés des formules suivantes I.A.I et I.D.I, 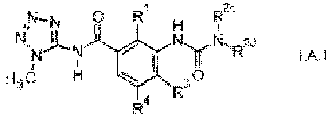 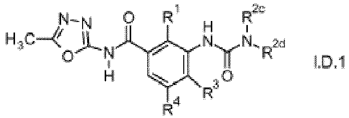 où
R¹ est choisi dans le groupe constitué par halogène et C₁-C₄-alkyle,
R^{2c} est choisi dans le groupe constitué par C₁-C₄-alkyle, C₃-C₇-cycloalkyle et C₁-C₄- halogénoalkyle,
R^{2d} est choisi dans le groupe constitué par C₁-C₆-alkyle, C₃-C₇-cycloalkyle, C₃-C₇-cycloalkyl-C₁-C₂-alkyle et C₁-C₆-halogénoalkyle,
R³ est choisi dans le groupe constitué par hydrogène, halogène et C₁-C₂-halogénoalkyle.
R⁴ est choisi dans le groupe constitué par fluor et chlore.

12. Composé selon la revendication 11, ou sel approprié sur le plan agricole correspondant, dans lequel R¹, R^{2c}, R^{2d}, R³ et R⁴ sont tels que définis dans les tableaux suivants :
| | **R¹** | **R^{2c}** | **R^{2d}** | **R³** | **R⁴** |
|---|---|---|---|---|---|
| 1. | CH₃ | CH₃ | CH₃ | H | Cl |
| 2. | CH₃ | CH₃ | CH₂CH₃ | H | Cl |
| 3. | CH₃ | CH₃ | CH₂CH₅ | H | Cl |
| 4. | CH₃ | CH₂CH₃ | CH₂CH₃ | H | Cl |
| 5. | CH₃ | CH₃ | CH₂CF₃ | H | Cl |
| 6. | CH₃ | CH₂CH₃, | CH₂CF₃ | H | Cl |
| 7. | CH₃ | CH₃ | CH₂CHF₂ | H | Cl |
| 8. | CH₃ | CH₂CH₃ | CH₂CHF₂ | H | Cl |
| 9. | CH₃ | CH₃ | CH(CH₃)₂ | H | Cl |
| 10. | CH₃ | c-C₃H₅ | c-C₃H₅ | H | Cl |
| 11. | Cl | CH₃ | CH₃ | H | Cl |
| 12. | Cl | CH₃ | CH₂CH₃ | H | Cl |
| 13. | Cl | CH₂CH₃ | CH₂CH₃ | H | Cl |
| 14. | Cl | CH₃ | CH₂CF₃ | H | Cl |
| 15. | Cl | CH₂CH₃ | CH₂CF₃ | H | Cl |
| 16. | Cl | CH₃ | CH₂CHF₂ | H | Cl |
| 17. | Cl | CH₂CH₃ | CH₂CHF₂ | H | Cl |
| 18. | Cl | CH₃ | CH(CH₃)₂ | H | Cl |
| 19. | Cl | c-C₃H₅ | c-C₃H₅ | H | Cl |
| 20. | CH₃ | CH₃ | CH₃ | Cl | Cl |
| 21. | CH₃ | CH₃ | CH₂CH₃ | Cl | Cl |
| 22. | CH₃ | CH₃ | CH₂CH₃ | Cl | Cl |
| 23. | CH₃ | CH₂CH₃ | CH₂CH₃ | Cl | Cl |
| 24. | CH₃ | CH₃ | CH₂CF₃ | Cl | Cl |
| 25. | CH₃ | CH₂CH₃ | CH₂CF₃ | Cl | Cl |
| 26. | CH₃ | CH₃ | CH₂CHF₂ | Cl | Cl |
| 27. | CH₃ | CH₂CH₃ | CH₂CHF₂ | Cl | Cl |
| 28. | CH₃ | CH₃ | CH(CH₃)₂ | Cl | Cl |
| 29. | CH₃ | c-C₃H₅ | c-C₃H₅ | Cl | Cl |
| 30. | Cl | CH₃ | CH₃ | Cl | Cl |
| 31. | Cl | CH₃ | CH₂CH₃ | Cl | Cl |
| 32. | Cl | CH₂CH₃ | CH₂CH₃ | Cl | Cl |
| 33. | Cl | CH₃ | CH₂CF₃ | Cl | Cl |
| 34. | Cl | CH₂CH₃ | CH₂CF₃ | Cl | Cl |
| 35. | Cl | CH₃ | CH₂CHF₂ | Cl | Cl |
| 36. | Cl | CH₂CH₃ | CH₂CHF₂ | Cl | Cl |
| 37. | Cl | CH₃ | CH(CH₃)₂ | Cl | Cl |
| 38. | Cl | c-C₃H₅ | c-C₃-H₅ | Cl | Cl |
| 39. | CH₃ | CH₃ | CH₃ | Br | Cl |
| 40. | CH₃ | CH₃ | CH₂CH₃ | Br | Cl |
| 41. | CH₃ | CH₂CH₃ | CH₂CH₃ | Br | Cl |
| 42. | CH₃ | CH₃ | CH₂CF₃ | Br | Cl |
| 43. | CH₃ | CH₂CH₃ | CH₂CF₃ | Br | Cl |
| 44. | CH₃ | CH₃ | CH₂CHF₂ | Br | Cl |
| 45. | CH₃ | CH₂CH₃ | CH₂CH₂ | Br | Cl |
| 46. | CH₃ | CH₃ | CH(CH₃)₂ | Br | Cl |
| 47. | CH₃ | c-C₃H₅ | c-C₃H₅ | Br | Cl |
| 48. | Cl | CH₃ | CH₃ | Br | Cl |
| 49. | Cl | CH₃ | CH₂CH₃ | Br | Cl |
| 50. | Cl | CH₂CH₃ | CH₂CH₃ | Br | Cl |
| 51. | Cl | CH₃ | CH₂CF₃ | Br | Cl |
| 52. | Cl | CH₂CH₃ | CH₂CF₃ | Br | Cl |
| 53. | Cl | CH₃ | CH₂CHF₂ | Br | Cl |
| 54. | Cl | CH₂CH₃ | CH₂CHF₂ | Br | Cl |
| 55. | Cl | CH₃ | CH(CH₃)₂ | Br | Cl |
| 56. | Cl | c-C₃H₅ | C-C₃H₅ | Br | Cl |
| 57. | CH₃ | CH₃ | CH₃ | CF₃ | Cl |
| 58. | CH₃ | CH₃ | CH₂CH₃ | CF₃ | Cl |
| 59. | CH₃ | CH₂CH₃ | CH₂CH₃ | CF₃ | Cl |
| 60. | CH₃ | CH₃ | CH₂CF₃ | CF₃ | Cl |
| 61. | CH₃ | CH₂CH₃ | CH₂CF₃ | CF₃ | Cl |
| 62. | CH₃ | CH₃ | CH₂CHF₂ | CF₃ | Cl |
| 63. | CH₃ | CH₂CH₃ | CH₂CHF₂, | CF₃ | Cl |
| 64. | CH₃ | CH₃ | CH(CH₃)₂ | CF₃ | Cl |
| 65. | CH₃ | c-C₃H₅ | c-C₃H₅ | CF₃ | Cl |
| 66. | Cl | CH₃ | CH₃ | CF₃ | Cl |
| 67. | Cl | CH₃ | CH₂CH₃ | CF₃ | Cl |
| 68. | Cl | CH₂CH₃ | CH₂CH₃ | CF₃ | Cl |
| 69. | Cl | CH₃ | CH₂CF₃ | CF₃ | Cl |
| 70. | Cl | CH₂CH₃ | C H₂CF₃ | CF₃ | Cl |
| 71. | Cl | CH₃ | CH₂CHF₂ | CF₃ | Cl |
| 72. | Cl | CH₂CH₃ | CH₂CHF₂ | CF₃ | Cl |
| 73. | Cl | CH₃ | CH (CH₃)₂ | CF₃ | Cl |
| 74. | Cl | c-C₃H₅, | c-C₃H₅ | CF₃ | Cl |
| 75. | CH₃ | CH₃ | CH₃ | H | F |
| 76. | CH₃ | CH₃ | CH₂CH₃ | H | F |
| 77. | CH₃ | CH₃ | CH₂CH₃ | H | F |
| 78. | CH₃ | CH₂CH₃ | CH₂CH₃ | H | F |
| 79. | CH₃ | CH₃ | CH₂CF₃ | H | F |
| 80. | CH₃ | CH₂CH₃ | CH₂CF₃ | H | F |
| 81. | CH₃ | CH₃ | CH₂CHF₂ | H | F |
| 82. | CH₃ | CH₂CH₃ | CH₂CHF₂ | H | F |
| 83. | CH₃ | CH₃ | CH(CH₃)₂ | H | F |
| 84. | CH₃ | c-C₃H₅ | c-C₃H₅ | H | F |
| 85. | Cl | CH₃ | CH₃ | H | F |
| 86. | Cl | CH₃ | CH₂CH₃ | H | F |
| 87. | Cl | CH₂CH₃ | CH₂CH₃ | H | F |
| 88. | Cl | CH₃ | CH₂CF₃ | H | F |
| 89. | Cl | CH₂CH₃ | CH₂CF₃ | H | F |
| 90. | Cl | CH₃ | CH₂CHF₂ | H | F |
| 91. | Cl | CH₂CH₃ | CH₂CHF₂ | H | F |
| 92. | Cl | CH₃ | CH(CH₃)₂ | H | F |
| 93. | Cl | c-C₃H₅ | c-C₃H₅ | H | F |
| 94. | CH₃ | CH₃ | CH₃ | Cl | F |
| 95. | CH₃ | CH₃ | CH₂CH₃ | Cl | F |
| 96. | CH₃ | CH₃ | CH₂CH₃ | Cl | F |
| 97. | CH₃ | CH₂CH₄ | CH₂CH₃ | Cl | F |
| 98. | CH₃ | CH₃ | CH₂CF₃ | Cl | F |
| 99. | CH₃ | CH₂CH₃ | CH₂CF₃ | Cl | F |
| 100. | CH₃ | CH₃ | CH₂CHF₂ | Cl | F |
| 101. | CH₃ | CH₂CH₃ | CH₂CHF₂ | Cl | F |
| 102. | CH₃ | CH₃ | CH(CH₃)₂ | Cl | F |
| 103. | CH₃ | c-C₃H₅ | c-C₃-H₅ | Cl | F |
| 104. | Cl | CH₃ | CH₃ | Cl | F |
| 105. | Cl | CH₃ | CH₂CH₃. | Cl | F |
| 106. | Cl | CH₂CH₃ | CH₂CH₃ | Cl | F |
| 107. | Cl | CH₃ | CH₂CF₃ | Cl | F |
| 108. | Cl | CH₂CH₃, | CH₂CF₃ | Cl | F |
| 109. | Cl | CH₃ | CH₂CHF₂ | Cl | F |
| 110. | Cl | CH₂CH₃. | CH₂CHF₂ | Cl | F |
| 111. | Cl | CH₃ | CH(CH₃)₂ | Cl | F |
| 112. | Cl | c-C₃H₅ | c-C₃H₅ | Cl | F |
| 113. | CH₃ | CH₃ | CH₃ | Br | F |
| 114. | CH₃ | CH₃ | CH₂CH₃ | Br | F |
| 115. | CH₃ | CH₂CH₃ | CH₂CH₃ | Br | F |
| 116. | CH₃ | CH₃ | CH₂CF₃ | Br | F |
| 117. | CH₃ | CH₂CH₃ | CH₂CF₃ | Br | F |
| 118. | CH₃ | CH₃ | CH₂CHF₂ | Br | F |
| 119. | CH₃ | CH₂CH₃ | CH₂CHF₂ | Br | F |
| 120. | CH₃ | CH₃ | CH(CH₃)₂ | Br | F |
| 121. | CH₃ | c-C₃H₅ | c-C₃H₅ | Br | F |
| 122. | Cl | CH₃ | CH₃ | Br | F |
| 123. | Cl | CH₃ | CH₂CH₃ | Br | F |
| 124. | Cl | CH₂CH₃ | CH₅CH₃ | Br | F |
| 125. | Cl | CH₃ | CH₂CF₃ | Br | F |
| 126. | Cl | CH₂CH₃ | CH₂CF₃ | Br | F |
| 127. | Cl | CH₃ | CH₂CHF₂ | Br | F |
| 128. | Cl | CH₂CH₃ | CH₂CHF₂ | Br | F |
| 129. | Cl | CH₃ | CH(CH₃)₂ | Br | F |
| 130. | Cl | c-C₃H₅ | c-C₃H₅ | Br | F |
| 131. | CH₃ | CH₃ | CH₃ | CF₃ | F |
| 132. | CH₃ | CH₃ | CH₂CH₃ | CF₃ | F |
| 133. | CH₃ | CH₂CH₃ | CH₂CH₃ | CF₃ | F |
| 134. | CH₃ | CH₃ | CH₂CF₃ | CF₃ | F |
| 135. | CH₃ | CH₂CH₃ | CH₂CF₃ | CF₃ | F |
| 136. | CH₃ | CH₃ | CH₂CHF₂ | CF₃ | F |
| 137. | CH₃ | CH₂CH₃ | CH₂CHF₂, | CF₃ | F |
| 138. | CH₃ | CH₃ | CH(CH₃)₂ | CF₃ | F |
| 139. | CH₃ | c-C₃H₅ | c-C₃H₅ | CF₃ | F |
| 140. | Cl | CH₃ | CH₃ | CF₃ | F |
| 141. | Cl | CH₃ | CH₂CH₃ | CF₃ | F |
| 142. | Cl | CH₂CH₃ | CH₂CH₃ | CF₃ | F |
| 143. | Cl | CH₃ | CH₂CF₃ | CF₃ | F |
| 144. | Cl | CH₂CH₃ | CH₂CF₃ | CF₃ | F |
| 145. | Cl | CH₃ | CH₂CHF₂ | CF₃ | F |
| 146. | Cl | CH₂CH₃ | CH₂CHF₂ | CF₃ | F |
| 147. | Cl | CH₃ | CH(CH₃)₂ | CF₃ | F |
| 148. | Cl | c-C₃H₅ | c-C₃H₅ | CF₃ | F |
dans lequel c-C₃H₅ est cyclopropyle,
| | **R¹** | **NR^{2c}R^{2d}** | **R³** | **R³** |
|---|---|---|---|---|
| 149. | CH₃ | pyrrolidin-1-yle | H | Cl |
| 150. | CH₃ | pipéridin-1-yle | H | Cl |
| 151. | CH₃ | morpholin-4-yle | H | Cl |
| 152. | CH₃ | 2,6-diméthylmorpholin-4-yle | H | Cl |
| 153. | Cl | pyrrolidin-1-yle | H | Cl |
| 154. | Cl | pipéridin-1-yle | H | Cl |
| 155. | Cl | morpholin-4-yle | H | Cl |
| 156. | Cl | 2,6-diméthylmorpholin-4-yle | H | Cl |
| 157. | CH₃ | pyrrolidin-1-yle | Cl | Cl |
| 158. | CH₃ | piperidin-1-yle | Cl | Cl |
| 159. | CH₃ | morpholin-4-yle | Cl | Cl |
| 160. | CH₃ | 2,6-diméthylmorpholin-4-yle | Cl | Cl |
| 161. | Cl | pyrrolidin-1-yle | Cl | Cl |
| 162. | Cl | pipéridin-1-yle | Cl | Cl |
| 163. | Cl | morpholin-4-yle | Cl | Cl |
| 164. | Cl | 2,6-diméthylmorpholin-4-yle | Cl | Cl |
| 165. | CH₃ | pyrrolidin-1-yle | Br | Cl |
| 166. | CH₃ | pipéridin-1-yle | Br | Cl |
| 167. | CH₃ | morpholin-4-yle | Br | Cl |
| 168. | CH₃ | 2,6-diméthylmorpholin-4-yle | Br | Cl |
| 169. | Cl | pyrrolidin-1-yle | Br | Cl |
| 170. | Cl | pipéridin-1-yle | Br | Cl |
| 171. | Cl | morpholin-4-yle | Br | Cl |
| 172. | Cl | 2,6-diméthylmorpholin-4-yle | Br | Cl |
| 173. | CH₃ | pyrrolidin-1-yle | CF₃ | Cl |
| 174. | CH₃ | pipéridin-1-yle | CF₃ | Cl |
| 175. | CH₃ | morpholin-4-yle | CF₃ | Cl |
| 176. | CH₃ | 2,6-diméthylmorpholin-4-yle | CF₃ | Cl |
| 177. | Cl | pyrrolidin-1-yle | CF₃ | Cl |
| 178. | Cl | pipéridin-1-yle | CF₃ | Cl |
| 179. | Cl | morpholin-4-yle | CF₃ | Cl |
| 180. | Cl | 2,6-diméthylmorpholin-4-yle | CF₃ | Cl |
| 181. | CH₃ | pyrrolidin-1-yle | H | F |
| 182. | CH₃ | pipéridin-1-yle | H | F |
| 183. | CH₃ | morpholin-4-yle | H | F |
| 184. | CH₃ | 2,6-diméthylmorpholin-4-yle | H | F |
| 185. | Cl | pyrrolidin-1-yle | H | F |
| 186. | Cl | pipéridin-1-yle | H | F |
| 187. | Cl | morpholin-4-yle | H | F |
| 188. | Cl | 2,6-diméthylmorpholin-4-yle | H | F |
| 189. | CH₃ | pyrrolidin-1-yle | Cl | F |
| 190. | CH₃ | pipéridin-1-yle | Cl | F |
| 191. | CH₃ | morpholin-4-yle | Cl | F |
| 192. | CH₃ | 2,6-diméthylmorpholin-4-yle | Cl | F |
| 193. | Cl | pyrrolidin-1-yle | Cl | F |
| 194. | Cl | pipéridin-1-yle | Cl | F |
| 195. | Cl | morpholin-4-yle | Cl | F |
| 196. | Cl | 2,6-diméthylmorpholin-4-yle | Cl | F |
| 197. | CH₃ | pyrrolidin-1-yle | Br | F |
| 198. | CH₃ | pipéridin-1-yle | Br | F |
| 199. | CH₃ | morpholin-4-yle | Br | F |
| 200. | CH₃ | 2,6-diméthylmorpholin-4-yle | Br | F |
| 201. | Cl | pyrrolidin-1-yle | Br | F |
| 202. | Cl | pipéridin-1-yle | Br | F |
| 203. | Cl | morpholin-4-yle | Br | F |
| 204. | Cl | 2,6-diméthylmorpholin-4-yle | Br | F |
| 205. | CH₃ | pyrrolidin-1-yle | CF₃ | F |
| 206. | CH₃ | pipéridin-1-yle | CF₃ | F |
| 207. | CH₃ | morpholin-4-yl | CF₃ | F |
| 208. | CH₃ | 2,6-diméthylmorpholin-4-yle | CF₃ | F |
| 209. | Cl | pyrrolidin-1-yle | CF₃ | F |
| 210. | Cl | pipéridin-1-yle | CF₃ | F |
| 211. | Cl | morpholin-4-yle | CF₃ | F |
| 212. | Cl | 2,6-diméthylmorpholin-4-yle | CF₃ | F |

13. Composition comprenant au moins un composé selon l'une quelconque des revendications 1 à 12, ou un sel approprié sur le plan agricole correspondant, et au moins un auxiliaire, qui est habituel pour la formulation de composés pour la protection de cultures.

14. Utilisation d'un composé selon l'une quelconque des revendications 1 à 12, ou d'un sel approprié sur le plan agricole correspondant, ou de la composition selon la revendication 13 pour la lutte contre de la végétation indésirable.

15. Procédé pour la lutte contre de la végétation indésirable qui comprend le fait de laisser agir une quantité efficace sur le plan herbicide d'au moins un composé selon l'une quelconque des revendications 1 à 12, ou d'un sel approprié sur le plan agricole correspondant, ou de la composition selon la revendication 13 sur des végétaux, sur leurs semences et/ou sur leur habitat.
